# EUROPEAN PATENT SPECIFICATION

(11) **EP 2 614 153 B1**
(45) Date of publication and mention of the grant of the patent: **12.04.2017**
(21) Application number: 11752246.6
(22) Date of filing: 07.09.2011
(51) Int. Cl.: C12Q 1/68

(54) **MOLECULAR METHOD FOR THE IDENTIFICATION OF MATING TYPE GENES OF TRUFFLES SPECIES**
MOLEKULARES VERFAHREN ZUR IDENTIFIZIERUNG VON PAARUNGSÄHNLICHEN GENEN VON TRÜFFELARTEN
PROCÉDÉ MOLÉCULAIRE PERMETTANT D'IDENTIFIER DES GÈNES DE TYPE SEXUEL CHEZ LES ESPÈCES DE TRUFFES

(30) Priority: 07.09.2010 EP 10175517
(43) Date of publication of application: 17.07.2013
(73) Proprietor: Institut National de la Recherche Agronomique, 75007 Paris (FR); Consiglio Nazionale delle Ricerche, 00185 Roma (IT)
(72) Inventor: MARTIN, Françis, F-54280 Champenoux (FR); MURAT-FURMINIEUX, Claude, F-54130 Saint Max (FR); PAOLOCCI, Francesco, I-06135 Perugia (IT); RUBINI, Andrea, I-06012 Cita di Castello (IT); RICCIONI, Claudia, I-06132 Perugia (IT); BELFIORI, Béatrice, I-06128 Perugia (IT); ARCIONI, Sergio, I-06132 Perugia (IT)
(74) Representative: Ipside
(86) International application number: PCT/EP2011/065501
(87) International publication number: WO 2012/032098

(56) References cited:
- MARTIN F ET AL: "Périgord black truffle genome uncovers evolutionary origins and mechanisms of symbiosis", NATURE, NATURE PUBLISHING GROUP, vol. 464, no. 7291, 15 April 2010 (2010-04-15), pages 1033-1038, XP002615247, ISSN: 1476-4687, DOI: DOI:10.1038/NATURE08867 [retrieved on 2010-03-28]
- STRANDBERG REBECKA ET AL: "Conflict between reproductive gene trees and species phylogeny among heterothallic and pseudohomothallic members of the filamentous ascomycete genus Neurospora", FUNGAL GENETICS AND BIOLOGY, vol. 47, no. 10, 1 July 2010 (2010-07-01), pages 869-878, XP002636676, ISSN: 1087-1845
- RICCIONI CLAUDIA ET AL: "Tuber melanosporum outcrosses: analysis of the genetic diversity within and among its natural populations under this new scenario", NEW PHYTOLOGIST, vol. 180, no. 2, 2008, pages 466-478, XP002636677, ISSN: 0028-646X
- MURAT CLAUDE ET AL: "Sex and truffles: first evidence of Perigord black truffle outcrosses", NEW PHYTOLOGIST, vol. 180, no. 2, 2008, pages 260-263, XP002636678, ISSN: 0028-646X
- SINGH GURJEET ET AL: "Intra-specific and inter-specific conservation of mating-type genes from the discomycete plant-pathogenic fungi Pyrenopeziza brassicae and Tapesia yallundae", CURRENT GENETICS, vol. 36, no. 5, November 1999 (1999-11), pages 290-300, XP002636679, ISSN: 0172-8083
- RUBINI ANDREA ET AL: "Tuber melanosporum: mating type distribution in a natural plantation and dynamics of strains of different mating types on the roots of nursery-inoculated host plants", NEW PHYTOLOGIST, vol. 189, no. 3, 2011, pages 723-735, XP002636680, ISSN: 0028-646X
- RUBINI ANDREA ET AL: "Isolation and characterization of MAT genes in the symbiotic ascomycete Tuber melanosporum", NEW PHYTOLOGIST, vol. 189, no. 3, 2011, pages 710-722, XP002636681, ISSN: 0028-646X
- DATABASE EMBL [Online] 30 June 2010 (2010-06-30), "Tuber melanosporum mRNA 5-prime sequence from clone SY0AAC59YF03 (SY0AAC59YF03FM1).", XP002636682, retrieved from EBI accession no. EM_EST:FP441239 Database accession no. FP441239
- DATABASE EMBL [Online] 30 June 2010 (2010-06-30), "Tuber melanosporum mRNA 3-prime sequence from clone SY0AAA25YN17 (SY0AAA25YN17RM1).", XP002636683, retrieved from EBI accession no. EM_EST:FP383886 Database accession no. FP383886

### SEQUENCE LISTING

<110> INSTITUT NATIONAL DE LA RECHERCHE AGRONOMIQUE
<120> MOLECULAR METHOD FOR THE IDENTIFICATION OF MATING TYPE GENES OF TRUFFLES SPECIES
<130> 24416PCT
<150> EP 10175517.1
   <151> 2010-09-07
<160> 64
<170> PatentIn version 3.5
<210> 1
   <211> 1062
   <212> DNA
   <213> Tuber melanosporum
<400> 1
   atgactgctt tgtccatgct caatatggcc gttgaagccg ggcctcttat tcaccaacgt      60
   tatgatggga tcggtctcgc cgagtatatt cccttgctcg ccaatggaac ttttttcttt      120
   gatgggtcgg atggagcccc gctccaggtc cgtcatctcc ttccagcaga ctggaagccc     180
   gtcaatggac cacttacggt cttgttcgat tccgcccccc ataccattat tgggactcac     240
   ttctctatcc acaatcatcc catttacaag cttaacgatg gttctatctt tgttgtcttg     300
   gacctggcca ccaagcacgg ttttgagttt accaatcaat ctttcatgga cccccagctt     360
   gaggtctccg gggaaaaccg tttcttgcgt ctcgttacca atgaccaaga cttcgcctac     420
   catggctcta gtcttagcat gattcatgac aagggcgtta agcgccctgc caatgcgtaa     480
   gtacaattct accaatgtac cagtttcgtg tgctcatata gtcataggtt tatcttgttc     540
   agaacggaca aaatgaagcc cctcaaggct cagtacccgg agatgggcaa caacgatatc     600
   tgtacgtggc tctcattttc cctttattta gatctctatt cttgctacca tttgcctaac     660
   gttttaatag ccaagattct cggtcgcatg tggcagaatt cctcggatga ggtcaaaaat     720
   gtttataagg taaaacatct cacctaactg ttagttagaa tcactaacac attaataggc     780
   aagggcccat cagcttgcgg tttcccacaa gttgatcaac ccggattaca aatactcccc     840
   gcgccgctct cgcgaaatca tgaggagggg ctccgggaat aaaggtcctt cggcccgtat     900
   cgaccttcat cgtgctggtg ttgcgaagcg tggaaaaaag aggatcatta cccaccttgg     960
   gttcctcact caacgtttcg cgactcaccc cactgctaac aatctcggtg atatcatcga    1020
   ggaagccgaa aaggtcttga aggcctttgg tatggtcaat tg                       1062
<210> 2
   <211> 1069
   <212> DNA
   <213> Tuber melanosporum
<400> 2
   atgcgtaatg ttaccttggg atgcgccttg tacctttcgg accgtgggta cattctcatg      60
   caagacagga tcgggtgtat ctgggtccag cacgagaact cccccattat cgttcaaccc     120
   cccggtggtg tcactttcac tgtcagcggc gaactcttgc cctacgacaa gtggctcggt     180
   gcaattcaat caaccgtccg tcatcaaggg cgagtccccc tcgaccccga agtcccttat     240
   gttgtgtcgg ttctgcgttt gtattgcgga tactataaac ctggacagcc gtggacggcc     300
   caggatgcca ctattccggc tgttatcgtc aatggtgatg aggccttggc ccgtattccc     360
   ccctctcagc accttcgtgc attgaatccc tacgttgcgc agagatgtaa gtaaatcaca     420
   tccctatttt gcctctattt ctaaccatct attagcctgg atttctaagt actgtaacgg     480
   ttatgggctc actcaagccg aaatttccaa tctcactcgt gatgtctggg tcgctgagac     540
   caataagtat atgtggcaaa acatcgcctc tctttatact gctgctaggg atcgtggtga     600
   ccccggcctc gtgctcgaag aatttatcga gaccgaattg gcgaaacacg gtcaccctac     660
   tacccctgag aagctcttgc gtgaagctgg ctttgtcccc aaggccccca tcgacaagga     720
   agcggccgat aaggctgctg caagaaggaa ggttcgtgcc aagcccgcac ccggggtgtc     780
   cagtttcact atcacccgtg tttacgtttc cagcaaccgt cagctcgatc tcaccgagga     840
   ggaagccgcc aacatcctcc atgctttcga catcgctagc gctgaggaga atggagactc     900
   tcaactcgac ccgcacctcc agcccgagat cgagatgcac atgaacactt tcacttctgg     960
   tggatacagc gtttttgtga gaagttcccc gaacagcata ttccgccact gcccaattct    1020
   aacattatct aaacagccta ctattggaga atcctccact ggattgtga               1069
<210> 3
   <211> 5486
   <212> DNA
   <213> Tuber melanosporum
<400> 3
   tgagcgattg cttgcctgca ggcaagtgat ccggacccac cactaggtgg gcccggtacg      60
   gggagcactg tctgtctgtt cgcttcactt gtgcggttcg aatgctttac ctggttgtgc     120
   gctctgagta cgagaaatgg gcgggcaagc tagtctgtct attcacgaag ggcgcaatga     180
   actatcacaa accaatcccg gattcttcta ctcttttgtc cctttctcac ggaggttctt     240
   tccagtgagc ttcgcccggt ctcgtctcgt ccgccattgc gtggttattt acagcggatg     300
   gcttaatccg cacccttgtc tatcatcgca atacggcatt ggctctggcg aaactatatc     360
   gtgtcgttcc gagggagaaa aaaaaatccc ttcattctac tttcccttcg tccactccga     420
   ttcactggca gatgaaaccg taccgcgcgc tcgggctctt tggtccctat tttctcaacc     480
   ggcgagggcg tcgttagtat gggggttgga aggctccttt ggcttgtttg tcgtgagtag     540
   ggagggcgct gtcattgctg agtagccttt cagcggggcc tgcgggggtg cttctttccg 600
   atcggtcttg cgaggggtgg tattgctatt gggcggtgtt ggccattcgg cggcgttgat 660
   gaaatttcta tcgggggttc tgtggcctct ggatacgtgg tgtcgtatat cttctggcgg 720
   tttctctgct gctaagtatg atatcgcact attcgtgggg acctgtcatt aagtctttac 780
   atagttgctc actatggtct taggaatcct tgcgctatcg tacaccactt tccttctatc 840
   tgaacaacac tctatatgtt tatctatcgt acagggggag aaaggtaacg cgtgggcaag 900
   aatgcacggc tggagaaaat aaaatatgat aggatgaaaa ccgcaaggta taataaacta 960
   gacttttttg ccacagcgac tagttccaaa ctctacagta ctcagggggc aaacccacgc 1020
   ccgactataa aagcaaatct ccaggggggc tcgcatcagc acggtgacat aagcgcattc 1080
   cgggctcgtc tcatgggatc tccttcgact ggaaggggtg atgcctaagg acgactttcg 1140
   tgggtagggt accccggaaa agaaggaacg gtgatgcctt cgatgaggtc ctgttttata 1200
   agccttaaat cgcgctaggg gttaatgctt tgtgggactg tagcagcgct accaccttct 1260
   gatggccgtg cctagatgcg aacgagagag gtgtttgacc gtcattgttt ggcctgtcgg 1320
   ggctgacatc ttccccgccg agtagtacct tcaccacctc cgcatgccca tcccaagcag 1380
   cgagcgagag cggtgtttgg ccgcaatcat ctggcctgtc agggttgacc tcttccctca 1440
   tgagtaacat tttcaccact tcttcatgtc catgaaaggc agcatgagag agaggtgatt 1500
   ggccgtggct gtctggcttg tcgggattga cctcttcccg catgagtagc attctcacca 1560
   cctcttcata tccggaccag acggcatgcg agagcggtgt tcggcaatag atatctggct 1620
   tgtcagggtt gacttcttcc cgcccgagaa agattttcac tactccctca tgtccagaac 1680
   cagcggcatg cgagagtggc gttcggccga gattgtctgg cttgtcgggg ttgacctctt 1740
   cccgctcgag taataatttc gcaactccct cataccccct ccaagcagca ttcgagagcg 1800
   gtgtttggcc gtcattatct ggctcgttgg gattaacatc ctcccgcccg agcagtattc 1860
   tcaccacttc ctcatgtcca aaccaagcag cgagcgagag cggtgtttgg ccgtcattgt 1920
   ccggcttgtc ggggttgacc tcttcccgcc cgagcagtat tttcaccact tcctcatgtc 1980
   cacaggcagt agcatgtgag agcggtgtct aggccttctc cgttcgttat attactatac 2040
   catcctactg atctcagcat ggcctaactt agccaagaac ccctctgaaa ccaccaggag 2100
   taacctctag caatgtcagc cacaatctct taatgtccag acccttaccg agaccggttg 2160
   tcgcattgga tgactgcacc tttgttctgg agttccatct tttaaacagc tttcccgaga 2220
   gctgcatacg ccacatcaaa cgtcgtagtc ctccttgtcc attcacctgg atgcacaagc 2280
   ggatagccga acaggggaca ttcattggat cggctggtag tgtttccaag gcatttggct 2340
   ttcctggttg gccgggcagt gggtgaagct accatacttc tgaattcaca tttcgcctaa 2400
   cggcctcctg gccattggat atccccccag gacggaaaga acggcttctg gatggccagg 2460
   attaaggtat ggtttcccag tccgccacac agatttatgg agatcatatt aaattcctca    2520
   atcagcaaac taccttgatt cacatatctg atcacaaaga attattgttc agggctcgga    2580
   taccatacct ctctatcaaa gaagcaatgt acaagagagg tggggttgga ggacggatgc    2640
   tgtaacagaa cccacatttc ccagctcacc ctgtttcatt aattcctttg ttcggaactt    2700
   ctccgacagt aaaaaaaaat tcgcggatct tttcgccagg aatcttggga acctaagggc    2760
   tctctcttcc tgccctgggg gaaatcttat tgtgtcataa tattattatg tgtgataata    2820
   ttatgacatt ttcaagatac cggtaatact attctcgggc cctagtaggg ttgagttgta    2880
   cgtgacggct tcttggcgtc actctatttt ttgggtcttg gcatttcccc tcgtgacagg    2940
   aggcccacta gggggatcca tcctttcctc cttctttcct ccgccgacaa ccgtcaagct    3000
   ccaccgacaa ccgtcaagtt ccaccgacaa ccgtcaagtt cgacagaaac attcggcctt    3060
   tctacttgca cctctccact cacgtcgttt ctttttttct gccactatga ctgctttgtc    3120
   catgctcaat atggccgttg aagccgggcc tcttattcac caacgttatg atgggatcgg    3180
   tctcgccgag tatattccct tgctcgccaa tggaactttt ttctttgatg ggtcggatgg    3240
   agccccgctc caggtccgtc atctccttcc agcagactgg aagcccgtca atggaccact    3300
   tacggtcttg ttcgattccg ccccccatac cattattggg actcacttct ctatccacaa    3360
   tcatcccatt tacaagctta acgatggttc tatctttgtt gtcttggacc tggccaccaa    3420
   gcacggtttt gagtttacca atcaatcttt catggacccc cagcttgagg tctccgggga    3480
   aaaccgtttc ttgcgtctcg ttaccaatga ccaagacttc gcctaccatg gctctagtct    3540
   tagcatgatt catgacaagg gcgttaagcg ccctgccaat gcgtaagtac aattctacca    3600
   atgtaccagt ttcgtgtgct catatagtca taggtttatc ttgttcagaa cggacaaaat    3660
   gaagcccctc aaggctcagt acccggagat gggcaacaac gatatctgta cgtggctctc    3720
   attttccctt tatttagatc tctattcttg ctaccatttg cctaacgttt taatagccaa    3780
   gattctcggt cgcatgtggc agaattcctc ggatgaggtc aaaaatgttt ataaggtaaa    3840
   acatctcacc taactgttag ttagaatcac taacacatta ataggcaagg gcccatcagc    3900
   ttgcggtttc ccacaagttg atcaacccgg attacaaata ctccccgcgc cgctctcgcg    3960
   aaatcatgag gaggggctcc gggaataaag gtccttcggc ccgtatcgac cttcatcgtg    4020
   ctggtgttgc gaagcgtgga aaaaagagga tcattaccca ccttgggttc ctcactcaac    4080
   gtttcgcgac tcaccccact gctaacaatc tcggtgatat catcgaggaa gccgaaaagg    4140
   tcttgaaggc ctttggtatg gtcaattgag tgatctctac ggagggactc atcggtacct    4200
   gggtggtata cttttaatgg gagggatgat cttgtctatc aaaggtcatt cattatctca    4260
   gtcgactaaa tttatgacct aaatagataa aaaatatctt ttaaaaccaa aatgaggggt    4320
   ccgaacacta catcatacag taaatatcaa gttgtgaagc ggaagagagc cgactatggg 4380
   aagaggcagt aagaatttaa ttctgactaa gtctggaaag tttacacagc acttgcacag 4440
   aagttatgct atggtggtgc catcaccgga aaaacacgcc aaaaattcgg gacctatggt 4500
   gccgtgactt agtttgagcg caagtacatg gttcgacaca aaagagtgtt acggagtagg 4560
   ggtgttgggg ttgattttgg ccattagcgt tatcgtatag attgtttgaa aacacgactc 4620
   gtattgctta acaagaatat ggagagagct atttttttaa ttttttttaa ttttgataga 4680
   tgtattggac tggaggccaa gcatctgatc aattggttat actatactcc tatttgttca 4740
   tagttgactg aggccctgtt tggctacaac ggttcctttc tgtttcccca tacaaagcga 4800
   ccgttgcaga acgggagggc aatagctttt atatgagaaa cggttccaaa atgaaacgga 4860
   accgaggaaa ttgtgatcta gtttcttgga tgtgccgctg ctactgaaat ttttgttata 4920
   aaggtatccg ttttatatct gactcccttc acacagaccc caatggcgcg aatccccgga 4980
   gtactttgct catgaaagtc gtcctgtcgt ccttgggcat caccccttcc agtcgaagga 5040
   gatcccatga gacgagcccg gaatgcactt atatgtcacc atatggacat gaacctccgt 5100
   tgagatttgc ttttacagtc gggcgttgtt ttatttcccg agtactttag agtttggaac 5160
   cagttctatt gctttgacaa agtctagttt attatacctt gcagttttca tcttatcaca 5220
   ttttattttc tctagccgta cattcttgcc ctcgcgtttt tttctcttgc acaactgata 5280
   gacatataag tgttcttcag atagaaggaa atggcgtacg atagcgcaag gattcctaag 5340
   accattgtga gaaactatgt aaagacttct gacaggtccc cacgaatagt gcaatatcat 5400
   acttagcagc agagaaacca tcaagaaggt agactacagc acatagtcag aggccacgga 5460
   actcctgaga cataagggta gggata 5486
<210> 4
   <211> 7509
   <212> DNA
   <213> Tuber melanosporum
<400> 4
   ggcttgcatg atatcagcga atgtagctcg aggacgtaaa tctgatagtt acactaaacg      60
   ttggatacca cgctggttca gagctagctc cttctccgct cgaggtagag gattaagttc     120
   accgccctct gaatggcatc tgatgatgtt ccgggcaact tctctcgggg cacctgtgtc     180
   gctagcaatt gtatgataac ttttgccttt ctgcatgaag ctcaatcatt cgwactctct     240
   catcgcttga ggccattcgc cgcttgcctg tttcttgacc ttgaggtatg gtgtatgatg     300
   ggaggtgtta gctcctataa ggaaatcgtg gccaagatac tagtgtcggg ttactaaaat     360
   aacctctgct gccgagaccg ttgcgaaagc tgggtagatt ctctctacat attctagtag     420
   atcttgacgc tgatttacgg atggataaaa aggaatttta tgtttttttt ggttttaaag     480
   ataccgaggg cgtttcattg atcctagata cataagttta atttaagtct acacgatcta 540
   taatacgctg agaatgcctc ccaactatgc ccttgattcc cgccgctccc acacggtcat 600
   accgtaatac agtaacttca tttttttttt gtacccacta gcctctttag gccttcatgg 660
   gaggtcatat cggctcacaa tccagtggag gattctccaa tagtaggctg tttagataat 720
   gttagaattg ggcagtggcg gaatatgctg ttcggggaac ttctcacaaa aacgctgtat 780
   ccaccagaag tgaaagtgtt catgtgcatc tcgatctcgg gctggaggtg cgggtcgagt 840
   tgagagtctc cattctcctc agcgctagcg atgtcgaaag catggaggat gttggcggct 900
   tcctcctcgg tgagatcgag ctgacggttg ctggaaacgt aaacacgggt gatagtgaaa 960
   ctggacaccc cgggtgcggg cttggcacga accttccttc ttgcagcagc cttatcggcc 1020
   gcttccttgt cgatgggggc cttggggaca aagccagctt cacgcaagag cttctcaggg 1080
   gtagtagggt gaccgtgttt cgccaattcg gtctcgataa attcttcgag cacgaggccg 1140
   gggtcaccac gatccctagc agcagtataa agagaggcga tgttttgcca catatactta 1200
   ttggtctcag cgacccagac atcacgagtg agattggaaa tttcggcttg agtgagccca 1260
   taaccgttac agtacttaga aatccaggct aatagatggt tagaaataga ggcaaaatag 1320
   ggatgtgatt tacttacatc tctgcgcaac gtagggattc aatgcacgaa ggtgctgaga 1380
   ggggggaata cgggccaagg cctcatcacc attgacgata acagccggaa tagtggcatc 1440
   ctgggccgtc cacggctgtc caggtttata gtatccgcaa tacaaacgca gaaccgacac 1500
   aacataaggg acttcggggt cgagggggac tcgcccttga tgacggacgg ttgattgaat 1560
   tgcaccgagc cacttgtcgt agggcaagag ttcgccgctg acagtgaaag tgacaccacc 1620
   ggggggttga acgataatgg gggagttctc gtgctggacc cagatacacc cgatcctgtc 1680
   ttgcatgaga atgtacccac ggtccgaaag gtacaaggcg catcccaagg taacattacg 1740
   cataatgagg cgtctctcga gcctgttctc ccactccgaa accttagggg ggggggagag 1800
   gttaacgacc atgctcagat tgacaggacg agacgacatt atgaaaggtg ggggggcggt 1860
   tggagacggg aaaagaaatt tggaccgaca aaaggaggat tgcgtatggg cttttcgatg 1920
   agtggagatt ggcctcagtg gctgttggtg gtggaagaaa ggagggtgga aggtctggca 1980
   acttttgaag gctacccgtc acgaagtggg atgccaaggc ccaaaaaaaa gagtgacgcc 2040
   aagaagctgt cacgtacaac ttagccctac tagggtctat cataatacat actatacgat 2100
   aataaattat tatgatatta tcataagatt ttccccagag caggtagaga gcccttaggt 2160
   ttccaagatt cctggcggaa agatcgggga acatttttct ttttttacgg tcagagttgc 2220
   tcgaatagaa aaatgagtca aggtatttga tgaagatctg atcgacattc gggagatgtt 2280
   tagaacaaag gaatttagtg gggatgagct gggaaatgtg ggttctgtta cagtatctgt 2340
   cccccaatct taactagccc tcttgtacat tgcctctttg gtaaaggggt atggtatctg 2400
   agccttgaac agtaattcct tgcgatagga tatatcgatc aggatagttt gctggctgga 2460
   aaatttaata tagtcttcat aaaatcgtgt gacggactgg gaaatcatac cttgagtttg 2520
   gccatccgga agcctctctt tctgctatga gggaatatca aatagccaaa aggccatggg 2580
   gtgaaatgag aatttagaaa tatgatagcg gtcccttgcc accaaaagac aaatgcttcg 2640
   gcaataccat cacccgatcc ggtggatgtc tccatgttcg gctatccgct tgagcatcca 2700
   ggggaataga caaggaagac taccacattt gatgcggtgt gtacggctgc cgagaaaact 2760
   gtttgaaaga tggaactcca gaacaaagga ttaactggtc aacgcaatac ctggttgccg 2820
   caagagtctg aagattaaag gattgtaact agtattgttg gaggttactt ctagcaattc 2880
   tagaggggtt tttggtaaag atagattata ctgatatcag tcggatgata tggcactaca 2940
   acgaaagaaa gtggtctaga ccatgagcgc aaaaactcca aaaagaattt cctgccggga 3000
   aattagaata tttaaccggt ttaaaaccgt aagatttgct ggtccaggac cagcggcaaa 3060
   ttgagagcca ttgtgagagc atctaagaac tcaaccttca gaactccagg ataagtagga 3120
   tgataaaagg gaaatggggt ttagaatggg tgcataaagc cttttgattc tggattaaga 3180
   tatggccaga taagggagtc tgaagcaatg tgcatttaat ctccgctcgg actcctagga 3240
   taaaaacgga catgacaagt aaattataat gaggaaaaaa gcttgattgg ttgtaggggt 3300
   cgattgtatg atcagaggtg ggggtactac aagtttatcg atataatatt gggaataact 3360
   gtgaaaatgg ttccagaata tagctgagta ttttacaaac atgcattagt tgcatatgag 3420
   atatcataga tactagagtc gccttgttca gtctcttggc tgtctttgaa tatctagata 3480
   taccgggaaa gaaaatggga aagaaaatat catcggcttc tttagtaaac tcttcaaaga 3540
   tagcaaaaac ccaacctcta cttacagcct gagcctatag cttggtatgg tactcattta 3600
   ccctttcttc cacagagcta tgcacttgca atatttcttc caactgtttg tttaaaatcc 3660
   atgaggttcg acatcaaatg aaaggggtaa tttccacatg cacaaaacat atattgtgtg 3720
   aatacctaca attgagcctg cataaacaca tgaacccaaa tcaattcagt aaattaaaac 3780
   ccatcaagta gttggtgatt tagctcatga aatttgtgaa gtttttttga gctaaaggga 3840
   aaagagaaat gttacgcaaa tttttttgtc acccttagct ggtcggctgt caatgcttga 3900
   tattgtgata taaattacta caggttgtat tgcgggaatg ttgacatatt gcagtacgac 3960
   atcttctaag gccatatgca tataagagat tactttatcg tactaagaaa gccaagacaa 4020
   tgatttctac tacttttttc tatattctct tacctcatat tccactatga tttacaagag 4080
   tcatgccaaa tcaagtattg tgtgatacct ggtgaaaaat atccatcaaa gcctcagagc 4140
   tgtaaatatc gcaaattgta aggtgagagg ggaatcacaa tgcataatat tatataaaca 4200
   ccacaactgc tctactgggg gtgccttatg ctaggacttt cctacatgcg acccattaac 4260
   agattttaaa aaagaaaaaa agtcactgag gatgccccac tgccacatta aaggagagag 4320
   tagttcttag atcttgtctc cctcagcttg cttagtgcac ctgtggacag aaactcaggt 4380
   gaagatagag aaagaggtat catgaatttt aatgcacagt attcattgca atttaattcc 4440
   gggcacacta agttatttat tcatttactc aatccctact gcaatccaag ctactcttgt 4500
   ccttgcccag gctatatctt gagggcgggg aacaaactta ttctcattgc accttctggg 4560
   cgctacgagc gaaagattga ctatttcatc aattttaaat cctaaataca agtatcatac 4620
   acatctactc aagtacaaga atccttgtaa cgattggaac aaaacaaatt ctgggactct 4680
   tgtcggacac tacgttaacg ctagtaagta acaaaaaatc tttttaatat tagtagtgtg 4740
   tgtgacatcc tcttagattt tgcgtgacac ccctgccgct actaggtctt agtactcgag 4800
   tagtagaata ataggagtag cgaccttcaa cgacagccaa gcgatcaaaa agagtatcgt 4860
   actcgagtat tcaaatggaa aatctacctt cctatgatac caggtgaggt cattgcggtg 4920
   gttcaatacg acgatcaaat cctgcgatta cgcataacaa gtgtgagtgc tcgagaagag 4980
   cgcgaatgca cagctgcctg gcctgcgtgg gtctgacaga tctgtccttg ctctatttat 5040
   ttgttttgtt tttcctgagc gcagcacttt ttaccctatg aggtatttat aataatatta 5100
   tggatattct ggaggaggtt ccaaaaaata tatcggaaat gcacttaggg gcaatggtac 5160
   taggattcca tcgcacacta tccgctaata gatatatcta ggtttctagt ttccataaat 5220
   atcagacgag aaaggcttac caaaatgacc aagggggttg ggtctgggag atgtctacgg 5280
   tgcgacgata gaacggataa aagcgcaggg cggggataaa tcgagactcg ggatggcagc 5340
   tttgatgtgg atcagtcatg cgcagcggct actcgggatg gacgagctct cccacgccct 5400
   ggcagttgag ctgaactcca ccgacttcaa cgctagcaac atctcttcga tgtcgacact 5460
   ggtgggttgc ttccaagggc ttattaaggt ggacaaggaa gcatcagccg tgcaactggc 5520
   ccactttact ctccaagagt acctttccgc tcatcccgat attttctgca gacgccatgc 5580
   ggcaatggca gagatctgct taacctatct gaattcccgg caaatcaagg acctttcgac 5640
   cgaccatctc ctgacacccc ggatgcaccc tttctagaat actgttccgt gcactgggga 5700
   atccatgcga aaacggaact ctcggatggc aggagatcgc ttgcgctgaa gctattctag 5760
   gagtttgatg gccacatatc cacagaattt cttttagcgc aagtggagaa tttaagttcc 5820
   caggggcgtg atacctgacc tccgtccagt gggttgtact gcgcatcgtt ctttggaatc 5880
   gtcgaggttg tggctgcttt gacatccgtg ggatgttggg atgccaaaga agggggttgt 5940
   ttggggtaac cgccacttgc acgggttcat aacgggtatg gggaggtggt ggaaatgcta 6000
   ctcgggttgg aacaggtcca tcccgacgag ccggataacg cgaccggaca ccgctctcac 6060
   atgttactgc ctgtgggcat gaagaagtgg tgaaaatact gctcctgttt gagcggggag 6120
   aggctagcca cgaaaagccg gacaatgacg gtcaaacacc actctctttc gctgcttggt 6180
   ttggacatgg ggaagtggtg agaatactac ttgggcggaa gatgttaatc ccaatgagcc      6240
   agataatgac ggccaaacac cgctctcgaa tgctgcttgg aggggtcatg agagagttgc      6300
   gaaaatatta ctcgggcggg aagaggtcaa cctcgaagag ccagacgagc tcggccgggc      6360
   gccgctcttg catgccgctg gtcatggaca tgagggagta gtgaaaattt tctcgggcgg      6420
   gaaggggtca acctcgaaaa gctagacaat tactcccgaa caccgctttt gcatgccgtt      6480
   tggtctagat atgaagaggt ggtgagtgtg ctactcatag ggggggtgtc gatctcggcg       6540
   aaccggacag cagcggccaa ataccgctct cgcatgctgc cgctcatggg cacgaggagg      6600
   tggtgaaaat gttactcatg tgggaagagg tcaaacccga caggccagat gatttcggcc      6660
   aaacactgcg ctcgcttgct gcttgggatg gacatggggg atgatgaaag tactactcgg      6720
   ccgggaggat gtcaaaccgg acaggccaga cgatagcggc aaaaccctgc tctagttggc      6780
   cgccgggatg ggcatgaggt agtggtgaaa atactactcg ggcgcgaagt tgtcagtcct      6840
   gacaaaccag ataatggcga ccaaacaccc ctctcgttcg cagctgggaa tgaccatcag      6900
   aaggtggtgg ccctgctaca gtcccacaaa gcgttaaccc ctagcgcggt ttaaggttta      6960
   taaaaaacaa cctcttcgaa ggcatcacct tccttttctt ccagagtact ttgctcgatg      7020
   ggggtcatcc tagggcatca tcctttccag tcgaaggaga tcccatgaga cgagcccgga      7080
   atgcgcttat atgccaccat gcggatgtga acctccctgg ggatttgctt ttatagtcgg      7140
   gcgtggattt ttttttctga gtacggtaga gtttggaact agttctatcg ccgtgacaaa      7200
   gtttagttta gtttattaca ccttgcagtc ctcatcctat catatttgat ttcctccagc      7260
   cgtacattct tgccctcacg ttttccctct cccttgcgca attgataaac atatagagtg      7320
   ttcatataga aggaaagtgg tgtacgagag gggaagggtt cttaagacca atgtgagcaa      7380
   ctatgtaagg actgaatgat aggtccccac gaatagtgcg atatcatact cagcagcaga      7440
   gaaaccatca agaaggtaac tacagcacat atccagaggc cccagacctg tgacataagg      7500
   atagggata                                                             7509
<210> 5
   <211> 12757
   <212> DNA
   <213> Tuber melanosporum
<400> 5
   gtgtgcgtgc ttgaagtgaa ttggagtaaa gtgaaggtgg tggtggtgaa agtggcgaat      60
   atgagagtat caaaggagga ggtgagcaga gcattgagct tattagtagg ttcagagcga     120
   gttgcgtgcg ctgtctctag ggttagagtt acagttagga tgcggcgttg tgtgtgtgtg     180
   ggcgtgggtg cgtgggtgga tatgtgggtg tgtgtgttat tcttatcatc tgagtttgtt     240
   gtcgtctttt caatttcact cgagtcgctg taggaggcag gagggagagt tgtacgagta     300
   ctgtagatgt agagcacctg tatcatatta tcatactaca cacagtacag tactgcacga 360
   gcacgagcac gaatacggtt tcagtgaatc tggagcttca ctcgtttgtg ttttttagct 420
   tcgtgcactt ttgtcaatag agtaattatt taatatcatg atggttatta taatatatta 480
   tcatacaggt ggtggagctg tatgataccc ttcgtatcat atccttatgc atcacacatc 540
   ctttgcataa caaagtagct ggttttaggc ctacagtgcc cacgcgcacg ggtactaccg 600
   gtaccactct actaccagga tagtcaatga agcttaccta acttgaacct gtctttttac 660
   cctcaattac catccagcta taaattttcc tctcccacga atccgatgct cgccactcgc 720
   cgtgctcttc gctatttacg gctgtcgaaa cggctctcgc tttcaaatcc cccctcccaa 780
   ttccgccata ttcacaccac acttcaaaac cgttcgaggg atgaggagga ggcagctcct 840
   tctcgtccct tgttgttgcc cccccccgcg ataacgataa aaccgcctgt ggaggaatat 900
   gttcgacaga tacgcgagca gtatggcgac tacctccccg ccggtctact ggaggagggc 960
   gaatacagaa tctacgagag atactatggc cgtccaatcc gactgctgca gccgggcgag 1020
   acgctccacc attttccacc tccaaagacg cccgcggagg aggccttata cgcacaggtt 1080
   atccagcgaa tcgcccaccc ccaaggggta gggatggaag aaggagaaga aggggaaggg 1140
   ggaggaggag ggggaggagg aggtgaggac cttcgtgcca atggttctcc accattcttt 1200
   cggacacacc cgctcactta cgccggtcga ttcgagtcgt ctcagattcg acttccgcca 1260
   tcggtaaggg acgggacaga gtctgtgatc gtggatgtcg ccgcctcgca tctagcggaa 1320
   tcggcgcata agactctcgg cggtccacac ctccaattct cgcccgtata ctcgaggaag 1380
   cggagtaagc acatgttgac tattcccttg aacccgatta acagtcgcat gtcaagtatg 1440
   gatgccgacg tgtttctcac tgccgtgatg ccggggctgt atgcgcagtg cttgagttcg 1500
   ttgaccgagc tgcggaaacg gttgggcggc gattgggtgc ttggcgggga tggggaaccg 1560
   ggtgttagga atgtgcttga tgtggggggt gggggcgctg gggtacttgc ttggcggagt 1620
   gttatcgaag tggaagagga ggtaaggaag gataggcttt ccggaagagc cgggaagccg 1680
   ccacctggtt cggatggggg ggctctgcct gcggcggggg agaggagtgg gatgcgagct 1740
   gttgttgtcg ccgggtccat cgctcttcgg gggaaggttt cgaggctctt ggacgatact 1800
   ctgtttattc cccgaatgcc ggatatccat ttcgaaaacc taaaccacac accgggtggc 1860
   ctggtggaga agaataaggc ccagccgaga aaactctacg atctgatcat tgccacaaac 1920
   aatctcctcc cgatagtgaa gtcgttttac cgccgccaag ccattgaaca actctggtcg 1980
   catctaaatc cgaacggcgg cgtccttttg atgatcgaaa gaggcacgcc aatgggcttc 2040
   gaggccatcg cctacgcccg ctccgttatt ctagaggact acatcaagga cgttggcgga 2100
   gcctttcaat ccctcccgac cacgaatgcc cccggttccg ccagcccccc tgtcgaaaag 2160
   ggacccggcg ctataatcgc cccgtgcacg aaccacgagc aatgccccat gttcgtctct 2220
   ggccctacgg atggcaccca acgcaaagat tattgccgct tctcacagcg ctatgagcgc 2280
   cccggctatc tgcagaaact catggaggag tcatccaaaa accacgagga cctcgagtac 2340
   tcatacgtcg ccttccgtcg tggtgtcgac caccgcaccg acacgaagaa caagatcagc 2400
   ccaacaatag acgactttga cataatcccc gtcgacggcg aaccccacac gacagttcaa 2460
   agcccatata cagtgtcaca actccgtaat tacagtctaa ccctcccgcg catcatcctc 2520
   ccacccatca aacgcgacgg gcacgtcata ctcgacgtgt gcactccgac aggcttcatc 2580
   gaacgctgga ttgtccctaa gagcgtcgga aaattggagt ttagagacgc gaggaagagc 2640
   ggatggggtg atctctgggc ccttggagcg aaaagtcgca caaggcggaa cataaagatt 2700
   ggcggtggcg acaagagatt taggtcaaag ttagttgcca tcaaggaccc tcatagcgat 2760
   agagtgaggg ctattgtcaa ggacaagacg accgccttct tgaaggctcg gaagaaggag 2820
   acaagggagg cgaggagggc cgcaaggctt cagaagaagc tagaggctag gatgaggcaa 2880
   gacgaaatgg ttcgtcagca aaaggaggag aaggaaagga agcagcgtga gaagttaatg 2940
   gcggagaagg gggccaggga ggtgcacgaa ggggttgttc agatgcatgg ggagaggaag 3000
   ggaaagaaac ggctggagaa gcaaatgcgg gaaagggagt aggaggcgac gaaggggggg 3060
   tgcaatcgca gttttgcgta atgtaaccgt actttcaggc cgccggtggg tggtgtgtat 3120
   atatgaggct tcccgacttt tgtaaattat atttctagct ccgaaaaagc gttatacata 3180
   acagccattg atggtggttc atgagttggt gcgtcattcg aaaggtcgag tgattgttgg 3240
   tgaaaggata ccacgccccc ccaccattcc cgaatccgcc aatctcttcc atcgcccgtc 3300
   cagcagttgt gagggtgggg ggacgtgtat cggtgtattt gtggtatact cgtgtgcagg 3360
   caagtgctgt cggtggcggg gttggcagga gatggcatga tatcttgaga aattttctgc 3420
   tagggggtgt ggtggcatat caacgtggca ttctctattg gaggattctg tcgtataccg 3480
   ttggatgggc tttccataca ttcctgcctt aggccaatcc gcttttcacc ctccgcccgg 3540
   ccgagccggc gcggtgtcct tcaaggcggg gcgtgggctc ggagaacggg tgtttgtcta 3600
   tcgagtgatg ttttgtggtc ttactgatta tagttatcaa tacatccaca tataccagga 3660
   gcttctgagc gattgcttgc ctgcaggcaa gtgatccgga cccaccacta ggtgggcccg 3720
   gtacggggag cactgtctgt ctgttcgctt cacttgtgcg gttcgaatgc tttacctggt 3780
   tgtgcgctct gagtacgaga aatgggcggg caagctagtc tgtctattca cgaagggcgc 3840
   aatgaactat cacaaaccaa tcccggattc ttctactctt ttgtcccttt ctcacggagg 3900
   ttctttccag tgagcttcgc ccggtctcgt ctcgtccgcc attgcgtggt tatttacagc 3960
   ggatggctta atccgcaccc ttgtctatca tcgcaatacg gcattggctc tggcgaaact 4020
   atatcgtgtc gttccgaggg agaaaaaaaa atcccttcat tctactttcc cttcgtccac 4080
   tccgattcac tggcagatga aaccgtaccg cgcgctcggg ctctttggtc cctattttct 4140
   caaccggcga gggcgtcgtt agtatggggg ttggaaggct cctttggctt gtttgtcgtg 4200
   agtagggagg gcgctgtcat tgctgagtag cctttcagcg gggcctgcgg gggtgcttct 4260
   ttccgatcgg tcttgcgagg ggtggtattg ctattgggcg gtgttggcca ttcggcggcg 4320
   ttgatgaaat ttctatcggg ggttctgtgg cctctggata cgtggtgtcg tatatcttct 4380
   ggcggtttct ctgctgctaa gtatgatatc gcactattcg tggggacctg tcattaagtc 4440
   tttacatagt tgctcactat ggtcttagga atccttgcgc tatcgtacac cactttcctt 4500
   ctatctgaac aacactctat atgtttatct atcgtacagg gggagaaagg taacgcgtgg 4560
   gcaagaatgc acggctggag aaaataaaat atgataggat gaaaaccgca aggtataata 4620
   aactagactt ttttgccaca gcgactagtt ccaaactcta cagtactcag ggggcaaacc 4680
   cacgcccgac tataaaagca aatctccagg ggggctcgca tcagcacggt gacataagcg 4740
   cattccgggc tcgtctcatg ggatctcctt cgactggaag gggtgatgcc taaggacgac 4800
   tttcgtgggt agggtacccc ggaaaagaag gaacggtgat gccttcgatg aggtcctgtt 4860
   ttataagcct taaatcgcgc taggggttaa tgctttgtgg gactgtagca gcgctaccac 4920
   cttctgatgg ccgtgcctag atgcgaacga gagaggtgtt tgaccgtcat tgtttggcct 4980
   gtcggggctg acatcttccc cgccgagtag taccttcacc acctccgcat gcccatccca 5040
   agcagcgagc gagagcggtg tttggccgca atcatctggc ctgtcagggt tgacctcttc 5100
   cctcatgagt aacattttca ccacttcttc atgtccatga aaggcagcat gagagagagg 5160
   tgattggccg tggctgtctg gcttgtcggg attgacctct tcccgcatga gtagcattct 5220
   caccacctct tcatatccgg accagacggc atgcgagagc ggtgttcggc aatagatatc 5280
   tggcttgtca gggttgactt cttcccgccc gagaaagatt ttcactactc cctcatgtcc 5340
   agaaccagcg gcatgcgaga gtggcgttcg gccgagattg tctggcttgt cggggttgac 5400
   ctcttcccgc tcgagtaata atttcgcaac tccctcatac cccctccaag cagcattcga 5460
   gagcggtgtt tggccgtcat tatctggctc gttgggatta acatcctccc gcccgagcag 5520
   tattctcacc acttcctcat gtccaaacca agcagcgagc gagagcggtg tttggccgtc 5580
   attgtccggc ttgtcggggt tgacctcttc ccgcccgagc agtattttca ccacttcctc 5640
   atgtccacag gcagtagcat gtgagagcgg tgtctaggcc ttctccgttc gttatattac 5700
   tataccatcc tactgatctc agcatggcct aacttagcca agaacccctc tgaaaccacc 5760
   aggagtaacc tctagcaatg tcagccacaa tctcttaatg tccagaccct taccgagacc 5820
   ggttgtcgca ttggatgact gcacctttgt tctggagttc catcttttaa acagctttcc 5880
   cgagagctgc atacgccaca tcaaacgtcg tagtcctcct tgtccattca cctggatgca 5940
   caagcggata gccgaacagg ggacattcat tggatcggct ggtagtgttt ccaaggcatt 6000
   tggctttcct ggttggccgg gcagtgggtg aagctaccat acttctgaat tcacatttcg 6060
   cctaacggcc tcctggccat tggatatccc cccaggacgg aaagaacggc ttctggatgg 6120
   ccaggattaa ggtatggttt cccagtccgc cacacagatt tatggagatc atattaaatt 6180
   cctcaatcag caaactacct tgattcacat atctgatcac aaagaattat tgttcagggc 6240
   tcggatacca tacctctcta tcaaagaagc aatgtacaag agaggtgggg ttggaggacg 6300
   gatgctgtaa cagaacccac atttcccagc tcaccctgtt tcattaattc ctttgttcgg 6360
   aacttctccg acagtaaaaa aaaattcgcg gatcttttcg ccaggaatct tgggaaccta 6420
   agggctctct cttcctgccc tgggggaaat cttattgtgt cataatatta ttatgtgtga 6480
   taatattatg acattttcaa gataccggta atactattct cgggccctag tagggttgag 6540
   ttgtacgtga cggcttcttg gcgtcactct attttttggg tcttggcatt tcccctcgtg 6600
   acaggaggcc cactaggggg atccatcctt tcctccttct ttcctccgcc gacaaccgtc 6660
   aagctccacc gacaaccgtc aagttccacc gacaaccgtc aagttcgaca gaaacattcg 6720
   gcctttctac ttgcacctct ccactcacgt cgtttctttt tttctgccac tatgactgct 6780
   ttgtccatgc tcaatatggc cgttgaagcc gggcctctta ttcaccaacg ttatgatggg 6840
   atcggtctcg ccgagtatat tcccttgctc gccaatggaa cttttttctt tgatgggtcg 6900
   gatggagccc cgctccaggt ccgtcatctc cttccagcag actggaagcc cgtcaatgga 6960
   ccacttacgg tcttgttcga ttccgccccc cataccatta ttgggactca cttctctatc 7020
   cacaatcatc ccatttacaa gcttaacgat ggttctatct ttgttgtctt ggacctggcc 7080
   accaagcacg gttttgagtt taccaatcaa tctttcatgg acccccagct tgaggtctcc 7140
   ggggaaaacc gtttcttgcg tctcgttacc aatgaccaag acttcgccta ccatggctct 7200
   agtcttagca tgattcatga caagggcgtt aagcgccctg ccaatgcgta agtacaattc 7260
   taccaatgta ccagtttcgt gtgctcatat agtcataggt ttatcttgtt cagaacggac 7320
   aaaatgaagc ccctcaaggc tcagtacccg gagatgggca acaacgatat ctgtacgtgg 7380
   ctctcatttt ccctttattt agatctctat tcttgctacc atttgcctaa cgttttaata 7440
   gccaagattc tcggtcgcat gtggcagaat tcctcggatg aggtcaaaaa tgtttataag 7500
   gtaaaacatc tcacctaact gttagttaga atcactaaca cattaatagg caagggccca 7560
   tcagcttgcg gtttcccaca agttgatcaa cccggattac aaatactccc cgcgccgctc 7620
   tcgcgaaatc atgaggaggg gctccgggaa taaaggtcct tcggcccgta tcgaccttca 7680
   tcgtgctggt gttgcgaagc gtggaaaaaa gaggatcatt acccaccttg ggttcctcac 7740
   tcaacgtttc gcgactcacc ccactgctaa caatctcggt gatatcatcg aggaagccga 7800
   aaaggtcttg aaggcctttg gtatggtcaa ttgagtgatc tctacggagg gactcatcgg 7860
   tacctgggtg gtatactttt aatgggaggg atgatcttgt ctatcaaagg tcattcatta     7920
   tctcagtcga ctaaatttat gacctaaata gataaaaaat atcttttaaa accaaaatga     7980
   ggggtccgaa cactacatca tacagtaaat atcaagttgt gaagcggaag agagccgact     8040
   atgggaagag gcagtaagaa tttaattctg actaagtctg gaaagtttac acagcacttg     8100
   cacagaagtt atgctatggt ggtgccatca ccggaaaaac acgccaaaaa ttcgggacct     8160
   atggtgccgt gacttagttt gagcgcaagt acatggttcg acacaaaaga gtgttacgga     8220
   gtaggggtgt tggggttgat tttggccatt agcgttatcg tatagattgt ttgaaaacac     8280
   gactcgtatt gcttaacaag aatatggaga gagctatttt tttaattttt tttaattttg     8340
   atagatgtat tggactggag gccaagcatc tgatcaattg gttatactat actcctattt     8400
   gttcatagtt gactgaggcc ctgtttggct acaacggttc ctttctgttt ccccatacaa     8460
   agcgaccgtt gcagaacggg agggcaatag cttttatatg agaaacggtt ccaaaatgaa     8520
   acggaaccga ggaaattgtg atctagtttc ttggatgtgc cgctgctact gaaatttttg     8580
   ttataaaggt atccgtttta tatctgactc ccttcacaca gaccccaatg gcgcgaatcc     8640
   ccggagtact ttgctcatga aagtcgtcct gtcgtccttg ggcatcaccc cttccagtcg     8700
   aaggagatcc catgagacga gcccggaatg cacttatatg tcaccatatg gacatgaacc     8760
   tccgttgaga tttgctttta cagtcgggcg ttgttttatt tcccgagtac tttagagttt     8820
   ggaaccagtt ctattgcttt gacaaagtct agtttattat accttgcagt tttcatctta     8880
   tcacatttta ttttctctag ccgtacattc ttgccctcgc gtttttttct cttgcacaac     8940
   tgatagacat ataagtgttc ttcagataga aggaaatggc gtacgatagc gcaaggattc     9000
   ctaagaccat tgtgagaaac tatgtaaaga cttctgacag gtccccacga atagtgcaat     9060
   atcatactta gcagcagaga aaccatcaag aaggtagact acagcacata gtcagaggcc     9120
   acggaactcc tgagacataa gggtagggat aaccaccgta cacgagaaga acaattagaa     9180
   taaaacagag ccattttata acaaatacta catgacaaag ggctaccaga atatattgat     9240
   gcgcagaggc ccatgagaga gagcatagga tccctatttc ggtacccgta gtttaagagg     9300
   gtgaaacatg ccctagatat tagtatattt tctagggaac tgctcttata tgatatgaaa     9360
   aatatacttg tcctctggcg tatgtcacat ccaccctgca agcttaacct ctttgggtgc     9420
   tcatcatagg caaggcccac tagtttcagg agccagtcag atattataca atagaatcta     9480
   tagatcggag gtcctaccga tttggatgtg attcgggtaa gtctgggaca ttctgttaag     9540
   attataccca ctaccgaagc cggaaagagt ccagatggct ggccaatatc cgaaggggca     9600
   taggccaaag ctatcaggtg ctctggcaca gttgcacatg ctcaggcaat caaactttga     9660
   agataccgta aaaacccttg ccgttcacta ccccaccggg ttgttaattt cctaatctcc     9720
   gccgtattca gacataagcg cttaagcaga aatagtgctt tcctgacacg gtacagccta 9780
   agccctgtgg aattgttggt tctcatgaac tagttttcgc aaccctgtta acatgtcgga 9840
   cgcatgtcgt gcgctgatgc ggcgaactac agggatattg gagacctacg gagatatcga 9900
   gtggaaagga tttttacagt attggtacat ggctgttaga ctctcttgcc ctccctcctt 9960
   ttgctacgca cgatgaagaa tagaacaata ccggtaggtc agatcagacg acgactctgc 10020
   ttggaataga gagatagaga aatagcatgg ctcgggcccc tgcgcccgtc agccacccgg 10080
   accgggccag ggttgagatt tgaaagaaga tttcactggg tcagcccggc agcactggta 10140
   cccagagccg cggatgtgcc agcctggggt tactcattcg gcgctcgaac ttcccccaat 10200
   aacccctccc ttacggcgtc tcgcccaaga ctagtggcat agggccaaaa tactctgtat 10260
   catatgatat catacgatat cgttgtccaa tgcaaaaaga ccgcaaaccc aaacctaatt 10320
   gaaggttgtg ataccactac aaaagtccga ggctcaatat tcccacccaa tcccatgtca 10380
   accccccaca aaaaattctc ctctcagcgc ccactccagt gtaccaaatt caacgatatc 10440
   ttccacacaa tacacccccg ccttccttcc taaaatgccc ccaagtatcc ccccaactaa 10500
   aaaaaaccga attcaatggc gatggctaac ttggaattgt aaaacgaaga tatcatgatc 10560
   cgccgcgagg tgatgttccg ggctggaatg atgcttgtca atgaaatgga agccactgcc 10620
   tggaaccctg gcaaccttat ggttgggatt gccatccata agaatccaag cgttcgtatg 10680
   gttaaagcgg ttgagtcggc caaggacttg ctccgtgaag tcgctgtgag cgaccataaa 10740
   ctgcgcaaaa tggatcgcat caatgatctt cagccactgg cgaatatcca taatacactt 10800
   ttacagcttg gaggattaat ctctttgaaa caacatttct acgaggaact tcaaggtatc 10860
   aaattacgcc ttcaggaact gcagagacac atgacagaca acattggcca ggccgttgca 10920
   gaagttcatg acttcatctg tttcgtcact tttgtcaact tgatccaccc gcaaaactgc 10980
   gtattaacct ccaaatccat caaggatgcg gagattctcg cctctaagaa ctcatccccc 11040
   gaggcactcc aggcgtttgt tgaactccgt caggtttacg atctgcagac tcccgttcca 11100
   gggtaccgtc taagcaaagc cagggagcta cctgcaatca ctaggtgcgc aatggagcaa 11160
   tggcacaaag gttacttttc tcatgcagcc gcaactggaa tagtaagtaa aaaaacatct 11220
   cttccatctc agcactcgta cctaataagc gccatcccac ttgatactaa tatcaaccca 11280
   cagcaagtta tcccattcca gatcatattc ggatggtgga gtgagttcaa tttcctcatt 11340
   tcaatttgat ggtgttatta gctgacatca agagatgttg tcttatagtt aattatcaac 11400
   gcaagtttgt tagtagctct caagagaaaa tcactccgat tgtgcaagaa cttatcaact 11460
   gcgcatatac ctcggaggcc agtgagccaa aagataagga cgagaaacta tggatgcaag 11520
   aagataagga ggcttttaag gctgttacta acctagctat gtctatgact gaattgtgca 11580
   aaaatatccc tctacccgag catgtcaaac cgaaccctca gaaagagaag cagttgccga 11640
   tttacaccca gttcaaagtc gagcctcatg ttcctccgat cttccaagaa ttccagaagc 11700
   tggtggctca aatttacgga ctttttgaag aaaactcgtg ctcaactttc actatcatga 11760
   ccaaagcgta ttatccaaaa gtggagaact accttaggtt ctctgaaaaa gaccagacaa 11820
   catcttctgt gaagcacttg aggtaagcta tcttttaagg ttccaggttt cttttgcact 11880
   aaagctaatt ttcaacagcg ccgcgaccta ctttttcagt tcgctccgtg aggtcccagt 11940
   tgtggttctc atcaataaga atgtaggggg tgtcgtgcca cgcaaggctg aggttccact 12000
   ccttgatgct gttagcttgg ccggagtgga tcccactatt gccgatgaag agctcttggc 12060
   ggagattact tgcaagaatt tgccacaagt tggcatgcac tgtaagtttt tcattatggt 12120
   actactggtt tcggatatgt tctgatctct gaacgtaaca cagtccttgt caggtatcgc 12180
   ggacaatctc accagtgccc gcgcaactaa attctactat ctctggtggc gtttgatcgc 12240
   agcgctctat cagcgaaaag atgacaaatt tcgctatccg ggtagtcttt agatagttcc 12300
   attcaaaatc aaactcttgt atcacaagat ttgccatatc tccatccaca ctttcattgt 12360
   aaatttatat caaaaaaaaa aagaccccat ccagccccct ggaagaacag aggaaggagg 12420
   ggactggttg agggcatttt tcttccctta tcaccaggta catggtgaac ctcccagtga 12480
   ggttggggag tttttatttt acaaagggat ctaattcggg atcatggctt caggcccatt 12540
   cggggtagcg attgataatt tagaggagta tgttattgtc cattggttcc actttgggga 12600
   ggataggagt ttcttttatt atttgggttt tctaggccat ctttccgggt tcttaccgcg 12660
   ctttttcccg cagtttgcga ataaattcaa agacccaaag aaaacaaagt gatttagacc 12720
   ccatcaaaaa gggcacctag ctctgggaat gtaatta 12757
<210> 6
   <211> 15256
   <212> DNA
   <213> Tuber melanosporum
<220>
   <221> misc_feature
   <222> (15085)..(15085)
   <223> n is a, c, g, or t
<220>
   <221> misc_feature
   <222> (15109)..(15109)
   <223> n is a, c, g, or t
<220>
   <221> misc_feature
   <222> (15121)..(15121)
   <223> n is a, c, g, or t
<400> 6
<210> 7
   <211> 25
   <212> DNA
   <213> Tuber melanosporum
<400> 7
   caggtccgtc atctccttcc agcag 25
<210> 8
   <211> 26
   <212> DNA
   <213> Tuber melanosporum
<400> 8
   ccacatgcga ccgagaatct tggcta 26
<210> 9
   <211> 25
   <212> DNA
   <213> Tuber melanosporum
<400> 9
   caatctcact cgtgatgtct gggtc 25
<210> 10
   <211> 26
   <212> DNA
   <213> Tuber melanosporum
<400> 10
   tctcgggctg gaggtgcggg tcgagt 26
<210> 11
   <211> 31
   <212> DNA
   <213> Tuber melanosporum
<400> 11
   tggtatatgt ggatgtattg ataactataa t 31
<210> 12
   <211> 26
   <212> DNA
   <213> Tuber melanosporum
<400> 12
   agagatagag aaatagcatg gctcgg 26
<210> 13
   <211> 29
   <212> DNA
   <213> Tuber melanosporum
<400> 13
   gtgtgcgtgc ttgaagtgaa ttggagtaa 29
<210> 14
   <211> 27
   <212> DNA
   <213> Tuber melanosporum
<400> 14
   taattacatt cccagagcta ggtgccc 27
<210> 15
   <211> 25
   <212> DNA
   <213> Tuber melanosporum
<400> 15
   aagtaacctt tgtgccattg ctcca 25
<210> 16
   <211> 27
   <212> DNA
   <213> Tuber melanosporum
<400> 16
   cagcatggtc tggttggacg ttttgat 27
<210> 17
   <211> 26
   <212> DNA
   <213> Tuber melanosporum
<400> 17
   ctatgcaccc aagtgtgatt gaggac 26
<210> 18
   <211> 26
   <212> DNA
   <213> Tuber melanosporum
<400> 18
   ccattcgttg agatgcgccg ttgcta 26
<210> 19
   <211> 27
   <212> DNA
   <213> Tuber melanosporum
<400> 19
   aggcggtatc agagcttttc catgcag 27
<210> 20
   <211> 32
   <212> DNA
   <213> Tuber melanosporum
<400> 20
   gccaacctct agttgggata tttgttcagg ac 32
<210> 21
   <211> 25
   <212> DNA
   <213> Tuber melanosporum
<400> 21
   gtgaatagac agactagctt gcccg 25
<210> 22
   <211> 26
   <212> DNA
   <213> Tuber melanosporum
<400> 22
   aatctcttaa tgtccagacc cttacc 26
<210> 23
   <211> 27
   <212> DNA
   <213> Tuber melanosporum
<400> 23
   tgaatcaagg tagtttgctg attgagg 27
<210> 24
   <211> 27
   <212> DNA
   <213> Tuber melanosporum
<400> 24
   tatcgtacac cactttcctt ctatctg 27
<210> 25
   <211> 25
   <212> DNA
   <213> Tuber melanosporum
<400> 25
   gatcttcagc cactggcgaa tatcc 25
<210> 26
   <211> 27
   <212> DNA
   <213> Tuber melanosporum
<400> 26
   gttcctccga tcttccaaga attccag 27
<210> 27
   <211> 27
   <212> DNA
   <213> Tuber melanosporum
<400> 27
   gccggaatag tggcatcctg ggccgtc 27
<210> 28
   <211> 28
   <212> DNA
   <213> Tuber melanosporum
<400> 28
   gacggacggt tgattgaatt gcaccgag 28
<210> 29
   <211> 26
   <212> DNA
   <213> Tuber melanosporum
<400> 29
   gatcgtggtg accccggcct cgtgct 26
<210> 30
   <211> 28
   <212> DNA
   <213> Tuber melanosporum
<400> 30
   cttgcgtgaa gctggctttg tccccaag 28
<210> 31
   <211> 24
   <212> DNA
   <213> Tuber melanosporum
<400> 31
   atcctaactt acagcatctc gcct 24
<210> 32
   <211> 28
   <212> DNA
   <213> Tuber melanosporum
<400> 32
   tatcggctca caatccagtg gaggattc 28
<210> 33
   <211> 22
   <212> DNA
   <213> Tuber melanosporum
<400> 33
   acggcccagg atgccactat tc 22
<210> 34
   <211> 12121
   <212> DNA
   <213> Tuber indicum
<400> 34
<210> 35
   <211> 9770
   <212> DNA
   <213> Tuber indicum
<400> 35
   tgggcgattg cttgcctgaa aacaagtgat ccgggggagc actgtctgtc tgttcgcttc      60
   acttgtgctg ttcgaatgct ttacctggtt gtgcgctctg agtacgagaa atgggcgagc     120
   aagctagtct gtctgttcac gaagggcgca atgaactatc acaaaccaat tccagattct     180
   tctactcttt tatccccttc tcacggaggt tctttccagt gagcttcgcc cggtcttgtc     240
   tcgtctacca ttgcatagtt atttacagcg gatggcgtaa tccgcaccct tgtctatgat     300
   cgcaatacgg cattggctct aacaaaacta tatcgtgtcg ttccgaggga gaaaaaaagt     360
   cccttcattc tactttccct tcctccactc cgattcactg gcagatgaaa ccgtaccgcg     420
   cgctcgggct ctttggtccc tattttctca accggcgagg gcgtcgttag tatggggatt     480
   ggaaggctcc tttggcttgt ttgccgtgag taggaagggc gctgtcattg ctgagtagcc     540
   tttcagcggg acctgcgggg gtgcttcttt ccgatcggtc ttgcgagggg tggtattggt     600
   attgggcggt gttggccatt cggcggcgtt gatgaaattt ctatcaggag ttctgtggcc     660
   tctggatacg tggtgtagta tatcttcttg gcggtttctc tgctgctgct aagtatgata     720
   tcgcactatt cgtggggacc tgtcattaaa tctttacata gttgctcaca atggtcttag     780
   gaatcctcgc gctaccgtac accactttcc ttctatctga acaacgctct atatgtttat      840
   gtatcgtaca gggggagaaa ggtaacgcgt gggcaagaat gcacggctgg agaaaataaa      900
   atatgatagg atgaaaactg caaggtataa taaactagac ttttttgcca cagcgataga       960
   gctagttcca aactctacag tactcagggg gcaaacccac gcccgactat aaaagcaaat     1020
   ctccggaggg gctcacatcc gcacggtgac atataagcgc attccgggct cgtctcatgg     1080
   gatctccttc gactggaagg ggtgatgccc aaagacgact ttcatgagta aagtaccccg     1140
   gaaaagaagg aacggtgatg ccttcgatga ggtcctgttt tataagcctt aaatcgcgct     1200
   aggggttaac gctttgtggg actgtagcag cgctaccacc ttctgatggt cgtgcctaga     1260
   tgcgaacgag agaggtgttt gaccgtcatt gtttggcctg tcggggctga catcttcccg     1320
   gccgagtagt accttcacca cctccgcatg cccaccccaa gcagcgagcg agagcggtgt     1380
   ttggccgcaa tcatctggcc tgtcggggtt gacctcttcc cgcatgagta aaattttcac     1440
   cacttcttca agtccatgag cggcagcatg agagagcggt gattggccgt ggctgtctga     1500
   cttgtcggga ttgacctctt cccgcatgag tagcattctc accacctctt catatccgga     1560
   ccaaacggcg tgcgagagcg gtgttcggca gtagatatct ggcttgtcgg ggttgacctc     1620
   ttcccgcccg agaaagattt tcactactcc ctcatgtcca taaccagcgg catgcgagag     1680
   tggcgttcga ccgagattgt ctggcttgtc ggggttgacc tcttcccgct cgagtaataa     1740
   tttcgcaact ccctcatacc ccctccaagc agcattcgag agcggtgttt ggccgtcatt     1800
   atctggttcg ttgggattaa catcctcccg cccgagcagt attctcacca cttcctcatg     1860
   tccaaaccaa gcagcgagcg agagcggtgt ttggccgtca ttgtccggct tgtcggggtt     1920
   gacctcttcc ctcccgagta gtattttcac cacttcctca tgtccgcagg cagtagcatg     1980
   tgagagcggt gtttggccgc gattatcggg ctcgccggga tcgacctgtt ccagcccaag     2040
   tagcattttc accacttcct catgtccgtt atgagcagcc catgcaagtg gcgtataccc     2100
   ccaacaatct ccttcgttga tatcccaaca tcccatctct attaaagcag ccacaacctc     2160
   gacaattcca aagaacgatg cgcagtgcag cccactgaat gggggccagg tatcgtgccc     2220
   tccgggacct aaattcccca cttgcgctaa aagaaattcg gtggatatgt ggccatcaaa     2280
   cccctggaat agcttcagcg caagtgatct cccgcaatcc gagagttccg ttttcgcatg     2340
   gactccccaa tacatagaac aatattctag aaagggtgca tccgaggtgt caggagatgg     2400
   atcggtcgaa aggtccttga cttgctggga attcagatag gtcaagcata tctctgccat     2460
   tgccgcatgg ggtctactga aaatatcggg atgagcggaa aggtactctt ggagagtaaa     2520
   gtggactagt cgcacggctg atgcctcctt ccccaccgta ataagccctt gacagcaacc     2580
   tatcagtgtc gacatcgaag ggatgttgtt agcattgaag tcggtggagc ccagctcgac     2640
   tgccagggcg cagcagagct cgtccgtcct gagcggccgc tgcgcatgac tgatccacat 2700
   caaggctgcc atcccgagtc tcgatttatc cccgccctgc gcctttatcc gtcctatcgt 2760
   cgcaccgtag gcatctccca gacccaaacc gtcggtcatt ttgctaagcc tttctcgtct 2820
   ccgataggtt gtcgattctt gtaagattgc ttcgatgttt agggaaacta gtataaacct 2880
   ggatatatct attagcggat agtgtgcaat gggaatccct ctgtcattgc cctacaaaca 2940
   tttccgagat cttcccaggg atctgctcta gaacgtctgc ctcaagaccc tcgtccatgg 3000
   catctggggt ttcgtcctcg tctagcctaa cacgaaggta tctgataata tcatctcttc 3060
   tagggcctat agacacactt gccactcgcc cggcaagacg cttttcgatc tcggcccgaa 3120
   tataggacct tccagtcagg aatattcgta tccccgggga cttcccaagg atttttttca 3180
   gcgaatcaag gagcttggct cgatgcactg cggcacattc gtccagggcg tcaatgcaca 3240
   taaatgtgcg ctgtgaggac gtgatggtct gtagcatctt tacgatatca ggaagttgcg 3300
   gtccccgccc gccaatggcc ctcctctgct cttgaaaagc ccgcgatatt tcctctggaa 3360
   tcctttccat tccgctaacg atctgtttaa gcaaggaacc cagcatattc gtcgcggact 3420
   gctcctttcg ggccgcaaag tcaaagtaaa aacaggtaac ggtagtgttt tgtcctctag 3480
   cctgatcgca cagcctgtcc actaccatcg aactgtcatc gcggcttatt agcactggcg 3540
   cacttctctc cccagagaat aactcttgtt acctgataaa tgtcttgccc acacctggat 3600
   ctccatggca aaacaacact gccttattgc cttcaccttc caaactccag tcataccagc 3660
   ttctgaattc gtcagtttgg ataagccatt cccccacatt gtccacacgg tgctcttgaa 3720
   tgtcgcgatg ccgtaaccta ggttccagcg gcgacaacca agctaggagt tgagaccgat 3780
   catcggcaac tgtgaagttg ttccagacat tgctaaaaga gctcgtatta ttgaagcagt 3840
   tgttattgct attgattgta tattgagaca tcttctgctc tttactgctt gcactattga 3900
   agggaagtat ttttctggtt tggttggttg acgatatcct tgggaggtca agaaaagcgg 3960
   ggtctagatg gggaaagcaa gggtctgctg gtgttaaagc agcatcgcag ataccgctac 4020
   tcgtacatgc agtactaact caagagtttt cgtccggtac cctttttaca ccaaaagtat 4080
   ctaacttcac caccctctct cacagcacaa tgtcaagggg aatatgccaa aatttcgcgg 4140
   tgtattggca aatctgtagc taatatggga gataatatta ttacagaatt gttgagtaat 4200
   agagccccat accggtacaa aaatctaggt taatttcaca caggccaacg ttattgtaaa 4260
   caatgggcac aactttgatc ctctacagtt ctacttctag agatttaaat gaataacagc 4320
   tatctagccg agggagggat ttggagtgtt ttcaacaggg tattgtttgg attgttaggt 4380
   atgttgcggt agtctgcgac atgataaaca ccatacttcg tggagaatga tataaagcgc 4440
   tgcgcccggg aaaagtaagg acaaaataaa tagacactgt taaatacgta gaacaaggtc 4500
   tttggcattc gagtgcaatt tgaaaacttg cggtcgatat tcgtgatcga gagattgtgg 4560
   ctgttgcatc gaactaccat atgactttcc taaataggaa ggcagactct tgattctttt 4620
   agatggcctg gcagttgttg aaggttctga actataggaa ctattggtac ttgtactaaa 4680
   tactaattcc taacaacgta cagggatgtc acgcaaaatc aaatcaaaca ttacacacta 4740
   atggtattaa agaatgtttt acttgctagc gccgtcatcg ttcctgatca cctggaattt 4800
   gtcagttcca atagacagac agctataagg gctcttgtag atgagtgttt gaggatttac 4860
   attgctatct cagcttcagg gagaacaaga aaggtatgtc attttttgcc cccacagccc 4920
   aatatgtgca gcgaaaatgg ttgttttttc tcccgtcaaa atacaatttt tgccgggata 4980
   aggggaccct aggtcgggtg gggttaaatg gcttggggta tcatatctag aaggaaattg 5040
   acaattagcg cgcattaaac tcacagctct ttggtgagct ttcctcgttg tctgtctgta 5100
   ggtgcacgtg gagcgatcca ggtatctggg gggacctggg aagcattccc ctcttattgt 5160
   ggtggtaggg cacctgcagt ggggaaactc cagcatataa ataaagttat aatataatat 5220
   tattacgatt cccgcctatc tcataattaa attctgcgat attcccagct ctggggtatt 5280
   attggtcgtt atctccacga tgtatcaccc gatgtttgat ttagcgtgac tgttgtaaat 5340
   cacagtggaa tgagagataa gagtatgtgg ggacaggtag aagtgacttg cttgacactc 5400
   tgggtggggt gaatacatac atataactca tagagatgtc atgctaaggt atatagacaa 5460
   ctctataaca caacttggat atggtggata aagtgtgacg gcctactaac tgggaccaca 5520
   aaggattcaa ttgtgtacct taagaacatg gaatacttat gctgtaatgt tttatgcatg 5580
   caggaatcac ccattcagta gatgtcgaat tttacagctt tcgaactaac actccattac 5640
   caaataagta tcgcgggtgc gcatttgtgg gaagagatgg gcattatacc ataaaccaca 5700
   ggttctaata agtgtttaga tattggctgc ttccagcgat tccatcagga cttaagatat 5760
   ttcctttcta gtacaactac taaatagaga aagacagcga agagatggaa cagagaggcc 5820
   ctagtgcccg cctcatgtgc aattacggta tgtttgtgag agacactccg ctatgatcta 5880
   ctaccttcac agatgttcct aacattatat aaacaaacgt gcaatatctc ggtctctgat 5940
   catacaatcc cctgtcctac atccaatcaa gttcctttcc tccgtgtaat ccacttgaca 6000
   tcttagcttc tatcccggga gtcggagcgg gaccgggggc acattgcccc aggctctctt 6060
   atctgacgat atcttcgtcc agaatcaaga gactttgtct gtcccttcta aactccgccc 6120
   ccttccatca tcctgccttg catgaagctc aggaggttga gtgcttagat gctccacaat 6180
   ggtttgctca atcggccgct gctcctgggc cagaagctct gacggcttag agttggttaa 6240
   agattctaat ttccaagctg aaaatccttt tgggggcggg ctttgctcgc tcgtgatcca 6300
   gaccttctcc gttcgttata ttactatacc gtcctactga tttcagaatg gcctaactta 6360
   gccaaaaacc cctctgaaac cactaggagt aactgctagc aatgttagcc acaatccctt 6420
   aatttccaga cccttaccga gaccggttgt cgcattggat ggctgctcct ttgttctgga 6480
   gttccatctt ttaaacagct ttcccgagag ctgcatacgc cgcatcaaac gtcgtagtcc 6540
   tccttgtcca ttcacctgga tgcacaagcg gatagccgaa tatgggacat ccaccggatc 6600
   ggctggtagt gttgcaaaag catttggctt tcctggttgg ccgggcagcg ggtgaagcta 6660
   ccatacttct gaattcacat ttcgcctaac ggcctcctgg ccattggata tccccccagg 6720
   acggaaagga cagcttctgg atggtcaggc ttaaggtatg gtttcccagt ccgccacaca 6780
   gatttatgga gatcatatta aattccgcaa tcagaaaaaa ctatcttgaa ccacatacgg 6840
   tatctgatcg caaagaatta ttgttcaggg ctcggatacc atacctctct atcaaagaag 6900
   caatgtacga gtacaagaga ggtggggttg ggggacggat gctgtaacag aacccacatt 6960
   tcctagctca ccctgtttca ttaattcctt tgttcggaac ttctcccgaa tgtcgatcgg 7020
   atcttcatca ataccttgac tcgttttcta ccatactcca gcatctccga ccgtaaaaaa 7080
   aaaaattcgc ggaccttttc gccaggaatc ttggaaacct aagggctctc ccttcctgcc 7140
   ctgggggaaa tcttatcgtg tcatatgtgt gataataata ttatgacatt ttcatgagaa 7200
   tactattctc gggccctaat agggttgagt tgtacgtgac ggcttcttgg cgtcactcta 7260
   ttttttgggt cttggcagtc cccctcgtga ctggaggcta caaaggggat ccatcctttc 7320
   cccttctttc ctccaccgac aaccgtcaag ttcgacagaa acattcggcc tttctacttg 7380
   cacctctcca ctcacgtcgt ttcctttttt tctgccacta tgacttcttc tttgtccaac 7440
   ctcaatatgg cccttgaagc cgggcctctt attcaccaac attatgatgg aatcggtctc 7500
   gccgagtata ttcccttgct cgccaacgga acctttttct ttgatgggtc ggatggagtc 7560
   tccctccagg tccgccacct ccttccagca gactggaagc ccgtcaatgg accacttgcg 7620
   gtcttgttcg attccgtccc acataccatt attgggactc acttttctat ccgcaatcac 7680
   cccatttata agcttaacga tggttctatc ttggtcgtct ttgacccggc cgctaagcac 7740
   ggtttcgagt ttaccaatca atctttcgtg gacccccagc ttgaggtctc cggagagaac 7800
   cgtttcttgc gtctcgttac caatgaccaa gacttcgcct accatggttc tagtcttagc 7860
   atgattcatg acaagggcgt taagcgccct gccaatgcgt aagtacaatt ctaccaatgt 7920
   accagtttcg tgtgctcatc tagtcatagg tttatcttat tcagaacgga caaaatgaag 7980
   cccctcaagg ctcagtaccc ggagatgggc aacaacgata tctgtatgtg gctcttattt 8040
   tccctttatt ttgatctcta ttcttggtac catttgccta acgttttaat agccaagatt 8100
   ctcggtcgca tgtggcagaa ttcctcggat gaggtcaaag atgtttataa ggtaaaacat 8160
   ctcacctaac tgttagttag aatcactaac acattaatag gcgagggccc atcagcttgc 8220
   ggtttcccat aagttgacca acccggatta caaatactcc ccgcgccgct ctcgcgaaat 8280
   catgaggagg gactccggga ataaaggtcc ttctccccgt atcgaccttc atcgtgctgg 8340
   tgttgcgaag cgtgaaaaaa agaggatcat tacccacctt gggttcctca ctcaacgttt      8400
   cgcaactcac cccactgcta acaatctcgg tgatatcatc gaggaagccg aaaaggtctt      8460
   gaaggccttt ggtatggtca attgagtgat ctcaacggag ggactcatca gtgcctgggt      8520
   gatatacttt caatgggagg gatgatcttg tctatcaaag gtcattatct cagtcgaata      8580
   aatttatatg acctaaataa ataaaaaata tgtttaaaaa aaaaaaaatg aggggtctga      8640
   acactatatc atacagtaaa atatcaagtt gtgaagcaga agagagtcga ctaccggtat      8700
   gggaagaggc agtgagaatc taattctgac tgtcttagtc tggaaagttc gtacagcact      8760
   tgcacacaag ttatgctatg gtggtgccat caccggaaaa acacgccaaa aattcgggac      8820
   ctatggtgcc gtgacttagt ttgcgcgcaa gtatgtactg tacatggtcc gacacaaaag      8880
   agtgttatgg agcaggggtg gtggggttga ttttggccat cagcgttatc gcacagattg      8940
   tttgaaaaca cgactcgcat tgctcaacaa ggacatggag agctattttt tttaattttt      9000
   ttttaatttt gatagatgta ttggactaga ggccaagcat ctgatcaatt ggttgtacta      9060
   tatttgctca tagttgactg aggccctgtt tggatacaat ggttcctttc tgtttccccg      9120
   tacgaggtga ccgttgcaga acgggagggc aatcgctttt atatgagaaa cagttccaaa      9180
   acgaaacgga accgttccat ccaaacaagg cctgagagaa gaggaaattg tgatcttctc      9240
   agggttaagt ttcttggatg tgtcgctgct actgaaactt ttgttatata ggtatccatt      9300
   ttatatctga ctccctccac acagacccca gtggagcgaa tccccggagc actttgctca      9360
   tgaaagtcgc ccttgggcat tactccttcc agtcgaagga gatctcatga gacgagctcg      9420
   gaatgtgctt atatgtcacc agacggacgt gaacctccct ggagatttgc ttttacagtc      9480
   gggcgttttt tgtttcccga gtactttaga gtttggaacc agttctattg ctttgacaag      9540
   gtctagttta ttataccttc ttgctctcgc gtttttttct ctcgcacaac tgatagacat      9600
   ataagtgttc tttagataga aggaaatggt gtacgatagc gcaaggattc ctaaggccat      9660
   tgtggacaac tatgtaagga cttatgacag gtccccacga atagtgcgat atcatactta      9720
   gcagcagaga aaccatcaag aaggtagact atagcacata ttcagaggcc                 9770
<210> 36
   <211> 10118
   <212> DNA
   <213> Tuber indicum
<400> 36
   tgggcgattg cttgcctgca gacaagtgat ccggacccat ccagtggtgg gtccggcacg      60
   gggggagcac tgtctgtctg ttcgcttcac ttgtgctgtt cgaatgcttt acctggttgt     120
   gcgctctgag tacgagaaat gggcgagcaa gctagtctgt ctattcacga agggcgcaat     180
   gaactatcac aaaccaatcg cagattcttc tactcttttg tccctttctc acggaggttc     240
   tttccagtga gcttcgcccg gtcttgtctc gtctaccatt gcatagttat ttacagcgga 300
   tggcgtaatc cgcacccttg tctatcatcg caatacggca ttggctctaa caaaactata 360
   tcgtgtcgtt ctgagggaga aaaaaagtcc cttcactcta ctttcccttc ctccactccg 420
   attcactggc agatgaaacc gtaccgcgcg ctcgggctct ttggtcccta ttttctcaat 480
   gggcgagggc gtcgttagta tgggggttgt taggctcctt tggcctattt gccgtgagta 540
   gggagggcgc tgtcattgct gagtagcctt tcagcggggc ctgcgggggt gcttctttcc 600
   gatcggtctt gcgaggggtg gtattggtat tgggcggtgt tggccattcg gcggcgttga 660
   tgaaatttct atcaggagtt ctgtggcctc tggatacatg gtgtagtata tcttattggc 720
   ggtttctctg ctgctgctaa gtatgatatc gcactattcg tggggacctg tcattaagtc 780
   tttacatagt tgctcacaat ggtcttagga atcctcgcgc catcgtacat cactttcctt 840
   ctatctgaac aacgctctat atgtttatct atcgtacggg gggagaaggg tgacgcgtgg 900
   gcaagaatgc acggctggag aaaataaaat atgataggat gaaaacttca aggtataata 960
   aactagactt ttttgccacg gcgatagaac taattccaaa ctttacagta ctcagggggc 1020
   aagcccacgc ccgactataa aagcaaatct ccaggggggc tcacatccgc acggtgacat 1080
   ataagcgcat tccgggctcg tctcatggga tctccttcga ctggaagggc tgatgcccaa 1140
   gggcgacttt catgagtaaa gtaccccgga aaagaaggaa cggtgatgcc ttcgatgagg 1200
   tcctgtttta taagccttaa atcgcgctag gggttaacgc tttgtgggac tgtagcagcg 1260
   ctaccacctt ctgatggccg tgcctagatg cgaacgagag aggtgtttga ccgtcattgt 1320
   ttggcctgtc ggggctgaca tcttcccggc cgagtagcac cttcaccacc tccgcatgcc 1380
   catcccaagc agcgagcgag agcggtgttt ggccgcaatc atctggcctg tcggggttga 1440
   cctcttcccg catgagtaac attttcacca cttcttcaag tccatgagcc gcagcatgag 1500
   agagtggcgt tcggccgaga ttgtctggct tgtcggggtt gacctcttcc cgctcgagta 1560
   ataatttcgc aactgcctca tgccccctcc aagcagcatt cgagagcggt gtttggccgt 1620
   cattatctgg ctcgttggga ttaacatcct cccgcccgag cagtattctc accacttcct 1680
   catgtccaaa ccaagcagcg agcgagagcg gtgtttggcc gtcattgtcc ggcttgtcgg 1740
   ggttgacctc ttcccgcccg agcagtattt tcaccacttc ctcatgtcca caggcagtag 1800
   catgtgagag cggtgtttgg ccgcgattat ccggctcgtc gggatcgacc tgttccagcc 1860
   caagtagcat tttcaccact tcctcatgtc cgttatgagc agcccatgca agtggcgtat 1920
   acccccaaca atctccttcg ttgatatccc aacatcccat ctctattaaa gcagccacaa 1980
   cctcgacaat tccaaagaaa gatgcacagt gcagcccact gaatggaggc caggtatcgt 2040
   gcccttcggg acctaaattc cccacttgcg ctaaaagaaa ttcggtggat atatggccat 2100
   caaactcctg gaatagcttc agcgcaagag atctcccgca atccgagagt tccgttttcg 2160
   catggactcc ccaatacata gaacaatatt ctagaaaggg tgcatccgag gtgtcaggag 2220
   atggatcggt cgaaaggtcc ttgacttgcc gggagttcag ataggtcaag catatctctg 2280
   ccattgccgc atggggtcta ctgaaaatat cgggatgagc ggaaaggtac tcttggaggg 2340
   taaagtggac tagtcgcacg gctgatgcct ccttgcccac cgtaataagc ccttgacagc 2400
   aacctactag tgtcgatatc gaagggatgt tgttagcatt gaagtcggtg gagcccagct 2460
   cgactgccag ggcgcggcag agctcgtccg tcctgagcgg ccgctgcgca tgactgatcc 2520
   acatcaaggc tgccatcccg agtctcgatt tatccccgcc ctgcgccttt atccgtccta 2580
   tcgtcgcacc gtaggcatct cccagaccca aaccgtcggt cattttgcta agcctgtctc 2640
   gtctccgata ggttgttgat tcttgtaaga ttgcttcgat gtttagggaa actagtataa 2700
   acctggatat atctattagc ggatagtgtg caatgggaat ccctctgtca ttgccctaca 2760
   aacatttccg agatcttccc tgggatctgc tctagaacgt ctgcctcaag accctcgtcc 2820
   atggcatccg gggtttcgtc ctcgcccagc ctaacacgaa ggtatctgat aatatcatct 2880
   cttctagggc ttatagacac acttgccgct cgcccgggaa gacgcttttc gatctcggcc 2940
   cgaatatagg accttccagt caggaatatt cgtatccccg gggacttccc aaggatttgt 3000
   ttcagcgaat caaggagctt ggctcgatgc actgcggcac attcgtccag ggcgtcaatg 3060
   cacataaatg tgcgctgtga ggacgtgatg gtctgtagca tctttacgat atcaggaagt 3120
   tgcggtcccc gcccgccgat ggccctcctc tgctcctgaa aagcccgcga tatttcctct 3180
   ggaatccttt ccattccgct aacgatctgt ttaagcaagg aacccagcat attcgtcgcg 3240
   gactgctcct ttcgggccgc aaagtcaaag taaaaacagg taacggtagt gttttgtcct 3300
   ctggcctgat cgcacagcct gtccactacc atcgaactgt catcgcggct tattagcact 3360
   ggcgcacttc tctccccaga gaataactct tgttacctga taaatgtctt gcccacacct 3420
   gggtctccat ggcaaaacaa cactgcctta ttgccttcac cttccaaact ccagtcatac 3480
   cagcttctga attcgtcagt ttggataagc cattccccca cattgtccac acggtgctct 3540
   tggatgtcgc gatgccgtaa cctaggttcc agcggcgaca accaagctag gagttgagac 3600
   cgatcatcgg caactgtgaa gttgttccag acattgctaa aagagctcgt attattgaag 3660
   cagttgttat tgctattgat tgtatatcga gacatcttcc gctctttact gcttgcacta 3720
   ttgaagggaa gtatttatct ggtttggttg gttgacgata tcctagggag gtcaagaaaa 3780
   gcggggtcta gctggggaaa gcaagggtct gctggtgtta aagcagcatc gcagataccg 3840
   ctactcgtac atgcagtact aactcaagag ttttggtccg gtaccctttt tacaccaaaa 3900
   gtatctaact tcaccaccct ctctcacagc acaatgccaa ggggaatatg ccaaaatttc 3960
   gcggtgtatt ggcaaatctg tagataatat gggagataat attattacag aattgttgag 4020
   tattatagag gggtggagaa aatgtcattt aagtgcgagg tattggttgt catgtacttt 4080
   tttgtataga gccccataca ggtacaaaaa tccaggttaa tttcacacag gccaacgtta 4140
   ttgtaaacaa tgggcacaac tttgatccct ttgatcctct acagttctac ttctagagat 4200
   ttaaataaat aacagctatc tagctgagag agggatttaa agtgttttca acgggtattg 4260
   ttgggattgt taggtatgtt gcggtagtct gcgacttgat aaacaccata cttcgtggag 4320
   aatgatataa agcgctgcgc ccgggaaaag taaggacgaa ataaatagac actgttaaat 4380
   acctagaaca aggtctctag cattagagtg caatttgaaa acttgcggtc gataaattat 4440
   tcgtgatcga gagattgtgg ctgttgcatc gaactaccat atgactttcc taaataggaa 4500
   ggcagactct tggtgcctgg cagttgaagg ttctgaaata ggaactattg gtactcgtac 4560
   taaatactaa ttcctagcaa cgtacaggga tgtcacgcaa aatcaaatca aacataacac 4620
   actaatggta ttaaagaatg ttttacttgc tagcgccgtc atagttcctt tcccaatcac 4680
   ctggaatttg ttagttccaa tagacagcta taagggctct tgtagatgag tgtttaagga 4740
   tttacattgc tatctcagct tcagcgagaa caagaaaaat gtcatttttt gcccccacag 4800
   cccaatatgt gcagcgaaaa tgtttgtttt ttctcccgtc aaaatacaat ttttgccagg 4860
   ataaggggac cctaggtcag ggtggggtta aacggcttgg ggtatcatat ctagaaggaa 4920
   attgtggatc cctggacaat tagcgcgcat taaactcaca gctctttggt gagctttcct 4980
   cgttgtcttt ctgtaggtgc acgtggagcc agccaggtat ctggggggac ctgggaagca 5040
   ttccctctta ttgtggtggt agggcctgca atggggaaac tccagcatat aaaatttaat 5100
   aaagttatat tataatatta ttattacgac tcccgcctat ctcataatta aattctgcga 5160
   tattcccagc tctggggtat tattggttgt tatctccatg atgtataacc cgatgtttga 5220
   tttagcgtga ctattgtaaa tcacagtgga atgaaagata agagtaaatg gggacaggtg 5280
   gtagaagtga cttgcttgac tttctgggtg gggtggatac atacatatag ctcatagaga 5340
   tgtcatgcta aggtatatag acaactctat gacacaactt ggatatggtg gataaagtgt 5400
   gacggcctac taactgagac cacaaaggat tcaattgtgt accttaagaa catggaatac 5460
   ttatgctgta atgttttgtg catgcaggaa tcaccctttc agtagatgtc gaattttaca 5520
   gctttcgaac tagcaatcca ttaccaaata agtatcgcgg gtgcgcattt gtgggaagag 5580
   atgggcatta taccataaac cacaggttgt aaaaagtgtt tagattttgg ctgcttccag 5640
   cgattccatc aggactcaag atatttcctt tccagtacaa ctactaaata gcaaaagata 5700
   gcgaagagat ggaagagaga ggccctagtg tccgcctcat gtgcaattac ggtatgtttg 5760
   tgagagacac tccgctatga tctactacct tcacagatgt tcctaacatt atatatacaa 5820
   acgtgcaata tcccgatctc tgatcacaca atcccctgtc ctacatccaa tcaagttcct 5880
   ttcctccgtg taatccactt gacatcttag cttctatccc gggagtcgga gcgggaccgg 5940
   gggcacattg ccccaggctc tcttatctga caatattttc gtccagaatc aagagacttt 6000
   gtccgtccct tctaaactcc gcccccttca atcatcctgc cttgcatgaa gctcaggagg 6060
   ttgagtgctt agatgctcca caatggtttg ctcaatcggc agcggctcct gagccagaag 6120
   ctctgatggc ttagagttgg ttaaagattt taatttccca gctgaaaatc cttttggggt 6180
   cgagctttgc tcgctcgtga tccagacctt ctccgttcgt tatattacta taccatccta 6240
   ctgatatcag aatggcctaa cttagccaaa aaacccctct gagaccacta ggagtaactg 6300
   ctagcaatgt tagccacaat ccttaatttc cagaccctta ccgagacccg ttgtagcatt 6360
   ggatggctgc tcctttgttc tggagttcca tcttttaaac agctttcccg agagctgcat 6420
   acgccgcatc aaacgccgta gtcctccttg tccattcacc tggatgcaca agcggatagc 6480
   cgaacatggg acatccgccg gatcggctgg tagttttgca aaagaatttg gctttcctgg 6540
   ttggcagggc agcgggtgaa gctaccatac ttctgaattc acatttcgcc taacggcctc 6600
   ctggccattg gatatccccc caggacggaa aggacagcct cgggatggtc aggcttaagg 6660
   taccggtatg gtttcccagc ccgccacaca gatttatgga gatcatatta aattcccaat 6720
   cagaaaaaac tatcttgatc cacatacggt atctgatcgc aaagaattat tgttcagggc 6780
   tcagatacca tacctctcta tcaaagaagc aatgtacgag tacaagagag gtggggttgg 6840
   gggacgggcg ctgtaacaga acccacattt cctagctcac cctgtttcat taatttcttt 6900
   gttcggaact tctcccgaat gtcaatcaga tcttcatcaa taccttgact cgttttctac 6960
   cgtactccag catctccgac cgtaaaaaaa aattcgcgga tctttttcaa gataatacta 7020
   ttctcgggcc ctaatagggt tgagttgtac gtgacggctt cttggcgtca ctctattttt 7080
   tgggtcttgg cagtccccct cgtgactaga ggcctacaaa ggagatccat cctttccccc 7140
   ttctttcctc caccgacaac cgtcaagttc aacagaaaca ttcggccttt caacttgcac 7200
   ctctccactc acgtcgtttc cttttttctg ccactatgac tatgacttct tctttgtcca 7260
   ttctcaatat ggccgttgaa gccgggcctc ttattcacca acgttatgat ggaatcggtc 7320
   ttgccgagta tattcccttg ctcgccaacg gaaccttttt ctttgatggg ccggatggag 7380
   tctccctcca agtccgccat ctccttccag cagactggaa gcccgtcaat ggaccactta 7440
   cggtcttgtt cgattccgcc ccccatacca ttattgggac tcatttttct atccgcaatc 7500
   atcccattta taagcttaac gatggttcta tctttgtcgt cttggacccg gccaccaagc 7560
   acggtttcga gtttaccgat caatctttcg tggaccccca gcttgaggtc tccggagaaa 7620
   accgtttctt gcgtctcgtt accaatgacc aagacttcgc ctaccatggt tctagtctta 7680
   gcatgattca tgacaagggc gttaagcgcc ctgccaatgc gtattgatac aattctacca 7740
   atgtaccagt ttcgtgtgct catctagtca taggttcatt ttattcagaa cggacaaaat 7800
   gaagcccctc aaggctcagt acccggagat gggcaacaac gatatctgta cgtggctctc 7860
   atttccccct tatttagatc tctattcttg gtaccatttg cctaacgttt taatagccaa 7920
   gattctcggt cgcatgtggc agaattcctc ggatgaggtc aaagatgttt ataaggtaaa 7980
   acatctcacc taactgttag ttagaatcac taacacatta ataggcaagg gcccatcagc 8040
   ttgcggtttc ccataagttg atcaacccgg attacaaata ctccccgcgc cgctctcgcg 8100
   aaatcatgag gagggactcc gggaataaag gtccttcccc ccgtatcgac cttcaccgtg 8160
   ctggcgttgc gaagcgtgga aaaaagagga tcgttaccca ccttgggttc ctcactcaac 8220
   gtttcgcgac tcaccccact gttaacaatc tcggtgatat catcgaggaa gccgaaaagg 8280
   tcttgaaggc ctttggtatg gtcaattgag tgatctcaac ggagggactc atcagtacct 8340
   gggtggtata ctttcaatgg gagggatgat cttgtctatc aaaggtcatt atctcagtcg 8400
   aataaaaaaa tttatatgac ctaaataaat aaaaaatatg tttaaaaaaa aaaaaaaaaa 8460
   taaggggtct gaacactata tcatacagta aaatatcaag ttgtgaagca gaagagagtc 8520
   gactaccggt atgggaagag gcagtgagag tttaattctg actatcttag tctggaaagt 8580
   tcgtacagta cttgcacata tgttatgtta tggtggtgct atcacctgag aaacacgcca 8640
   aaaattcggg acctatggtg ccgtgactta gtttgcgcgc aaatatgtac tgtacatggt 8700
   ccgacataaa agagtgttac ggagcagggg tggtggggtt gattttggcc atcagcgtta 8760
   tcgcacagat tgtttgaaaa cacggctcgc attgctcaac aagaacatgg agagagctat 8820
   ttttttaatt ttgataaatg tattggacta gaggccaaga atctgatcaa ttggttatac 8880
   tatatttgtt cacagttgac tgaggccctg tttggataca acggtccctt tctgtttccc 8940
   cgtacgaggt gaccgttgca gaacgggagg gcaatcactt ttatatgaga aacagttcca 9000
   aaacgagacg gaaccgttcc atccaaacaa ggcctgggag aagaggaaat tgtgatcttc 9060
   tcaggtttaa gtttcttgga tgtgtcgctg ctactgaaaa ttttggtata taggtatccg 9120
   ttttatatct gactccctcc acacagaccc cagtggagtg aatacccgga gcactttgct 9180
   catgaaagtc gcccttgggc atcacccctt ccagtcaagg agatctcatg agacgagctc 9240
   ggaatgtgct tattgtcacc atacggacat gaacctccct ggagatttgc ttttacagtc 9300
   tggcgttttt ttgtttcccg agtactttag agtttggaac tagttctatt gctttgacaa 9360
   ggtctagttt attataactt cttgccctcg cgttttttct cttgcacaac taatagacat 9420
   ataagtgttc ttcagataga aggaaatggc gtacgatagc gcaaggattc ctaagaccat 9480
   tgtgagcaac tatgtaaaga cttatgacag gtccccacga atagtgcgat atcatactta 9540
   gcagcagaga aaccatcaag aaggtagact acagtgttga aaatcacgaa gcttccactg 9600
   tttagagtat gaggcgatgg ttgtttggca atgttttaga aacacaatgg tggaacatgc 9660
   taaaggaacg agtcaacgaa agagtcaacg ggtaagtaag agtacgtagg aactagcgtt      9720
   agtgcaagga gaagtgtttg ggatgtttta ggagttagca ctagggatgc cggaagtaac      9780
   gggttacact ccggaggtct agtcgacaac caggtgtcgc actatgggag ataggaaact      9840
   ggagaagatg cacgaaggta tcgggactgt ctggaacagc gatagcctag gagaaacgtt      9900
   tcggaggatg ttcgaaggag ttgtgtggat ggaactgtgt ggcatcatga agaaaagatg      9960
   gtatggaatt ctgcgaggca gatggacgaa gaaaatgttt gcaaaggaac agcgcgtgta     10020
   tatataggac acctccggca gcatagaaca aacaagaatc gcagacactc tatacctatc     10080
   atagccttat tatttgatac agcacatatt cagaggcc                             10118
<210> 37
   <211> 7583
   <212> DNA
   <213> Tuber indicum
<220>
   <221> misc_feature
   <222> (3894)..(3894)
   <223> n is a, c, g, or t
<220>
   <221> misc_feature
   <222> (6700)..(6700)
   <223> n is a, c, g, or t
<400> 37
   ggcttgcatg atatcagcga atgtagctcg gggactaaat ctgatagtta cactaaacgt 60
   tggataccac gctcgttcag agctagctcc ttctccgctc gaggtagagg attaagttca 120
   caggcctctg aatggcatct gatgatgttc cgggcaactt ctctcggggc atctgtgtcg 180
   ctagcaattg tatgataact tttgccttct gcatgaagct caatcattcg tactctctca 240
   tcgcttgcgg ccattctccg cttgcctgtt gcttgacctt gaggtatggt gtatgatggg 300
   aggtgttggc tcccataaag aagtcgtggc taagatacta gtgtcgggtt actaaataac 360
   ctctactgct gagaccgttg cgaaagctgg gtaggttctc tctacatatt ctagtagata 420
   ttgacgctga tttgcgggag gaaaaaaagg aattttatgt tttttttggt tttaaagata 480
   ccgagggcgt ttcattgatc ctagatacat aagtttaatt taagtctacg cgatctataa 540
   tacgctgaga atgcctccca actatgccct taatttccgc cgctcccaca cagtcatact 600
   gtgatacagt aacttcagtt tttttttgta cccactagcc tctttaggcc ttctcgggag 660
   gtcatatcgg ttcacaatcc agtggaggat tctccaatag gaggctgttt agatcatgtt 720
   agaattgggc gctggtggaa tatgttgttc ggggaacttc tcacaaaaac gctgtatcca 780
   ccagaagtga aagtgttcat gtgcatctcg gtctcgggct ggaagtgcgg gtcgagttga 840
   gagtctccat tctcctcagc gctagcgatg tcgaaagcat tgaggatgtt ggcggcttcc 900
   tcctcggtga gatcgagctg atggttgctg gaaacgtaaa cacgggtgat ggtgaatctg 960
   gacaccgcgg atgcgggctt ggcacgagcc ttccttcttg cagcagcctt atcggccgct 1020
   tccttgtcga tgggggcctt ggggacaaag ccagcttcac gcaagagctt ctcaggggta 1080
   gtagggtgac cgtgtttcgc caattcggtc tcgataaatt cttcgagcac gagaccaggg 1140
   tcaccacgat ccctagcagc agtataaaga gaggcaatgt tttgccacat atacttattg 1200
   gtctcagcga cccagacatc acgagtgaga ttggaaattt cggcttgagt gagcccataa 1260
   ccgttacagt acttagaaat ccaggctaat agatggttag aaatagaggc aaaataggga 1320
   tgtgatttac ttacatctct gcgcaacgta gggattcaat gcacgaaggt gctgagaggg 1380
   ggggatgcgg gccaaggcct catcaccatt gacgataaca gccggaatag tggcatcctg 1440
   ggccgtccac ggctgtccag gtttatagta tccgcaatac aaacgtagaa ccgacacaac 1500
   ataagggact tcggggtcga gggggactcg ccctcgatga cggacggttg attgaattgc 1560
   accaagccac ttgtcgtagg gcaagagttc gccgctgaca gtgaaagtga caccaccggg 1620
   gggttgaacg ataatggggg agttctcgtg ttggacccag atgcacccga tcctgtcttg 1680
   catgagaatg tacccacggt ccgaaaggta caaggcgcat cccaaggtag cattacgcat 1740
   aatgaggcgt ctctcgagcc tgttctccca ctccaaaacc ttaggggggg gggagaggtt 1800
   aacgaccatg ctcagattga caggacgaga cgacattatg aaaggcgggg ggggtggaga 1860
   cgggaaaaga aatttgaacc gacaaaagga ggattgcgta tgggcgtttc aatgagtgga 1920
   gattggcctc aatggctgtt gatggaagaa aggagggtgg aaggtctggc ctcttttgta 1980
   tgctacccgt cgaagtggga tgccaaggcc caaaaaaaag agtgacgcca agaagccgtc 2040
   acgtacaact cagccctact agggtctatc ataagatttt tcccagagca ggtagagagc 2100
   ccttaggttt ctgggattcc tcgcggaaag atcggggaac atttttgtat ttacggtcag 2160
   agttgctcga atagaaagac gagtcaaggt atttgatgaa gatctgatcg acattcggga 2220
   gaagtttaaa acaaaggaat tagtgggggt gagtgggaaa tgtgggttct gttaaagtat 2280
   ctgtccccca atcttaacta cccctcttgt acattgcctc tttggtaaag gggtatggta 2340
   tctgaacctt gaacaataat tccttgcgat aatatatatc gatcaggaca gtttgctgac 2400
   tggaaaattt aatatagtct ccataaaacc gtgtgaccga ctcggactgg gaaatcatac 2460
   cttaagtctg gccatccgga agccgctctt tctgctatgg ggtaatatca aatagccaag 2520
   aggccatcag gcgaaatgag aattcagaaa tatgatagcc ctcccctgcc aaccaagaga 2580
   caaatacttc ggcaatacca ccacccgatc tggtggatgt ctccatgttc ggctatccgc 2640
   ttgagtatcc aggggaatag acaaggaaga ctaccacatt tgatgcggtg tgtgcggctg 2700
   ccgagaaaac tgtttgaaag atggaactcc agaacaaagg attaactggt caatgcaaca 2760
   cctggtcgct gcaagggtct gaagattaaa ggattgtaac tagtattgtt ggaggttact 2820
   cctagcaatt tcagaggggt ttttggtaaa gatagactac agtcgcaccc cgctgtcccg 2880
   gacctcgctc tcccggatcc cccgttgtcc gggacgaaat tctctgagct ctgaattttt 2940
   ttgcaccaga acgtaccccg ctgtccagga cagattctgc ggattttggc catccgggac 3000
   agcgaggtgc gactgtatac tgatatcagt aggatggtat ggcactacaa tgaaagaaag 3060
   tggtctggac catgagctca aaaactccaa agagaatttc cggccgggaa attataatat 3120
   ttaaccggtt taaaaccgtg agatcctctg gcccaggacc agcggccgat tgagagagct 3180
   gttgtgagag catcttagca ctcaaccatt agaactccat gataggtagg atgataaaag 3240
   gggaatgggt ttagaatggg tgcacaaagc ttcttgattc tggattaaga tatgactaca 3300
   taagggagtc tgaaacaatg tacactctat ctccgcttgg acttatagga taaaaatgga 3360
   catgttaagt gaattataat gaggaaaaaa gcctgattgg ttgtagggat cgattgtatg 3420
   atcaggggtg ggagtactac aagtttgtct atatagtatt gggaataact gtaaaaatag 3480
   ttccagaata tggctgagta tttcacaaac atgcattagt tgaatatgag gtagatacta 3540
   gagtcacctt gttcagtccc ttggctgtct ttgaatatct ggaaaatata ccaggaaaga 3600
   aagtgggaaa aacaatatct tcaacttctt tggcaaactc tttggaagta gcaaaaccta 3660
   acctctcctt acagcctgag cctgtagctt gatatggtac tcatttactc tttcatccgc 3720
   atagctatgc acttgcaata tctcttccaa ctgtttgttt aaaaaccatg aggtttgaca 3780
   tcaaatgaag gagtaatttc caggtgcaca aaaaatatat tgtgtgaata cctacaattg 3840
   agcccaaatc gatttagtaa attgaaaccc atcaagtagt tggtaatcta tctnatgaag 3900
   ttttttgagc tgaagggaaa agagaaatgt taggcaaatt tttttgtgac ccttagctgg 3960
   tcggctgtca atgcttgata ttgtcatcca aaccaccaca gattgtattg ctgggatgtt 4020
   gacatgttgc agtacgacat ctcctaaggc gatatgcata tcagtgatta ccttatcgta 4080
   ctaagaaaga caagacagtc atttctacta cttgcttttt tattctctta cttcatattc 4140
   taccatgatt tacaggggtc atgctaaatt aaatattgtg tgataccttg tgaaaaatgt 4200
   ccaccaaagc cacagagctg tgaatatcgc aaattgtagg gtgagaggga aatcacaatg 4260
   cataacttta taataattat aaacaccaca actgctccac tggggtgcct tatgctagga 4320
   ctttcctcca tgcgaccctt tttgcatcat aaaacaaata ataacagatt ttacaaaaga 4380
   aaaaataaaa accaatgagg atgccccacc gccacattaa aggagaaagt agttcttaga 4440
   tcttgtctcc ctcaattcac ttagtgcacc tgtggataga aacttaggtg aagatagaga 4500
   aagaggtatc atgagtttta atgtacatta ttcattgcaa tttaattccg ggcacactaa 4560
   gttatttatt tatttattca atccctactg caatccaagc tactcttgtt cttgccaaga 4620
   ctatatcttg gggcggggga acaaactatt ttcattgcac cttttgggcg ctacgagcaa 4680
   aaaattgacc attaaatttt catcaattta aaatcctaaa tacaagtatc atacacatct 4740
   actcaagtac aagaatcctt gtaactattg gaacaaaaca aattctggga ctcttgtcgg 4800
   acactacgtt aacgctagta agtaacaaaa aatcttttaa tattagaagt gtgtgtgaca 4860
   tccacttaga ttttgcgtga catccctgcc gctaccagac cttagtactc gagtagtaga 4920
   ataagaggag tagcgacctt caacgacagc caagccatca aaaagggtat cgtactcgag 4980
   tgttcaaata gaaaatctac cttcctgtga taccaggtga ggtcattgtg gtggttcaat 5040
   acaacaatca agtcctgcga ttacgcataa caagtgtgag tactcgagaa gagcgcgaat 5100
   acacggctgc ctggcccgcg tgggtctaac agatctgtcc ttgctctatt tatttgtttt 5160
   gtttttccta agcgcagcac tttttaccct atgaggtatt tataataata ttatagatat 5220
   tctggaggag gttcccaaaa atatatcgga aatgcaccta ggggcagtgg tactaggatt 5280
   cccaccgcac actatctgct agtggatatg tctaggtttc tagtttccat aaacaccaga 5340
   cgagaaaggc ttaccaaaac gacagagggg gttgggtctg ggagatgtct acggtgcgac 5400
   gatagaacgg ataaaagcgc agggcgggga taaatcgaga ctcgggatgg caactttgat 5460
   gtggatcagt catgcgcaga ggctgctcgg gacggacgag ctctcccacg ccctggcagt 5520
   tgagctgagc tccaccaact tcaacgctag caacatctct tcgatgtcga tactgctggg 5580
   ttgtttctaa gggcttatta cggtggacaa ggaagcatca gccgtgcaac tggcccactt 5640
   tactctccaa gagtaccttt ccgctcatcc cgatattttc tgcagacccc atgcggcgat 5700
   ggcagagatc tgcttgacct atctgaattc ccggcaaatc aaggaccttt cgaccgaccc 5760
   atctcctaac accccgggtg caccctttct agaatattgt tctgtgcact ggggaatcca 5820
   tgcgaaaacg gaactctggg attgcaggag atcgcttgcg ctgaagctat tctaggagtt 5880
   tgatggccac atatccacag aatttctttt agcgcaagtg gcgaatttaa gtcccaaggg 5940
   gcgtgatacc tggcctccgt tcagtgggct gtactgcgca tcgttcttcg gaatcgtgga 6000
   gattatggct gctttaacat acgtgggatg ktgggatacc ggggaaggag gttgtttggg 6060
   gtaaccgccg cttgcacggg ctgttcataa cgggcatggg ggggtggtgg aaatgctatt 6120
   cgggttggaa aaggtccatc cggacgagct ggataaccgc gaccggacac cgctctcaca 6180
   tgttactgcc tgtcggcatg aagaagtggt gaaaatattg ctcctgtttg agcgggaaga 6240
   ggatagccac aaaaagccgg acaatgacgg tcaaacaaca ctctctttcg ctgctcggtt 6300
   tggacatgag gaagtggtgg gaatactact tgggtgggaa gatgttaatc ccaatgagct 6360
   agataatgac ggccaaacac cgctctcgaa tgctgcttgg aggggtcatg agagagttgc 6420
   gaagatatta ctcgggcggg aagaggtcaa cctcgaagag ccagacgagc tcggccgggc 6480
   gccgctcttg catgccgctg gtcatggaca tgagggagta gtgaaaattt ttctcgggcg 6540
   ggaagaggtc aacctcggga agctagacaa ttactcccaa acaccgcttt tgcatgcgtt    6600
   tggtctggat atgaagaggt ggtgagagtg ctactcatac ggggagtgtc gataccggcg    6660
   aaccggacat cagcggccaa ataccgctct cgcatgctgn cgctcatggg cacgaggagg    6720
   tggtagaaat gttactcatg tgggaagagg tcaaacccga caggccagat gatttcggcc    6780
   aaacacttgc tgcttgggat ggacatgggg ggatgatgaa gtactactcg gccgggaaga    6840
   tgtcaaaccg gacaggccag acgatagcgg taaaaccctg ctcttgttgg cctcttggga    6900
   taggaatgag ggagcggtga aaatactact tgggcgcgaa gttgtcggcc ccgacgaacc    6960
   ggataatgac gaccaaatac ccctctcgtt cgcaactggg aatgaccatc agaaggcggt    7020
   ggcccacaaa gcgttaaccc ctagcgcggt ttaaggttta taaaaagcaa cctcttcgaa    7080
   gggatcacct tccttttctt acagagtact ttgctcgtgg gggtcgacct agagcatcac    7140
   cctttccagt cgaaggagat cccatgagac gagtctggaa tgcgcttata tgccaccatg    7200
   cggacgtgaa cctctctggg gatttgcttt tatagtcggg cgtgggtttt ttttctgagt    7260
   acggtagagt ttggaactag ttctattgcc gtgacaaagt ctagtttagt ttattacacc    7320
   ttgcagtcct catcctatca tatttgattt cctccagccg tacattcttg ccctcacgtt    7380
   ttttctctcc cttgcgcaat tgataagcat atagagtgtt gttcatatag aaggaaagtg    7440
   gtgtatgaga ggggaagggt tcttaagacc aatgtgagca gctatgtaag gactgaatga    7500
   taggtcccca cgaatagtgc gatatcatac ttagcagcag agaaaccatc aggaaggtaa    7560
   actacagcac atattcagat gcc                                           7583
<210> 38
   <211> 7742
   <212> DNA
   <213> Tuber indicum
<400> 38
   ggcttgcatg atatcagcga atgtagctcg gggactaaat ctgatagtta cactaaacgt     60
   tggataccac gctcgttcag agctagctcc ttctccgctc gaggtagagg attaagttca    120
   caggcctctg aatggcatct gatgatgttc cgggcaactt ctctcggggc atctgtgtcg    180
   ctagcaattg tatgataact tttgccttct gcatgaagct caatcattcg tactctctca    240
   tcgcttgcgg ccattctccg cttgcctgtt gcttgacctt gaggtatggt gtatgatggg    300
   aggtgttggc tcccataaag aagtcgtggc taagatacta gtgtcgggtt actaaataac    360
   ctctactgct gagaccgttg cgaaagctgg gtaggttctc tctacatatt ctagtagata    420
   ttgacgctga tttgcgggag gaaaaaaagg aattttatgt tttttttggt tttaaagata    480
   ccgagggcgt ttcattgatc ctagatacat aagtttaatt taagtctacg cgatctataa    540
   tacgctgaga atgcctccca actatgccct taatttccgc cgctcccaca cagtcatact    600
   gtgatacagt aacttcagtt tttttttgta cccactagcc tctttaggcc ttctcgggag 660
   gtcatatcgg ttcacaatcc agtggaggat tctccaatag gaggctgttt agatcatgtt 720
   agaattgggc gctggtggaa tatgttgttc ggggaacttc tcacaaaaac gctgtatcca 780
   ccagaagtga aagtgttcat gtgcatctcg gtctcgggct ggaagtgcgg gtcgagttga 840
   gagtctccat tctcctcagc gctagcgatg tcgaaagcat tgaggatgtt ggcggcttcc 900
   tcctcggtga gatcgagctg atggttgctg gaaacgtaaa cacgggtgat ggtgaatctg 960
   gacaccgcgg atgcgggctt ggcacgagcc ttccttcttg cagcagcctt atcggccgct 1020
   tccttgtcga tgggggcctt ggggacaaag ccagcttcac gcaagagctt ctcaggggta 1080
   gtagggtgac cgtgtttcgc caattcggtc tcgataaatt cttcgagcac gagaccaggg 1140
   tcaccacgat ccctagcagc agtataaaga gaggcaatgt tttgccacat atacttattg 1200
   gtctcagcga cccagacatc acgagtgaga ttggaaattt cggcttgagt gagcccataa 1260
   ccgttacagt acttagaaat ccaggctaat agatggttag aaatagaggc aaaataggga 1320
   tgtgatttac ttacatctct gcgcaacgta gggattcaat gcacgaaggt gctgagaggg 1380
   ggggatgcgg gccaaggcct catcaccatt gacgataaca gccggaatag tggcatcctg 1440
   ggccgtccac ggttgtccag gtttatagta tccgcaatac aaacgtagaa ccgacacaac 1500
   ataagggact tcggggtcga gggggactcg ccctcgatga cggacggttg attgaattgc 1560
   accaagccac ttgtcgtagg gcaagagttc gccgctgaca gtgaaagtga caccaccggg 1620
   gggttgaacg atgatggggg agttctcgtg ttggacccag atgcacccga tcctgtcttg 1680
   catgagaatg tacccacggt ccgaaaggta caaggcgcat cccaaggtag cattacgcat 1740
   aatgaggcgt ctctcgagcc tgttctccca ctccaaaacc ttaggggggg gggagaggtt 1800
   aacgaccatg ctcagattga caggacgaga cgacattatg aaaggcgggg ggggtggaga 1860
   cgggaaaaga aatttgaacc gacaaaagga ggattgcgta tgggcgtttc aatgagtgga 1920
   gattggcctc agtggctgtt gatggaagaa aggagggtgg aaggtctggc ctcttttgta 1980
   tgctacccgt cgaagtggga tgccaaggcc caaaaaaaag agtgacgcca agaagccgtc 2040
   acgtacaact cagccctact agggtctatc ataagatttt tcccagagca ggtagagagc 2100
   ccttaggttt ctaagattcc tcgcggaaag atcggggaac atttttgtat ttacggtcag 2160
   agttgctcga atagaaagac gagtcaaggt atttgatgaa gatctgatcg acattcggga 2220
   gaagtttaaa acaaaggaat tagtgggggt gagtgggaaa tgtgggttct gttaaagtat 2280
   ctgtccccca atcttaacta cccctcttgt acattgcctc tttggtaaag gggtatggta 2340
   tctgaacctt gaacaataat tccttgcgat aatatatatc gatcaggaca gtttgctgac 2400
   tggaaaattt aatatagtct ccataaaacc gtgtgaccga ctcggactgg gaaatcatac 2460
   cttaagtctg gccatccgga agccgctctt tctgctatgg ggtaatatca aatagccaag 2520
   aggccatcag gcgaaatgag aattcagaaa tatgatagcc ctcccctgcc aaccaagaga 2580
   caaatacttc ggcaatacca ccacccgatc tggtggatgt ctccatgttc ggctatccgc 2640
   ttgagtatcc aggggaatag acaaggaaga ctaccacatt tgatgcggtg tgtgcggctg 2700
   ccgagaaaac tgtttgaaag atggaactcc agaacaaagg attaactggt caatgcaaca 2760
   cctggtcgct gcaagggtct gaagattaaa ggattgtaac tagtattgtt ggaggttact 2820
   cctagcaatt tcagaggggt ttttggtaaa gatagactac agtcgcaccc cgctgtcccg 2880
   gacctcgctc tcccggatcc cccgttgtcc gggacgaaat tctctgagct ctgaattttt 2940
   ttgcaccaga acgtaccccg ctgtccaggg cagattctgc ggattttggc catccgggac 3000
   agcgaggtgc gactgtatac tgatatcagt aggatggtat ggcactacaa tgaaagaaag 3060
   tggtctggac catgagctca aaaactccaa agagaatttc cggccgggaa attataatat 3120
   ttaaccggtt taaaaccgtg agatcctctg gcccaggacc agcggccgat tgagagagct 3180
   gttgtgagag catcttagca ctcaaccatt agaactccat gataggtagg atgataaaag 3240
   gggaatgggg tttagaatgg gtgcacaaag cttcttgatt ctggattaag atatgactac 3300
   ataagggagt ctgaaacaat gtacactcta tctccgcttg gacttatagg ataaaaatag 3360
   acatgttaag tgaattataa tgaggaaaaa agcctgattg gttgtaggga tcgattgtat 3420
   gatcaggggt gggagtacta caagtttgtc tatatagtat tgggaataac tgtaaaaata 3480
   gtttcagaat atggctgagt atttcacaaa catgcattag ttgaatatga ggtagatact 3540
   agagtcacct tgttcagtcc cttggctgtc tttgaatatc tggaaaatat accaggaaag 3600
   aaagtgggaa aaacaatatc ttcaacttct ttggcaaact gtttggaagt agcaaaacct 3660
   aacctctcct tacagcctga gcctgtagct tgatatggta ctcatttact ctttcataaa 3720
   taaataaaca aataaacccg tcggacgggg ggggggggct ggctggctgg ctggctggac 3780
   tgggctggcg gttgtgtatg tgtgagattt gctattttac cttccacagt accatcatca 3840
   tcatacttgc ttactttttt acttttaact tcatccgcat agctatgcac ttgcaatatc 3900
   tcttccaact gtttgtttaa aaaccatgag gtttgacatc aaatgaagga gtaatttcca 3960
   ggtgcacaaa aaatatattg tgtgaatacc tacaattgag cccaaatcga tttagtaaat 4020
   tgaaacccat caagtagttg gtaatctatc ttatgaagtt ttttgagctg aagggaaaag 4080
   agaaatgtta ggcaaatttt tttgtgaccc ttagctggtc ggctgtcaat gcttgatatt 4140
   gtcatccaaa ccaccacaga ttgtattgct gggatgttga catgttgcag tacgacatct 4200
   cctaaggcga tatgcatatc agagattacc ttatcgtact aagaaagaca agacagtcat 4260
   ttctactact tgctttttta ttctcttact tcatattcta ccatgattta caggggtcat 4320
   gctaaattaa atattgtgtg ataccttgtg aaaaatgtcc accaaagcta tagagctgtg 4380
   aatatcgcaa attgtagggt gagagggaaa tcacaatgca taactttata ataattataa 4440
   acaccacaac tgctccactg gggtgcctta tgctaggact ttcctccatg cgaccctttt 4500
   tgcatcataa aacaaataat aacagatttt acaaaagaaa aaataaaaac caatgaggat 4560
   gccccaccgc cacattaaag gagaaagtag ttcttagatc ttgtctctct caattcactt 4620
   agtgcacctg tggatagaaa cttaggtgaa gatagagaaa gaggtatcat gagttttaat 4680
   gtacattatt cattgcaatt taattccggg cacactaagt tatttattta tttattcaat 4740
   ccctactgca atccaagcta ctcttgttct tgccaagact atatcttggg ggcggggaac 4800
   aaactatttt cattgcacct tttgggcgct acgagcaaaa aattgaccat caaattttcg 4860
   tcaatttaaa atcctaaata caagtatcat acacatctac tcaagtacaa gaatccttgt 4920
   aactattgga acaaaacaaa ttctgggact cttgtcggac actacgttaa cgctagtaag 4980
   taacaaaaaa tctttttaat attagaagtg tgtgtgacat ccacttagat tttgcgtgac 5040
   atccctgccg ctaccagacc ttagtactcg agtagtagaa taagaggagt agcgaccttc 5100
   aacgacagcc aagccatcaa aaagggtatc gtactcgagt gttcaaatag aaaatctacc 5160
   ttcctgtgat accaggtgag gtcattgtgg tggttcaata caacaatcaa gtcctgcgat 5220
   tacgcataac aagtgtgagt actcgagaag agcgcgaata cacggctgcc tggcccgcgt 5280
   gggtctaaca gatctgtcct tgctctattt atttgttttg tttttcctaa gcgcagcact 5340
   ttttacccta tgaggtattt ataataatat tatagatatt ctggaggagg ttcccaaaaa 5400
   tatatcggaa atgcacctag gggcagtggt actaggattc ccaccgcaca ctatctgcta 5460
   gtggatatgt ctaggtttct agtttccata aacaccagac gagaaaggct taccaaaacg 5520
   acagaggggg ttgggtctgg gagatgtcta cggtgcgacg atagaacgga taaaagcgca 5580
   gggcggggat aaatcgagac tcgggatggc aactttgatg tggatcagtc atgcgcagag 5640
   gctgctcggg acggacgagc tctcccacgc cctggcagtt gagctgagct ccaccaactt 5700
   caacgctagc aacatctctt cgatgtcgat actgctgggt tgtttctaag ggcttattac 5760
   ggtggacaag gaagcatcag ccgtgcaact ggcccacttt actctccaag agtacctttc 5820
   cgctcatccc gatattttct gcagacccca tgcggcgatg gtagagatct gcttgaccta 5880
   tctgaattcc cggcaaatca aggacctttc gaccgaccca tctcctgaca ccccgggtgc 5940
   accctttcta gaatattgtt ctgtgcactg gggaatccat gcgaaaacgg aactctggga 6000
   ttgtaggaga tcgcttgcgc tgaagctatt ctaggagttt gatggccaca tatccacaga 6060
   atttctttta gcgcaagtgg cgaatttaag tcccaagggg cgtgatacct ggcctccgtt 6120
   cagtgggctg tactgcgcat cgttcttcgg aatcgtcgag attatggctg ctttaacata 6180
   cgtgggatgt tgggataccg gggaaggagg ttgtttgggg taaccgccgc ttgcacgggc 6240
   tgttcataac gggcatgggg aggtggtgga aatgctattc gggttggaaa aggtccatcc 6300
   ggacgagctg gataaccgcg accggacacc gctctcacat gttactgcct gtcggcatga      6360
   agaagtggtg aaaatattgc tcctgtttga gcgggaagag gatagccaca aaaatccgga      6420
   caatgacggt caaacaacac tctctttcgc tgctcggttt ggacatgagg aagtggtggg      6480
   aatactactt gggtgggaag atgttaatcc caatgagcta gataatgacg gccaaacacc      6540
   gctctcgaat gctgcttgga ggggtcatga gagagttgcg aagatattac tcgggcggga      6600
   agaggtcaac ctcgaagagc cagacgagct cggccgggcg ccgctcttgc atgccgctgg      6660
   tcatggacat gagggagtag tgaaaatttt tctcgggcgg gaagaggtca acctcaggaa      6720
   gctagacaat tactcccaaa caccgctttt gcatgcgttt ggtctggata tgaagaggtg      6780
   gtgagagtgc tactcatacg gggagtgtcg ataccggcga accggacatc agcggccaaa      6840
   taccgctctc gcatgctgcc gctcatgggc acgaggaggt ggtagaaatg ttactcatgt      6900
   gggaagaggt caaacccgac aggccagatg atttcggcca aacacttgct gcttgggatg      6960
   gacatggggg gatgatgaag tactactcgg ccgggaagat gtcaaaccgg acaggccaga      7020
   cgatagcggt aaaaccctgc tcttgttggc ctcttgggat aggaatgagg gagcggtgaa      7080
   aatactactt gggcgcgaag ttgtcggccc cgacgaaccg gataatgacg accaaatacc      7140
   cctctcgttc gcaactggga atggccatca gaaggcggtg gcccacaaag cgttaacccc      7200
   tagcgcggtt taaggtttat aaaaaacaac ctcttcgaag gcatcacctt ccttttctta       7260
   cagagtactt tgctcgtagg ggtcgaccta gagcatcacc ctttccagtc gaaggagatc      7320
   ccatgagacg agtctggaat gcgcttatat gccaccatgc ggacgtgaac ctctctgggg      7380
   atttgctttt atagtcgggc gtgggttttt tttctgagta cggtagagtt tggaactagt      7440
   tctattgccg tgacaaagtc tagtttagtt tattacacct tgcagtcctc atcctatcat      7500
   atttgatttc ctccagccgt acattcttgc cctcacgttt tttctctccc ttgcgcaatt      7560
   gataagcata tagagtgttg ttcatataga aggaaagtgg tgtatgagag gggaagggtt      7620
   cttaagacca atgtgagcag ctatgtaagg actgaatgat aggtccccac gaatagtgcg      7680
   atatcatact tagcagcaga gaaaccatca ggaaggtaaa ctacagcaca tattcagatg      7740
   cc                                                                   7742
<210> 39
   <211> 7667
   <212> DNA
   <213> Tuber indicum
<400> 39
   ggcttgcatg atatcagcga atgtagctcg gggacgtaat ctgataatta cactaaacgt      60
   tggataccac gcaggttcag agctagctcc ttctccgctc gaggtagagg attaagttca     120
   ccggcctctg aatgacatct gatgatgttc cgggcaactt ctctcggggc atctgtgtcg     180
   ctagcaattg tatgataact tttgccttct gcatgaagct caatcattcg tactctctca      240
   tcgcttgcgg ccattcgccg cttgcctgtt gcttgacctt gaggtatggt gtataatggg      300
   aggtgttggc tcccataaag aaatcgtggc taagatacta gtgacgggtt actaaatagc      360
   ctctactgct gagaccgttg cgaaagctga gtaggttctc tctacatatt ctagtagatc      420
   ttgacgctga tttgtggaag gaaaaaaaga aattttatgt tttaagagat tttatgtttt      480
   aagaaatttt atgttttaag aaattttatg ctttaagaaa ttttattttt taagaaattt      540
   tatgctttaa gaaattttat gttttaagaa attttatgtt tttaagaaat ttcatgtttt      600
   aagaaatttt atgttttttt tggttttaaa gatactgagg gcgtttcatt gatcctaggt      660
   acataagttt aatttaagtc tacacgatct ataatacgct gagaatgcct cccagctatg      720
   cccttgattt ccgccgctcc cacacagtca tactgtaata cagtaacttc agtttttttt      780
   tgtacctgct agcctcttta ggctgtctcg ggaagtcata taggttcaca atccagtgga      840
   ggattctcca atagtaggct gtttagatca tgttagaatt gggggctggc ggaatatgct      900
   gttcggggaa cttctcacaa aaacgctgta tccaccagaa gtgaaagtgt tcatgtgcat      960
   ctcgttctcg ggctggaagt gcgggtcgag ttgagagtct ccattctcct cagcgctagt     1020
   gatgtcaaaa gcatggagga tgttggcggc ttcctcctcg gtgagatcga gctgacggtt     1080
   gctggaaacg taaacacggg tgatagtgaa tctggacact gcgggtgcgg gcttggcacg     1140
   agccttcctt cttgcagcag ccttatcggc cgcttccttg tcgatggggg ccttggggac     1200
   aaagccagct tcacgcaaga gcttctcagg ggtagtaggg tgaccatgtt tcgccaattc     1260
   ggtctcgata aattcttcga gcacgagacc agggtcacca cgatccctag cagcagtata     1320
   aagagaggcg atgttttgcc acatatactt attggtctca gcgacccaga cgccacgagt     1380
   gagattggaa atttcggctt gagtgagccc ataaccgtta cagtacttag aaatccaggc     1440
   taatagatgg ttagaaatag aggcaaaata gggatgtgat ttacttacat ctctgagcaa     1500
   cgtagggatt caatgcacga aggtgctgag aggggggaat acgggccaag gcctcatcac     1560
   cattgacgat aacagccgga atagtggcat cctgggcggt ccacggctgt ccaggtctat     1620
   agtatccgca atacaaacgc agaaccgaca caacataagg gacttcgggg tcgaggggga     1680
   ctcgcccttg atgacggacg gttgattgaa ttgcaccgag ccacttgtca tagggcaaga     1740
   gttcgccgct gacagtgaaa gtgacaccac cggggggttg aacgataatg ggggagttct     1800
   catgttggac ccagatgcac ccgatcctgt cttgcatgag gatgtaccca cggtccgaaa     1860
   ggtacaaggc gcatcccaag gtagcattgc gcataatgag gcgtctctcg agcctgttct     1920
   cccactccaa aaccttaggg ggggggagag gttaacgacc atgctcagat tgacaggacg     1980
   agacgacatt atgaaaggcg gggggggggg tggagacggg aaaagaaatt tgaaccgaca     2040
   aaaggaggat tgcgtatggg cgtttcaatg agtggagatt agcctcagtg gctgttgatg 2100
   ttgatggaag aaaggagggt ggaaggtctg gcctcagttg tatgctacct cacgaagtgg 2160
   gatgccaagg cccaaaaaaa agagtgacgc caagaagccg tcatgtacaa ctcagcccta 2220
   ctagggtcta tcatagtaca tacaataata ataataatat tattatgata aataatatta 2280
   ttattatgat atcataatat ttttcccaga gcaggtagag aacccttagg tttctaagat 2340
   tcctggcgga aagattgggg aacatttttg tatttacggt cagagttgct cgaatagaaa 2400
   aacgagtaaa ggtatttgat gaagatctga ttgacattcg ggagaagttt agaacaaagg 2460
   aattagtggg gatgagctgg gaaatgtggg ttctgttaca gtatctgtca cccaatctta 2520
   actacccctc ttgtacattg cctctttggt agggggggat ggtatctgag gcttgaacaa 2580
   taattccttg cgataatata tatcgatcag gacagtttgc tgactggaaa gtctaatata 2640
   tactatatag tcttcataaa accgtgtgac cgactgggaa atcatacctt aagtctggcc 2700
   atccggaagc cgctctttct gctatggggg aatatcaaat agccaagagg ccatcaggtg 2760
   aaatgagaat tcagaaatat gatagccttc ccctgccaac caagagacaa atgtttcggc 2820
   aataccacca cctgatctgg tggatgtctc catgttcggc tatccgcttg agtatccagg 2880
   ggaatagaca aggaagacta ccacatttta tgcagtgtgt gcggctgccg agaaaactgt 2940
   ttgaaagatg gaactccaga acaaaggatt aactggtcaa cgcaacacct ggttgatgca 3000
   agggtctgaa gattaaagga ttgtaactag tattgttgga cgttacttct agcaatttca 3060
   gagtggtttt gggtaaagat agactatact gatattggta ggatggtata gcactacaac 3120
   gaaagaaagt ggtctggaca atgagcacaa aaactctaaa gagaatttcc gggtgggaaa 3180
   ttagaatatt taactagttt aaaaccataa gattctctat cccaagacct gttgaaaatc 3240
   acgaagcttc catggtttgg atgatgaggt gtaggttgtt tggccttggt ttggaaacgc 3300
   aatggcggaa taagctaagg aaagggtcgg cgtgtgggtg agaactagca gttggtgtaa 3360
   ggagaagtgt ttgggatgtt ttaggagtta gcactaggga tactggaagt aacaggttat 3420
   gctccggagg tccagtcgac aaccaggtgt cgcactatag gagataggaa actggagaac 3480
   aaccacaaaa gtatcagggc tgtctggaat agcgatagct taggggaaac gttttggagg 3540
   atgtttgaag gagttgtgtg aaaggaacgg tgtggcaaca tgaagaaaag atggtatgga 3600
   attctgcgag gcagatggat gaagaaaata tttgcaaagg aatagcttct gtatatagag 3660
   gacacctccg gcagcataga acaaacaaga atcgcagaca ctctctacct atcatagcct 3720
   tattatttga agaccagcag ctgattgaga gagctgttgt gagagcatct aagcactcaa 3780
   cctgcagaac tccatgatag gtaggatgat aaaaggggaa tggggtttag aatgggtata 3840
   taaagcctct tgattctgga ttaggatatg gctagataat gttgtttttt gtcacatctc 3900
   ttccaactat ttatttaaaa accacgagat tggacattaa aatttaatga aggagtgatt 3960
   tcctcatgca caaaatatat attgtgtgaa tacctacaat tgagactgca taaacacatg 4020
   agcctaaatc aatttagtaa attaaaatcc atcaagtagt tggtgatcta gcttatgaag 4080
   tttgtgaagt tttttgagct gaagggaaaa gagaaaggtt aggcaaattt ttttgtgacc 4140
   cttagctggt tggctgtcaa tgcttgatat tgtcatccaa accaccacag attgtattgt 4200
   ggggatgttg acatgttgca gtacgacatc tcctaagacc gtatgcatat aagagattac 4260
   tttatcctac taaaagagac aagacagtcg tttctactac ttgcttctgt attctcttac 4320
   ctcatattca accataattt acaagagtca tgccaaatca aatgttgtgt gatacctggt 4380
   gaaaaatgtc cacctaagcc tcagctgtga atatcgcaaa ttgtaaggtg agaggggaat 4440
   cacaatgcat aactttatat tataaacacc acaactgctc cactggggta ccttatacta 4500
   ggactttcct acatgcaact ctttttgcat cataaaacaa ataagaacag attttacaaa 4560
   agaaaaaaaa aaaactagtg aggatgccct accgcgacgt taaaggagaa agtagttctc 4620
   agatcttgtc tccctcagct cgcttagtgt acctgtggac agaaactcat gtgaagatag 4680
   agaaagaggt atcatgaatt ttaatgcaga ttattcattg caatttaatt ccgggcatac 4740
   taatttattt attcatttac tcaatcccta ctgcaatcca agctactctt gtccttgcca 4800
   aggctatctt gagcgccgtg aacaaactta ttttcattgc accttttggg cgctatgagc 4860
   aaaagattga ctatttcatc aatttaaaat cctaaataca aatatcatac acatctactc 4920
   aagtactgta caagaatcct tgtaactatt ggaacaaaat aaattctggg actcttgtcg 4980
   gacactacgt taacgctagt aagtaacaat aaatctttta aaattactag tgtgtgacat 5040
   ccacttagat tttgcgtgac atccctgccg ctactagacc ttagtactcg agtaatagaa 5100
   taataggagt cgcgaccttc aacgacaagg gtatcgtact cgagtattca aatagaaaat 5160
   ctaccttcct atgataccag gtgaggtctt tgctgtggtt caatacgaca atcaagtccc 5220
   gcgattacgc ataacaagtg tgagtactcg agaagagtgc gaatacacgg ctgcctggcc 5280
   tgcgtgggtc taacagatct atccttgctc tatttatttg ttttgttttt cctgagcgca 5340
   gcacttttta ccctatgagg tatttataat aatattatag atattctgga ggaggttccc 5400
   aaaaatatat cggaaatgca cttaggggca atggtactag gattcccacc gcacactatc 5460
   cgctaataga tatatctagg tttctagttt ccataaacac cagatgagaa aggcttacca 5520
   aaacgacaga gggggttggg tctgggggat gcctacggtg cgacgataga acggataaaa 5580
   gcgcagggcg gggataaatc gagactcggg atggcaactt tgatgtggat cagtcatgcg 5640
   cagaggctgc tcgggacgga cgagctctcc cacgccctgg cagttgagct gagctccacc 5700
   gacttcaacg ctagcaacat ctcttcgatg tcgacactgc tgggttactt ctaagggctt 5760
   attacggtgg acaaggaagc atcagccgtg caactggccc aatttactct ccaagagtac 5820
   ttttccgctc atcccgatat tttctgcaga ccccatgggg caatggcaga gatatacttg 5880
   acctatctga attcccggca aatcaaggac ctttcgaccg acccatctcc tgacaccccg 5940
   gatgcaccct ttctagaata ttgttctgtg cactggggaa tccatgcgaa aacggaactc 6000
   tcggattgca ggagatcgct tgcgctgaag ctattctagg agtttgatgg ccacatatcc 6060
   acagaatttc ttttagcgca agtggagaat ttaagtccca aggggcgtga tacctttcct 6120
   ccgttcagtg ggctgtactg cgcatcgttc ttcggaaccg tcgaggttat ggctgcttta 6180
   acatacgtgg gatgttggga tactcggggt ggaacaggtc catcccgacg agctggataa 6240
   ccgcgaccgg acaccgctct cacatgttgc tgcctgtggg catggggggg gtggtgaaaa 6300
   tactgttcct gtttgagcag gaagaggata gccacgaaaa gccggacaat gacggtcaag 6360
   caccactctc tttcgctgct tggtttggac atgaagaagt ggtgagaata ctacttgggc 6420
   gggaagatgt aaatcccaat gagccagata atgacggcca aacaccgctc tcgaatgcta 6480
   cttggagggg tcatgagaga gttgcgaaga tattactcgg gcgggatgag gtcaacctcg 6540
   aagagccaga tgagctcggc cgggcgccgc tcttgcatgc cgctggtcat ggacatgagg 6600
   gagtagtgaa aatttttctc gggcgggaag gggtcaacct cgaaaagcta gacaattact 6660
   cccgaacacc gcttttgcat gcgtttggtc tggatatgaa gaggtggtga gagtgctact 6720
   catacggagg gtgtcgatcc tggcgaaccg gacagcagcg gccaaatacc gctctcgcgt 6780
   gctgccgctc atgggcacga ggaggtggta aaaatgttac tcatgtggga agaggtcaaa 6840
   cccgacacgc cagatgattt cggccaaaca cttgctgctt gggatggaca tggggggata 6900
   atgaagtact actcggccgg gaagatgtca aaccggacag cggtaaaacc ctgctctcgt 6960
   ggcctcttgg gatgggaatg agagagtggt gaatatacta cttgggcgcg aagttatcgg 7020
   ccccgacgaa ccggataatg acgaccaaac acccctctcg ttcgcagctg ggaatgacca 7080
   tcagaaggtg gtggccctgc tacagtctta caaatcgtta actcctagcg cggtttaagg 7140
   tttataaaaa gcagcctctt cgaaggcatc accttccttt tcttacagag tactttgctc 7200
   gtgggggtcg tcctagggca tcaccctttc cagtcgaagg agatcccatg agacgagtcc 7260
   ggaatgcgct tatatgccac catgcggacg tgaacctccc tggggatttg cttttatagt 7320
   cgggtgtggg ttttttttct gaatacggta gaggttggaa ctagttctat tgccgtgaca 7380
   aagcctagtt tagtttatta caccttgcag tcctcatcct atcatatttg atttcctcca 7440
   gccgtacatt cttgccttca cgttttttct ctcccttgcg caattgataa gcatatagag 7500
   tattgttcat atagaaggaa agtggtgtac gagaggggaa gggttcttaa ggccaatgtg 7560
   agcagctatg taagggctga atgatagatc ctcacgaata gtgcgatatc atacttagca 7620
   gcagagaaac catcaagaag gtaaactaca gcacatattc agaggcc 7667
<210> 40
   <211> 1065
   <212> DNA
   <213> Tuber indicum
<400> 40
   atgacttctt ctttgtccaa cctcaatatg gcccttgaag ccgggcctct tattcaccaa      60
   cgttatgatg gaatcggtct cgccgagtat attcccttgc tcgccaacgg aacctttttc     120
   tttgatgggt cggatggagt ctccctccag gtccgccacc tccttccagc agactggaag     180
   cccgtcaatg gaccacttgc ggtcttgttc gattccgtcc cacataccat tattgggact     240
   cacttttcta tccgcaatca ccccatttat aagcttaacg atggttctat cttggtcgtc     300
   tttgacccgg ccgctaagca cggtttcgag tttaccaatc aatctttcgt ggacccccag     360
   cttgaggtct ccggagagaa ccgtttcttg cgtctcgtta ccaatgacca agacttcgcc     420
   taccatggtt ctagtcttag catgattcat gacaagggcg ttaagcgccc tgccaatgcg     480
   taagtacaat tctaccaatg taccagtttc gtgtgctcat ctagtcatag gtttatctta     540
   ttcagaacgg acaaaatgaa gcccctcaag gctcagtacc cggagatggg caacaacgat     600
   atctgtatgt ggctcttatt ttccctttat tttgatctct attcttggta ccatttgcct     660
   aacgttttaa tagccaagat tctcggtcgc atgtggcaga attcctcgga tgaggtcaaa     720
   gatgtttata aggtaaaaca tctcacctaa ctgttagtta gaatcactaa cacattaata     780
   ggcgagggcc catcagcttg cggtttccca taagttgacc aacccggatt acaaatactc     840
   cccgcgccgc tctcgcgaaa tcatgaggag ggactccggg aataaaggtc cttctccccg     900
   tatcgacctt catcgtgctg gtgttgcgaa gcgtgaaaaa aagaggatca ttacccacct     960
   tgggttcctc actcaacgtt tcgcaactca ccccactgct aacaatctcg gtgatatcat    1020
   cgaggaagcc gaaaaggtct tgaaggcctt tggtatggtc aattg                   1065
<210> 41
   <211> 732
   <212> DNA
   <213> Tuber indicum
<400> 41
   atgacttctt ctttgtccaa cctcaatatg gcccttgaag ccgggcctct tattcaccaa 60
   cattatgatg gaatcggtct cgccgagtat attcccttgc tcgccaacgg aacctttttc 120
   tttgatgggt cggatggagt ctccctccag gtccgccacc tccttccagc agactggaag 180
   cccgtcaatg gaccacttgc ggtcttgttc gattccgtcc cacataccat tattgggact 240
   cacttttcta tccgcaatca ccccatttat aagcttaacg atggttctat cttggtcgtc 300
   tttgacccgg ccgctaagca cggtttcgag tttaccaatc aatctttcgt ggacccccag 360
   cttgaggtct ccggagagaa ccgtttcttg cgtctcgtta ccaatgacca agacttcgcc 420
   taccatggtt ctagtcttag catgattcat gacaagggcg ttaagcgccc tgccaatgcg 480
   taagtacaat tctaccaatg taccagtttc gtgtgctcat ctagtcatag gtttatctta 540
   ttcagaacgg acaaaatgaa gcccctcaag gctcagtacc cggagatggg caacaacgat 600
   atctgtatgt ggctcttatt ttccctttat tttgatctct attcttggta ccatttgcct 660
   aacgttttaa tagccaagat tctcggtcgc atgtggcaga attcctcgga tgaggtcaaa 720
   gatgtttata ag 732
<210> 42
   <211> 1067
   <212> DNA
   <213> Tuber indicum
<400> 42
   atgacttctt ctttgtccat tctcaatatg gccgttgaag ccgggcctct tattcaccaa      60
   cgttatgatg gaatcggtct tgccgagtat attcccttgc tcgccaacgg aacctttttc     120
   tttgatgggc cggatggagt ctccctccaa gtccgccatc tccttccagc agactggaag     180
   cccgtcaatg gaccacttac ggtcttgttc gattccgccc cccataccat tattgggact     240
   catttttcta tccgcaatca tcccatttat aagcttaacg atggttctat ctttgtcgtc     300
   ttggacccgg ccaccaagca cggtttcgag tttaccgatc aatctttcgt ggacccccag     360
   cttgaggtct ccggagaaaa ccgtttcttg cgtctcgtta ccaatgacca agacttcgcc     420
   taccatggtt ctagtcttag catgattcat gacaagggcg ttaagcgccc tgccaatgcg     480
   tattgataca attctaccaa tgtaccagtt tcgtgtgctc atctagtcat aggttcattt     540
   tattcagaac ggacaaaatg aagcccctca aggctcagta cccggagatg ggcaacaacg     600
   atatctgtac gtggctctca tttccccctt atttagatct ctattcttgg taccatttgc     660
   ctaacgtttt aatagccaag attctcggtc gcatgtggca gaattcctcg gatgaggtca     720
   aagatgttta taaggtaaaa catctcacct aactgttagt tagaatcact aacacattaa     780
   taggcaaggg cccatcagct tgcggtttcc cataagttga tcaacccgga ttacaaatac     840
   tccccgcgcc gctctcgcga aatcatgagg agggactccg ggaataaagg tccttccccc     900
   cgtatcgacc ttcaccgtgc tggcgttgcg aagcgtggaa aaaagaggat cgttacccac     960
   cttgggttcc tcactcaacg tttcgcgact caccccactg ttaacaatct cggtgatatc    1020
   atcgaggaag ccgaaaaggt cttgaaggcc tttggtatgg tcaattg                  1067
<210> 43
   <211> 1069
   <212> DNA
   <213> Tuber indicum
<400> 43
   tcacaatcca gtggaggatt ctccaatagg aggctgttta gatcatgtta gaattgggcg      60
   ctggtggaat atgttgttcg gggaacttct cacaaaaacg ctgtatccac cagaagtgaa     120
   agtgttcatg tgcatctcgg tctcgggctg gaagtgcggg tcgagttgag agtctccatt     180
   ctcctcagcg ctagcgatgt cgaaagcatt gaggatgttg gcggcttcct cctcggtgag     240
   atcgagctga tggttgctgg aaacgtaaac acgggtgatg gtgaatctgg acaccgcgga     300
   tgcgggcttg gcacgagcct tccttcttgc agcagcctta tcggccgctt ccttgtcgat     360
   gggggccttg gggacaaagc cagcttcacg caagagcttc tcaggggtag tagggtgacc      420
   gtgtttcgcc aattcggtct cgataaattc ttcgagcacg agaccagggt caccacgatc     480
   cctagcagca gtataaagag aggcaatgtt ttgccacata tacttattgg tctcagcgac     540
   ccagacatca cgagtgagat tggaaatttc ggcttgagtg agcccataac cgttacagta     600
   cttagaaatc caggctaata gatggttaga aatagaggca aaatagggat gtgatttact     660
   tacatctctg cgcaacgtag ggattcaatg cacgaaggtg ctgagagggg gggatgcggg     720
   ccaaggcctc atcaccattg acgataacag ccggaatagt ggcatcctgg gccgtccacg     780
   gctgtccagg tttatagtat ccgcaataca aacgtagaac cgacacaaca taagggactt     840
   cggggtcgag ggggactcgc cctcgatgac ggacggttga ttgaattgca ccaagccact      900
   tgtcgtaggg caagagttcg ccgctgacag tgaaagtgac accaccgggg ggttgaacga     960
   taatggggga gttctcgtgt tggacccaga tgcacccgat cctgtcttgc atgagaatgt    1020
   acccacggtc cgaaaggtac aaggcgcatc ccaaggtagc attacgcat                1069
<210> 44
   <211> 1069
   <212> DNA
   <213> Tuber indicum
<400> 44
   tcacaatcca gtggaggatt ctccaatagg aggctgttta gatcatgtta gaattgggcg 60
   ctggtggaat atgttgttcg gggaacttct cacaaaaacg ctgtatccac cagaagtgaa 120
   agtgttcatg tgcatctcgg tctcgggctg gaagtgcggg tcgagttgag agtctccatt 180
   ctcctcagcg ctagcgatgt cgaaagcatt gaggatgttg gcggcttcct cctcggtgag 240
   atcgagctga tggttgctgg aaacgtaaac acgggtgatg gtgaatctgg acaccgcgga 300
   tgcgggcttg gcacgagcct tccttcttgc agcagcctta tcggccgctt ccttgtcgat 360
   gggggccttg gggacaaagc cagcttcacg caagagcttc tcaggggtag tagggtgacc 420
   gtgtttcgcc aattcggtct cgataaattc ttcgagcacg agaccagggt caccacgatc 480
   cctagcagca gtataaagag aggcaatgtt ttgccacata tacttattgg tctcagcgac 540
   ccagacatca cgagtgagat tggaaatttc ggcttgagtg agcccataac cgttacagta 600
   cttagaaatc caggctaata gatggttaga aatagaggca aaatagggat gtgatttact 660
   tacatctctg cgcaacgtag ggattcaatg cacgaaggtg ctgagagggg gggatgcggg 720
   ccaaggcctc atcaccattg acgataacag ccggaatagt ggcatcctgg gccgtccacg 780
   gttgtccagg tttatagtat ccgcaataca aacgtagaac cgacacaaca taagggactt 840
   cggggtcgag ggggactcgc cctcgatgac ggacggttga ttgaattgca ccaagccact 900
   tgtcgtaggg caagagttcg ccgctgacag tgaaagtgac accaccgggg ggttgaacga 960
   tgatggggga gttctcgtgt tggacccaga tgcacccgat cctgtcttgc atgagaatgt 1020
   acccacggtc cgaaaggtac aaggcgcatc ccaaggtagc attacgcat 1069
<210> 45
   <211> 1069
   <212> DNA
   <213> Tuber indicum
<400> 45
   tcacaatcca gtggaggatt ctccaatagt aggctgttta gatcatgtta gaattggggg      60
   ctggcggaat atgctgttcg gggaacttct cacaaaaacg ctgtatccac cagaagtgaa     120
   agtgttcatg tgcatctcgt tctcgggctg gaagtgcggg tcgagttgag agtctccatt     180
   ctcctcagcg ctagtgatgt caaaagcatg gaggatgttg gcggcttcct cctcggtgag     240
   atcgagctga cggttgctgg aaacgtaaac acgggtgata gtgaatctgg acactgcggg     300
   tgcgggcttg gcacgagcct tccttcttgc agcagcctta tcggccgctt ccttgtcgat     360
   gggggccttg gggacaaagc cagcttcacg caagagcttc tcaggggtag tagggtgacc     420
   atgtttcgcc aattcggtct cgataaattc ttcgagcacg agaccagggt caccacgatc     480
   cctagcagca gtataaagag aggcgatgtt ttgccacata tacttattgg tctcagcgac     540
   ccagacgcca cgagtgagat tggaaatttc ggcttgagtg agcccataac cgttacagta     600
   cttagaaatc caggctaata gatggttaga aatagaggca aaatagggat gtgatttact     660
   tacatctctg agcaacgtag ggattcaatg cacgaaggtg ctgagagggg ggaatacggg     720
   ccaaggcctc atcaccattg acgataacag ccggaatagt ggcatcctgg gcggtccacg     780
   gctgtccagg tctatagtat ccgcaataca aacgcagaac cgacacaaca taagggactt     840
   cggggtcgag ggggactcgc ccttgatgac ggacggttga ttgaattgca ccgagccact      900
   tgtcataggg caagagttcg ccgctgacag tgaaagtgac accaccgggg ggttgaacga     960
   taatggggga gttctcatgt tggacccaga tgcacccgat cctgtcttgc atgaggatgt     1020
   acccacggtc cgaaaggtac aaggcgcatc ccaaggtagc attgcgcat                1069
<210> 46
   <211> 13341
   <212> DNA
   <213> Tuber indicum
<400> 46
   cttcacggcg gggcgtgggc tcggagaacg ggtgtttgtc tatcgagtga tgttttgtga 60
   tcttactgat tttagttatc aatacatcca catataccag gagcttctgg gcgattgctt 120
   gcctgaaaac aagtgatccg ggggagcact gtctgtctgt tcgcttcact tgtgctgttc 180
   gaatgcttta cctggttgtg cgctctgagt acgagaaatg ggcgagcaag ctagtctgtc 240
   tgttcacgaa gggcgcaatg aactatcaca aaccaattcc agattcttct actcttttat 300
   ccccttctca cggaggttct ttccagtgag cttcgcccgg tcttgtctcg tctaccattg 360
   catagttatt tacagcggat ggcgtaatcc gcacccttgt ctatgatcgc aatacggcat 420
   tggctctaac aaaactatat cgtgtcgttc cgagggagaa aaaaagtccc ttcattctac 480
   tttcccttcc tccactccga ttcactggca gatgaaaccg taccgcgcgc tcgggctctt 540
   tggtccctat tttctcaacc ggcgagggcg tcgttagtat gggggttgga aggctccttt 600
   ggcttgtttg ccgtgagtag gaagggcgct gtcattgctg agtagccttt cagcgggacc 660
   tgcgggggtg cttctttccg atcggtcttg cgaggggtgg tattggtatt gggcggtgtt 720
   ggccattcgg cggcgttgat gaaatttcta tcaggagttc tgtggcctct ggatacgtgg 780
   tgtagtatat cttcttggcg gtttctctgc tgctgctaag tatgatatcg cactattcgt 840
   ggggacctgt cattaaatct ttacatagtt gctcacaatg gtcttaggaa tcctcgcgct 900
   accgtacacc actttccttc tatctgaaca acgctctata tgtttatgta tcgtacaggg 960
   ggagaaaggt aacgcgtggg caagaaatgc acggctggag aaaataaaat atgataggat 1020
   gaaaactgca aggtataata aactagactt ttttgccaca gcgatagagc tagttccaaa 1080
   ctctacagta ctcagggggc aaacccacgc ccgactataa aagcaaatct ccggaggggc 1140
   tcacatccgc acggtgacat ataagcgcat tccgggctcg tctcatggga tctccttcga 1200
   ctggaagggg tgatgcccaa agacgacttt catgagtaaa gtaccccgga aaagaaggaa 1260
   cggtgatgcc ttcgatgagg tcctgtttta taagccttaa atcgcgctag gggttaacgc 1320
   tttgtgggac tgtagcagcg ctaccacctt ctgatggccg tgcctagatg cgaacgagag 1380
   aggtgtttga ccgtcattgt ttggcctgtc ggggctgaca tcttcccggc cgagtagtac 1440
   cttcaccacc tccgcatgcc caccccaagc agcgagcgag agcggtgttt ggccgcaatc 1500
   atctggcctg tcggggttga cctcttcccg catgagtaaa attttcacca cttcttcaag 1560
   tccatgagcg gcagcatgag agagcggtga ttggccgtgg ctgtctgact tgtcgggatt 1620
   gacctcttcc cgcatgagta gcattctcac cacctcttca tatccggacc aaacggcgtg 1680
   cgagagcggt gttcggcagt agatatctgg cttgtcgggg ttgacctctt cccgcccgag 1740
   aaagattttc actactccct catgtccata accagcggca tgcgagagtg gcgttcgacc 1800
   gagattgtct ggcttgtcgg ggttgacctc ttcccgctcg agtaataatt tcgcaactcc 1860
   ctcatacccc ctccaagcag cattcgagag cggtgtttgg ccgtcattat ctggttcgtt 1920
   gggattaaca tcctcccgcc cgagcagtat tctcaccact tcctcatgtc caaaccaagc 1980
   agcgagcgag agcggtgttt ggccgtcatt gtccggcttg tcggggttga cctcttccct 2040
   cccgagtagt attttcacca cttcctcatg tccgcaggca gtagcatgtg agagcggtgt 2100
   ttggccgcga ttatcgggct cgccgggatc gacctgttcc agcccaagta gcattttcac 2160
   cacttcctca tgtccgttat gagcagccca tgcaagtggc gtataccccc aacaatctcc 2220
   ttcgttgata tcccaacatc ccatctctat taaagcagcc acaacctcga caattccaaa 2280
   gaacgatgcg cagtgcagcc cactgaatgg gggccaggta tcgtgccctc cgggacctaa 2340
   attccccact tgcgctaaaa gaaattcggt ggatatgtgg ccatcaaacc cctggaatag 2400
   cttcagcgca agtgatctcc cgcaatccga gagttccgtt ttcgcatgga ctccccaata 2460
   catagaacaa tattctagaa agggtgcatc cgaggtgtca ggagatggat cggtcgaaag 2520
   gtccttgact tgctgggaat tcagataggt caagcatatc tctgccattg ccgcatgggg 2580
   tctactgaaa atatcgggat gagcggaaag gtactcttgg agagtaaagt ggactagtcg 2640
   cacggctgat gcctccttcc ccaccgtaat aagcccttga cagcaaccta tcagtgtcga 2700
   catcgaaggg atgttgttag cattgaagtc ggtggagccc agctcgactg ccagggcgca 2760
   gcagagctcg tccgtcctga gcggccgctg cgcatgactg atccacatca aggctgccat 2820
   cccgagtctc gatttatccc cgccctgcgc ctttatccgt cctatcgtcg caccgtaggc 2880
   atctcccaga cccaaaccgt cggtcatttt gctaagcctt tctcgtctcc gataggttgt 2940
   cgattcttgt aagattgctt cgatgtttag ggaaactagt ataaacctgg atatatctat 3000
   tagcggatag tgtgcaatgg gaatccctct gtcattgccc tacaaacatt tccgagatct 3060
   tcccagggat ctgctctaga acgtctgcct caagaccctc gtccatggca tctggggttt 3120
   cgtcctcgtc tagcctaaca cgaaggtatc tgataatatc atctcttcta gggcctatag 3180
   acacacttgc cactcgcccg gcaagacgct tttcgatctc ggcccgaaat ataggacctt 3240
   ccagtcagga atattcgtat ccccggggac ttcccaagga tttttttcag cgaatcaagg 3300
   agcttggctc gatgcactgc ggcacattcg tccagggcgt caatgcacat aaatgtgcgc 3360
   tgtgaggacg tgatggtctg tagcatcttt acgatatcag gaagttgcgg tccccgcccg 3420
   ccaatggccc tcctctgctc ttgaaaagcc cgcgatattt cctctggaat cctttccatt 3480
   ccgctaacga tctgtttaag caaggaaccc agcatattcg tcgcggactg ctcctttcgg 3540
   gccgcaaagt caaagtaaaa acaggtaacg gtagtgtttt gtcctctagc ctgatcgcac 3600
   agcctgtcca ctaccatcga actgtcatcg cggcttatta gcactggcgc acttctctcc 3660
   ccagagaata actcttgtta cctgataaat gtcttgccca cacctggatc tccatggcaa 3720
   aacaacactg ccttattgcc ttcaccttcc aaactccagt cataccagct tctgaattcg 3780
   tcagtttgga taagccattc ccccacattg tccacacggt gctcttgaat gtcgcgatgc 3840
   cgtaacctag gttccagcgg cgacaaccaa gctaggagtt gagaccgatc atcggcaact 3900
   gtgaagttgt tccagacatt gctaaaagag ctcgtattat tgaagcagtt gttattgcta 3960
   ttgattgtat attgagacat cttctgctct ttactgcttg cactattgaa gggaagtatt 4020
   tttctggttt ggttggttga cgatatcctt gggaggtcaa gaaaagcggg gtctagatgg 4080
   ggaaagcaag ggtctgctgg tgttaaagca gcatcgcaga taccgctact cgtacatgca 4140
   gtactaactc aagagttttc gtccggtacc ctttttacac caaaagtatc taacttcacc 4200
   accctctctc acagcacaat gtcaagggga atatgccaaa atttcgcggt gtattggcaa 4260
   atctgtagct aatatgggag ataatattat tacagaattg ttgagtaata gagccccata 4320
   ccggtacaaa aatctaggtt aatttcacac aggccaacgt tattgtaaac aatgggcaca 4380
   actttgatcc tctacagttc tacttctaga gatttaaatg aataacagct atctagccga 4440
   gggagggatt tggagtgttt tcaacagggt attgtttgga ttgttaggta tgttgcggta 4500
   gtctgcgaca tgataaacac catacttcgt ggagaatgat ataaagcgct gcgcccggga 4560
   aaagtaagga caaaataaat agacactgtt aaatacgtag aacaaggtct ttggcattcg 4620
   agtgcaattt gaaaacttgc ggtcgatatt cgtgatcgag agattgtggc tgttgcatcg 4680
   aactaccata tgactttcct aaataggaag gcagactctt gattctttta gatggcctgg 4740
   cagttgttga aggttctgaa ctatccctta gagagctgaa tcaacgaaat tcagtgccgc 4800
   caagatattg cgcaactgtg ctgtctaaga catagggtga ataagcacta agcgacgtcg 4860
   tgagaagctg aatctcgtga gaagattaag aaaacaaatc ccaatcgttc agaattctac 4920
   tagaaatgcc acgaacaaga aaatcaagtg cacaaacggc tgctagagga gtggtgtccc 4980
   agtcggttca ccagagagag cctttagctg tcctatcagt gaacggtagg aagccaagcc 5040
   gaatatgttc atagaatgct aaacgtttca cagcaggtgc agaatctcga atgggaccag 5100
   tggggccatc acatctgcat atggatacca taatgcctat gggtgagatg ggaaagggaa 5160
   agggaaaagc aaaggagtcg ggtggggagt cactctctgc ggccgaaaag attcgaaatc 5220
   ttcctcctcc gccacaccac ataccattcc aaggactatt tccaccgcac caagctagag 5280
   ccataggacc tgcaactgga gtaactgacc cgtatcggtt attctcactg tttttttcgc 5340
   cggagcaact ggagattctg gcgaggcata ccaatattta tgcaagtatg catgatgctg 5400
   gcctgcaaga cagtaggcac cctcttgttc ggaagtggta tccaacaact ccctctgagc 5460
   ttcgaatatt gttagcgatc ttgattcatc tgggtatatc aagaggtgct agtccaaaac 5520
   tcttttggag aaaggttgga ggtgttgttc cggaaccgat gtgcagaatg agatatatac 5580
   gctttcagca gctaaagcgc tatttgcata tctcagagcc atctgagtcc ccaattccta 5640
   ctcaaaagtg gtggaaaaag ctcgaacctt taaatagcag tattcgaaaa acctcaaaaa 5700
   gagtgtttcc ttccgtccac caatgtggct gtcgatgaga tgatgatacg cttccttggc 5760
   cgctcagccc acacaattaa aatgccaaat aaacctatta gtttaggata taaggtactg 5820
   gcattgtgtg atgcaggata cacctatgac tgggaactta cttcacgtat tgcgagcttt 5880
   gcaacctccg aacagcaaaa aatgccatat cctctttctc ctacctcctc cgctgtactt 5940
   cagatgctta ccacactacc ctaccgtacc cactttttta ccgcctatat ggacaattac 6000
   ttctcaaata tacgcctatt tgcacgttta ctggattatg gaattggagc ctgtggtaca 6060
   gtgcggtgca gcagcaaaga ttttcctccc tctctcaata tcagcaaaga taaggctgct 6120
   ggtatcttga aatggaattt tgctactgga gttgttgtac gcgaaatagt tgatacagca 6180
   aaaaagataa agggagacag cccacccaag ctacataggt gggggattcc tccggttatg 6240
   gcttttttgt ggcaggataa taacacggtg catttattat ctactgttca tgatcttgag 6300
   ccatcaactt cctggataaa gaaacttcgc agaaggccac gggaaacaag taccaatgct 6360
   gctgctgctc ggaagccctt ttctgctggg gagcatcgca agctattggc aattcccaag 6420
   atcgacaatg actataacca attcatgggt agtgtggata ttgcagatca gcttcgatca 6480
   tatttttcta cgcaacgtat tgtgcgtcgg aactggcaac ctttttttta ctggttactt 6540
   gatactgcaa tcattaatgc ctaccgaatc gcgcgaacca atggctctaa aaccacccac 6600
   cgtacatttc gctcctccct tacaagttcc cttcttactg ccggtcagaa acactcctcc 6660
   cctgagccaa aatttacatt cctctaccga actcgacgac ggtatcgcct acgcaaccca 6720
   gtacgtcaaa tatatattac gaagaactca cagaagccag aaccggtagg tggaggggga 6780
   actttaaggg gccatgtact ggtacggaaa gctactcgag catggtgcct atggtgccgc 6840
   tggcgtcaca agcaaggtgg ctcggaggtg aaaatccacc aagtgcgatc tcagtgtgaa 6900
   cggtgtcatg tagccctttg tcattcatgt tttcccttat atcatggtgt aggaagctct 6960
   ttggtatagg aagctcttcg gtctaggttc tctttttttt ttttttttat atcactttgt 7020
   ctatttagta atccagatcc ctgctctggc ttgctgtcga ttgcgcaata tcttggcggc 7080
   actgaatttc gttgtttcag ctctcgaagg gataggaact attggtactt gtactaaata 7140
   ctaattccta acaacgtaca gggatgtcac gcaaaatcaa atcaaacatt acacactaat 7200
   ggtattaaag aatgttttac ttgctagcgc cgtcatcgtt cctgatcacc tggaatttgt 7260
   cagttccaat agacagacag ctataagggc tcttgtagat gagtgtttga ggatttacat 7320
   tgctatctca gcttcaggga gaacaagaaa ggtatgtcat tttttgcccc cacagcccaa 7380
   tatgtgcagc gaaaatggtt gttttttctc ccgtcaaaat acaatttttg ccgggataag 7440
   gggaccctag gtcgggtggg gttaaatggc ttggggtatc atatctagaa ggaaattgac 7500
   aattagcgcg cattaaactc acagctcttt ggtgagcttt cctcgttgtc tgtctgtagg 7560
   tgcacgtgga gcgatccagg tatctggggg gacctgggaa gcattcccct cttattgtgg 7620
   tggtagggca cctgcagtgg ggaaactcca gcatataaat aaagttataa tataatatta 7680
   ttacgattcc cgcctatctc ataattaaat tctgcgatat tcccagctct ggggtattat 7740
   tggtcgttat ctccacgatg tatcacccga tgtttgattt agcgtgactg ttgtaaatca 7800
   cagtggaatg agagataaga gtatgtgggg acaggtagaa gtgacttgct tgacactctg 7860
   ggtggggtga atacatacat ataactcata gagatgtcat gctaaggtat atagacaact 7920
   ctataacaca acttggatat ggtggataaa gtgtgacggc ctactaactg ggaccacaaa 7980
   ggattcaatt gtgtacctta agaacatgga atacttatgc tgtaatgttt tatgcatgca 8040
   ggaatcaccc attcagtaga tgtcgaattt tacagctttc gaactaacac tccattacca 8100
   aataagtatc gcgggtgcgc atttgtggga agagatgggc attataccat aaaccacagg 8160
   ttctaataag tgtttagata ttggctgctt ccagcgattc catcaggact taagatattt 8220
   cctttctagt acaactacta aatagagaaa gacagcgaag agatggaaca gagaggccct 8280
   agtgcccgcc tcatgtgcaa ttacggtatg tttgtgagag acactccgct atgatctact 8340
   accttcacag atgttcctaa cattatataa acaaacgtgc aatatctcgg tctctgatca 8400
   tacaatcccc tgtcctacat ccaatcaagt tcctttcctc cgtgtaatcc acttgacatc 8460
   ttagcttcta tcccgggagt cggagcggga ccgggggcac attgccccag gctctcttat 8520
   ctgacgatat cttcgtccag aatcaagaga ctttgtctgt cccttctaaa ctccgccccc 8580
   ttccatcatc ctgccttgca tgaagctcag gaggttgagt gcttagatgc tccacaatgg 8640
   tttgctcaat cggccgctgc tcctgggcca gaagctctga cggcttagag ttggttaaag 8700
   attctaattt ccaagctgaa aatccttttg gggcgggctt ttgctcgctc gtgatccaga 8760
   ccttctccgt tcgttatatt actataccgt cctactgatt tcagaatggc ctaacttagc 8820
   caaaaacccc tctgaaacca ctaggagtaa ctgctagcaa tgttagccac aatcccttaa 8880
   tttccagacc cttaccgaga ccggttgtcg cattggatgg ctgctccttt gttctggagt 8940
   tccatctttt aaacagcttt cccgagagct gcatacgccg catcaaacgt cgtagtcctc 9000
   cttgtccatt cacctggatg cacaagcgga tagccgaata tgggacatcc accggatcgg 9060
   ctggtagtgt tgcaaaagca tttggctttc ctggttggcc gggcagcggg tgaagctacc 9120
   atacttctga attcacattt cgcctaacgg cctcctggcc attggatatc cccccaggac 9180
   ggaaaggaca gcttctggat ggtcaggctt aaggtatggt ttcccagtcc gccacacaga 9240
   tttatggaga tcatattaaa ttccgcaatc agaaaaaact atcttgaacc acatacggta 9300
   tctgatcgca aagaattatt gttcagggct cggataccat acctctctat caaagaagca 9360
   atgtacgagt acaagagagg tggggttggg ggacggatgc tgtaacagaa cccacatttc 9420
   ctagctcacc ctgtttcatt aattcctttg ttcggaactt ctcccgaatg tcgatcggat 9480
   cttcatcaat accttgactc gttttctacc atactccagc atctccgacc gtaaaaaaaa 9540
   aatttcgcgg atcttttcgc caggaatctt ggaaacctaa gggctctccc ttcctgccct 9600
   gggggaaatc ttatcgtgtc atatgtgtga taataatatt atgacatttt catgagaata 9660
   ctattctcgg gccctaatag ggttgagttg tacgtgacgg cttcttggcg tcactctatt 9720
   ttttgggtct tggcagtccc cctcgtgact ggaggctaca aaggggatcc atcctttccc 9780
   cttctttcct ccaccgacaa ccgtcaagtt cgacagaaac attcggcctt tctacttgca 9840
   cctctccact cacgtcgttt cctttttttc tgccactatg acttcttctt tgtccaacct 9900
   caatatggcc cttgaagccg ggcctcttat tcaccaacgt tatgatggaa tcggtctcgc 9960
   cgagtatatt cccttgctcg ccaacggaac ctttttcttt gatgggtcgg atggagtctc 10020
   cctccaggtc cgccacctcc ttccagcaga ctggaagccc gtcaatggac cacttgcggt 10080
   cttgttcgat tccgtcccac ataccattat tgggactcac ttttctatcc gcaatcaccc 10140
   catttataag cttaacgatg gttctatctt ggtcgtcttt gacccggccg ctaagcacgg 10200
   tttcgagttt accaatcaat ctttcgtgga cccccagctt gaggtctccg gagagaaccg 10260
   tttcttgcgt ctcgttacca atgaccaaga cttcgcctac catggttcta gtcttagcat 10320
   gattcatgac aagggcgtta agcgccctgc caatgcgtaa gtacaattct accaatgtac 10380
   cagtttcgtg tgctcatcta gtcataggtt tatcttattc agaacggaca aaatgaagcc 10440
   cctcaaggct cagtacccgg agatgggcaa caacgatatc tgtatgtggc tcttattttc 10500
   cctttatttt gatctctatt cttggtacca tttgcctaac gttttaatag ccaagattct 10560
   cggtcgcatg tggcagaatt cctcggatga ggtcaaagat gtttataagg taaaacatct 10620
   cacctaactg ttagttagaa tcactaacac attaataggc gagggcccat cagcttgcgg 10680
   tttcccataa gttgaccaac ccggattaca aatactcccc gcgccgctct cgcgaaatca 10740
   tgaggaggga ctccgggaat aaaggtcctt ctccccgtat cgaccttcat cgtgctggtg 10800
   ttgcgaagcg tgaaaaaaag aggatcatta cccaccttgg gttcctcact caacgtttcg 10860
   caactcaccc cactgctaac aatctcggtg atatcatcga ggaagccgaa aaggtcttga 10920
   aggcctttgg tatggtcaat tgagtgatct caacggaggg actcatcagt gcctgggtga 10980
   tatactttca atgggaggga tgatcttgtc tatcaaaggt cattatctca gtcgaataaa 11040
   tttatatgac ctaaataaat aaaaaatatg tttaaaaaaa aaaaaatgag gggtctgaac 11100
   actatatcat acagtaaaat atcaagttgt gaagcagaag agagtcgact accggtatgg 11160
   gaagaggcag tgagaatcta attctgactg tcttagtctg gaaagttcgt acagcacttg 11220
   cacacaagtt atgctatggt ggtgccatca ccggaaaaac acgccaaaaa ttcgggacct 11280
   atggtgccgt gacttagttt gcgcgcaagt atgtactgta catggtccga cacaaaagag 11340
   tgttatggag caggggtggt ggggttgatt ttggccatca gcgttatcgc acagattgtt 11400
   tgaaaacacg actcgcattg ctcaacaagg acatggagag ctattttttt taattttttt 11460
   ttaattttga tagatgtatt ggactagagg ccaagcatct gatcaattgg ttgtactata 11520
   tttgctcata gttgactgag gccctgtttg gatacaatgg ttcctttctg tttccccgta 11580
   cgaggtgacc gttgcagaac gggagggcaa tcgcttttat atgagaaaca gttccaaaac 11640
   gaaacggaac cgttccatcc aaacaaggcc tgagagaaga ggaaattgtg atcttctcag 11700
   ggttaagttt cttggatgtg tcgctgctac tgaaactttt gttatatagg tatccatttt 11760
   atatctgact ccctccacac agaccccagt ggagcgaatc cccggagcac tttgctcatg 11820
   aaagtcgccc ttgggcatta ctccttccag tcgaaggaga tctcatgaga cgagctcgga 11880
   atgtgcttat atgtcaccag acggacgtga acctccctgg agatttgctt ttacagtcgg 11940
   gcgttttttg tttcccgagt actttagagt ttggaaccag ttctattgct ttgacaaggt 12000
   ctagtttatt ataccttctt gctctcgcgt ttttttctct cgcacaactg atagacatat 12060
   aagtgttctt cagatagaag gaaatggtgt acgatagcgc aaggattcct aaggccattg 12120
   tggacaacta tgtaaggact tatgacaggt ccccacgaat agtgcgatat catacttagc 12180
   agcagagaaa ccatcaagaa ggtagactat agcacatatt cagaggccac ggaactcctg 12240
   agacataagg gtagggataa ccactgcaca cgagaagaac aatcagaata aaacagagcc 12300
   attttataac aaatactaca tgacaaaggg ctaccagaat ctattgatga gcagaggcgc 12360
   atgagagaaa gcataggatc ccgatttcgg tacccgtagt ttaagggggt agaacatgcc 12420
   ctagatctta gtatattttc tagggaactg ctattatatg ataataatat tattatgata 12480
   aatatacttg tcctctagtg tgtgtaacat ccaccctgca agcttaaccg ctttgggtgc 12540
   tcatcatagg ggaggtccac tagttccagg agccagtcag atattttaca atagaatcta 12600
   tagatcggag gtcttaccaa tttggctgcg gttcgggtga gtctgggaca ttctgttaag 12660
   attataccca ctaccgaatc cggacagagt ccagatggcc gcttaaccac tatccgaagg 12720
   gccctaggcc aaagctatca ggagtgtgct ctagcacagt tacacatgct taggcaatca 12780
   aactttgaag ataccgtaaa aacccttgtc gttcgctacc ccaccgggtt gctaatctcc 12840
   agattcagac atagcgctta agcaggaata gtgctttcct gacacggtac agcctaagtc 12900
   ctgtggcatt gttggttctc atgaactagt tttcgcaacc ctgttatcct gtcggatgta 12960
   tgatagcatg tcgtgcgctg atgcgccgac ccacagggat attggagacc tacggagata 13020
   tagaatcgaa aggattttta cagtattggt agatggctgt tagactctct tgccctccct 13080
   ccttttgcta cgcacgatga agaatagaac aatatcggta ggtcagatca aacgacgact 13140
   ttgcttggaa ttgagagata gagaaatagc ttggctcggg cccctgcgcc cgtcagccac 13200
   ccggaccggg tcagggttgg gatttgaaag aagatttccc tgggtcagtc cggcagcact 13260
   ggtatcacag atgtgtcagc ctggggttac tgattcggcg ctcgaacctc ccccaataac 13320
   ccctcccttg cagcatcgcc c 13341
<210> 47
   <211> 10992
   <212> DNA
   <213> Tuber indicum
<400> 47
   gtccttcacg gcggggcgtg ggctcggaga acgggtgttt gtctatcgag tgatgttttg 60
   tgatcttact gattttagtt atcaatacat ccacatatac caggagcttc tgggcgattg 120
   cttgcctgaa aacaagtgat ccgggggagc actgtctgtc tgttcgcttc acttgtgctg 180
   ttcgaatgct ttacctggtt gtgcgctctg agtacgagaa atgggcgagc aagctagtct 240
   gtctgttcac gaagggcgca atgaactatc acaaaccaat tccagattct tctactcttt 300
   tatccccttc tcacggaggt tctttccagt gagcttcgcc cggtcttgtc tcgtctacca 360
   ttgcatagtt atttacagcg gatggcgtaa tccgcaccct tgtctatgat cgcaatacgg 420
   cattggctct aacaaaacta tatcgtgtcg ttccgaggga gaaaaaaagt cccttcattc 480
   tactttccct tcctccactc cgattcactg gcagatgaaa ccgtaccgcg cgctcgggct 540
   ctttggtccc tattttctca accggcgagg gcgtcgttag tatggggatt ggaaggctcc 600
   tttggcttgt ttgccgtgag taggaagggc gctgtcattg ctgagtagcc tttcagcggg 660
   acctgcgggg gtgcttcttt ccgatcggtc ttgcgagggg tggtattggt attgggcggt 720
   gttggccatt cggcggcgtt gatgaaattt ctatcaggag ttctgtggcc tctggatacg 780
   tggtgtagta tatcttcttg gcggtttctc tgctgctgct aagtatgata tcgcactatt 840
   cgtggggacc tgtcattaaa tctttacata gttgctcaca atggtcttag gaatcctcgc 900
   gctaccgtac accactttcc ttctatctga acaacgctct atatgtttat gtatcgtaca 960
   gggggagaaa ggtaacgcgt gggcaagaat gcacggctgg agaaaataaa atatgatagg 1020
   atgaaaactg caaggtataa taaactagac ttttttgcca cagcgataga gctagttcca 1080
   aactctacag tactcagggg gcaaacccac gcccgactat aaaagcaaat ctccggaggg 1140
   gctcacatcc gcacggtgac atataagcgc attccgggct cgtctcatgg gatctccttc 1200
   gactggaagg ggtgatgccc aaagacgact ttcatgagta aagtaccccg gaaaagaagg 1260
   aacggtgatg ccttcgatga ggtcctgttt tataagcctt aaatcgcgct aggggttaac 1320
   gctttgtggg actgtagcag cgctaccacc ttctgatggt cgtgcctaga tgcgaacgag 1380
   agaggtgttt gaccgtcatt gtttggcctg tcggggctga catcttcccg gccgagtagt 1440
   accttcacca cctccgcatg cccaccccaa gcagcgagcg agagcggtgt ttggccgcaa 1500
   tcatctggcc tgtcggggtt gacctcttcc cgcatgagta aaattttcac cacttcttca      1560
   agtccatgag cggcagcatg agagagcggt gattggccgt ggctgtctga cttgtcggga      1620
   ttgacctctt cccgcatgag tagcattctc accacctctt catatccgga ccaaacggcg      1680
   tgcgagagcg gtgttcggca gtagatatct ggcttgtcgg ggttgacctc ttcccgcccg      1740
   agaaagattt tcactactcc ctcatgtcca taaccagcgg catgcgagag tggcgttcga      1800
   ccgagattgt ctggcttgtc ggggttgacc tcttcccgct cgagtaataa tttcgcaact      1860
   ccctcatacc ccctccaagc agcattcgag agcggtgttt ggccgtcatt atctggttcg      1920
   ttgggattaa catcctcccg cccgagcagt attctcacca cttcctcatg tccaaaccaa      1980
   gcagcgagcg agagcggtgt ttggccgtca ttgtccggct tgtcggggtt gacctcttcc      2040
   ctcccgagta gtattttcac cacttcctca tgtccgcagg cagtagcatg tgagagcggt      2100
   gtttggccgc gattatcggg ctcgccggga tcgacctgtt ccagcccaag tagcattttc      2160
   accacttcct catgtccgtt atgagcagcc catgcaagtg gcgtataccc ccaacaatct      2220
   ccttcgttga tatcccaaca tcccatctct attaaagcag ccacaacctc gacaattcca      2280
   aagaacgatg cgcagtgcag cccactgaat gggggccagg tatcgtgccc tccgggacct      2340
   aaattcccca cttgcgctaa aagaaattcg gtggatatgt ggccatcaaa cccctggaat      2400
   agcttcagcg caagtgatct cccgcaatcc gagagttccg ttttcgcatg gactccccaa      2460
   tacatagaac aatattctag aaagggtgca tccgaggtgt caggagatgg atcggtcgaa      2520
   aggtccttga cttgctggga attcagatag gtcaagcata tctctgccat tgccgcatgg      2580
   ggtctactga aaatatcggg atgagcggaa aggtactctt ggagagtaaa gtggactagt      2640
   cgcacggctg atgcctcctt ccccaccgta ataagccctt gacagcaacc tatcagtgtc      2700
   gacatcgaag ggatgttgtt agcattgaag tcggtggagc ccagctcgac tgccagggcg      2760
   cagcagagct cgtccgtcct gagcggccgc tgcgcatgac tgatccacat caaggctgcc      2820
   atcccgagtc tcgatttatc cccgccctgc gcctttatcc gtcctatcgt cgcaccgtag      2880
   gcatctccca gacccaaacc gtcggtcatt ttgctaagcc tttctcgtct ccgataggtt      2940
   gtcgattctt gtaagattgc ttcgatgttt agggaaacta gtataaacct ggatatatct      3000
   attagcggat agtgtgcaat gggaatccct ctgtcattgc cctacaaaca tttccgagat      3060
   cttcccaggg atctgctcta gaacgtctgc ctcaagaccc tcgtccatgg catctggggt      3120
   ttcgtcctcg tctagcctaa cacgaaggta tctgataata tcatctcttc tagggcctat      3180
   agacacactt gccactcgcc cggcaagacg cttttcgatc tcggcccgaa tataggacct      3240
   tccagtcagg aatattcgta tccccgggga cttcccaagg atttttttca gcgaatcaag      3300
   gagcttggct cgatgcactg cggcacattc gtccagggcg tcaatgcaca taaatgtgcg      3360
   ctgtgaggac gtgatggtct gtagcatctt tacgatatca ggaagttgcg gtccccgccc 3420
   gccaatggcc ctcctctgct cttgaaaagc ccgcgatatt tcctctggaa tcctttccat 3480
   tccgctaacg atctgtttaa gcaaggaacc cagcatattc gtcgcggact gctcctttcg 3540
   ggccgcaaag tcaaagtaaa aacaggtaac ggtagtgttt tgtcctctag cctgatcgca 3600
   cagcctgtcc actaccatcg aactgtcatc gcggcttatt agcactggcg cacttctctc 3660
   cccagagaat aactcttgtt acctgataaa tgtcttgccc acacctggat ctccatggca 3720
   aaacaacact gccttattgc cttcaccttc caaactccag tcataccagc ttctgaattc 3780
   gtcagtttgg ataagccatt cccccacatt gtccacacgg tgctcttgaa tgtcgcgatg 3840
   ccgtaaccta ggttccagcg gcgacaacca agctaggagt tgagaccgat catcggcaac 3900
   tgtgaagttg ttccagacat tgctaaaaga gctcgtatta ttgaagcagt tgttattgct 3960
   attgattgta tattgagaca tcttctgctc tttactgctt gcactattga agggaagtat 4020
   ttttctggtt tggttggttg acgatatcct tgggaggtca agaaaagcgg ggtctagatg 4080
   gggaaagcaa gggtctgctg gtgttaaagc agcatcgcag ataccgctac tcgtacatgc 4140
   agtactaact caagagtttt cgtccggtac cctttttaca ccaaaagtat ctaacttcac 4200
   caccctctct cacagcacaa tgtcaagggg aatatgccaa aatttcgcgg tgtattggca 4260
   aatctgtagc taatatggga gataatatta ttacagaatt gttgagtaat agagccccat 4320
   accggtacaa aaatctaggt taatttcaca caggccaacg ttattgtaaa caatgggcac 4380
   aactttgatc ctctacagtt ctacttctag agatttaaat gaataacagc tatctagccg 4440
   agggagggat ttggagtgtt ttcaacaggg tattgtttgg attgttaggt atgttgcggt 4500
   agtctgcgac atgataaaca ccatacttcg tggagaatga tataaagcgc tgcgcccggg 4560
   aaaagtaagg acaaaataaa tagacactgt taaatacgta gaacaaggtc tttggcattc 4620
   gagtgcaatt tgaaaacttg cggtcgatat tcgtgatcga gagattgtgg ctgttgcatc 4680
   gaactaccat atgactttcc taaataggaa ggcagactct tgattctttt agatggcctg 4740
   gcagttgttg aaggttctga actataggaa ctattggtac ttgtactaaa tactaattcc 4800
   taacaacgta cagggatgtc acgcaaaatc aaatcaaaca ttacacacta atggtattaa 4860
   agaatgtttt acttgctagc gccgtcatcg ttcctgatca cctggaattt gtcagttcca 4920
   atagacagac agctataagg gctcttgtag atgagtgttt gaggatttac attgctatct 4980
   cagcttcagg gagaacaaga aaggtatgtc attttttgcc cccacagccc aatatgtgca 5040
   gcgaaaatgg ttgttttttc tcccgtcaaa atacaatttt tgccgggata aggggaccct 5100
   aggtcgggtg gggttaaatg gcttggggta tcatatctag aaggaaattg acaattagcg 5160
   cgcattaaac tcacagctct ttggtgagct ttcctcgttg tctgtctgta ggtgcacgtg 5220
   gagcgatcca ggtatctggg gggacctggg aagcattccc ctcttattgt ggtggtaggg 5280
   cacctgcagt ggggaaactc cagcatataa ataaagttat aatataatat tattacgatt 5340
   cccgcctatc tcataattaa attctgcgat attcccagct ctggggtatt attggtcgtt 5400
   atctccacga tgtatcaccc gatgtttgat ttagcgtgac tgttgtaaat cacagtggaa 5460
   tgagagataa gagtatgtgg ggacaggtag aagtgacttg cttgacactc tgggtggggt 5520
   gaatacatac atataactca tagagatgtc atgctaaggt atatagacaa ctctataaca 5580
   caacttggat atggtggata aagtgtgacg gcctactaac tgggaccaca aaggattcaa 5640
   ttgtgtacct taagaacatg gaatacttat gctgtaatgt tttatgcatg caggaatcac 5700
   ccattcagta gatgtcgaat tttacagctt tcgaactaac actccattac caaataagta 5760
   tcgcgggtgc gcatttgtgg gaagagatgg gcattatacc ataaaccaca ggttctaata 5820
   agtgtttaga tattggctgc ttccagcgat tccatcagga cttaagatat ttcctttcta 5880
   gtacaactac taaatagaga aagacagcga agagatggaa cagagaggcc ctagtgcccg 5940
   cctcatgtgc aattacggta tgtttgtgag agacactccg ctatgatcta ctaccttcac 6000
   agatgttcct aacattatat aaacaaacgt gcaatatctc ggtctctgat catacaatcc 6060
   cctgtcctac atccaatcaa gttcctttcc tccgtgtaat ccacttgaca tcttagcttc 6120
   tatcccggga gtcggagcgg gaccgggggc acattgcccc aggctctctt atctgacgat 6180
   atcttcgtcc agaatcaaga gactttgtct gtcccttcta aactccgccc ccttccatca 6240
   tcctgccttg catgaagctc aggaggttga gtgcttagat gctccacaat ggtttgctca 6300
   atcggccgct gctcctgggc cagaagctct gacggcttag agttggttaa agattctaat 6360
   ttccaagctg aaaatccttt tgggggcggg ctttgctcgc tcgtgatcca gaccttctcc 6420
   gttcgttata ttactatacc gtcctactga tttcagaatg gcctaactta gccaaaaacc 6480
   cctctgaaac cactaggagt aactgctagc aatgttagcc acaatccctt aatttccaga 6540
   cccttaccga gaccggttgt cgcattggat ggctgctcct ttgttctgga gttccatctt 6600
   ttaaacagct ttcccgagag ctgcatacgc cgcatcaaac gtcgtagtcc tccttgtcca 6660
   ttcacctgga tgcacaagcg gatagccgaa tatgggacat ccaccggatc ggctggtagt 6720
   gttgcaaaag catttggctt tcctggttgg ccgggcagcg ggtgaagcta ccatacttct 6780
   gaattcacat ttcgcctaac ggcctcctgg ccattggata tccccccagg acggaaagga 6840
   cagcttctgg atggtcaggc ttaaggtatg gtttcccagt ccgccacaca gatttatgga 6900
   gatcatatta aattccgcaa tcagaaaaaa ctatcttgaa ccacatacgg tatctgatcg 6960
   caaagaatta ttgttcaggg ctcggatacc atacctctct atcaaagaag caatgtacga 7020
   gtacaagaga ggtggggttg ggggacggat gctgtaacag aacccacatt tcctagctca 7080
   ccctgtttca ttaattcctt tgttcggaac ttctcccgaa tgtcgatcgg atcttcatca     7140
   ataccttgac tcgttttcta ccatactcca gcatctccga ccgtaaaaaa aaaaattcgc     7200
   ggaccttttc gccaggaatc ttggaaacct aagggctctc ccttcctgcc ctgggggaaa 7260
   tcttatcgtg tcatatgtgt gataataata ttatgacatt ttcatgagaa tactattctc 7320
   gggccctaat agggttgagt tgtacgtgac ggcttcttgg cgtcactcta ttttttgggt 7380
   cttggcagtc cccctcgtga ctggaggcta caaaggggat ccatcctttc cccttctttc 7440
   ctccaccgac aaccgtcaag ttcgacagaa acattcggcc tttctacttg cacctctcca 7500
   ctcacgtcgt ttcctttttt tctgccacta tgacttcttc tttgtccaac ctcaatatgg 7560
   cccttgaagc cgggcctctt attcaccaac attatgatgg aatcggtctc gccgagtata 7620
   ttcccttgct cgccaacgga acctttttct ttgatgggtc ggatggagtc tccctccagg 7680
   tccgccacct ccttccagca gactggaagc ccgtcaatgg accacttgcg gtcttgttcg 7740
   attccgtccc acataccatt attgggactc acttttctat ccgcaatcac cccatttata 7800
   agcttaacga tggttctatc ttggtcgtct ttgacccggc cgctaagcac ggtttcgagt 7860
   ttaccaatca atctttcgtg gacccccagc ttgaggtctc cggagagaac cgtttcttgc 7920
   gtctcgttac caatgaccaa gacttcgcct accatggttc tagtcttagc atgattcatg 7980
   acaagggcgt taagcgccct gccaatgcgt aagtacaatt ctaccaatgt accagtttcg 8040
   tgtgctcatc tagtcatagg tttatcttat tcagaacgga caaaatgaag cccctcaagg 8100
   ctcagtaccc ggagatgggc aacaacgata tctgtatgtg gctcttattt tccctttatt 8160
   ttgatctcta ttcttggtac catttgccta acgttttaat agccaagatt ctcggtcgca 8220
   tgtggcagaa ttcctcggat gaggtcaaag atgtttataa ggtaaaacat ctcacctaac 8280
   tgttagttag aatcactaac acattaatag gcgagggccc atcagcttgc ggtttcccat 8340
   aagttgacca acccggatta caaatactcc ccgcgccgct ctcgcgaaat catgaggagg 8400
   gactccggga ataaaggtcc ttctccccgt atcgaccttc atcgtgctgg tgttgcgaag 8460
   cgtgaaaaaa agaggatcat tacccacctt gggttcctca ctcaacgttt cgcaactcac 8520
   cccactgcta acaatctcgg tgatatcatc gaggaagccg aaaaggtctt gaaggccttt 8580
   ggtatggtca attgagtgat ctcaacggag ggactcatca gtgcctgggt gatatacttt 8640
   caatgggagg gatgatcttg tctatcaaag gtcattatct cagtcgaata aatttatatg 8700
   acctaaataa ataaaaaata tgtttaaaaa aaaaaaaatg aggggtctga acactatatc 8760
   atacagtaaa atatcaagtt gtgaagcaga agagagtcga ctaccggtat gggaagaggc 8820
   agtgagaatc taattctgac tgtcttagtc tggaaagttc gtacagcact tgcacacaag 8880
   ttatgctatg gtggtgccat caccggaaaa acacgccaaa aattcgggac ctatggtgcc 8940
   gtgacttagt ttgcgcgcaa gtatgtactg tacatggtcc gacacaaaag agtgttatgg 9000
   agcaggggtg gtggggttga ttttggccat cagcgttatc gcacagattg tttgaaaaca 9060
   cgactcgcat tgctcaacaa ggacatggag agctattttt tttaattttt ttttaatttt 9120
   gatagatgta ttggactaga ggccaagcat ctgatcaatt ggttgtacta tatttgctca 9180
   tagttgactg aggccctgtt tggatacaat ggttcctttc tgtttccccg tacgaggtga 9240
   ccgttgcaga acgggagggc aatcgctttt atatgagaaa cagttccaaa acgaaacgga 9300
   accgttccat ccaaacaagg cctgagagaa gaggaaattg tgatcttctc agggttaagt 9360
   ttcttggatg tgtcgctgct actgaaactt ttgttatata ggtatccatt ttatatctga 9420
   ctccctccac acagacccca gtggagcgaa tccccggagc actttgctca tgaaagtcgc 9480
   ccttgggcat tactccttcc agtcgaagga gatctcatga gacgagctcg gaatgtgctt 9540
   atatgtcacc agacggacgt gaacctccct ggagatttgc ttttacagtc gggcgttttt 9600
   tgtttcccga gtactttaga gtttggaacc agttctattg ctttgacaag gtctagttta 9660
   ttataccttc ttgctctcgc gtttttttct ctcgcacaac tgatagacat ataagtgttc 9720
   tttagataga aggaaatggt gtacgatagc gcaaggattc ctaaggccat tgtggacaac 9780
   tatgtaagga cttatgacag gtccccacga atagtgcgat atcatactta gcagcagaga 9840
   aaccatcaag aaggtagact atagcacata ttcagaggcc acggaactcc tgagacataa 9900
   gggtagggat aaccactgca cacgagaaga acaatcagaa taaaacagag ccattttata 9960
   acaaatacta catgacaaag ggctaccaga atctattgat gagcagaggc gcatgagaga 10020
   aagcatagga tcccgatttc ggtacccgta gtttaagggg gtagaacatg ccctagatct 10080
   tagtatattt tctagggaac tgctattata atattatgat aataatatta ttatgataaa 10140
   tatacttgtc ctctagtgtg tgtaacatcc accctgcaag cttaaccgct ttgggtgctc 10200
   atcatagggg aggtccacta gttccaggag ccagtcagat attttacaat agaatctata 10260
   gatcggaggt cttaccaatt tggctgcggt tcgggtgagt ctgggacatt ctgttaagat 10320
   tatacccact accgaatccg gacagagtcc agatggccgc ttaaccacta tccgaagggc 10380
   cctaggccaa agctatcagg agtgtgctct agcacagtta cacatgctta ggcaatcaaa 10440
   ctttgaagat accgtaaaaa cccttgtcgt tcgctacccc accgggttgc taatctccag 10500
   attcagacat agcgcttaag caggaatagt gctttcctga cacggtacag cctaagtcct 10560
   gtggcattgt tggttctcat gaactagttt tcgcaaccct gttatcctgt cggatgtatg 10620
   atagcatgtc gtgcgctgat gcgccgaccc acagggatat tggagaccta cggagatata 10680
   gaatcgaaag gatttttaca gtattggtag atggctgtta gactctcttg ccctccctcc 10740
   ttttgctacg cacgatgaag aatagaacaa tatcggtagg tcagatcaaa cgacgacttt 10800
   gcttggaatt gagagataga gaaatagctt ggctcgggcc cctgcgcccg tcagccaccc 10860
   ggaccgggtc agggttggga tttgaaagaa gatttccctg ggtcagtccg gcagcactgg 10920
   tatcataccc aaagtcacag atgtgtcagc ctggggttac tgattcggcg ctcgaacctc 10980
   ccccaataac cc 10992
<210> 48
   <211> 13263
   <212> DNA
   <213> Tuber indicum
<400> 48
   gggcgtgggg ctcggggaac gggtgttttt ctatcgagtg attttttgtg atcttactgg 60
   ttttggctat caatacatcc acatatacca ggagcttctg ggcgattgct tgcctgcaga 120
   caagtgatcc ggacccatcc agtggtgggt ccggcacggg gggagcactg tctgtctgtt 180
   cgcttcactt gtgctgttcg aatgctttac ctggttgtgc gctctgagta cgagaaatgg 240
   gcgagcaagc tagtctgtct attcacgaag ggcgcaatga actatcacaa accaatcgca 300
   gattcttcta ctcttttgtc cctttctcac ggaggttctt tccagtgagc ttcgcccggt 360
   cttgtctcgt ctaccattgc atagttattt acagcggatg gcgtaatccg cacccttgtc 420
   tatcatcgca atacggcatt ggctctaaca aaactatatc gtgtcgttct gagggagaaa 480
   aaaagtccct tcactctact ttcccttcct ccactccgat tcactggcag atgaaaccgt 540
   accgcgcgct cgggctcttt ggtccctatt ttctcaatgg gcgagggcgt cgttagtatg 600
   ggggttgtta ggctcctttg gcctatttgc cgtgagtagg gagggcgctg tcattgctga 660
   gtagcctttc agcggggcct gcgggggtgc ttctttccga tcggtcttgc gaggggtggt 720
   attggtattg ggcggtgttg gccattcggc ggcgttgatg aaatttctat caggagttct 780
   gtggcctctg gatacatggt gtagtatatc ttattggcgg tttctctgct gctgctaagt 840
   atgatatcgc actattcgtg gggacctgtc attaagtctt tacatagttg ctcacaatgg 900
   tcttaggaat cctcgcgcca tcgtacatca ctttccttct atctgaacaa cgctctatat 960
   gtttatctat cgtacggggg gagaagggtg acgcgtgggc aagaatgcac ggctggagaa 1020
   aataaaatat gataggatga aaacttcaag gtataataaa ctagactttt ttgccacggc 1080
   gatagaacta attccaaact ttacagtact cagggggcaa gcccacgccc gactataaaa 1140
   gcaaatctcc aggggggctc acatccgcac ggtgacatat aagcgcattc cgggctcgtc 1200
   tcatgggatc tccttcgact ggaagggctg atgcccaagg gcgactttca tgagtaaagt 1260
   accccggaaa agaaggaacg gtgatgcctt cgatgaggtc ctgttttata agccttaaat 1320
   cgcgctaggg gttaacgctt tgtgggactg tagcagcgct accaccttct gatggccgtg 1380
   cctagatgcg aacgagagag gtgtttgacc gtcattgttt ggcctgtcgg ggctgacatc 1440
   ttcccggccg agtagcacct tcaccacctc cgcatgccca tcccaagcag cgagcgagag 1500
   cggtgtttgg ccgcaatcat ctggcctgtc ggggttgacc tcttcccgca tgagtaacat 1560
   tttcaccact tcttcaagtc catgagccgc agcatgagag agtggcgttc ggccgagatt 1620
   gtctggcttg tcggggttga cctcttcccg ctcgagtaat aatttcgcaa ctgcctcatg 1680
   ccccctccaa gcagcattcg agagcggtgt ttggccgtca ttatctggct cgttgggatt 1740
   aacatcctcc cgcccgagca gtattctcac cacttcctca tgtccaaacc aagcagcgag 1800
   cgagagcggt gtttggccgt cattgtccgg cttgtcgggg ttgacctctt cccgcccgag 1860
   cagtattttc accacttcct catgtccaca ggcagtagca tgtgagagcg gtgtttggcc 1920
   gcgattatcc ggctcgtcgg gatcgacctg ttccagccca agtagcattt tcaccacttc 1980
   ctcatgtccg ttatgagcag cccatgcaag tggcgtatac ccccaacaat ctccttcgtt 2040
   gatatcccaa catcccatct ctattaaagc agccacaacc tcgacaattc caaagaaaga 2100
   tgcacagtgc agcccactga atggaggcca ggtatcgtgc ccttcgggac ctaaattccc 2160
   cacttgcgct aaaagaaatt cggtggatat atggccatca aactcctgga atagcttcag 2220
   cgcaagagat ctcccgcaat ccgagagttc cgttttcgca tggactcccc aatacataga 2280
   acaatattct agaaagggtg catccgaggt gtcaggagat ggatcggtcg aaaggtcctt 2340
   gacttgccgg gagttcagat aggtcaagca tatctctgcc attgccgcat ggggtctact 2400
   gaaaatatcg ggatgagcgg aaaggtactc ttggagggta aagtggacta gtcgcacggc 2460
   tgatgcctcc ttgcccaccg taataagccc ttgacagcaa cctactagtg tcgatatcga 2520
   agggatgttg ttagcattga agtcggtgga gcccagctcg actgccaggg cgcggcagag 2580
   ctcgtccgtc ctgagcggcc gctgcgcatg actgatccac atcaaggctg ccatcccgag 2640
   tctcgattta tccccgccct gcgcctttat ccgtcctatc gtcgcaccgt aggcatctcc 2700
   cagacccaaa ccgtcggtca ttttgctaag cctgtctcgt ctccgatagg ttgttgattc 2760
   ttgtaagatt gcttcgatgt ttagggaaac tagtataaac ctggatatat ctattagcgg 2820
   atagtgtgca atgggaatcc ctctgtcatt gccctacaaa catttccgag atcttccctg 2880
   ggatctgctc tagaacgtct gcctcaagac cctcgtccat ggcatccggg gtttcgtcct 2940
   cgcccagcct aacacgaagg tatctgataa tatcatctct tctagggctt atagacacac 3000
   ttgccgctcg cccgggaaga cgcttttcga tctcggcccg aatataggac cttccagtca 3060
   ggaatattcg tatccccggg gacttcccaa ggatttgttt cagcgaatca aggagcttgg 3120
   ctcgatgcac tgcggcacat tcgtccaggg cgtcaatgca cataaatgtg cgctgtgagg 3180
   acgtgatggt ctgtagcatc tttacgatat caggaagttg cggtccccgc ccgccgatgg 3240
   ccctcctctg ctcctgaaaa gcccgcgata tttcctctgg aatcctttcc attccgctaa 3300
   cgatctgttt aagcaaggaa cccagcatat tcgtcgcgga ctgctccttt cgggccgcaa 3360
   agtcaaagta aaaacaggta acggtagtgt tttgtcctct ggcctgatcg cacagcctgt 3420
   ccactaccat cgaactgtca tcgcggctta ttagcactgg cgcacttctc tccccagaga 3480
   ataactcttg ttacctgata aatgtcttgc ccacacctgg gtctccatgg caaaacaaca 3540
   ctgccttatt gccttcacct tccaaactcc agtcatacca gcttctgaat tcgtcagttt 3600
   ggataagcca ttcccccaca ttgtccacac ggtgctcttg gatgtcgcga tgccgtaacc 3660
   taggttccag cggcgacaac caagctagga gttgagaccg atcatcggca actgtgaagt 3720
   tgttccagac attgctaaaa gagctcgtat tattgaagca gttgttattg ctattgattg 3780
   tatatcgaga catcttccgc tctttactgc ttgcactatt gaagggaagt atttatctgg 3840
   tttggttggt tgacgatatc ctagggaggt caagaaaagc ggggtctagc tggggaaagc 3900
   aagggtctgc tggtgttaaa gcagcatcgc agataccgct actcgtacat gcagtactaa 3960
   ctcaagagtt ttggtccggt acccttttta caccaaaagt atctaacttc accaccctct 4020
   ctcacagcac aatgccaagg ggaatatgcc aaaatttcgc ggtgtattgg caaatctgta 4080
   gataatatgg gagataatat tattacagaa ttgttgagta ttatagaggg gtggagaaaa 4140
   tgtcatttaa gtgcgaggta ttggttgtca tgtacttttt tgtatagagc cccatacagg 4200
   tacaaaaatc caggttaatt tcacacaggc caacgttatt gtaaacaatg ggcacaactt 4260
   tgatcccttt gatcctctac agttctactt ctagagattt aaataaataa cagctatcta 4320
   gctgagagag ggatttaaag tgttttcaac gggtattgtt gggattgtta ggtatgttgc 4380
   ggtagtctgc gacttgataa acaccatact tcgtggagaa tgatataaag cgctgcgccc 4440
   gggaaaagta aggacgaaat aaatagacac tgttaaatac ctagaacaag gtctctagca 4500
   ttagagtgca atttgaaaac ttgcggtcga taaattattc gtgatcgaga gattgtggct 4560
   gttgcatcga actaccatat gactttccta aataggaagg cagactcttg gtgcctggca 4620
   gttgaaggtt ctgaaatagg aactattggt actcgtacta aatactaatt cctagcaacg 4680
   tacagggatg tcacgcaaaa tcaaatcaaa cataacacac taatggtatt aaagaatgtt 4740
   ttacttgcta gcgccgtcat agttcctttc ccaatcacct ggaatttgtt agttccaata 4800
   gacagctata agggctcttg tagatgagtg tttaaggatt tacattgcta tctcagcttc 4860
   agcgagaaca agaaaaatgt cattttttgc ccccacagcc caatatgtgc agcgaaaatg 4920
   tttgtttttt ctcccgtcaa aatacaattt ttgccaggat aaggggaccc taggtcaggg 4980
   tggggttaaa cggcttgggg tatcatatct agaaggaaat tgtggatccc tggacaatta 5040
   gcgcgcatta aactcacagc tctttggtga gctttcctcg ttgtctttct gtaggtgcac 5100
   gtggagccag ccaggtatct ggggggacct gggaagcatt ccctcttatt gtggtggtag 5160
   ggcctgcaat ggggaaactc cagcatataa aatttaataa agttatatta taatattatt 5220
   attacgactc ccgcctatct cataattaaa ttctgcgata ttcccagctc tggggtatta 5280
   ttggttgtta tctccatgat gtataacccg atgtttgatt tagcgtgact attgtaaatc 5340
   acagtggaat gaaagataag agtaaatggg gacaggtggt agaagtgact tgcttgactt 5400
   tctgggtggg gtggatacat acatatagct catagagatg tcatgctaag gtatatagac 5460
   aactctatga cacaacttgg atatggtgga taaagtgtga cggcctacta actgagacca 5520
   caaaggattc aattgtgtac cttaagaaca tggaatactt atgctgtaat gttttgtgca 5580
   tgcaggaatc accctttcag tagatgtcga attttacagc tttcgaacta gcaatccatt 5640
   accaaataag tatcgcgggt gcgcatttgt gggaagagat gggcattata ccataaacca 5700
   caggttgtaa aaagtgttta gattttggct gcttccagcg attccatcag gactcaagat 5760
   atttcctttc cagtacaact actaaatagc aaaagatagc gaagagatgg aagagagagg 5820
   ccctagtgtc cgcctcatgt gcaattacgg tatgtttgtg agagacactc cgctatgatc 5880
   tactaccttc acagatgttc ctaacattat atatacaaac gtgcaatatc ccgatctctg 5940
   atcacacaat cccctgtcct acatccaatc aagttccttt cctccgtgta atccacttga 6000
   catcttagct tctatcccgg gagtcggagc gggaccgggg gcacattgcc ccaggctctc 6060
   ttatctgaca atattttcgt ccagaatcaa gagactttgt ccgtcccttc taaactccgc     6120
   ccccttcaat catcctgcct tgcatgaagc tcaggaggtt gagtgcttag atgctccaca     6180
   atggtttgct caatcggcag cggctcctga gccagaagct ctgatggctt agagttggtt 6240
   aaagatttta atttcccagc tgaaaatcct tttggggtcg agctttgctc gctcgtgatc 6300
   cagaccttct ccgttcgtta tattactata ccatcctact gatatcagaa tggcctaact 6360
   tagccaaaaa acccctctga gaccactagg agtaactgct agcaatgtta gccacaatcc 6420
   ttaatttcca gacccttacc gagacccgtt gtagcattgg atggctgctc ctttgttctg 6480
   gagttccatc ttttaaacag ctttcccgag agctgcatac gccgcatcaa acgccgtagt 6540
   cctccttgtc cattcacctg gatgcacaag cggatagccg aacatgggac atccgccgga 6600
   tcggctggta gttttgcaaa agaatttggc tttcctggtt ggcagggcag cgggtgaagc 6660
   taccatactt ctgaattcac atttcgccta acggcctcct ggccattgga tatcccccca 6720
   ggacggaaag gacagcctcg ggatggtcag gcttaaggta ccggtatggt ttcccagccc 6780
   gccacacaga tttatggaga tcatattaaa ttcccaatca gaaaaaacta tcttgatcca 6840
   catacggtat ctgatcgcaa agaattattg ttcagggctc agataccata cctctctatc 6900
   aaagaagcaa tgtacgagta caagagaggt ggggttgggg gacgggcgct gtaacagaac 6960
   ccacatttcc tagctcaccc tgtttcatta atttctttgt tcggaacttc tcccgaatgt 7020
   caatcagatc ttcatcaata ccttgactcg ttttctaccg tactccagca tctccgaccg 7080
   taaaaaaaaa ttcgcggatc tttttcaaga taatactatt ctcgggccct aatagggttg 7140
   agttgtacgt gacggcttct tggcgtcact ctattttttg ggtcttggca gtccccctcg     7200
   tgactagagg cctacaaagg agatccatcc tttccccctt ctttcctcca ccgacaaccg     7260
   tcaagttcaa cagaaacatt cggcctttca acttgcacct ctccactcac gtcgtttcct 7320
   tttttctgcc actatgacta tgacttcttc tttgtccatt ctcaatatgg ccgttgaagc 7380
   cgggcctctt attcaccaac gttatgatgg aatcggtctt gccgagtata ttcccttgct 7440
   cgccaacgga acctttttct ttgatgggcc ggatggagtc tccctccaag tccgccatct 7500
   ccttccagca gactggaagc ccgtcaatgg accacttacg gtcttgttcg attccgcccc 7560
   ccataccatt attgggactc atttttctat ccgcaatcat cccatttata agcttaacga 7620
   tggttctatc tttgtcgtct tggacccggc caccaagcac ggtttcgagt ttaccgatca 7680
   atctttcgtg gacccccagc ttgaggtctc cggagaaaac cgtttcttgc gtctcgttac 7740
   caatgaccaa gacttcgcct accatggttc tagtcttagc atgattcatg acaagggcgt 7800
   taagcgccct gccaatgcgt attgatacaa ttctaccaat gtaccagttt cgtgtgctca 7860
   tctagtcata ggttcatttt attcagaacg gacaaaatga agcccctcaa ggctcagtac 7920
   ccggagatgg gcaacaacga tatctgtacg tggctctcat ttccccctta tttagatctc 7980
   tattcttggt accatttgcc taacgtttta atagccaaga ttctcggtcg catgtggcag 8040
   aattcctcgg atgaggtcaa agatgtttat aaggtaaaac atctcaccta actgttagtt 8100
   agaatcacta acacattaat aggcaagggc ccatcagctt gcggtttccc ataagttgat 8160
   caacccggat tacaaatact ccccgcgccg ctctcgcgaa atcatgagga gggactccgg 8220
   gaataaaggt ccttcccccc gtatcgacct tcaccgtgct ggcgttgcga agcgtggaaa 8280
   aaagaggatc gttacccacc ttgggttcct cactcaacgt ttcgcgactc accccactgt 8340
   taacaatctc ggtgatatca tcgaggaagc cgaaaaggtc ttgaaggcct ttggtatggt 8400
   caattgagtg atctcaacgg agggactcat cagtacctgg gtggtatact ttcaatggga 8460
   gggatgatct tgtctatcaa aggtcattat ctcagtcgaa taaaaaaatt tatatgacct 8520
   aaataaataa aaaatatgtt taaaaaaaaa aaaaaaaata aggggtctga acactatatc 8580
   atacagtaaa atatcaagtt gtgaagcaga agagagtcga ctaccggtat gggaagaggc 8640
   agtgagagtt taattctgac tatcttagtc tggaaagttc gtacagtact tgcacatatg 8700
   ttatgttatg gtggtgctat cacctgagaa acacgccaaa aattcgggac ctatggtgcc 8760
   gtgacttagt ttgcgcgcaa atatgtactg tacatggtcc gacataaaag agtgttacgg 8820
   agcaggggtg gtggggttga ttttggccat cagcgttatc gcacagattg tttgaaaaca 8880
   cggctcgcat tgctcaacaa gaacatggag agagctattt ttttaatttt gataaatgta 8940
   ttggactaga ggccaagaat ctgatcaatt ggttatacta tatttgttca cagttgactg 9000
   aggccctgtt tggatacaac ggtccctttc tgtttccccg tacgaggtga ccgttgcaga 9060
   acgggagggc aatcactttt atatgagaaa cagttccaaa acgagacgga accgttccat 9120
   ccaaacaagg cctgggagaa gaggaaattg tgatcttctc aggtttaagt ttcttggatg 9180
   tgtcgctgct actgaaaatt ttggtatata ggtatccgtt ttatatctga ctccctccac 9240
   acagacccca gtggagtgaa tacccggagc actttgctca tgaaagtcgc ccttgggcat 9300
   caccccttcc agtcaaggag atctcatgag acgagctcgg aatgtgctta ttgtcaccat 9360
   acggacatga acctccctgg agatttgctt ttacagtctg gcgttttttt gtttcccgag 9420
   tactttagag tttggaacta gttctattgc tttgacaagg tctagtttat tataacttct 9480
   tgccctcgcg ttttttctct tgcacaacta atagacatat aagtgttctt cagatagaag 9540
   gaaatggcgt acgatagcgc aaggattcct aagaccattg tgagcaacta tgtaaagact 9600
   tatgacaggt ccccacgaat agtgcgatat catacttagc agcagagaaa ccatcaagaa 9660
   ggtagactac agtgttgaaa atcacgaagc ttccactgtt tagagtatga ggcgatggtt 9720
   gtttggcaat gttttagaaa cacaatggtg gaacatgcta aaggaacgag tcaacgaaag 9780
   agtcaacggg taagtaagag tacgtaggaa ctagcgttag tgcaaggaga agtgtttggg 9840
   atgttttagg agttagcact agggatgccg gaagtaacgg gttacactcc ggaggtctag 9900
   tcgacaacca ggtgtcgcac tatgggagat aggaaactgg agaagatgca cgaaggtatc 9960
   gggactgtct ggaacagcga tagcctagga gaaacgtttc ggaggatgtt cgaaggagtt 10020
   gtgtggatgg aactgtgtgg catcatgaag aaaagatggt atggaattct gcgaggcaga 10080
   tggacgaaga aaatgtttgc aaaggaacag cgcgtgtata tataggacac ctccggcagc 10140
   atagaacaaa caagaatcgc agacactcta tacctatcat agccttatta tttgatacag 10200
   cacatattca gaggccacgg aactcctgag acataagggt agggataacc accgcacacc 10260
   agaagaacaa tcagaataaa acagagccat tttataacaa atactacatg acaaagggat 10320
   accagaatct attgatgcgc agaggcccat gacagaaagc ataggatccc gatttcggta 10380
   cccgtagttt aagggtgtag aacatgccct gggtcttagt ctattttcta gggaactgca 10440
   attatactat aataatatta ttatgataaa tatacttgtc ctctggtgta tgtcacatcc 10500
   accctgcaag cttaaccgct ttgggtgctc atcatagggg aggcccacta gttccaagag 10560
   ccagtcggat attatacaat agaatctata gatcggagat cttaccaatt tggctgcggt 10620
   tcgggtaagt ctgggacatt ctgttaagat tatacccact accgaatccg gacagagtcc 10680
   agatggccgc ttagccacta tccgaagggc cctaggtcaa agctatcagg tgttctagca 10740
   cagctgcgca tgctcaggaa atcaaactct gaagataccg taaaaaccct tgccttcgct 10800
   accccaccgg gttgctaatc tccagattca gacatagcgt ttaagcagga atagtgcttt 10860
   cctgacacgg tacagcctaa gtcctctggc attgttggtt ctcatgaact agttttcgca 10920
   accctgttaa cctgtcagac gtatgatagc atgtcgtgcg ctgatgcggc gaccaacagg 10980
   gatattggag acctacggag atatagaatc gaaaggattt ttccagtatt ggtactcgta 11040
   atgtcgtgac gtttgtgaac acgaccgggc ttgcattagt gtacattagt gtgaatacaa 11100
   gggggtaaca gacgagaaat gaacttaatg tagactagtt agtaaatagg ggtacttcag 11160
   tgaatagggg tacttgagta tatgaagttc gtgatcaata gtagcaatac agtgaatcca 11220
   atatccagca aaaatgccaa ctccacgagg tcctacaact tctttgagca agcgaggtca 11280
   gattttgggt tatgcaattc tcgatgggcg acagaaaatg acattgaggg agatatcgaa 11340
   aaagacagga atacttgaaa gtacttgttc gaatattatc cgaactgcac gcgagcgagc 11400
   gagtgtgaat ggcatccaag atctctgcgt aactgagaat ctaaagccat tgcctaccgc 11460
   actcaaagga tcaaatcagg ctcttaccga ggaagaaaag ggtcatcttg ttgaaacagc 11520
   actgaaggat gctgagcact gtcgaatgac ctttacgcag cttgctgaag ctggtatgta 11580
   ttttatatta aaattcccta taatacataa atgtaactaa aaaaaaaaaa aaaaaaagcc 11640
   aatttagata tcagtcggca gactgtatct aagatactag ctgaaaataa aatccatcgc 11700
   cgaaagccaa caacaaaacc ctcactcaat gcagctcaac aggcagcccg gcttgccttt 11760
   tgctatgagc atcggaacta cgattggcac tctgtaattt ttacagacga gtcttatttt 11820
   gaaactggca accttcgcca gcgccgtgcc cgtggtgttt tacgccgggc tggtgaggcc 11880
   taccggcctc aaaacattca gcgaaagttt gctcaaggag caacggtcat gttttggggg 11940
   gctattttgt atggtaaatc aggtataatt tatttattat tattattgtt attgtttatt 12000
   actatttctc tttctcttac tatttttatt atttttaatg tacatatagg ctaatataat 12060
   atattaaaaa aggaaatgaa ctaccatatc atttatatgc ttcaccatat gaaacagcgg 12120
   gacagcggag agagtcatca attatcctac agcgagaata cgagttcgag ttagcagaaa 12180
   atactcgcgc tactgtactt ggacatgtag tcccatttcc aacacctgaa ttaaaggtgc 12240
   ggaaggtaga gagaaaggga gggatagatt ggtatatcta tcgtgaaaga attttaaatc 12300
   cccttcttta ccctttcacc cagcaagcaa tacaggactt tcctgatcgg aatattaaaa 12360
   taatggagga taatgcgcct gcgcatatcc accattatca taatattccg cgagagcgtt 12420
   tggggctacg gaagctagtc tggccggcca actcccccga cttgaatccc atagagacta 12480
   tatggactga gcttaaagat aagctgatgg atcaaattgg tccacggatg actgctcgtg 12540
   atatccgcca tgtacttgaa caagtaagct ttaccttccc ttttattctt ttactactaa 12600
   caatttacat aggagtggag aaactatcca cgagaacgtg tgaaccacca tatagaatct 12660
   atgtcttcac gaatggaagc ctgtattgca gataatggtg gaaacaactt taatttttaa 12720
   tattaaattt tgtatttctt tctttctctt ttgattccat gagtgctgta ttcaccatat 12780
   ataattcgta atatattatc atacctttat atccccctcc ctagtttcgc caagcataag 12840
   cctcatatgc acgctaatgc aagcccggtc gtgttcacaa acgtcacgac attacgagta 12900
   gatggctgtt agactccctt gcctccctcc ttttgctacg cacgatgaag aatagaacaa    12960
   tatcggtagg ttatcataga tcaaacgacg actttgcttg gaattgagaa atagagaaat    13020
   agcatggctc gggcccctgc gcccgtcagc cacccagacc ggaccagggt tgggatttga    13080
   aaggagattt ccctgggtca gtccggcagc attggtatca tacccaaagt cccagctgtg    13140
   tcagcctggg gttactgatt cggcgctcga atctccccca ataacccctc ccttgcagca    13200
   tctcgcccaa agctagtagc atagggccaa aatgctatgt atcataataa tattattatg    13260
   ata                                                                  13263
<210> 49
   <211> 8730
   <212> DNA
   <213> Tuber indicum
<220>
   <221> misc_feature
   <222> (3897)..(3897)
   <223> n is a, c, g, or t
<220>
   <221> misc_feature
   <222> (6703)..(6703)
   <223> n is a, c, g, or t
<400> 49
   ttcggcttgc atgatatcag cgaatgtagc tcggggacta aatctgatag ttacactaaa     60
   cgttggatac cacgctcgtt cagagctagc tccttctccg ctcgaggtag aggattaagt    120
   tcacaggcct ctgaatggca tctgatgatg ttccgggcaa cttctctcgg ggcatctgtg    180
   tcgctagcaa ttgtatgata acttttgcct tctgcatgaa gctcaatcat tcgtactctc    240
   tcatcgcttg cggccattct ccgcttgcct gttgcttgac cttgaggtat ggtgtatgat    300
   gggaggtgtt ggctcccata aagaagtcgt ggctaagata ctagtgtcgg gttactaaat    360
   aacctctact gctgagaccg ttgcgaaagc tgggtaggtt ctctctacat attctagtag    420
   atattgacgc tgatttgcgg gaggaaaaaa aggaatttta tgtttttttt ggttttaaag    480
   ataccgaggg cgtttcattg atcctagata cataagttta atttaagtct acgcgatcta    540
   taatacgctg agaatgcctc ccaactatgc ccttaatttc cgccgctccc acacagtcat    600
   actgtgatac agtaacttca gttttttttt gtacccacta gcctctttag gccttctcgg    660
   gaggtcatat cggttcacaa tccagtggag gattctccaa taggaggctg tttagatcat    720
   gttagaattg ggcgctggtg gaatatgttg ttcggggaac ttctcacaaa aacgctgtat    780
   ccaccagaag tgaaagtgtt catgtgcatc tcggtctcgg gctggaagtg cgggtcgagt    840
   tgagagtctc cattctcctc agcgctagcg atgtcgaaag cattgaggat gttggcggct    900
   tcctcctcgg tgagatcgag ctgatggttg ctggaaacgt aaacacgggt gatggtgaat    960
   ctggacaccg cggatgcggg cttggcacga gccttccttc ttgcagcagc cttatcggcc 1020
   gcttccttgt cgatgggggc cttggggaca aagccagctt cacgcaagag cttctcaggg 1080
   gtagtagggt gaccgtgttt cgccaattcg gtctcgataa attcttcgag cacgagacca 1140
   gggtcaccac gatccctagc agcagtataa agagaggcaa tgttttgcca catatactta 1200
   ttggtctcag cgacccagac atcacgagtg agattggaaa tttcggcttg agtgagccca 1260
   taaccgttac agtacttaga aatccaggct aatagatggt tagaaataga ggcaaaatag 1320
   ggatgtgatt tacttacatc tctgcgcaac gtagggattc aatgcacgaa ggtgctgaga 1380
   gggggggatg cgggccaagg cctcatcacc attgacgata acagccggaa tagtggcatc 1440
   ctgggccgtc cacggctgtc caggtttata gtatccgcaa tacaaacgta gaaccgacac 1500
   aacataaggg acttcggggt cgagggggac tcgccctcga tgacggacgg ttgattgaat 1560
   tgcaccaagc cacttgtcgt agggcaagag ttcgccgctg acagtgaaag tgacaccacc 1620
   ggggggttga acgataatgg gggagttctc gtgttggacc cagatgcacc cgatcctgtc 1680
   ttgcatgaga atgtacccac ggtccgaaag gtacaaggcg catcccaagg tagcattacg 1740
   cataatgagg cgtctctcga gcctgttctc ccactccaaa accttagggg ggggggagag 1800
   gttaacgacc atgctcagat tgacaggacg agacgacatt atgaaaggcg gggggggtgg 1860
   agacgggaaa agaaatttga accgacaaaa ggaggattgc gtatgggcgt ttcaatgagt 1920
   ggagattggc ctcaatggct gttgatggaa gaaaggaggg tggaaggtct ggcctctttt 1980
   gtatgctacc cgtcgaagtg ggatgccaag gcccaaaaaa aagagtgacg ccaagaagcc 2040
   gtcacgtaca actcagccct actagggtct atcataagat ttttcccaga gcaggtagag 2100
   agcccttagg tttctgggat tcctcgcgga aagatcgggg aacatttttg tatttacggt 2160
   cagagttgct cgaatagaaa gacgagtcaa ggtatttgat gaagatctga tcgacattcg 2220
   ggagaagttt aaaacaaagg aattagtggg ggtgagtggg aaatgtgggt tctgttaaag 2280
   tatctgtccc ccaatcttaa ctacccctct tgtacattgc ctctttggta aaggggtatg 2340
   gtatctgaac cttgaacaat aattccttgc gataatatat atcgatcagg acagtttgct 2400
   gactggaaaa tttaatatag tctccataaa accgtgtgac cgactcggac tgggaaatca 2460
   taccttaagt ctggccatcc ggaagccgct ctttctgcta tggggtaata tcaaatagcc 2520
   aagaggccat caggcgaaat gagaattcag aaatatgata gccctcccct gccaaccaag 2580
   agacaaatac ttcggcaata ccaccacccg atctggtgga tgtctccatg ttcggctatc 2640
   cgcttgagta tccaggggaa tagacaagga agactaccac atttgatgcg gtgtgtgcgg 2700
   ctgccgagaa aactgtttga aagatggaac tccagaacaa aggattaact ggtcaatgca 2760
   acacctggtc gctgcaaggg tctgaagatt aaaggattgt aactagtatt gttggaggtt 2820
   actcctagca atttcagagg ggtttttggt aaagatagac tacagtcgca ccccgctgtc 2880
   ccggacctcg ctctcccgga tcccccgttg tccgggacga aattctctga gctctgaatt 2940
   tttttgcacc agaacgtacc ccgctgtcca ggacagattc tgcggatttt ggccatccgg 3000
   gacagcgagg tgcgactgta tactgatatc agtaggatgg tatggcacta caatgaaaga 3060
   aagtggtctg gaccatgagc tcaaaaactc caaagagaat ttccggccgg gaaattataa 3120
   tatttaaccg gtttaaaacc gtgagatcct ctggcccagg accagcggcc gattgagaga 3180
   gctgttgtga gagcatctta gcactcaacc attagaactc catgataggt aggatgataa 3240
   aaggggaatg ggtttagaat gggtgcacaa agcttcttga ttctggatta agatatgact 3300
   acataaggga gtctgaaaca atgtacactc tatctccgct tggacttata ggataaaaat 3360
   ggacatgtta agtgaattat aatgaggaaa aaagcctgat tggttgtagg gatcgattgt 3420
   atgatcaggg gtgggagtac tacaagtttg tctatatagt attgggaata actgtaaaaa 3480
   tagttccaga atatggctga gtatttcaca aacatgcatt agttgaatat gaggtagata 3540
   ctagagtcac cttgttcagt cccttggctg tctttgaata tctggaaaat ataccaggaa 3600
   agaaagtggg aaaaacaata tcttcaactt ctttggcaaa ctctttggaa gtagcaaaac 3660
   ctaacctctc cttacagcct gagcctgtag cttgatatgg tactcattta ctctttcatc 3720
   cgcatagcta tgcacttgca atatctcttc caactgtttg tttaaaaacc atgaggtttg 3780
   acatcaaatg aaggagtaat ttccaggtgc acaaaaaata tattgtgtga atacctacaa 3840
   ttgagcccaa atcgatttag taaattgaaa cccatcaagt agttggtaat ctatctnatg 3900
   aagttttttg agctgaaggg aaaagagaaa tgttaggcaa atttttttgt gacccttagc 3960
   tggtcggctg tcaatgcttg atattgtcat ccaaaccacc acagattgta ttgctgggat 4020
   gttgacatgt tgcagtacga catctcctaa ggcgatatgc atatcagtga ttaccttatc 4080
   gtactaagaa agacaagaca gtcatttcta ctacttgctt ttttattctc ttacttcata 4140
   ttctaccatg atttacaggg gtcatgctaa attaaatatt gtgtgatacc ttgtgaaaaa 4200
   tgtccaccaa agccacagag ctgtgaatat cgcaaattgt agggtgagag ggaaatcaca 4260
   atgcataact ttataataat tataaacacc acaactgctc cactggggtg ccttatgcta 4320
   ggactttcct ccatgcgacc ctttttgcat cataaaacaa ataataacag attttacaaa 4380
   agaaaaaata aaaaccaatg aggatgcccc accgccacat taaaggagaa agtagttctt 4440
   agatcttgtc tccctcaatt cacttagtgc acctgtggat agaaacttag gtgaagatag 4500
   agaaagaggt atcatgagtt ttaatgtaca ttattcattg caatttaatt ccgggcacac 4560
   taagttattt atttatttat tcaatcccta ctgcaatcca agctactctt gttcttgcca 4620
   agactatatc ttgggggcgg ggaacaaact attttcattg caccttttgg gcgctacgag 4680
   caaaaaattg accattaaat tttcatcaat ttaaaatcct aaatacaagt atcatacaca 4740
   tctactcaag tacaagaatc cttgtaacta ttggaacaaa acaaattctg ggactcttgt 4800
   cggacactac gttaacgcta gtaagtaaca aaaaatcttt taatattaga agtgtgtgtg 4860
   acatccactt agattttgcg tgacatccct gccgctacca gaccttagta ctcgagtagt 4920
   agaataagag gagtagcgac cttcaacgac agccaagcca tcaaaaaggg tatcgtactc 4980
   gagtgttcaa atagaaaatc taccttcctg tgataccagg tgaggtcatt gtggtggttc 5040
   aatacaacaa tcaagtcctg cgattacgca taacaagtgt gagtactcga gaagagcgcg 5100
   aatacacggc tgcctggccc gcgtgggtct aacagatctg tccttgctct atttatttgt 5160
   tttgtttttc ctaagcgcag cactttttac cctatgaggt atttataata atattataga 5220
   tattctggag gaggttccca aaaatatatc ggaaatgcac ctaggggcag tggtactagg 5280
   attcccaccg cacactatct gctagtggat atgtctaggt ttctagtttc cataaacacc 5340
   agacgagaaa ggcttaccaa aacgacagag ggggttgggt ctgggagatg tctacggtgc 5400
   gacgatagaa cggataaaag cgcagggcgg ggataaatcg agactcggga tggcaacttt 5460
   gatgtggatc agtcatgcgc agaggctgct cgggacggac gagctctccc acgccctggc 5520
   agttgagctg agctccacca acttcaacgc tagcaacatc tcttcgatgt cgatactgct 5580
   gggttgtttc taagggctta ttacggtgga caaggaagca tcagccgtgc aactggccca 5640
   ctttactctc caagagtacc tttccgctca tcccgatatt ttctgcagac cccatgcggc 5700
   gatggcagag atctgcttga cctatctgaa ttcccggcaa atcaaggacc tttcgaccga 5760
   cccatctcct aacaccccgg gtgcaccctt tctagaatat tgttctgtgc actggggaat 5820
   ccatgcgaaa acggaactct gggattgcag gagatcgctt gcgctgaagc tattctagga 5880
   gtttgatggc cacatatcca cagaatttct tttagcgcaa gtggcgaatt taagtcccaa 5940
   ggggcgtgat acctggcctc cgttcagtgg gctgtactgc gcatcgttct tcggaatcgt 6000
   ggagattatg gctgctttaa catacgtggg atgktgggat accggggaag gaggttgttt 6060
   ggggtaaccg ccgcttgcac gggctgttca taacgggcat gggggggtgg tggaaatgct 6120
   attcgggttg gaaaaggtcc atccggacga gctggataac cgcgaccgga caccgctctc 6180
   acatgttact gcctgtcggc atgaagaagt ggtgaaaata ttgctcctgt ttgagcggga 6240
   agaggatagc cacaaaaagc cggacaatga cggtcaaaca acactctctt tcgctgctcg 6300
   gtttggacat gaggaagtgg tgggaatact acttgggtgg gaagatgtta atcccaatga 6360
   gctagataat gacggccaaa caccgctctc gaatgctgct tggaggggtc atgagagagt 6420
   tgcgaagata ttactcgggc gggaagaggt caacctcgaa gagccagacg agctcggccg 6480
   ggcgccgctc ttgcatgccg ctggtcatgg acatgaggga gtagtgaaaa tttttctcgg 6540
   gcgggaagag gtcaacctcg ggaagctaga caattactcc caaacaccgc ttttgcatgc 6600
   gtttggtctg gatatgaaga ggtggtgaga gtgctactca tacggggagt gtcgataccg 6660
   gcgaaccgga catcagcggc caaataccgc tctcgcatgc tgncgctcat gggcacgagg 6720
   aggtggtaga aatgttactc atgtgggaag aggtcaaacc cgacaggcca gatgatttcg 6780
   gccaaacact tgctgcttgg gatggacatg gggggatgat gaagtactac tcggccggga 6840
   agatgtcaaa ccggacaggc cagacgatag cggtaaaacc ctgctcttgt tggcctcttg 6900
   ggataggaat gagggagcgg tgaaaatact acttgggcgc gaagttgtcg gccccgacga 6960
   accggataat gacgaccaaa tacccctctc gttcgcaact gggaatgacc atcagaaggc 7020
   ggtggcccac aaagcgttaa cccctagcgc ggtttaaggt ttataaaaag caacctcttc 7080
   gaagggatca ccttcctttt cttacagagt actttgctcg tgggggtcga cctagagcat 7140
   caccctttcc agtcgaagga gatcccatga gacgagtctg gaatgcgctt atatgccacc 7200
   atgcggacgt gaacctctct ggggatttgc ttttatagtc gggcgtgggt tttttttctg 7260
   agtacggtag agtttggaac tagttctatt gccgtgacaa agtctagttt agtttattac 7320
   accttgcagt cctcatccta tcatatttga tttcctccag ccgtacattc ttgccctcac 7380
   gttttttctc tcccttgcgc aattgataag catatagagt gttgttcata tagaaggaaa 7440
   gtggtgtatg agaggggaag ggttcttaag accaatgtga gcagctatgt aaggactgaa 7500
   tgataggtcc ccacgaatag tgcgatatca tacttagcag cagagaaacc atcaggaagg 7560
   taaactacag cacatattca gatgccacag acctgagaca taaggatagg gataaacact 7620
   gtacacgaga aggacaacta gaataaaaca gagccatttt ataacaaata ctacatgaca 7680
   aagggctacc agaatctatt gatacgcaga ggctcatgag agaaagtata agatcccaat 7740
   ttcggtaccc gtagtttaag agggtgaaac atgccctaga tcttagtata ttttctaggg 7800
   aactgctctt ataatatgat atgaaaaata tacttgtcct ctagcgtgtg tcacatccac 7860
   cctgcaagac taacatcttc gggtgctcac cataggcgag gcccactagt tccaggagcc 7920
   agtcagatat tatacaatag aatctataga tctgaggtcc taccgatttg gctgtgattc 7980
   gggtgagtct gggacattct gttaagatta tacccactac cgaagcctga cagagtccag 8040
   atggccgcct ggccaatatc cgaaggatcc taggccaaag ctatcaggtg ttctagcaca 8100
   gctgcacatg ctcaggaaat caaactctga agataccgta aaacaccatg ccgttcgcta 8160
   ccccaccagg tcgctgatat ccatattcag acataagcgc ttaagcagga atagtgcttt 8220
   cctgatacgg tacagcctaa gtcttgtggc attgttggct ctcatgaact cgcaaccctg 8280
   ttaacttgtt ggacgtatga tagcatgtcg tgcgctgatg cggcgaccta cagggatatt 8340
   ggagacctac ggaaatattg aatcgaaagg atttttacag tattggtaca tggccgttag 8400
   actctcttgc cctccctcct tttgctaccc acgatggaaa atagaacaat accggtaggt 8460
   catatcaaat gacgactctg cttggaatag agagatagaa aaacagcatg gctcgggcca 8520
   ctgctccctt tcagccaccc ggaccgggcc agagttggga tttgaaagaa gatttccctg      8580
   gatcagcccg gcagcactgt acctaaagcc acacgctatg acagcctagg gttactcact      8640
   cggcactcga acctccccca ataacccctc ccttacagca tctcgcccaa aactagtatc      8700
   atagggctaa aatgctatgt atcatatgac                                        8730
<210> 50
   <211> 9016
   <212> DNA
   <213> Tuber indicum
<400> 50
   tccgcccggc cgagccggcg cggtgtcctt cacggcgggg cgtgggctcg gagaacgggt 60
   gtttgtctat cgagtgatgt tttgtgatct tactgatttt agttatcaat acatccacat 120
   atacgaggag cttcggcttg catgatatca gcgaatgtag ctcggggact aaatctgata 180
   gttacactaa acgttggata ccacgctcgt tcagagctag ctccttctcc gctcgaggta 240
   gaggattaag ttcacaggcc tctgaatggc atctgatgat gttccgggca acttctctcg 300
   gggcatctgt gtcgctagca attgtatgat aacttttgcc ttctgcatga agctcaatca 360
   ttcgtactct ctcatcgctt gcggccattc tccgcttgcc tgttgcttga ccttgaggta 420
   tggtgtatga tgggaggtgt tggctcccat aaagaagtcg tggctaagat actagtgtcg 480
   ggttactaaa taacctctac tgctgagacc gttgcgaaag ctgggtaggt tctctctaca 540
   tattctagta gatattgacg ctgatttgcg ggaggaaaaa aaggaatttt atgttttttt 600
   tggttttaaa gataccgagg gcgtttcatt gatcctagat acataagttt aatttaagtc 660
   tacgcgatct ataatacgct gagaatgcct cccaactatg cccttaattt ccgccgctcc 720
   cacacagtca tactgtgata cagtaacttc agtttttttt tgtacccact agcctcttta 780
   ggccttctcg ggaggtcata tcggttcaca atccagtgga ggattctcca ataggaggct 840
   gtttagatca tgttagaatt gggcgctggt ggaatatgtt gttcggggaa cttctcacaa 900
   aaacgctgta tccaccagaa gtgaaagtgt tcatgtgcat ctcggtctcg ggctggaagt 960
   gcgggtcgag ttgagagtct ccattctcct cagcgctagc gatgtcgaaa gcattgagga 1020
   tgttggcggc ttcctcctcg gtgagatcga gctgatggtt gctggaaacg taaacacggg 1080
   tgatggtgaa tctggacacc gcggatgcgg gcttggcacg agccttcctt cttgcagcag 1140
   ccttatcggc cgcttccttg tcgatggggg ccttggggac aaagccagct tcacgcaaga 1200
   gcttctcagg ggtagtaggg tgaccgtgtt tcgccaattc ggtctcgata aattcttcga 1260
   gcacgagacc agggtcacca cgatccctag cagcagtata aagagaggca atgttttgcc 1320
   acatatactt attggtctca gcgacccaga catcacgagt gagattggaa atttcggctt 1380
   gagtgagccc ataaccgtta cagtacttag aaatccaggc taatagatgg ttagaaatag 1440
   aggcaaaata gggatgtgat ttacttacat ctctgcgcaa cgtagggatt caatgcacga 1500
   aggtgctgag agggggggat gcgggccaag gcctcatcac cattgacgat aacagccgga 1560
   atagtggcat cctgggccgt ccacggttgt ccaggtttat agtatccgca atacaaacgt 1620
   agaaccgaca caacataagg gacttcgggg tcgaggggga ctcgccctcg atgacggacg 1680
   gttgattgaa ttgcaccaag ccacttgtcg tagggcaaga gttcgccgct gacagtgaaa 1740
   gtgacaccac cggggggttg aacgatgatg ggggagttct cgtgttggac ccagatgcac 1800
   ccgatcctgt cttgcatgag aatgtaccca cggtccgaaa ggtacaaggc gcatcccaag 1860
   gtagcattac gcataatgag gcgtctctcg agcctgttct cccactccaa aaccttaggg 1920
   gggggggaga ggttaacgac catgctcaga ttgacaggac gagacgacat tatgaaaggc 1980
   ggggggggtg gagacgggaa aagaaatttg aaccgacaaa aggaggattg cgtatgggcg 2040
   tttcaatgag tggagattgg cctcagtggc tgttgatgga agaaaggagg gtggaaggtc 2100
   tggcctcttt tgtatgctac ccgtcgaagt gggatgccaa ggcccaaaaa aaagagtgac 2160
   gccaagaagc cgtcacgtac aactcagccc tactagggtc tatcataaga tttttcccag 2220
   agcaggtaga gagcccttag gtttctaaga ttcctcgcgg aaagatcggg gaacattttt 2280
   gtatttacgg tcagagttgc tcgaatagaa agacgagtca aggtatttga tgaagatctg 2340
   atcgacattc gggagaagtt taaaacaaag gaattagtgg gggtgagtgg gaaatgtggg 2400
   ttctgttaaa gtatctgtcc cccaatctta actacccctc ttgtacattg cctctttggt 2460
   aaaggggtat ggtatctgaa ccttgaacaa taattccttg cgataatata tatcgatcag 2520
   gacagtttgc tgactggaaa atttaatata gtctccataa aaccgtgtga ccgactcgga 2580
   ctgggaaatc ataccttaag tctggccatc cggaagccgc tctttctgct atggggtaat 2640
   atcaaatagc caagaggcca tcaggcgaaa tgagaattca gaaatatgat agccctcccc 2700
   tgccaaccaa gagacaaata cttcggcaat accaccaccc gatctggtgg atgtctccat 2760
   gttcggctat ccgcttgagt atccagggga atagacaagg aagactacca catttgatgc 2820
   ggtgtgtgcg gctgccgaga aaactgtttg aaagatggaa ctccagaaca aaggattaac 2880
   tggtcaatgc aacacctggt cgctgcaagg gtctgaagat taaaggattg taactagtat 2940
   tgttggaggt tactcctagc aatttcagag gggtttttgg taaagataga ctacagtcgc 3000
   accccgctgt cccggacctc gctctcccgg atcccccgtt gtccgggacg aaattctctg 3060
   agctctgaat ttttttgcac cagaacgtac cccgctgtcc agggcagatt ctgcggattt 3120
   tggccatccg ggacagcgag gtgcgactgt atactgatat cagtaggatg gtatggcact 3180
   acaatgaaag aaagtggtct ggaccatgag ctcaaaaact ccaaagagaa tttccggccg 3240
   ggaaattata atatttaacc ggtttaaaac cgtgagatcc tctggcccag gaccagcggc 3300
   cgattgagag agctgttgtg agagcatctt agcactcaac cattagaact ccatgatagg 3360
   taggatgata aaaggggaat ggggtttaga atgggtgcac aaagcttctt gattctggat 3420
   taagatatga ctacataagg gagtctgaaa caatgtacac tctatctccg cttggactta 3480
   taggataaaa atagacatgt taagtgaatt ataatgagga aaaaagcctg attggttgta 3540
   gggatcgatt gtatgatcag gggtgggagt actacaagtt tgtctatata gtattgggaa 3600
   taactgtaaa aatagtttca gaatatggct gagtatttca caaacatgca ttagttgaat 3660
   atgaggtaga tactagagtc accttgttca gtcccttggc tgtctttgaa tatctggaaa 3720
   atataccagg aaagaaagtg ggaaaaacaa tatcttcaac ttctttggca aactgtttgg 3780
   aagtagcaaa acctaacctc tccttacagc ctgagcctgt agcttgatat ggtactcatt 3840
   tactctttca taaataaata aacaaataaa cccgtcggac gggggggggg ggctggctgg 3900
   ctggctggct ggactgggct ggcggttgtg tatgtgtgag atttgctatt ttaccttcca 3960
   cagtaccatc atcatcatac ttgcttactt ttttactttt aacttcatcc gcatagctat 4020
   gcacttgcaa tatctcttcc aactgtttgt ttaaaaacca tgaggtttga catcaaatga 4080
   aggagtaatt tccaggtgca caaaaaatat attgtgtgaa tacctacaat tgagcccaaa 4140
   tcgatttagt aaattgaaac ccatcaagta gttggtaatc tatcttatga agttttttga 4200
   gctgaaggga aaagagaaat gttaggcaaa tttttttgtg acccttagct ggtcggctgt 4260
   caatgcttga tattgtcatc caaaccacca cagattgtat tgctgggatg ttgacatgtt 4320
   gcagtacgac atctcctaag gcgatatgca tatcagagat taccttatcg tactaagaaa 4380
   gacaagacag tcatttctac tacttgcttt tttattctct tacttcatat tctaccatga 4440
   tttacagggg tcatgctaaa ttaaatattg tgtgatacct tgtgaaaaat gtccaccaaa 4500
   gctatagagc tgtgaatatc gcaaattgta gggtgagagg gaaatcacaa tgcataactt 4560
   tataataatt ataaacacca caactgctcc actggggtgc cttatgctag gactttcctc 4620
   catgcgaccc tttttgcatc ataaaacaaa taataacaga ttttacaaaa gaaaaaataa 4680
   aaaccaatga ggatgcccca ccgccacatt aaaggagaaa gtagttctta gatcttgtct 4740
   ctctcaattc acttagtgca cctgtggata gaaacttagg tgaagataga gaaagaggta 4800
   tcatgagttt taatgtacat tattcattgc aatttaattc cgggcacact aagttattta 4860
   tttatttatt caatccctac tgcaatccaa gctactcttg ttcttgccaa gactatatct 4920
   tgggggcggg gaacaaacta ttttcattgc accttttggg cgctacgagc aaaaaattga 4980
   ccatcaaatt ttcgtcaatt taaaatccta aatacaagta tcatacacat ctactcaagt 5040
   acaagaatcc ttgtaactat tggaacaaaa caaattctgg gactcttgtc ggacactacg 5100
   ttaacgctag taagtaacaa aaaatctttt taatattaga agtgtgtgtg acatccactt 5160
   agattttgcg tgacatccct gccgctacca gaccttagta ctcgagtagt agaataagag 5220
   gagtagcgac cttcaacgac agccaagcca tcaaaaaggg tatcgtactc gagtgttcaa 5280
   atagaaaatc taccttcctg tgataccagg tgaggtcatt gtggtggttc aatacaacaa 5340
   tcaagtcctg cgattacgca taacaagtgt gagtactcga gaagagcgcg aatacacggc 5400
   tgcctggccc gcgtgggtct aacagatctg tccttgctct atttatttgt tttgtttttc 5460
   ctaagcgcag cactttttac cctatgaggt atttataata atattataga tattctggag 5520
   gaggttccca aaaatatatc ggaaatgcac ctaggggcag tggtactagg attcccaccg 5580
   cacactatct gctagtggat atgtctaggt ttctagtttc cataaacacc agacgagaaa 5640
   ggcttaccaa aacgacagag ggggttgggt ctgggagatg tctacggtgc gacgatagaa 5700
   cggataaaag cgcagggcgg ggataaatcg agactcggga tggcaacttt gatgtggatc 5760
   agtcatgcgc agaggctgct cgggacggac gagctctccc acgccctggc agttgagctg 5820
   agctccacca acttcaacgc tagcaacatc tcttcgatgt cgatactgct gggttgtttc 5880
   taagggctta ttacggtgga caaggaagca tcagccgtgc aactggccca ctttactctc 5940
   caagagtacc tttccgctca tcccgatatt ttctgcagac cccatgcggc gatggtagag 6000
   atctgcttga cctatctgaa ttcccggcaa atcaaggacc tttcgaccga cccatctcct 6060
   gacaccccgg gtgcaccctt tctagaatat tgttctgtgc actggggaat ccatgcgaaa 6120
   acggaactct gggattgtag gagatcgctt gcgctgaagc tattctagga gtttgatggc 6180
   cacatatcca cagaatttct tttagcgcaa gtggcgaatt taagtcccaa ggggcgtgat 6240
   acctggcctc cgttcagtgg gctgtactgc gcatcgttct tcggaatcgt cgagattatg 6300
   gctgctttaa catacgtggg atgttgggat accggggaag gaggttgttt ggggtaaccg 6360
   ccgcttgcac gggctgttca taacgggcat ggggaggtgg tggaaatgct attcgggttg 6420
   gaaaaggtcc atccggacga gctggataac cgcgaccgga caccgctctc acatgttact 6480
   gcctgtcggc atgaagaagt ggtgaaaata ttgctcctgt ttgagcggga agaggatagc 6540
   cacaaaaatc cggacaatga cggtcaaaca acactctctt tcgctgctcg gtttggacat 6600
   gaggaagtgg tgggaatact acttgggtgg gaagatgtta atcccaatga gctagataat 6660
   gacggccaaa caccgctctc gaatgctgct tggaggggtc atgagagagt tgcgaagata 6720
   ttactcgggc gggaagaggt caacctcgaa gagccagacg agctcggccg ggcgccgctc 6780
   ttgcatgccg ctggtcatgg acatgaggga gtagtgaaaa tttttctcgg gcgggaagag 6840
   gtcaacctca ggaagctaga caattactcc caaacaccgc ttttgcatgc gtttggtctg 6900
   gatatgaaga ggtggtgaga gtgctactca tacggggagt gtcgataccg gcgaaccgga 6960
   catcagcggc caaataccgc tctcgcatgc tgccgctcat gggcacgagg aggtggtaga 7020
   aatgttactc atgtgggaag aggtcaaacc cgacaggcca gatgatttcg gccaaacact 7080
   tgctgcttgg gatggacatg gggggatgat gaagtactac tcggccggga agatgtcaaa 7140
   ccggacaggc cagacgatag cggtaaaacc ctgctcttgt tggcctcttg ggataggaat 7200
   gagggagcgg tgaaaatact acttgggcgc gaagttgtcg gccccgacga accggataat 7260
   gacgaccaaa tacccctctc gttcgcaact gggaatggcc atcagaaggc ggtggcccac 7320
   aaagcgttaa cccctagcgc ggtttaaggt ttataaaaaa caacctcttc gaaggcatca 7380
   ccttcctttt cttacagagt actttgctcg taggggtcga cctagagcat caccctttcc 7440
   agtcgaagga gatcccatga gacgagtctg gaatgcgctt atatgccacc atgcggacgt 7500
   gaacctctct ggggatttgc ttttatagtc gggcgtgggt tttttttctg agtacggtag 7560
   agtttggaac tagttctatt gccgtgacaa agtctagttt agtttattac accttgcagt 7620
   cctcatccta tcatatttga tttcctccag ccgtacattc ttgccctcac gttttttctc 7680
   tcccttgcgc aattgataag catatagagt gttgttcata tagaaggaaa gtggtgtatg 7740
   agaggggaag ggttcttaag accaatgtga gcagctatgt aaggactgaa tgataggtcc 7800
   ccacgaatag tgcgatatca tacttagcag cagagaaacc atcaggaagg taaactacag 7860
   cacatattca gatgccacag acctgagaca taaggatagg gataaacacc gtacacgaga 7920
   aggacaacta gaataaaaca gagccatttt ataacaaata ctacatgaca aagggctacc 7980
   agaatctatt gatacgcaga ggctcatgag agaaagtata agatcccaat ttcggtaccc 8040
   gtagtttaag agggtgaaac atgccctaga tcttagtata ttttctaggg aactgctctt 8100
   ataatatgat atgaaaaata tacttgtcct ctagcgtgtg tcacatccac cctgcaagac 8160
   taacatcttc gggtgctcac cataggcgag gcccactagt tccaggagcc agtcagatat 8220
   tatacaatag aatctataga tctgaggtcc taccgatttg gctgtgattc gggtgagtct 8280
   gggacattct gttaagatta tacccactac cgaagcctga cagagtccag atggccgcct 8340
   ggccaatatc cgaaggatcc taggccaaag ctatcaggtg ttctagcaca gctgcacatg 8400
   ctcaggaaat caaactctga agataccgta aaacaccatg ccgttcgcta ccccaccagg 8460
   tcgctgatat ccatattcag acataagcgc ttaagcagga atagtgcttt cctgacacgg 8520
   tacagcctaa gtcttgtggc attgttggct ctcatgaact cgcaaccctg ttaacttgtc 8580
   ggacgtatga tagcatgtcg tgcgctgatg cggcgaccta cagggatatt ggagacctac 8640
   ggaaatatcg aatcgaaagg atttttacag tattggtaca tggccgttag actctcttgc 8700
   cctccctcct tttgctaccc acgatggaaa atagaacaat accggtaggt catatcaaat 8760
   gacgactctg cttggaatag agagatagaa aaacagcatg gctcgggcca ctgctccctt 8820
   tcagccaccc ggatcgggcc agagttggga tttgaaagaa gatttccctg gatcagcccg 8880
   gcagcactgt acctaaagcc acacgctgtg acagcctagg gttactcact cggcactcga 8940
   acctccccca ataacccctc ccttacagca tctcgcccaa aactagtatc atagggctaa 9000
   aatactatgt atcata 9016
<210> 51
   <211> 9062
   <212> DNA
   <213> Tuber indicum
<400> 51
   atccgctttt cgccctccgc ccggctgagc cggcgcgctg tccttcacgg cggggcgtgg 60
   gctcggggaa cgggtgtttt tctatcgagt gatgttttgt gatcttactg gttttggcta 120
   tcaatacatc cacatatacg aggagcttcg gcttgcatga tatcagcgaa tgtagctcgg 180
   ggacgtaatc tgataattac actaaacgtt ggataccacg caggttcaga gctagctcct 240
   tctccgctcg aggtagagga ttaagttcac cggcctctga atgacatctg atgatgttcc 300
   gggcaacttc tctcggggca tctgtgtcgc tagcaattgt atgataactt ttgccttctg 360
   catgaagctc aatcattcgt actctctcat cgcttgcggc cattcgccgc ttgcctgttg 420
   cttgaccttg aggtatggtg tataatggga ggtgttggct cccataaaga aatcgtggct 480
   aagatactag tgacgggtta ctaaatagcc tctactgctg agaccgttgc gaaagctgag 540
   taggttctct ctacatattc tagtagatct tgacgctgat ttgtggaagg aaaaaaagaa 600
   attttatgtt ttaagagatt ttatgtttta agaaatttta tgttttaaga aattttatgc 660
   tttaagaaat tttatttttt aagaaatttt atgctttaag aaattttatg ttttaagaaa 720
   ttttatgttt ttaagaaatt tcatgtttta agaaatttta tgtttttttt ggttttaaag 780
   atactgaggg cgtttcattg atcctaggta cataagttta atttaagtct acacgatcta 840
   taatacgctg agaatgcctc ccagctatgc ccttgatttc cgccgctccc acacagtcat 900
   actgtaatac agtaacttca gttttttttt gtacctgcta gcctctttag gctgtctcgg 960
   gaagtcatat aggttcacaa tccagtggag gattctccaa tagtaggctg tttagatcat 1020
   gttagaattg ggggctggcg gaatatgctg ttcggggaac ttctcacaaa aacgctgtat 1080
   ccaccagaag tgaaagtgtt catgtgcatc tcgttctcgg gctggaagtg cgggtcgagt 1140
   tgagagtctc cattctcctc agcgctagtg atgtcaaaag catggaggat gttggcggct 1200
   tcctcctcgg tgagatcgag ctgacggttg ctggaaacgt aaacacgggt gatagtgaat 1260
   ctggacactg cgggtgcggg cttggcacga gccttccttc ttgcagcagc cttatcggcc 1320
   gcttccttgt cgatgggggc cttggggaca aagccagctt cacgcaagag cttctcaggg 1380
   gtagtagggt gaccatgttt cgccaattcg gtctcgataa attcttcgag cacgagacca 1440
   gggtcaccac gatccctagc agcagtataa agagaggcga tgttttgcca catatactta 1500
   ttggtctcag cgacccagac gccacgagtg agattggaaa tttcggcttg agtgagccca 1560
   taaccgttac agtacttaga aatccaggct aatagatggt tagaaataga ggcaaaatag 1620
   ggatgtgatt tacttacatc tctgagcaac gtagggattc aatgcacgaa ggtgctgaga 1680
   ggggggaata cgggccaagg cctcatcacc attgacgata acagccggaa tagtggcatc 1740
   ctgggcggtc cacggctgtc caggtctata gtatccgcaa tacaaacgca gaaccgacac 1800
   aacataaggg acttcggggt cgagggggac tcgcccttga tgacggacgg ttgattgaat 1860
   tgcaccgagc cacttgtcat agggcaagag ttcgccgctg acagtgaaag tgacaccacc 1920
   ggggggttga acgataatgg gggagttctc atgttggacc cagatgcacc cgatcctgtc 1980
   ttgcatgagg atgtacccac ggtccgaaag gtacaaggcg catcccaagg tagcattgcg 2040
   cataatgagg cgtctctcga gcctgttctc ccactccaaa accttagggg gggggagagg 2100
   ttaacgacca tgctcagatt gacaggacga gacgacatta tgaaaggcgg gggggggggt 2160
   ggagacggga aaagaaattt gaaccgacaa aaggaggatt gcgtatgggc gtttcaatga 2220
   gtggagatta gcctcagtgg ctgttgatgt tgatggaaga aaggagggtg gaaggtctgg 2280
   cctcagttgt atgctacctc acgaagtggg atgccaaggc ccaaaaaaaa gagtgacgcc 2340
   aagaagccgt catgtacaac tcagccctac tagggtctat catagtacat acaataataa 2400
   taataatatt attatgataa ataatattat tattatgata tcataatatt tttcccagag 2460
   caggtagaga acccttaggt ttctaagatt cctggcggaa agattgggga acatttttgt 2520
   atttacggtc agagttgctc gaatagaaaa acgagtaaag gtatttgatg aagatctgat 2580
   tgacattcgg gagaagttta gaacaaagga attagtgggg atgagctggg aaatgtgggt 2640
   tctgttacag tatctgtcac ccaatcttaa ctacccctct tgtacattgc ctctttggta 2700
   gggggggatg gtatctgagg cttgaacaat aattccttgc gataatatat atcgatcagg 2760
   acagtttgct gactggaaag tctaatatat actatatagt cttcataaaa ccgtgtgacc 2820
   gactgggaaa tcatacctta agtctggcca tccggaagcc gctctttctg ctatggggga 2880
   atatcaaata gccaagaggc catcaggtga aatgagaatt cagaaatatg atagccttcc 2940
   cctgccaacc aagagacaaa tgtttcggca ataccaccac ctgatctggt ggatgtctcc 3000
   atgttcggct atccgcttga gtatccaggg gaatagacaa ggaagactac cacattttat 3060
   gcagtgtgtg cggctgccga gaaaactgtt tgaaagatgg aactccagaa caaaggatta 3120
   actggtcaac gcaacacctg gttgatgcaa gggtctgaag attaaaggat tgtaactagt 3180
   attgttggac gttacttcta gcaatttcag agtggttttg ggtaaagata gactatactg 3240
   atattggtag gatggtatag cactacaacg aaagaaagtg gtctggacaa tgagcacaaa 3300
   aactctaaag agaatttccg ggtgggaaat tagaatattt aactagttta aaaccataag 3360
   attctctatc ccaagacctg ttgaaaatca cgaagcttcc atggtttgga tgatgaggtg 3420
   taggttgttt ggccttggtt tggaaacgca atggcggaat aagctaagga aagggtcggc 3480
   gtgtgggtga gaactagcag ttggtgtaag gagaagtgtt tgggatgttt taggagttag 3540
   cactagggat actggaagta acaggttatg ctccggaggt ccagtcgaca accaggtgtc 3600
   gcactatagg agataggaaa ctggagaaca accacaaaag tatcagggct gtctggaata 3660
   gcgatagctt aggggaaacg ttttggagga tgtttgaagg agttgtgtga aaggaacggt 3720
   gtggcaacat gaagaaaaga tggtatggaa ttctgcgagg cagatggatg aagaaaatat 3780
   ttgcaaagga atagcttctg tatatagagg acacctccgg cagcatagaa caaacaagaa 3840
   tcgcagacac tctctaccta tcatagcctt attatttgaa gaccagcagc tgattgagag 3900
   agctgttgtg agagcatcta agcactcaac ctgcagaact ccatgatagg taggatgata 3960
   aaaggggaat ggggtttaga atgggtatat aaagcctctt gattctggat taggatatgg 4020
   ctagataatg ttgttttttg tcacatctct tccaactatt tatttaaaaa ccacgagatt 4080
   ggacattaaa atttaatgaa ggagtgattt cctcatgcac aaaatatata ttgtgtgaat 4140
   acctacaatt gagactgcat aaacacatga gcctaaatca atttagtaaa ttaaaatcca 4200
   tcaagtagtt ggtgatctag cttatgaagt ttgtgaagtt ttttgagctg aagggaaaag 4260
   agaaaggtta ggcaaatttt tttgtgaccc ttagctggtt ggctgtcaat gcttgatatt 4320
   gtcatccaaa ccaccacaga ttgtattgtg gggatgttga catgttgcag tacgacatct 4380
   cctaagaccg tatgcatata agagattact ttatcctact aaaagagaca agacagtcgt 4440
   ttctactact tgcttctgta ttctcttacc tcatattcaa ccataattta caagagtcat 4500
   gccaaatcaa atgttgtgtg atacctggtg aaaaatgtcc acctaagcct cagctgtgaa 4560
   tatcgcaaat tgtaaggtga gaggggaatc acaatgcata actttatatt ataaacacca 4620
   caactgctcc actggggtac cttatactag gactttccta catgcaactc tttttgcatc 4680
   ataaaacaaa taagaacaga ttttacaaaa gaaaaaaaaa aaactagtga ggatgcccta 4740
   ccgcgacgtt aaaggagaaa gtagttctca gatcttgtct ccctcagctc gcttagtgta 4800
   cctgtggaca gaaactcatg tgaagataga gaaagaggta tcatgaattt taatgcagat 4860
   tattcattgc aatttaattc cgggcatact aatttattta ttcatttact caatccctac 4920
   tgcaatccaa gctactcttg tccttgccaa ggctatcttg agcgccgtga acaaacttat 4980
   tttcattgca ccttttgggc gctatgagca aaagattgac tatttcatca atttaaaatc 5040
   ctaaatacaa atatcataca catctactca agtactgtac aagaatcctt gtaactattg 5100
   gaacaaaata aattctggga ctcttgtcgg acactacgtt aacgctagta agtaacaata 5160
   aatcttttaa aattactagt gtgtgacatc cacttagatt ttgcgtgaca tccctgccgc 5220
   tactagacct tagtactcga gtaatagaat aataggagtc gcgaccttca acgacaaggg 5280
   tatcgtactc gagtattcaa atagaaaatc taccttccta tgataccagg tgaggtcttt 5340
   gctgtggttc aatacgacaa tcaagtcccg cgattacgca taacaagtgt gagtactcga 5400
   gaagagtgcg aatacacggc tgcctggcct gcgtgggtct aacagatcta tccttgctct 5460
   atttatttgt tttgtttttc ctgagcgcag cactttttac cctatgaggt atttataata 5520
   atattataga tattctggag gaggttccca aaaatatatc ggaaatgcac ttaggggcaa 5580
   tggtactagg attcccaccg cacactatcc gctaatagat atatctaggt ttctagtttc 5640
   cataaacacc agatgagaaa ggcttaccaa aacgacagag ggggttgggt ctgggggatg 5700
   cctacggtgc gacgatagaa cggataaaag cgcagggcgg ggataaatcg agactcggga 5760
   tggcaacttt gatgtggatc agtcatgcgc agaggctgct cgggacggac gagctctccc 5820
   acgccctggc agttgagctg agctccaccg acttcaacgc tagcaacatc tcttcgatgt 5880
   cgacactgct gggttacttc taagggctta ttacggtgga caaggaagca tcagccgtgc 5940
   aactggccca atttactctc caagagtact tttccgctca tcccgatatt ttctgcagac 6000
   cccatggggc aatggcagag atatacttga cctatctgaa ttcccggcaa atcaaggacc 6060
   tttcgaccga cccatctcct gacaccccgg atgcaccctt tctagaatat tgttctgtgc 6120
   actggggaat ccatgcgaaa acggaactct cggattgcag gagatcgctt gcgctgaagc 6180
   tattctagga gtttgatggc cacatatcca cagaatttct tttagcgcaa gtggagaatt 6240
   taagtcccaa ggggcgtgat acctttcctc cgttcagtgg gctgtactgc gcatcgttct 6300
   tcggaaccgt cgaggttatg gctgctttaa catacgtggg atgttgggat actcggggtg 6360
   gaacaggtcc atcccgacga gctggataac cgcgaccgga caccgctctc acatgttgct 6420
   gcctgtgggc atgggggggg tggtgaaaat actgttcctg tttgagcagg aagaggatag 6480
   ccacgaaaag ccggacaatg acggtcaagc accactctct ttcgctgctt ggtttggaca 6540
   tgaagaagtg gtgagaatac tacttgggcg ggaagatgta aatcccaatg agccagataa 6600
   tgacggccaa acaccgctct cgaatgctac ttggaggggt catgagagag ttgcgaagat 6660
   attactcggg cgggatgagg tcaacctcga agagccagat gagctcggcc gggcgccgct 6720
   cttgcatgcc gctggtcatg gacatgaggg agtagtgaaa atttttctcg ggcgggaagg 6780
   ggtcaacctc gaaaagctag acaattactc ccgaacaccg cttttgcatg cgtttggtct 6840
   ggatatgaag aggtggtgag agtgctactc atacggaggg tgtcgatcct ggcgaaccgg 6900
   acagcagcgg ccaaataccg ctctcgcgtg ctgccgctca tgggcacgag gaggtggtaa 6960
   aaatgttact catgtgggaa gaggtcaaac ccgacacgcc agatgatttc ggccaaacac 7020
   ttgctgcttg ggatggacat ggggggataa tgaagtacta ctcggccggg aagatgtcaa 7080
   accggacagc ggtaaaaccc tgctctcgtg gcctcttggg atgggaatga gagagtggtg 7140
   aatatactac ttgggcgcga agttatcggc cccgacgaac cggataatga cgaccaaaca 7200
   cccctctcgt tcgcagctgg gaatgaccat cagaaggtgg tggccctgct acagtcttac 7260
   aaatcgttaa ctcctagcgc ggtttaaggt ttataaaaag cagcctcttc gaaggcatca 7320
   ccttcctttt cttacagagt actttgctcg tgggggtcgt cctagggcat caccctttcc 7380
   agtcgaagga gatcccatga gacgagtccg gaatgcgctt atatgccacc atgcggacgt 7440
   gaacctccct ggggatttgc ttttatagtc gggtgtgggt tttttttctg aatacggtag 7500
   aggttggaac tagttctatt gccgtgacaa agcctagttt agtttattac accttgcagt 7560
   cctcatccta tcatatttga tttcctccag ccgtacattc ttgccttcac gttttttctc 7620
   tcccttgcgc aattgataag catatagagt attgttcata tagaaggaaa gtggtgtacg 7680
   agaggggaag ggttcttaag gccaatgtga gcagctatgt aagggctgaa tgatagatcc 7740
   tcacgaatag tgcgatatca tacttagcag cagagaaacc atcaagaagg taaactacag 7800
   cacatattca gaggccacag acctgagaca taaggatagg gataaacacc gtacacaaga 7860
   agaacaacta gaataaaaca gagtcatttt ataaaatact acatggcaaa gggctaccag 7920
   aatctattga tacgcagagg ctcatgagag aaagtataag atcccaattt cggtactcgt 7980
   agtttaagag ggtgaaacag gccctagatc ttagcatatt ttctagggga ctgctcttat 8040
   aataatatta tgatattatt aggaaaaata tacttgtcct ttagcgtatg tcacatccac 8100
   cctgcaagct taacctcttt gggtgctcac cataggcaag gcccactagt tccaggaccc 8160
   agtcagatat tatacaatag aatctataga tctgaggtcc taccgatttg gctgtggttc 8220
   gggtgagtct gggacattct gttaagatta tacccactac ggaagcagga cagagtccag 8280
   atggccgcct ggcctggctg gccaatatcc gaagggtcct aggccaaagc tatcaggtgt 8340
   tctagcacag ctgcgcatgc tcaggaaatc aaactctgaa gataccgtat aacaccttgc 8400
   cattcgctac cccaccaggt cgctaatctc catattcaga cataagtgct taagcaggaa 8460
   tagtactttc ctgacacggt acagcctaag tcttgtggca ttgttggctc tcatgaacta 8520
   gttttcgcaa ccctgtaaac ttgtcggacg tatgatagca tgtcgtgcgc tgatgcggcg 8580
   acctacaggg atattggaga cctacggaaa tatcgaatcg aaaggatttt tacagtattg 8640
   gtacatggcc gttagactct cttgcactcc ccccttttgc tacccacgat ggaaaagaga 8700
   acaataccgg taggtcagat caaacgacga ctctgcttgg aatagagaga tagagaaaca 8760
   gcatggctcg ggccactgcc cccgtcagcc acccgggccg ggccagagtt gggatttgaa 8820
   agaagatttc cctccggtag cactgtacct aaagccacac gctgtgctag cctagggtta 8880
   ctcactcggc actcgaacct ccccaaataa cccctccctt acagcatctc gcccaaaact 8940
   agtatcatag ggctaaaata ctatgtatca tatgacacca cacgatatcg ttgtcccatg 9000
   caaaaagacc gcaaacccaa acctaattga aggttgtgat cccactacaa aagtcagggg 9060
   at 9062
<210> 52
   <211> 9136
   <212> DNA
   <213> Tuber borchii
<220>
   <221> misc_feature
   <222> (534)..(534)
   <223> n is a, c, g, or t
<220>
   <221> misc_feature
   <222> (537)..(537)
   <223> n is a, c, g, or t
<220>
   <221> misc_feature
   <222> (1276)..(1277)
   <223> n is a, c, g, or t
<400> 52
   gataccccta cataggtttg tgcgtttgtt tttttcaaca tgcccttacc attctcaacc 60
   aataccaact ttatcataaa acggagttca caaggctaaa gaggaaccra ttttaaaaca 120
   gttttgtcct aggtttttct ccgaggactt tgttgttggg gccagttagt tattagactg 180
   ggttgcggcg agagtcgatt tcaagcgccc tgcaccgcag agcatcgaag aatagcccaa 240
   ttaagctgag gcgtgaaacc aatacctgca tgaaagtggt taaaattttg tggcgcagtg 300
   tagcacaaaa tgacaggaca gataagatag tctggcacct aactagtcct atgactttag 360
   tctacactac gccacaaaat attaatcact ttcatgcacc cccgagcaca gcactagtac 420
   agtactagta cttgtacttg ggtgtgaggg taagtggtcg gaaagtaacc caattctcgg 480
   tgcaaattct aagggataca ggaaggcatt gagggagatt gaagataatt tganaanaag 540
   atagtggggg agagaaagga gataaagaag gaaaggaggg atggagcaaa catctaaatt 600
   ggatatataa agcctgtagt agtataaata tactcgaaaa gttttaaaaa aaaaaaaaaa 660
   gtgatcggaa aaaaaaagtg gtttttccga tcactttttg ctccaggctg gtactcgggt 720
   atggatgtaa aaatcgagtt ctgtaagtaa ccctgtcgtt agaatgaggt actgagttgg 780
   ctagaataga agaaaagatc ggcaagtcag ggtcaccaag tacccggacc aaggctgggc 840
   agatgcaaca tggcagtgat ggtcctttct ttttcttttt ctgctttcta tatttgagtc 900
   tgtaaagttt gatagggaat tgagctatta ggggtttgga atttagaaga ctttcaataa 960
   gagcagtaac aactacaagc agcttccgca atgattaaga tgcaaccayy ccaggttttt 1020
   agctccttyc aaggtctagc tttttccatg acgccaattg cacgattccc gagattcctt 1080
   gttcatatcc ctgctktgct cgatttttac atccctaccc gagtactagg ggctttcaaa 1140
   gtcaggaatg ggggccaacg gtaaaatgat cgaacactca aagccacgca ttagttctgc 1200
   gttagggcga catattaact gtagccaact gtatcaaagc atttcaagtg acataatagc 1260
   cggtcccctc ctattnnatc gcgcatagts ggaaaagcaa gtgtgaccta cactgtaaaa 1320
   aaaaaagccg acgtgtcaca cctgcttgag cgtcacactt gcttgagcgt cacacttgct 1380
   tttcccgact atgagcgatt gcgaaatagg aggggaaccg gctattagta tcattctaaa 1440
   ttatttcggt ttttggggta gagaaacggt gaacaaaaga gacaggatcg cacgtacagt 1500
   aagaatgatt acaggtgaat cacgaggcta ggggagtaat ttcagttagt cgcagcgtta 1560
   aaccccccta ccaggttcat ggttcctccc atgcagtcta cgaaatttaa tggaagagac 1620
   cccctgctgt gtctacattg gagaaaacct caggtgaaaa aaatcaaacg caaacaaata 1680
   ctgacaaagg tcatacaagt gaaagcttcc tcgcctcctt actaaaaaag gtggaaaaat 1740
   accccacctt taagcgtgat caatagcgcg agtcacagcc caccaacagg cctctcgaac 1800
   agatgctgat tagtaggccc cctgtgagga aacgggtagt agtcagcaca gcagcaacag 1860
   ttcgtccaag taaaccactt gaaagtttct tatttaggtt taactgttcg agtagaggac 1920
   ctgctcgagt gaactatgag gcctaatcgt aggatttaaa agtaccagct atgttatccc 1980
   aaataggaag gcagatcttc tactcaagta cggtacaagt agtattgtat ttgtatccca 2040
   ctctcattcc tacaacctcc agtgggcagg gatgtcaagc aaaatctaag caaacatcac 2100
   aagcaacttg aatggagtta atttacactt ttgagcgaat acagcgcaag agaactcgca 2160
   actccttttt agcgagagtt tcacgcatct tatcgctaag cgcgctagga aaaaatcagg 2220
   cgaaataagg tcttgggatc ggagcttttt tttattgtag tgacaggtct tcaatagaat 2280
   agcctaatga cgttgtttaa acgagatctg ttaagatgat catgttgtaa cactggtgtg 2340
   ggttttgggg gctgaggggt agcttcaccc ggtatcactc tgtattttgt ttgattttgc 2400
   gtgacccaac caccttactg accttcctaa ataggaaggc aggcttttct actctttttg 2460
   gtgcttggct gtccttgaag tttctgtaat actgctggct cgttccgcta ctaatccttg 2520
   gcagtgcgta gggatgtcac acagagctta agcaaacctc ctacacttct aatatgatac 2580
   taaagaattt ttacttgcta aagctgctgt ctaagtaatt agggtgttta agtaaaattt 2640
   aagaattacg cttccctttc atctcacaat ttgtgatgcc cccagctcta gggtgttgga 2700
   tgttttcgtt gggtgtcaat cgatgtttga tttcgcgtga ccgctgcaaa tcaaggtgga 2760
   atgggagaca aaaagtatat gggacgagtg tggaggtgac ttgcttgact ttcctttgcg 2820
   ggataagcag atcttttata cggtatgtaa aactcctagg ggtatcatac atataggcat 2880
   ctctttagtg atgcgctgga tggcgagatc caatagcgat ggccaccagc tagtgatcgc 2940
   aaagtacttc cccagtgttt ttcttttttc tcgctagaac aaacttcaca agcttcataa 3000
   accagagcgc caacaactta gcgaagctca attcaccgga tgaatttagg cctctgcctt 3060
   tgcgccggct caattatgta atttgagaac attagatact cgcgctgtgt atgttttatg 3120
   cgagcagaaa tcatcccctc gtcatagccc ccaagtaaac aaactgtcgt aagaactcga 3180
   ataactgacc tcccgcaaag acgtcccaaa tttcctctct tggatagggg ggcgagcgca 3240
   cagcgccaac tggcctcctc agcctgagct taagacagaa taaaaagaaa aagtaccaga 3300
   ccttcccagc agaggcaatt caaacatcct agcgtgaggt tcaattgttc gagtccttat 3360
   ggtgctgtaa aagaagtgtc gcgggtgggc ggttgtggga aagaaagagt actcaagtcg 3420
   ccggattgaa gatgctcccg tcttagtagt gacgcactaa aaaaacgtat cttcaatcca 3480
   gcgacgcata ctgtagagtg ctataggccg caagttaaag gaggagttta gattctttgt 3540
   tccttcggaa tggtctagga aaggatttga agacttttcc ttttcaagta tagcatccaa 3600
   atagcaaaaa gggcagcgag gagattggcc aaggcagctc taaaatccgc ttgatatgca 3660
   gcccccgcat agttgtggga gactgtctgc tataatctac gagtagaact ttcccagata 3720
   ttcctaagac tacatatata aaggcgcagt atcctgtttc tggtcatacg actcactctt 3780
   ctgcaactga tcaaacttct ttcctccata taattagctt gaaatctcgg tttctatcct 3840
   ggtggtccaa gcgggggcgt cgggcacatt gcttctggct ctcctatctg gcgatatctt 3900
   catccagaat caggagactt agtgcgcccc tttcaaatcc taccctcctt ccgtcatcct 3960
   ccctggtatg aatctttgga ggttggtcaa acactctcac agcggctctt tcaactggcc 4020
   gctggccctg ggcaagaggc tctgacggtt tagaaccggt tagggattct aatttctctg 4080
   ccgaaaatcc ttcttagggg cgttcactca ctcacaatcc aaggcttcgc cttgtgctat 4140
   gatactatga tcccaccaat cttagcatgg tctaaccaga aggccctctg aaactatcac 4200
   gtgcaaccgg caacagcgct agccacaatt ccttaatctc cagacctttc gctgtccggt 4260
   tgttgcatcg ggcacctgct cctttgttct ggagttccat ctttcaaatc gcgctctctg 4320
   cgcccgcaca cgccgcctca aatagggcag cccttctgac ctattcgcct ggatgcgcaa 4380
   ggagtagctg aacgtgggga catatagcgg atcggttggt agtgttatcg aatcattcag 4440
   cccccgaccg gttggggaga aaactacttt tgaattctta tttcgcgtga cagcttcttg 4500
   gttattggat attccctcag aatggaaaaa acggcttccg aagtttgaga tatgatattc 4560
   cggtctgcca taccgtattg tggaggttat tcctattaca tttccaatta gcaaaatatc 4620
   ctaatttata tatcgaatca caactaatta ttcctctagg gggctctacc aaagtagtaa 4680
   agtacaggac tgacttgggg gacggataat agaacccgca tttctcagtt catcctgttt 4740
   cactaattcc tttgttcgaa gctcctcctg aatattgaac catcattaac tatcccgact 4800
   tgttttccgc atgagcatcc ttggccgcaa gaaggaaaac attggttgat attttcgccg 4860
   agaatcttag aaacctaagg gctctctccc tgccctagtg gaaagctcac catacatact 4920
   atgatatttt cacgataata ctatgctcag accctagtag ggcttgctgg agttgtacgt 4980
   gacttcttct tagggtcacc cttttttggg cgcacccacc cccttcgtga ctagtagtct 5040
   atataagaga tcccaactgc ccctcttctc tcttccatca acaaccactc agcagaaaca 5100
   acatccatac agcaatcctc cctccgccgg ttgatttttc aaattcatct ttcctcccca 5160
   tttttatctt tcatcatgtc agcgagaccg gtcaatctca ctatggtggt cactacctcc 5220
   ccgcctccaa agctctttga gtgggagaac aaggttcaga ggcgcctcgt tatgcgtaat 5280
   gccactttgg gttgcgcctt gtatctctcg gaccgtgggt atatcctcat gcaggacaga 5340
   atcggttgta tmtgggtcca gcacgaaaac tcccccgtca ttgttcaacc tccgggtggt 5400
   attactttta acgtcagtgg tgagctcttg ccttacgaca agtggctcgg tgcaatccag 5460
   tcaaccgtcc gccatcaggg gcgagttccc ctcgatcctg aagtccccta tgttgtttct 5520
   gttctacgtc tttattgcgg atactatcag cccggacaac cgtggacggc ccaagatagc 5580
   gctatccccg ccgccatcat caacggtgat cagacccttg ctcgtgttcc acgttctcag 5640
   caattacgtg ctttgaatcc ctatgttgcg cagagatgta agtaaatcac actcctacag 5700
   tccctaggca aaagtgttcg tgaatttgct tagtcagatg tctcacattt tcacgaacac 5760
   ttgctgtatt ttgtccctgg tactaattaa atctccatcc atcagcttgg atctctaaat 5820
   actgcaacgg ttatgggctc acacaggctg aaatttccaa tcttactcgc gaagtctggg 5880
   ttgcggagcc caacaagtat atgtggcaaa acatcgcctc tctttatact gctgctaggg 5940
   atcgcagcga gcctggtctt gtgctagaag acttcattga gactgaattg gcgaaacacg 6000
   gccaccctag taccccagaa aagctgctcc gtgaagctgg gtttgtcccc aagactccaa 6060
   tcgacaagga agccgccgat aaggctgccg caagaaggaa agctcgcgcc acgcccaaat 6120
   tgtctaactt cgctctcacc cgcgtttact tgggtaacga ccaccagctc agccttaccg 6180
   aggatgaagc tgccgatatt cttcgggctt tcgatggata tgaaccgaac ttgggtgacg 6240
   gacactcgca aatcggccct catgtccagc acttgctggc gatggaaatg caagacaagt 6300
   ttacccacac ttccgttggt tccaatggct tcgtgagtga taccgtgaat agtatgattt 6360
   acttgttgct caattctaac atttacccga acagcatact atgggagaat cctccacagg 6420
   attgtaaccg gatatgatcc ccttggagat ctgaggaggc tgcaggtgcg aaaaaaaaaa 6480
   aatggtggaa aaactgtgta cgacgggaag cagggtatag ctagaaggca tactcggcgg 6540
   gttatggatc aatgtagact taaattaagc ttatctgtct gggaccaata gaaagccctt 6600
   agtaacttta aatccagata tcaatgcttt cttccataaa tcagcgttcg cgacttcgat 6660
   gtactaggcc gttttttttt taagtattcg gatatggaaa atataagtaa tctagtcatg 6720
   gtataccggt acagtaagag cttgagcaat taaaccgtgt gcgaggtagt ccaaaatttc 6780
   ctcctctaga aaatgagaga aaaacacact aaaaaacctg ccctccgaga caaataaaaa 6840
   gaaaaataac accacacctt actaggagag ccaactccaa catcccagca catagttcaa 6900
   ttaccggatg ccagtgggct ttcaatgccc cgaatgcatc gtctaacggt attccagcat     6960
   cgcgagattc tggcactgta agtacaagta cagaagaaag acaccatgat agtcaagcgc     7020
   tcgcaagcgg ccaggtcggc ctgcaaatag ccggtaggct ttcagtccgg ccaggccaaa     7080
   gtgagacaga cctgggcttc ctaggccagg ctttgggaaa gccggcaggc ttcatgggtc     7140
   cctggcggca ccggtaccgt aaaaacttga ataattggac ggtgtgctat gtcgctgggt     7200
   tttcctcttt cagtaaggtc aagaaatttt cgtttttttt tccgtcccgg aaggggaaag     7260
   gggctgtgtt tttgctgtgc gttttgctcc ctttccagag aagggaattc tggactacat     7320
   agcagaccgt ccaattattc aagtttttac ggtaggcttt cggtatattc catgtgacta     7380
   gtcaatattt tcgtaatgtg tgggtgatat ctgcagatat ttgaagatac cgccagatat     7440
   ctgaagatgc cgccagatat ctgaaggtgc ctgtaggttt ctgcttatta ttcaggcttg     7500
   ggccacacca ttcgatttaa aacatattct gcttggcttc agatcttcta gagtttagcg     7560
   agaaagtagt atcttatttt tcattcactt tcaccctttt tctcctaatt gacaaactaa     7620
   ctactgtaac tagcggtaaa gacagaagca cataaacttg aacgaagtcg tcctcccatt     7680
   ctcccatcca tcgcaccaca tatcctttct ctgcgacctg tatacccctc tccctttcat      7740
   ggacacctaa actaagttag caaaagaacc gacatgcctt tcaaagaact cccacggctc     7800
   ataacctcca cgccgagcgt ccaaccttca gcacgtctga ctccttcgag atcgtactgc     7860
   atctggcggt gcgtccatcc aatttggcaa tacagctcgg agtagacgcg gtcggcccca     7920
   gcgcacctgg tgggggcggc cgggggttgt tctcatatac cgcccggggt gggcgtgaag     7980
   ggatagctct gggtatagga ggatgctacc cacgggagtg aaagcgcgac ttagaggaaa     8040
   acaattgcat ggcagagcga gaaagacaca ggggaaaggt agcactagaa gagtgcaatt     8100
   acgaatttga atgagtagga tgtaaggagg ccgctatgcg ttgtgagagt tttcaatgaa     8160
   ctttgttagc acaagagtct gtagggggga atgggagaca ggggcaagag ggagagaacg      8220
   cgtgcaatag tccctagagc actggttggt agatgggaag ctcgtggttt atgtgtactc     8280
   atgtctttac ctctaattag ttgatttatt tattgaataa gggcatacaa gcaggagtca     8340
   agttgaatca gaagaacttt cccccaattg tgctgtgtgt tggggatgaa tcttccatgc     8400
   ttggtgttgg tttgtgcttg gtgtttcgtt tgtgcttggt gtttcgtttc attctccagc     8460
   tgaatcacat cttatcactc gaaagcctgc agatatctgc agtccggcca ggcccggtca     8520
   gatgagcttc agaagccggg ctggccgctt gcgaacactg gtctcactgg ggcccccaga      8580
   ctaatgcgaa caccgcctag agtatgatac ccaatgccag acgctctgcc acccgggggg     8640
   tcactaaggg gctgtttgga gtcacaactt tggggccgca aaccattgtt tgtaactaca     8700
   ggtcttattt ggcagatggg aattacaaac aatggtttac aacaaccagc agcaattttt     8760
   ttgctgttta tgaaataggg cacagggttc tttcaaccat ggtttgtatc atagttaacc     8820
   aaacttcaca aagttgtcac gccctaactc tctacccaac cctatcattg ccccacacaa 8880
   aaaaccgcaa aaccaaatct aattgaaggg cgtcaccacc tataaaaatc cgaggcctca 8940
   cactccctca taatcctata tcccccctgt accaaaactc tcttcttctc aacacccact 9000
   ccagagtact caacctcaca acaccctccg catattggtc agccagtctg ccaatatcag 9060
   tattcgactt acagttgacc tctccacaca tacgcttgtt ttctttgcta aaatgccccc 9120
   aagtatcccc ccggct 9136
<210> 53
   <211> 1156
   <212> DNA
   <213> Tuber borchii
<400> 53
   atgcgtaatg ccactttggg ttgcgccttg tatctctcgg accgtgggta tatcctcatg 60
   caggacagaa tcggttgtat mtgggtccag cacgaaaact cccccgtcat tgttcaacct 120
   ccgggtggta ttacttttaa cgtcagtggt gagctcttgc cttacgacaa gtggctcggt 180
   gcaatccagt caaccgtccg ccatcagggg cgagttcccc tcgatcctga agtcccctat 240
   gttgtttctg ttctacgtct ttattgcgga tactatcagc ccggacaacc gtggacggcc 300
   caagatagcg ctatccccgc cgccatcatc aacggtgatc agacccttgc tcgtgttcca 360
   cgttctcagc aattacgtgc tttgaatccc tatgttgcgc agagatgtaa gtaaatcaca 420
   ctcctacagt ccctaggcaa aagtgttcgt gaatttgctt agtcagatgt ctcacatttt 480
   cacgaacact tgctgtattt tgtccctggt actaattaaa tctccatcca tcagcttgga 540
   tctctaaata ctgcaacggt tatgggctca cacaggctga aatttccaat cttactcgcg 600
   aagtctgggt tgcggagccc aacaagtata tgtggcaaaa catcgcctct ctttatactg 660
   ctgctaggga tcgcagcgag cctggtcttg tgctagaaga cttcattgag actgaattgg 720
   cgaaacacgg ccaccctagt accccagaaa agctgctccg tgaagctggg tttgtcccca 780
   agactccaat cgacaaggaa gccgccgata aggctgccgc aagaaggaaa gctcgcgcca 840
   cgcccaaatt gtctaacttc gctctcaccc gcgtttactt gggtaacgac caccagctca 900
   gccttaccga ggatgaagct gccgatattc ttcgggcttt cgatggatat gaaccgaact 960
   tgggtgacgg acactcgcaa atcggccctc atgtccagca cttgctggcg atggaaatgc 1020
   aagacaagtt tacccacact tccgttggtt ccaatggctt cgtgagtgat accgtgaata 1080
   gtatgattta cttgttgctc aattctaaca tttacccgaa cagcatacta tgggagaatc 1140
   ctccacagga ttgtaa 1156
<210> 54
   <211> 9381
   <212> DNA
   <213> Tuber borchii
<220>
   <221> misc_feature
   <222> (7267)..(7267)
   <223> n is a, c, g, or t
<220>
   <221> misc_feature
   <222> (7709)..(7709)
   <223> n is a, c, g, or t
<400> 54
   ctgttactca tcaggtatgg tgccgggact tgaaatcaac cctcatgggt aattcatttt 60
   gcttaataat acacacaaaa tagtggggct tggcaaaagt gtcttcaaaa accatcctac 120
   atgggtaatt caaaatttta ctatctccgc taatattgct ctgattgaaa attagaaaac 180
   atagaattag agaggagggt cttctgagca cttctatgaa gccatatcct attaaagatg 240
   tggttgtggt tgaaaacttt cgaagattgt tcttgtgttg gtctttgggt gatttcaagt 300
   gcccggcacc ataccgaaga aagtccaatt gagccgccga gagtttgtga aaccgctctt 360
   gctcttgtgt ggcactgttt tttttttgtt aagtccttga tttattcgta gctaactaac 420
   aggaacatca tggcaagcac cccaaggtat ggcctaaaaa ccctatataa caaaagaaac 480
   ttcttcgtcc cctaaataac aaataaacga taaaacatca ctatacaatc cttaactaaa 540
   gcaaagcttt catatgatca tgaagccaaa aaggcatctc tcgtaaaagg aaaaaccccc 600
   atcttcaccg ggaaatttat ccttccctcc ttcacataag acataaagtc ctccatcaat 660
   ttccttccca cctttccccc ttcccacaat ttagactctg gtctgatgtc gaaagctcgc 720
   gggtccttat tctctctcgc cctggccatt gctgggcatt ccattatatg cgctggtgtt 780
   tccaggctcg aaagacacct cccacaggtg gtgtcccgat ccagattcga acaatccatc 840
   tgagctccga ggtaactctt gttcgacctc atttggtgta gccttgacgc catgaactta 900
   gggagtgtca taaaaaacca acattcccta tatggcggcc tgtatccgta gtaggggggg 960
   tactcccatc ctacccattt ctgatgcatc tctcttctca ccctttctct cgctttgaaa 1020
   tgaaccttct tgtgtggcac tgtactcgta tcatcctagt actagtgcgg ccgatagata 1080
   aaatagtcac gtgggtctgt taaggcgctt gacaccctat atatagttta tctatcgcgc 1140
   aggagggagg gaggaggaaa ggaagaacgg aaaacgtgta ggtaaaaatt cacagctaaa 1200
   taaacaacgg agtatgatag gatgaaaact acaagctata aactaggctt cccacagcaa 1260
   taggaccact tccccccagg gaccaaactc tactgtgact tgtaatcaga cttggaaatc 1320
   ctcacctggc ataaaagcga atccctagcg agtttcgcgt gtgcatgtga catatacgtg 1380
   cattccccgg gcccatctca tcggattccc tccagctgca aaggatgacg tccatggaca 1440
   atttctataa gtgacatact ccgaaaggga aggatggcag cgatttcgac gaggcggtgt 1500
   ctcctacacc tcaaactgcc ccaggggttg ccgctttgcg cgactgtagc agcgctgcca 1560
   ctctgttatg gctgccccta gcagcgaacg agagcggtgt ttggccgaaa ttatttggtt 1620
   tttcggggtt aacatcttcc cgcatgagcg gtattttgac cacttcttca tgcccatacc 1680
   aagcagcgaa tgagagcagt gtttggccac cattatccgg cctgtcgagg ttgacccctt 1740
   cccatccgag tagtattttc accacttcct catgcccatt ccaagcggcg tacgagagtg 1800
   gtgtcctgcc acgattatct tgttcgtcag agttaacatc ttcttgccta tgccttctgc 1860
   tcgggaatga atctcggggt tttatctgct tcctctatta atgacaagtt taggcgcatt 1920
   tcacaatcca agggagcact actgggctag tagccatatc cccaactcta gaagatggtg 1980
   aagcaccaac caatatgggg tgccaagtta aactggttta cgagcgatgg ataaataaag 2040
   cggtgtcacc gtgagaggta cttttttgtc gcataatata agggcgtgag ggcctaagga 2100
   taattgtcag agtttgccgc gtacgggacg ctcgggcact cagtccatgg gtttcttagg 2160
   ctgcctggcg tagggcgtcc gctcagccaa aaaaatgacg gactatgacc cacaattcgt 2220
   cccgccctac tgctcacaac ttgatgttta tcttatggtt tggtgttcgg atttctcact 2280
   ctcattttaa gaaatttttt gtttatttgg accgtacaag tttatttaag tgaacaaatg 2340
   accctttaca aacagctaga tcatccctcc cattaaaagt aaatcaccca agtaacccat 2400
   aagctccccc cattgagatc attcaattaa tcaaaccgaa agatttcaga accctctccg 2460
   cttcctcgat aatatcgccc aaggcgtttg cgctgggctg ggaggcgaag cggtcggtga 2520
   gggctccaag atgggtagaa gctctcttct tcgcacgctt cgcgacccca gcacggtgaa 2580
   ggtcggcacg ggtcgaaggg cccttgttgg cgcacgccct cctcataatt tcatgagagc 2640
   ggcgcggggt gtatttatag ttgggattga ccaacttgtg agaagccgcg agttggcggg 2700
   cccttgccta ttaatgtgtt agtgattcca actaacaaac agtcaagtga gatgttatac 2760
   tttataaaca tctttggctt cgtccgggga atcctgccac atgcgaccga gaatttttgc 2820
   tattgaaact attagggaaa tggtactaag agcggagagt gaaaaagggg agaatgagaa 2880
   ccacatacaa atatcgttgt taccaacccc aggatactga gccttgaccg cattaacatt 2940
   ctccgttcta aacaagatga atctatgatt gtatcagcac atgaaatcgg tacgttacag 3000
   tcgcaccccg ctataacgaa ggccaaaaat tgggaatttt gttagttata gcgaggtttt 3060
   ggaagaaatt ccttatagca agggccaccc gcataggagc aggagtgtaa attcccagag 3120
   ggttcattcg taatagcgag gtttccctta taagcggtcc attatagcgg ggcacgactg 3180
   tagtaggatt gtacttacgc attagcgggg cgcttaacac ccttatcgcg gatgaaacta 3240
   agattagcgc catgagaggc aaagtcttga tcgttgacag caagacgcaa aaaacggttt 3300
   tcgccggtaa tctcgtgctg gggatcctcg aaggaatgat cggtgacgtt gcaattcatg 3360
   tcggtagtag cgggatcgaa cacaagaaga atagaaccat cgttaagctt gtaaatcggg 3420
   cgattgcgga tagtaaggtg ggtcccaata atggtgtgga gggccgaatc aaacgagacg 3480
   ataagaggtc cagaggcagg cttccagttg atcggaagca tatggtggat ctggagctgc 3540
   gctccatccg atccatcgaa ataaaacttg ttgctggcga gcatagaaag atactcacag 3600
   aggtcgactc catcgtagcg ttggtgaata agaggcccgg cctcgacggc catctcgaga 3660
   gcagagaggg agctcatatt gaagttaatg aaaataaaat gagttggaaa atatgccgtg 3720
   ataggagcac taccagtagg aaggttgaat gttgcgatgt tgaatttgtt gttggtcgtg 3780
   ttggttgttg gtggaagaaa gaagagggac agttgggatc tcttatatag actactggtc 3840
   acgaaggggg tgcgcccaaa caagtctgac cctaaaaaga ggtcaggtac aaattcaaca 3900
   agccctacaa gggtctaagc atagtattat cgtgaaaata tcatagtatg tatggtgagc 3960
   tttccactag ggcagtgaga gaacccttag gtttctaaga ttcttggcga aaatatcaac 4020
   gaatgttttc cttcttgcag ccaaggacgc tcatgaggaa aacaagtcga gatggttaat 4080
   gatgatcggt ttaatattcg ggaggaactt cgaacaaagg aattggtgaa acaggatgaa 4140
   ctgagaaatg cgggttctat tatccatccc caaagtcagt cctgtgcttt gctgctctgg 4200
   taagccccct agaggaataa cctcggacgg aagccgttct ttccattctg agggagtatc 4260
   atatccaata gccaagaagc tgccacgcga aataagaatt gaaaagtagt tttctcccca 4320
   accggtcggg ggctggatga ttcgataata ctaccaaccg atctggtaga tgtccccacg 4380
   ttcagctacc ccttgcgcat ccaggcgaat agaggagggc cgccctaccc gaggcggtgt 4440
   gcgcgggcgc cgagagggcg gtttgaaaga tggaactcca gaacaaagga gcaggtgccc 4500
   gatgcaacaa cagggcagca aaggctctgg agcttaagga attgtgacta gagctgttgg 4560
   cggttgcacg tgataatttc ggaagttgtt ctggctagac cgtgctgata ttagtgggat 4620
   catagtacca tagcacaatt gtgagtgggt aaactgagca cccctgagaa ggattttcag 4680
   cagagaaatt agaatcccca accagttcta aaccgtcaga gccccttgcc cagggccagc 4740
   agccagctga aggagccgct gtgagaatgt ttgaacacca acctccaaag cttcatacaa 4800
   gggaggatga cggaaggagg gtaggattta aaaggagcgc gctaagtctc ttgattctgg 4860
   atgaagatat cgccagatag gagaaccaga agcaatgtgc ccgacgcccc cgcttagacc 4920
   accaggatag aaatcgaaat ttcaagctaa ttatatggag gaaagaagct tgatcggctg 4980
   tggaagagtg agccgtatga ccagagacag gacactgcgc ctttatatat gcagtctaag 5040
   ggacatctgc gaaggtgcta gattatccgg ttccagcact ctagtcctgt tacggaacga 5100
   tattagtcat taagcactgc tagctgcccc tcaatctggc cttagtgtgc tgaaaccgaa 5160
   ttttactatt tcacaccgcc tgggatagca gactgtctcc catcactatg cggaggctgc 5220
   atatcaagcg gatattagag cccctcgctg ccctttttgc tatttggatg ctataccgga 5280
   aaaggaaaag tcttcaaatc ttttcccaga cccttccgaa gtaacgaaga atctgaactc 5340
   ctcctttagc tcgcggccta tagcactcta ctgtttccgg gctttcccgt caggtgttct 5400
   tttgcgggca gttggcaatt gggtttgctg agcttccggt gaaggtagcc agcagatgaa 5460
   agtgaaagaa agcgaagaga gataactgaa gaggatgaag atgccttgat ggtcgtgatt 5520
   gagatattct caagtcacga agcaaggacg gatgaagaat gatggaatgc gttctttcag 5580
   aagaagaaag atcccacgat gctcattgtt tctaggagga gttgtttcct cgtttaaata 5640
   acatgaggac tgctaactga gctacctagt tctttattgc gtaaaagcat acgcaatgtt 5700
   caccaaagtc tatgaaaaac tcgctgagcc atccggacgc acgacactac aatacttttg 5760
   tttagtgcgt cactactaaa acgggagtat cttcaatccg gcgacttgag tactctttct 5820
   ttcccacaac cgcccatcct cgacacttct tctacaacac cgtaagaact cgaacaattg 5880
   aacctcaagc taggatgttg gaattgcctc tgctggtaag gtctggctct ttttcttttt 5940
   tgtctgtctt gagctcaggc tgaggaggtc agttttccct gagcactcgc ccccctattt 6000
   aagagaggaa acttgggacg tctttgcgcg aggtcaatta ttcgagttct tacggcagtt 6060
   tgtttacttg gaggctatgg cgaggggatg gtttctgctc gcataaaaca tacacagcgc 6120
   aagtatccaa tgttctcaag ttacataatt aagcgggtgt aaaggcaggg gcccgaattc 6180
   atccggtgaa ttgagcttcg ctaagttgtt ggtgctctgg tttatgaagt ttgcgaagtt 6240
   tgttcttcta gcgagaaaaa gaaaaccact ggggaagtac tttgcgatca ccagctggcg 6300
   gccatcgcta ttggatatcg ccatctgacg tatcacaggt cgtgttaaag agatgcctat 6360
   atgtatatga cacccctagg agtcttgagt accttactta agagatctgc ttatcccgca 6420
   aagaaagtca agcaagtcac ccccaccact cgtcccttat acattttgtc tcccattcca 6480
   ccttgatttg cagcggtcac gcgaaatcga acatcgattg acacccagcg aaagcatcta 6540
   tcaacaccgc ctagcactga aggcatcaca aattatgaga tgaaaaggaa agcgtaattc 6600
   ttgaatttta cttaaacacc aaagctactt cgctaatgat gcctagccac cacaaataaa 6660
   agaaacccct agactccctc ccctgccctt agagcacttg tagaaacgat gcgtcaagct 6720
   cgccaaagag atgtcacgag ttttaatgta tggtattggt ccaaaagggt tgtaattcta 6780
   gacgctctaa ccatctaccg taaaacctca aataaaagaa ccgtgtgcta tgtagtcgag 6840
   aattacccac ttgagaaaag gggctaaaaa cacagcaaaa actgagctgc tcaggtcgcc 6900
   tgagagtcat aaaaaaacaa aaaaaacacc agaccttacc agaggaggca attactcgga 6960
   tagggtcaaa aagggcggaa aactttcggt ggcgaaacaa ggatagaggc gctaatcgat 7020
   caggaggaag tagaaaaaat ggggggtgtt ataggatgcc attaacctgg agatgttgca 7080
   gtgtagtcac cattgtattc catctatggg tcttattaag aatctgatca tataaggcca 7140
   aattaatacc aaaggagaag gaatcagggc ttttggaata gttctttaag agactcatat 7200
   aaatgcatag gttcaattgc acattggcaa tttcatcatc atttaaatgg tcaagtatca 7260
   tactagnata cttatttctt caacctggat ttctggtgtc cgccaaggca agaatttttc 7320
   cactgttgca acctacttcc taactacccc acctgatcct gctgatcttt ttttccgccc 7380
   tttttgaccc tatccgagta attgcctcct ctggtaaggt ctggtgtttt ttttgttttt 7440
   ttattgtact tctcaggcga cctgagcagc tcagtttttg ctgtgttttt agcccctttt 7500
   ctcaagtggg taattctcgg actacatagc acacggttct tttatttgag gttttacggt 7560
   attcatctgc ctcaatccca gcccaagttg ctcttgtctt ggccaaaatc ctaggcgtga 7620
   cttgctctaa ttgcttggac accagctagc aagtaaaaaa ttctttagta tgatatcata 7680
   ttagcagtgt gggaggtttg cttaggctnt gcgtgacctc cctacgcact gccagggatt 7740
   agtagcagaa cgagccaaca ttaccagcgc ggctgataga aaacatagtc acgtgagttt 7800
   gataaggcgc ttaacagcaa gaaggttcac caaatttttt ttattaggtt cacgtgacta 7860
   ttttacctgc cggccgcgcc cgtacagaaa cttcaaggac aaccaagcac ccaaaagagt 7920
   agaaaatctg ccttcctatt taggaaggcc agtaaggtgg ttgggccacg caaaatcaaa 7980
   caaaatgccg agtgataccg cgtgaagctg cccctcagcg ccccaaaacc catacgagtg 8040
   ttacaacatg ataatcctaa tacaacgcca ttacgctatt ctgttgaata tgtcttgtca 8100
   ctacaataaa aaacagctcc gatcctagga ctttatttcg cctggtttgt tcccagcgca 8160
   cttagcgaca agatacgtga ggctctcact taaaaggagt tgcgagttct cttgcgctgt 8220
   attcgcccaa aagtgtaaat tagctccatt caagttgctt gtgatgtttg cttagagcct 8280
   gtttggatac aacagttccg ttccgttccc ccatacaaat ggcccttttc cttccgtttc 8340
   aaaacggaaa gaacgctctt ttcacagtag aaatggtcgc ccgacggaac ggccccattt 8400
   ttgccaaaaa ggttcttaag ctttctagct ctcaccatcg agtatgacag tcaaagacat 8460
   tgttggtcaa tgggagcaga agttcacgtg acctacttag cactcggcga cctgagtaca 8520
   gaaacacgaa agtaacaagt ttacaaacta gacaaaagta caatgccagt ttaaaagtct 8580
   acctgtagga tttgtatgga aattttggca gaagggcact tttgtgggtt cacgcaagct 8640
   ataactacag ttataccttc taaatctcga tacgtctaca gaaattgtta gatctcccac 8700
   ctagaaaaat ataggtctgt ttaaactagt gcccatgccg tgaggccaca gaaaaaaaac 8760
   agggctcccc atacaaaaca catggctaat gaccaaatat atcaccaaac taggctcggg 8820
   tagggtgctc tgtcggggtt gggaatggag aaaaaaaggg gtcagcaaga tcctatgggg 8880
   cagtgagcag acggttcgtg tgagtggaaa ataatgagtt ctggcagatg caaacaattc 8940
   tgagtggata ctaaacacat tatcttaccg acaataagag atcccgagcg gatgattaag 9000
   aattaccctt agtgaaaact agctttatag agattacttg aagctccctg aagccctcca 9060
   gaatatctga gttggagttt aaagcagaca ctaatatact tggtccgggc ccctaatttc 9120
   tacaactccc tctttgatgg ggcccggcta gcaattttcg ttttaattta aattcacaac 9180
   tctctagcat aggagggaga aaatatgaag taaaactgcg ggaaatctta tagtatcata 9240
   ccagagatgc acaaatattt aataaaacta tctaaagaca caacccaggt ggtagtaaac 9300
   tccggcaccc cccaactgat agaggatagt tatcaagggc tactgggaat agcagaattg 9360
   aacgaccccg gccccaacag g 9381
<210> 55
   <211> 1255
   <212> DNA
   <213> Tuber borchii
<400> 55
   atgagctccc tctctgctct cgagatggcc gtcgaggccg ggcctcttat tcaccaacgc      60
   tacgatggag tcgacctctg tgagtatctt tctatgctcg ccagcaacaa gttttatttc     120
   gatggatcgg atggagcgca gctccagatc caccatatgc ttccgatcaa ctggaagcct     180
   gcctctggac ctcttatcgt ctcgtttgat tcggccctcc acaccattat tgggacccac     240
   cttactatcc gcaatcgccc gatttacaag cttaacgatg gttctattct tcttgtgttc     300
   gatcccgcta ctaccgacat gaattgcaac gtcaccgatc attccttcga ggatccccag     360
   cacgagatta ccggcgaaaa ccgttttttg cgtcttgctg tcaacgatca agactttgcc    420
   tctcatggcg ctaatcttag tttcatccgc gataagggtg ttaagcgccc cgctaatgcg     480
   taagtacaat cctactacag tcgtgccccg ctataatgga ccgcttataa gggaaacctc     540
   gctattacga atgaaccctc tgggaattta cactcctgct cctatgcggg tggcccttgc     600
   tataaggaat ttcttccaaa acctcgctat aactaacaaa attcccaatt tttggccttc     660
   gttatagcgg ggtgcgactg taacgtaccg atttcatgtg ctgatacaat catagattca     720
   tcttgtttag aacggagaat gttaatgcgg tcaaggctca gtatcctggg gttggtaaca     780
   acgatatttg tatgtggttc tcattctccc ctttttcact ctccgctctt agtaccattt     840
   ccctaatagt ttcaatagca aaaattctcg gtcgcatgtg gcaggattcc ccggacgaag     900
   ccaaagatgt ttataaagta taacatctca cttgactgtt tgttagttgg aatcactaac     960
   acattaatag gcaagggccc gccaactcgc ggcttctcac aagttggtca atcccaacta    1020
   taaatacacc ccgcgccgct ctcatgaaat tatgaggagg gcgtgcgcca acaagggccc    1080
   ttcgacccgt gccgaccttc accgtgctgg ggtcgcgaag cgtgcgaaga agagagcttc    1140
   tacccatctt ggagccctca ccgaccgctt cgcctcccag cccagcgcaa acgccttggg    1200
   cgatattatc gaggaagcgg agagggttct gaaatctttc ggtttgatta attga         1255
<210> 56
   <211> 14624
   <212> DNA
   <213> Tuber borchii
<220>
   <221> misc_feature
   <222> (358)..(358)
   <223> n is a, c, g, or t
<220>
   <221> misc_feature
   <222> (1562)..(1562)
   <223> n is a, c, g, or t
<220>
   <221> misc_feature
   <222> (1565)..(1565)
   <223> n is a, c, g, or t
<220>
   <221> misc_feature
   <222> (2304)..(2305)
   <223> n is a, c, g, or t
<400> 56
   agactctcgt ccatggcatc cggrgtctcr ycctcgctca gtctagyacg aargtatctr      60
   atratatcat ctttkctggg gcctatagak asrcttatca cymgtccggs ragacrcttt     120
   tcgryctcwg ccsgaatatg kggccttcsg styayraata ttygtgtkcc sggggacttc     180
   tcaaggatty gtttywsyga atcaaggakc ttraytckgt gsactrcyrc acattcgtcm     240
   agggcgtcaa tgcacatraa trtgsgctgy gaggasgtga trgcctgcaa catsttcacg     300
   atatcaggaa gtcgcrgtgy cckyccaycc atrgcctttt yctsctcttg aaaggmtngt     360
   gatatttcct cagggatcct ttccattccg ctaacgacct gtttcagcaa ggaacccagc     420
   acactcattg cagactgctc ctttcgggcc gcgaagtcaa agtagaaaca tgtaacggcg     480
   gtattttttt cgacggtctg atcgcttagc ttatccatta ccagcgaact gacatcgtga     540
   cctgtcagca ccccgcgctc tctttgcttc ccggagaata gtctttgtta ccttataaac     600
   gtcttgccga cccccggacc tccatagcaa aacaagactg ctttatcgcc ttcgccttcg     660
   ccactacagt cataccagct tctgaattct tctgtttgca tgagccactc ccccacattg     720
   tcgacacgcc gctcttgaac atcccgatgc cgtgacctag gcgacagggg cgacagccag     780
   cttaggagtt gagactgatc atcggcaata gtacagttgt tccacacctt attataagag     840
   ttcgtggtat tgaggcagtt ggtgctatga ctgatggtat attgagacat cttccgttct     900
   ttgctgcttg gggtgttgta grggaggagt tatcgagtat cgcctgttgt cgacattccc     960
   gggagccgaa gaaaagtggg gtctagcgaa agaaaaagcg ggtctgctgg cgcttaagca    1020
   acgttgcaga tacccctaca taggtttgtg cgtttgtttt tttcaacatg cccttaccat    1080
   tctcaaccaa taccaacttt atcataaaac ggagttcaca aggctaaaga ggaaccratt 1140
   ttaaaacagt tttgtcctag gtttttctcc gaggactttg ttgttggggc cagttagtta 1200
   ttagactggg ttgcggcgag agtcgatttc aagcgccctg caccgcagag catcgaagaa 1260
   tagcccaatt aagctgaggc gtgaaaccaa tacctgcatg aaagtggtta aaattttgtg 1320
   gcgcagtgta gcacaaaatg acaggacaga taagatagtc tggcacctaa ctagtcctat 1380
   gactttagtc tacactacgc cacaaaatat taatcacttt catgcacccc cgagcacagc 1440
   actagtacag tactagtact tgtacttggg tgtgagggta agtggtcgga aagtaaccca 1500
   attctcggtg caaattctaa gggatacagg aaggcattga gggagattga agataatttg 1560
   anaanaagat agtgggggag agaaaggaga taaagaagga aaggagggat ggagcaaaca 1620
   tctaaattgg atatataaag cctgtagtag tataaatata ctcgaaaagt tttaaaaaaa 1680
   aaaaaaaagt gatcggaaaa aaaaagtggt ttttccgatc actttttgct ccaggctggt 1740
   actcgggtat ggatgtaaaa atcgagttct gtaagtaacc ctgtcgttag aatgaggtac 1800
   tgagttggct agaatagaag aaaagatcgg caagtcaggg tcaccaagta cccggaccaa 1860
   ggctgggcag atgcaacatg gcagtgatgg tcctttcttt ttctttttct gctttctata 1920
   tttgagtctg taaagtttga tagggaattg agctattagg ggtttggaat ttagaagact 1980
   ttcaataaga gcagtaacaa ctacaagcag cttccgcaat gattaagatg caaccayycc 2040
   aggtttttag ctccttycaa ggtctagctt tttccatgac gccaattgca cgattcccga 2100
   gattccttgt tcatatccct gctktgctcg atttttacat ccctacccga gtactagggg 2160
   ctttcaaagt caggaatggg ggccaacggt aaaatgatcg aacactcaaa gccacgcatt 2220
   agttctgcgt tagggcgaca tattaactgt agccaactgt atcaaagcat ttcaagtgac 2280
   ataatagccg gtcccctcct attnnatcgc gcatagtsgg aaaagcaagt gtgacctaca 2340
   ctgtaaaaaa aaaagccgac gtgtcacacc tgcttgagcg tcacacttgc ttgagcgtca 2400
   cacttgcttt tcccgactat gagcgattgc gaaataggag gggaaccggc tattagtatc 2460
   attctaaatt atttcggttt ttggggtaga gaaacggtga acaaaagaga caggatcgca 2520
   cgtacagtaa gaatgattac aggtgaatca cgaggctagg ggagtaattt cagttagtcg 2580
   cagcgttaaa cccccctacc aggttcatgg ttcctcccat gcagtctacg aaatttaatg 2640
   gaagagaccc cctgctgtgt ctacattgga gaaaacctca ggtgaaaaaa atcaaacgca 2700
   aacaaatact gacaaaggtc atacaagtga aagcttcctc gcctccttac taaaaaaggt 2760
   ggaaaaatac cccaccttta agcgtgatca atagcgcgag tcacagccca ccaacaggcc 2820
   tctcgaacag atgctgatta gtaggccccc tgtgaggaaa cgggtagtag tcagcacagc 2880
   agcaacagtt cgtccaagta aaccacttga aagtttctta tttaggttta actgttcgag 2940
   tagaggacct gctcgagtga actatgaggc ctaatcgtag gatttaaaag taccagctat 3000
   gttatcccaa ataggaaggc agatcttcta ctcaagtacg gtacaagtag tattgtattt 3060
   gtatcccact ctcattccta caacctccag tgggcaggga tgtcaagcaa aatctaagca 3120
   aacatcacaa gcaacttgaa tggagttaat ttacactttt gagcgaatac agcgcaagag 3180
   aactcgcaac tcctttttag cgagagtttc acgcatctta tcgctaagcg cgctaggaaa 3240
   aaatcaggcg aaataaggtc ttgggatcgg agcttttttt tattgtagtg acaggtcttc 3300
   aatagaatag cctaatgacg ttgtttaaac gagatctgtt aagatgatca tgttgtaaca 3360
   ctggtgtggg ttttgggggc tgaggggtag cttcacccgg tatcactctg tattttgttt 3420
   gattttgcgt gacccaacca ccttactgac cttcctaaat aggaaggcag gcttttctac 3480
   tctttttggt gcttggctgt ccttgaagtt tctgtaatac tgctggctcg ttccgctact 3540
   aatccttggc agtgcgtagg gatgtcacac agagcttaag caaacctcct acacttctaa 3600
   tatgatacta aagaattttt acttgctaaa gctgctgtct aagtaattag ggtgtttaag 3660
   taaaatttaa gaattacgct tccctttcat ctcacaattt gtgatgcccc cagctctagg 3720
   gtgttggatg ttttcgttgg gtgtcaatcg atgtttgatt tcgcgtgacc gctgcaaatc 3780
   aaggtggaat gggagacaaa aagtatatgg gacgagtgtg gaggtgactt gcttgacttt 3840
   cctttgcggg ataagcagat cttttatacg gtatgtaaaa ctcctagggg tatcatacat 3900
   ataggcatct ctttagtgat gcgctggatg gcgagatcca atagcgatgg ccaccagcta 3960
   gtgatcgcaa agtacttccc cagtgttttt cttttttctc gctagaacaa acttcacaag 4020
   cttcataaac cagagcgcca acaacttagc gaagctcaat tcaccggatg aatttaggcc 4080
   tctgcctttg cgccggctca attatgtaat ttgagaacat tagatactcg cgctgtgtat 4140
   gttttatgcg agcagaaatc atcccctcgt catagccccc aagtaaacaa actgtcgtaa 4200
   gaactcgaat aactgacctc ccgcaaagac gtcccaaatt tcctctcttg gatagggggg 4260
   cgagcgcaca gcgccaactg gcctcctcag cctgagctta agacagaata aaaagaaaaa 4320
   gtaccagacc ttcccagcag aggcaattca aacatcctag cgtgaggttc aattgttcga 4380
   gtccttatgg tgctgtaaaa gaagtgtcgc gggtgggcgg ttgtgggaaa gaaagagtac 4440
   tcaagtcgcc ggattgaaga tgctcccgtc ttagtagtga cgcactaaaa aaacgtatct 4500
   tcaatccagc gacgcatact gtagagtgct ataggccgca agttaaagga ggagtttaga 4560
   ttctttgttc cttcggaatg gtctaggaaa ggatttgaag acttttcctt ttcaagtata 4620
   gcatccaaat agcaaaaagg gcagcgagga gattggccaa ggcagctcta aaatccgctt 4680
   gatatgcagc ccccgcatag ttgtgggaga ctgtctgcta taatctacga gtagaacttt 4740
   cccagatatt cctaagacta catatataaa ggcgcagtat cctgtttctg gtcatacgac 4800
   tcactcttct gcaactgatc aaacttcttt cctccatata attagcttga aatctcggtt 4860
   tctatcctgg tggtccaagc gggggcgtcg ggcacattgc ttctggctct cctatctggc 4920
   gatatcttca tccagaatca ggagacttag tgcgcccctt tcaaatccta ccctccttcc 4980
   gtcatcctcc ctggtatgaa tctttggagg ttggtcaaac actctcacag cggctctttc 5040
   aactggccgc tggccctggg caagaggctc tgacggttta gaaccggtta gggattctaa 5100
   tttctctgcc gaaaatcctt cttaggggcg ttcactcact cacaatccaa ggcttcgcct 5160
   tgtgctatga tactatgatc ccaccaatct tagcatggtc taaccagaag gccctctgaa 5220
   actatcacgt gcaaccggca acagcgctag ccacaattcc ttaatctcca gacctttcgc 5280
   tgtccggttg ttgcatcggg cacctgctcc tttgttctgg agttccatct ttcaaatcgc 5340
   gctctctgcg cccgcacacg ccgcctcaaa tagggcagcc cttctgacct attcgcctgg 5400
   atgcgcaagg agtagctgaa cgtggggaca tatagcggat cggttggtag tgttatcgaa 5460
   tcattcagcc cccgaccggt tggggagaaa actacttttg aattcttatt tcgcgtgaca 5520
   gcttcttggt tattggatat tccctcagaa tggaaaaaac ggcttccgaa gtttgagata 5580
   tgatattccg gtctgccata ccgtattgtg gaggttattc ctattacatt tccaattagc 5640
   aaaatatcct aatttatata tcgaatcaca actaattatt cctctagggg gctctaccaa 5700
   agtagtaaag tacaggactg acttggggga cggataatag aacccgcatt tctcagttca 5760
   tcctgtttca ctaattcctt tgttcgaagc tcctcctgaa tattgaacca tcattaacta 5820
   tcccgacttg ttttccgcat gagcatcctt ggccgcaaga aggaaaacat tggttgatat 5880
   tttcgccgag aatcttagaa acctaagggc tctctccctg ccctagtgga aagctcacca 5940
   tacatactat gatattttca cgataatact atgctcagac cctagtaggg cttgctggag 6000
   ttgtacgtga cttcttctta gggtcaccct tttttgggcg cacccacccc cttcgtgact 6060
   agtagtctat ataagagatc ccaactgccc ctcttctctc ttccatcaac aaccactcag 6120
   cagaaacaac atccatacag caatcctccc tccgccggtt gatttttcaa attcatcttt 6180
   cctccccatt tttatctttc atcatgtcag cgagaccggt caatctcact atggtggtca 6240
   ctacctcccc gcctccaaag ctctttgagt gggagaacaa ggttcagagg cgcctcgtta 6300
   tgcgtaatgc cactttgggt tgcgccttgt atctctcgga ccgtgggtat atcctcatgc 6360
   aggacagaat cggttgtatm tgggtccagc acgaaaactc ccccgtcatt gttcaacctc 6420
   cgggtggtat tacttttaac gtcagtggtg agctcttgcc ttacgacaag tggctcggtg 6480
   caatccagtc aaccgtccgc catcaggggc gagttcccct cgatcctgaa gtcccctatg 6540
   ttgtttctgt tctacgtctt tattgcggat actatcagcc cggacaaccg tggacggccc 6600
   aagatagcgc tatccccgcc gccatcatca acggtgatca gacccttgct cgtgttccac 6660
   gttctcagca attacgtgct ttgaatccct atgttgcgca gagatgtaag taaatcacac 6720
   tcctacagtc cctaggcaaa agtgttcgtg aatttgctta gtcagatgtc tcacattttc 6780
   acgaacactt gctgtatttt gtccctggta ctaattaaat ctccatccat cagcttggat 6840
   ctctaaatac tgcaacggtt atgggctcac acaggctgaa atttccaatc ttactcgcga 6900
   agtctgggtt gcggagccca acaagtatat gtggcaaaac atcgcctctc tttatactgc 6960
   tgctagggat cgcagcgagc ctggtcttgt gctagaagac ttcattgaga ctgaattggc 7020
   gaaacacggc caccctagta ccccagaaaa gctgctccgt gaagctgggt ttgtccccaa 7080
   gactccaatc gacaaggaag ccgccgataa ggctgccgca agaaggaaag ctcgcgccac 7140
   gcccaaattg tctaacttcg ctctcacccg cgtttacttg ggtaacgacc accagctcag 7200
   ccttaccgag gatgaagctg ccgatattct tcgggctttc gatggatatg aaccgaactt 7260
   gggtgacgga cactcgcaaa tcggccctca tgtccagcac ttgctggcga tggaaatgca 7320
   agacaagttt acccacactt ccgttggttc caatggcttc gtgagtgata ccgtgaatag 7380
   tatgatttac ttgttgctca attctaacat ttacccgaac agcatactat gggagaatcc 7440
   tccacaggat tgtaaccgga tatgatcccc ttggagatct gaggaggctg caggtgcgaa 7500
   aaaaaaaaaa tggtggaaaa actgtgtacg acgggaagca gggtatagct agaaggcata 7560
   ctcggcgggt tatggatcaa tgtagactta aattaagctt atctgtctgg gaccaataga 7620
   aagcccttag taactttaaa tccagatatc aatgctttct tccataaatc agcgttcgcg 7680
   acttcgatgt actaggccgt ttttttttta agtattcgga tatggaaaat ataagtaatc 7740
   tagtcatggt ataccggtac agtaagagct tgagcaatta aaccgtgtgc gaggtagtcc 7800
   aaaatttcct cctctagaaa atgagagaaa aacacactaa aaaacctgcc ctccgagaca 7860
   aataaaaaga aaaataacac cacaccttac taggagagcc aactccaaca tcccagcaca 7920
   tagttcaatt accggatgcc agtgggcttt caatgccccg aatgcatcgt ctaacggtat 7980
   tccagcatcg cgagattctg gcactgtaag tacaagtaca gaagaaagac accatgatag 8040
   tcaagcgctc gcaagcggcc aggtcggcct gcaaatagcc ggtaggcttt cagtccggcc 8100
   aggccaaagt gagacagacc tgggcttcct aggccaggct ttgggaaagc cggcaggctt 8160
   catgggtccc tggcggcacc ggtaccgtaa aaacttgaat aattggacgg tgtgctatgt 8220
   cgctgggttt tcctctttca gtaaggtcaa gaaattttcg tttttttttc cgtcccggaa 8280
   ggggaaaggg gctgtgtttt tgctgtgcgt tttgctccct ttccagagaa gggaattctg 8340
   gactacatag cagaccgtcc aattattcaa gtttttacgg taggctttcg gtatattcca 8400
   tgtgactagt caatattttc gtaatgtgtg ggtgatatct gcagatattt gaagataccg 8460
   ccagatatct gaagatgccg ccagatatct gaaggtgcct gtaggtttct gcttattatt 8520
   caggcttggg ccacaccatt cgatttaaaa catattctgc ttggcttcag atcttctaga 8580
   gtttagcgag aaagtagtat cttatttttc attcactttc accctttttc tcctaattga 8640
   caaactaact actgtaacta gcggtaaaga cagaagcaca taaacttgaa cgaagtcgtc 8700
   ctcccattct cccatccatc gcaccacata tcctttctct gcgacctgta tacccctctc 8760
   cctttcatgg acacctaaac taagttagca aaagaaccga catgcctttc aaagaactcc 8820
   cacggctcat aacctccacg ccgagcgtcc aaccttcagc acgtctgact ccttcgagat 8880
   cgtactgcat ctggcggtgc gtccatccaa tttggcaata cagctcggag tagacgcggt 8940
   cggccccagc gcacctggtg ggggcggccg ggggttgttc tcatataccg cccggggtgg 9000
   gcgtgaaggg atagctctgg gtataggagg atgctaccca cgggagtgaa agcgcgactt 9060
   agaggaaaac aattgcatgg cagagcgaga aagacacagg ggaaaggtag cactagaaga 9120
   gtgcaattac gaatttgaat gagtaggatg taaggaggcc gctatgcgtt gtgagagttt 9180
   tcaatgaact ttgttagcac aagagtctgt aggggggaat gggagacagg ggcaagaggg 9240
   agagaacgcg tgcaatagtc cctagagcac tggttggtag atgggaagct cgtggtttat 9300
   gtgtactcat gtctttacct ctaattagtt gatttattta ttgaataagg gcatacaagc 9360
   aggagtcaag ttgaatcaga agaactttcc cccaattgtg ctgtgtgttg gggatgaatc 9420
   ttccatgctt ggtgttggtt tgtgcttggt gtttcgtttg tgcttggtgt ttcgtttcat 9480
   tctccagctg aatcacatct tatcactcga aagcctgcag atatctgcag tccggccagg 9540
   cccggtcaga tgagcttcag aagccgggct ggccgcttgc gaacactggt ctcactgggg 9600
   cccccagact aatgcgaaca ccgcctagag tatgataccc aatgccagac gctctgccac 9660
   ccggggggtc actaaggggc tgtttggagt cacaactttg gggccgcaaa ccattgtttg 9720
   taactacagg tcttatttgg cagatgggaa ttacaaacaa tggtttacaa caaccagcag 9780
   caattttttt gctgtttatg aaatagggca cagggttctt tcaaccatgg tttgtatcat 9840
   agttaaccaa acttcacaaa gttgtcacgc cctaactctc tacccaaccc tatcattgcc 9900
   ccacacaaaa aaccgcaaaa ccaaatctaa ttgaagggcg tcaccaccta taaaaatccg 9960
   aggcctcaca ctccctcata atcctatatc ccccctgtac caaaactctc ttcttctcaa 10020
   cacccactcc agagtactca acctcacaac accctccgca tattggtcag ccagtctgcc 10080
   aatatcagta ttcgacttac agttgacctc tccacacata cgcttgtttt ctttgctaaa 10140
   atgcccccaa gtatcccccc ggctgaaaaa agaaatgaac ttaataataa taattaacac 10200
   aatcatagac gaggacatca ggctccgccg ggaggccctg tttcgggccg gaaggttgct 10260
   tgctaacgaa atggaagcca ccgcctggaa ccctggcaac ttaatggttg gaatggccat 10320
   tcataagaac ccaagcatta gcacggttaa agctgttgag tcggcccgag acttgcttcg 10380
   tgaagtcact gtgagtgaca atagattacg caagatggat cccatcaatg atctccaacc 10440
   gctggcgaat atccataaca cacttttaca gattggagga ttagtttcct tgaaacaacg 10500
   cctctatgag gcacttcaag atatcaagct gcgcctccag gaactccaaa gatatacggg 10560
   agacaacatt ggccaggcag ctgcagaagt tcatgacttc atctgtttcg tgacttttgt 10620
   caacttgatc catccgcaac aatgcatatt aacctcggaa tccatcaagg atgccgagat 10680
   cattgcctcc aagattccgt tttttgaggc tcgtcaggcc tttgtcgaac tccgccaagc 10740
   ttaccaactg cagactcctg ttccggggta tcgtctaagc aaagccaggc agttgcctgc 10800
   gatcactaga tgcattatgg agcagtggta taaagattac ctttctcacg cggccgcaac 10860
   tggaacagta agcaaaagaa taccttttct agatcagcgc tcgtacaatg tttccctacg 10920
   tcgcgagcac ccggaaagga gagcactggc tacaaaatgc tttgaactac ggtttaaaac 10980
   aataatataa gcacgaaaga caagccggta agtgttgagt gctcgctacg ccgggggctc 11040
   gcaaagtagg gaaacatagt atctaataag ccccaccccg cctcatactg acattaacgt 11100
   atagaaaatt atcccattcc agatcgtgtt cggatggtgg agtcagttca attcccttat 11160
   tcaggtttgc tagcactacc agctgacaat aaacgatctt gggttatagt taattaccaa 11220
   cgcaagttta ttagtagcgc tcaagagaaa actactccga tcgtgcaaga acttatcgac 11280
   caagcctata tctcgaaggc caagattcca gaggatgaag acgagcaacc atggatgcag 11340
   gaagataagg aagcttttaa ggccgccact tctctggcta tgtctatgac caaactatgc 11400
   aaaaatatcc ctctacctaa gcatgttaaa ccagaccctc agagagaaga acaattgcca 11460
   attcacaccc agttcaatgc ccgtcctcaa acccccccga tcacccgaga gctccagaag 11520
   ctgctggctc aaatttatgg cctttttgaa ggaaattcgt gcgcagcttt tactatcatg 11580
   accaaagcgt attataccaa agaggaaaac tattttagat tctcggccca ggaccagaaa 11640
   acgtcttctg tcaagcactt gaagtgagtt attgcccaag tctcaagatt tgttctacat 11700
   caaagctaat tttcaacagc gccgcgacct actttttcaa ttcgctccgc caggttaggg 11760
   ttgtggttct gaaggatcag tctggcagga gtgctgggga aggcaaggtt gaggttccgc 11820
   tccttgacgc agttagtgcg gtctgtcggg accccaccat cacagacgaa gagcatctgg 11880
   cagccattac ctgcaagagt ttaccacaag ttggcatgca ttgtaggttc ccattatcac 11940
   tagtttcgga tatgtgctaa tctcagaatg taatcctggc aatagtcctt gttagatatc 12000
   gcgggcccgt tggccggggc cgttaaattt tactattccc agtagcactt gacaacgtcc 12060
   tctatcaatt aaaatgtgcc aaagtctact actatccgag ctatgtcttt agataatctg 12120
   aaatctttgt actcacttgt gaattcaaat gagagctcaa aggaagcagt gaaaccatct 12180
   gtaagggaat catctaggtc agccccttag actagtggtg ttatagaacg ttagagtttg 12240
   gagaaataga catttccaat catattcata ctcttgctct agcccgtggg ccttcgtggg 12300
   ccgtgtcatg ctgtagagga ccggcccacg gcaggcactg gcaatgaaga cagcaaaacc 12360
   tcccatatga gacaaaatcc aggatatatt actacgagga ctacaaaaaa aaaaaacgcc 12420
   ctctagagcc agctttgtct atccacctac ctaccaataa cgcccccaac atccgggtcc 12480
   tccctcaatg cggccttaac ctcctcctca ccaatcaaaa acctaattct ccccccgccg 12540
   ccgccccgct tctccacact gagaactcca gattcaacca accccttgac cgcggcttct 12600
   attccgccaa ctcttggccc accgccaagc tctttttccg caacgccctg atttcgaacg 12660
   atcctttccg cttcatcaag gacatcacgg agaacacctt cgccgactcc ggagcgacgg 12720
   gtacggagta atagagccgt gatgaagatc ttggaggaaa gggaagtggt tttcaggaac 12780
   acttgcaaag gggaagaggt agcttcctgg attgcctgtt taacaacggc aatggttact 12840
   ttcccaccgg ctttgaggcc gccgccgcgc agtggggaga cgcccccgcc cttccgcctg 12900
   gacggcgaag acggtacttg ctcgtctcct ccctcgttgg aggagtattc gacaatctcg 12960
   actgcgcgcc tgcatatatc tagggctctt ctagcatcgc cagatacagc agcgactttt 13020
   ctactagcga actgaacggc gtctgggtcc acaatgttac caggtacacc gctgaggcgg 13080
   gactgaatta tcgtcattag ctgcgaatgg gtgtagccag gaaatgtaat cctagtaaga 13140
   cctgttaaac aatttagctg catttctcaa gagttaaggg tgtctgtgcc aacctaatct 13200
   cgacgagatc ttattgctca aggttctctc gggcaagtcc atggtattcg caaccgccaa 13260
   aacaataagc ctagaatacc gtcccccggg ccaattgaag aaattgtaca tgactccttg 13320
   gccccgcgta actaattgat caagttcgtc catcaagacg acacaaggaa cccttcgagg 13380
   actgggggtg ctgaactctg actccaaaag tcccaaggct tgtgaggggc ttacacgatc 13440
   acctttaatc gcctcccaaa gaagagagta cgcctgatgg ggatccgtga ccttcattcc 13500
   attgatctca acaaaggtaa agtcgtccaa ttcttccaag gtcactaagt actgcagttg 13560
   tgaaataacc tcacgaactg tagcagtttt tccagttcca ggagttccgg agatgtaaat 13620
   acacgatccg gttccctcgg caatagcagc agaaaggtga gtgtacacct ctgtaaactc 13680
   attctcacga caaggcaaag aaggtggaag ggtagacacg tgcagccggt ggcgtgcaag 13740
   tgcataaggg gttgacaagt gagtacctgg ggacagtgta cgagttgaaa gaggagtaaa 13800
   gagcagaggt ttcttcacca ctattctaga gagatgttag caattataca gaccaatcga 13860
   cagattgtct aacctcttat gtgtaggagt actgtatttc ttcgagacgc ttccaggagt 13920
   ccgtctagtt ggagtagtaa gagcagatgt cttccttttc ttacttggag tgcggggagc 13980
   tttagtccgt cgactaaaat tcgggtcatc ctccaccaca tcgtccaact ctgcaatttc 14040
   accctcatcc aacatatccc gatcccattc ctgatctgtc tcgtgatcct catcacttgc 14100
   tttgcgcctt ttcggtctcc gtacgccttg cccgacagat ttgttccctc tccgcttaac 14160
   caccttagtc tcattataca ccatctcccc aatggcggca atatcagtgt ccctcccttt 14220
   ccaaaattcc tcccaaataa actcaggtgt ataagttgca gttctaggat tacacccccg 14280
   tcggcaaaca aaaaccttcc catactcctt ggatttcttc tgcacgccgt tgggatattc 14340
   ttcgttatat ttctgaaggg aaagaacctt tgctcggcca tttatcgacg gcagagggat 14400
   caggttatag gaggggttaa cgtaaagttc attctattcc caaagtcagc acactgaacg 14460
   tgtaaaagaa acaagcaaac aaaccggtaa ataatccatc cgcttctttt ccttattctt 14520
   aacctcgaac tcagtggaaa accacatgac atttaccatc atttcaaccc cacccccatc 14580
   ctcaatcttt ccctcttcgt caaactcaca tatcatcccg acat 14624
<210> 57
   <211> 11180
   <212> DNA
   <213> Tuber borchii
<220>
   <221> misc_feature
   <222> (8629)..(8629)
   <223> n is a, c, g, or t
<220>
   <221> misc_feature
   <222> (9071)..(9071)
   <223> n is a, c, g, or t
<400> 57
   aagcttgaca gagctcgccc gcttgtaacg gccgctctgc atgactgatc cacattaaag      60
   ctgccatccc gagtaccgat ttattcccac cctgcgcctt tatccgttct atcgtcgctt     120
   cgtaagcatc tcccaacccc aatccatcgg tgattttgct gagcctttct cgcctctgat     180
   cgattgtcgg ttcttgcaag attgcctcaa tatttaagga aacgagaaga aacctagata     240
   tatttgttag cagagaatgt ggggtaggac tcccagtacc attgccctac gtacatttcc     300
   gatatacccc cgggaatcct ctctagaatg tcggcctcta gactctcgtc catagcatct     360
   agagtttcgt cctcgctcag cctagcacga aggtatctaa tgatatcatc tttgttgggg     420
   cctatagaca cacttatcac tcgtccggaa agacgctttt cgatctcagc acaaacatgg     480
   ggccttccgg ttatgaatac acgtgtgccc ggggacttcc ccaggatctg ttccagtgaa     540
   tcaaggattt tgatccgatg gactgccgca cattcgtcca gggcgtcaat gcacataaat     600
   gtgtgttgcg aggaggtgac ggcctgcagc atcttcacga tctctggaag tcccggcgac     660
   tttccaccaa tggccgtttt ctgctcttga aaggctcgtg atatttcttc tggaatcctt     720
   tccattccgc taacgacttg ttttagcaag gaacccagca cactcgtcgc ggaatgctcc     780
   ttgcgggccg cgaagtcgaa ataaaaacat gtaacagtgg ggccttgtcc tttagtctga     840
   tcgcacagcg tgtccactac cagcgaacta acaccgcggc ttgttagcac tgctgctctt     900
   cttccccgaa gaatggtttt tgttacctga taaatgtttt gccgacccct ggatctccat     960
   aacaaagcaa gactgcctta tccccttcac cttctccact acaatcatac cagcttttaa    1020
   attgctcggt ctgtataacc cattccccca cgttatcgac gcggcgctcc tgaacatccc 1080
   gatgccgtga cctgggctct agggatgaca accagcttag gagttgggac cgatcatcga 1140
   caatagtata gttgttccac acattattaa aagagttcgt attattgaag gagttgttac 1200
   tgtcattgat ggtatattga gacatcttcc gctctttacc gcttgcaatg ttgaagagga 1260
   gttatcgagt aacttacggc tggttgatgg tattcctggg aatcgaagaa aagcatggtg 1320
   cagctcaaaa gcggggcttg ctggcgttaa agcagccttg cactgttact catcaggtat 1380
   ggtgccggga cttgaaatca accctcatgg gtaattcatt ttgcttaata atacacacaa 1440
   aatagtgggg cttggcaaaa gtgtcttcaa aaaccatcct acatgggtaa ttcaaaattt 1500
   tactatctcc gctaatattg ctctgattga aaattagaaa acatagaatt agagaggagg 1560
   gtcttctgag cacttctatg aagccatatc ctattaaaga tgtggttgtg gttgaaaact 1620
   ttcgaagatt gttcttgtgt tggtctttgg gtgatttcaa gtgcccggca ccataccgaa 1680
   gaaagtccaa ttgagccgcc gagagtttgt gaaaccgctc ttgctcttgt gtggcactgt 1740
   tttttttttg ttaagtcctt gatttattcg tagctaacta acaggaacat catggcaagc 1800
   accccaaggt atggcctaaa aaccctatat aacaaaagaa acttcttcgt cccctaaata 1860
   acaaataaac gataaaacat cactatacaa tccttaacta aagcaaagct ttcatatgat 1920
   catgaagcca aaaaggcatc tctcgtaaaa ggaaaaaccc ccatcttcac cgggaaattt 1980
   atccttccct ccttcacata agacataaag tcctccatca atttccttcc cacctttccc 2040
   ccttcccaca atttagactc tggtctgatg tcgaaagctc gcgggtcctt attctctctc 2100
   gccctggcca ttgctgggca ttccattata tgcgctggtg tttccaggct cgaaagacac 2160
   ctcccacagg tggtgtcccg atccagattc gaacaatcca tctgagctcc gaggtaactc 2220
   ttgttcgacc tcatttggtg tagccttgac gccatgaact tagggagtgt cataaaaaac 2280
   caacattccc tatatggcgg cctgtatccg tagtaggggg ggtactccca tcctacccat 2340
   ttctgatgca tctctcttct caccctttct ctcgctttga aatgaacctt cttgtgtggc 2400
   actgtactcg tatcatccta gtactagtgc ggccgataga taaaatagtc acgtgggtct 2460
   gttaaggcgc ttgacaccct atatatagtt tatctatcgc gcaggaggga gggaggagga 2520
   aaggaagaac ggaaaacgtg taggtaaaaa ttcacagcta aataaacaac ggagtatgat 2580
   aggatgaaaa ctacaagcta taaactaggc ttcccacagc aataggacca cttcccccca 2640
   gggaccaaac tctactgtga cttgtaatca gacttggaaa tcctcacctg gcataaaagc 2700
   gaatccctag cgagtttcgc gtgtgcatgt gacatatacg tgcattcccc gggcccatct 2760
   catcggattc cctccagctg caaaggatga cgtccatgga caatttctat aagtgacata 2820
   ctccgaaagg gaaggatggc agcgatttcg acgaggcggt gtctcctaca cctcaaactg 2880
   ccccaggggt tgccgctttg cgcgactgta gcagcgctgc cactctgtta tggctgcccc 2940
   tagcagcgaa cgagagcggt gtttggccga aattatttgg tttttcgggg ttaacatctt 3000
   cccgcatgag cggtattttg accacttctt catgcccata ccaagcagcg aatgagagca 3060
   gtgtttggcc accattatcc ggcctgtcga ggttgacccc ttcccatccg agtagtattt 3120
   tcaccacttc ctcatgccca ttccaagcgg cgtacgagag tggtgtcctg ccacgattat 3180
   cttgttcgtc agagttaaca tcttcttgcc tatgccttct gctcgggaat gaatctcggg 3240
   gttttatctg cttcctctat taatgacaag tttaggcgca tttcacaatc caagggagca 3300
   ctactgggct agtagccata tccccaactc tagaagatgg tgaagcacca accaatatgg 3360
   ggtgccaagt taaactggtt tacgagcgat ggataaataa agcggtgtca ccgtgagagg 3420
   tacttttttg tcgcataata taagggcgtg agggcctaag gataattgtc agagtttgcc 3480
   gcgtacggga cgctcgggca ctcagtccat gggtttctta ggctgcctgg cgtagggcgt 3540
   ccgctcagcc aaaaaaatga cggactatga cccacaattc gtcccgccct actgctcaca 3600
   acttgatgtt tatcttatgg tttggtgttc ggatttctca ctctcatttt aagaaatttt 3660
   ttgtttattt ggaccgtaca agtttattta agtgaacaaa tgacccttta caaacagcta 3720
   gatcatccct cccattaaaa gtaaatcacc caagtaaccc ataagctccc cccattgaga 3780
   tcattcaatt aatcaaaccg aaagatttca gaaccctctc cgcttcctcg ataatatcgc 3840
   ccaaggcgtt tgcgctgggc tgggaggcga agcggtcggt gagggctcca agatgggtag 3900
   aagctctctt cttcgcacgc ttcgcgaccc cagcacggtg aaggtcggca cgggtcgaag 3960
   ggcccttgtt ggcgcacgcc ctcctcataa tttcatgaga gcggcgcggg gtgtatttat 4020
   agttgggatt gaccaacttg tgagaagccg cgagttggcg ggcccttgcc tattaatgtg 4080
   ttagtgattc caactaacaa acagtcaagt gagatgttat actttataaa catctttggc 4140
   ttcgtccggg gaatcctgcc acatgcgacc gagaattttt gctattgaaa ctattaggga 4200
   aatggtacta agagcggaga gtgaaaaagg ggagaatgag aaccacatac aaatatcgtt 4260
   gttaccaacc ccaggatact gagccttgac cgcattaaca ttctccgttc taaacaagat 4320
   gaatctatga ttgtatcagc acatgaaatc ggtacgttac agtcgcaccc cgctataacg 4380
   aaggccaaaa attgggaatt ttgttagtta tagcgaggtt ttggaagaaa ttccttatag 4440
   caagggccac ccgcatagga gcaggagtgt aaattcccag agggttcatt cgtaatagcg 4500
   aggtttccct tataagcggt ccattatagc ggggcacgac tgtagtagga ttgtacttac 4560
   gcattagcgg ggcgcttaac acccttatcg cggatgaaac taagattagc gccatgagag 4620
   gcaaagtctt gatcgttgac agcaagacgc aaaaaacggt tttcgccggt aatctcgtgc 4680
   tggggatcct cgaaggaatg atcggtgacg ttgcaattca tgtcggtagt agcgggatcg 4740
   aacacaagaa gaatagaacc atcgttaagc ttgtaaatcg ggcgattgcg gatagtaagg 4800
   tgggtcccaa taatggtgtg gagggccgaa tcaaacgaga cgataagagg tccagaggca 4860
   ggcttccagt tgatcggaag catatggtgg atctggagct gcgctccatc cgatccatcg 4920
   aaataaaact tgttgctggc gagcatagaa agatactcac agaggtcgac tccatcgtag 4980
   cgttggtgaa taagaggccc ggcctcgacg gccatctcga gagcagagag ggagctcata 5040
   ttgaagttaa tgaaaataaa atgagttgga aaatatgccg tgataggagc actaccagta 5100
   ggaaggttga atgttgcgat gttgaatttg ttgttggtcg tgttggttgt tggtggaaga 5160
   aagaagaggg acagttggga tctcttatat agactactgg tcacgaaggg ggtgcgccca 5220
   aacaagtctg accctaaaaa gaggtcaggt acaaattcaa caagccctac aagggtctaa 5280
   gcatagtatt atcgtgaaaa tatcatagta tgtatggtga gctttccact agggcagtga 5340
   gagaaccctt aggtttctaa gattcttggc gaaaatatca acgaatgttt tccttcttgc 5400
   agccaaggac gctcatgagg aaaacaagtc gagatggtta atgatgatcg gtttaatatt 5460
   cgggaggaac ttcgaacaaa ggaattggtg aaacaggatg aactgagaaa tgcgggttct 5520
   attatccatc cccaaagtca gtcctgtgct ttgctgctct ggtaagcccc ctagaggaat 5580
   aacctcggac ggaagccgtt ctttccattc tgagggagta tcatatccaa tagccaagaa 5640
   gctgccacgc gaaataagaa ttgaaaagta gttttctccc caaccggtcg ggggctggat 5700
   gattcgataa tactaccaac cgatctggta gatgtcccca cgttcagcta ccccttgcgc 5760
   atccaggcga atagaggagg gccgccctac ccgaggcggt gtgcgcgggc gccgagaggg 5820
   cggtttgaaa gatggaactc cagaacaaag gagcaggtgc ccgatgcaac aacagggcag 5880
   caaaggctct ggagcttaag gaattgtgac tagagctgtt ggcggttgca cgtgataatt 5940
   tcggaagttg ttctggctag accgtgctga tattagtggg atcatagtac catagcacaa 6000
   ttgtgagtgg gtaaactgag cacccctgag aaggattttc agcagagaaa ttagaatccc 6060
   caaccagttc taaaccgtca gagccccttg cccagggcca gcagccagct gaaggagccg 6120
   ctgtgagaat gtttgaacac caacctccaa agcttcatac aagggaggat gacggaagga 6180
   gggtaggatt taaaaggagc gcgctaagtc tcttgattct ggatgaagat atcgccagat 6240
   aggagaacca gaagcaatgt gcccgacgcc cccgcttaga ccaccaggat agaaatcgaa 6300
   atttcaagct aattatatgg aggaaagaag cttgatcggc tgtggaagag tgagccgtat 6360
   gaccagagac aggacactgc gcctttatat atgcagtcta agggacatct gcgaaggtgc 6420
   tagattatcc ggttccagca ctctagtcct gttacggaac gatattagtc attaagcact 6480
   gctagctgcc cctcaatctg gccttagtgt gctgaaaccg aattttacta tttcacaccg 6540
   cctgggatag cagactgtct cccatcacta tgcggaggct gcatatcaag cggatattag 6600
   agcccctcgc tgcccttttt gctatttgga tgctataccg gaaaaggaaa agtcttcaaa 6660
   tcttttccca gacccttccg aagtaacgaa gaatctgaac tcctccttta gctcgcggcc 6720
   tatagcactc tactgtttcc gggctttccc gtcaggtgtt cttttgcggg cagttggcaa 6780
   ttgggtttgc tgagcttccg gtgaaggtag ccagcagatg aaagtgaaag aaagcgaaga 6840
   gagataactg aagaggatga agatgccttg atggtcgtga ttgagatatt ctcaagtcac 6900
   gaagcaagga cggatgaaga atgatggaat gcgttctttc agaagaagaa agatcccacg 6960
   atgctcattg tttctaggag gagttgtttc ctcgtttaaa taacatgagg actgctaact 7020
   gagctaccta gttctttatt gcgtaaaagc atacgcaatg ttcaccaaag tctatgaaaa 7080
   actcgctgag ccatccggac gcacgacact acaatacttt tgtttagtgc gtcactacta 7140
   aaacgggagt atcttcaatc cggcgacttg agtactcttt ctttcccaca accgcccatc 7200
   ctcgacactt cttctacaac accgtaagaa ctcgaacaat tgaacctcaa gctaggatgt 7260
   tggaattgcc tctgctggta aggtctggct ctttttcttt tttgtctgtc ttgagctcag 7320
   gctgaggagg tcagttttcc ctgagcactc gcccccctat ttaagagagg aaacttggga 7380
   cgtctttgcg cgaggtcaat tattcgagtt cttacggcag tttgtttact tggaggctat 7440
   ggcgagggga tggtttctgc tcgcataaaa catacacagc gcaagtatcc aatgttctca 7500
   agttacataa ttaagcgggt gtaaaggcag gggcccgaat tcatccggtg aattgagctt 7560
   cgctaagttg ttggtgctct ggtttatgaa gtttgcgaag tttgttcttc tagcgagaaa 7620
   aagaaaacca ctggggaagt actttgcgat caccagctgg cggccatcgc tattggatat 7680
   cgccatctga cgtatcacag gtcgtgttaa agagatgcct atatgtatat gacaccccta 7740
   ggagtcttga gtaccttact taagagatct gcttatcccg caaagaaagt caagcaagtc 7800
   acccccacca ctcgtccctt atacattttg tctcccattc caccttgatt tgcagcggtc 7860
   acgcgaaatc gaacatcgat tgacacccag cgaaagcatc tatcaacacc gcctagcact 7920
   gaaggcatca caaattatga gatgaaaagg aaagcgtaat tcttgaattt tacttaaaca 7980
   ccaaagctac ttcgctaatg atgcctagcc accacaaata aaagaaaccc ctagactccc 8040
   tcccctgccc ttagagcact tgtagaaacg atgcgtcaag ctcgccaaag agatgtcacg 8100
   agttttaatg tatggtattg gtccaaaagg gttgtaattc tagacgctct aaccatctac 8160
   cgtaaaacct caaataaaag aaccgtgtgc tatgtagtcg agaattaccc acttgagaaa 8220
   aggggctaaa aacacagcaa aaactgagct gctcaggtcg cctgagagtc ataaaaaaac 8280
   aaaaaaaaca ccagacctta ccagaggagg caattactcg gatagggtca aaaagggcgg 8340
   aaaactttcg gtggcgaaac aaggatagag gcgctaatcg atcaggagga agtagaaaaa 8400
   atggggggtg ttataggatg ccattaacct ggagatgttg cagtgtagtc accattgtat 8460
   tccatctatg ggtcttatta agaatctgat catataaggc caaattaata ccaaaggaga 8520
   aggaatcagg gcttttggaa tagttcttta agagactcat ataaatgcat aggttcaatt 8580
   gcacattggc aatttcatca tcatttaaat ggtcaagtat catactagna tacttatttc 8640
   ttcaacctgg atttctggtg tccgccaagg caagaatttt tccactgttg caacctactt 8700
   cctaactacc ccacctgatc ctgctgatct ttttttccgc cctttttgac cctatccgag 8760
   taattgcctc ctctggtaag gtctggtgtt ttttttgttt ttttattgta cttctcaggc 8820
   gacctgagca gctcagtttt tgctgtgttt ttagcccctt ttctcaagtg ggtaattctc 8880
   ggactacata gcacacggtt cttttatttg aggttttacg gtattcatct gcctcaatcc 8940
   cagcccaagt tgctcttgtc ttggccaaaa tcctaggcgt gacttgctct aattgcttgg 9000
   acaccagcta gcaagtaaaa aattctttag tatgatatca tattagcagt gtgggaggtt 9060
   tgcttaggct ntgcgtgacc tccctacgca ctgccaggga ttagtagcag aacgagccaa 9120
   cattaccagc gcggctgata gaaaacatag tcacgtgagt ttgataaggc gcttaacagc 9180
   aagaaggttc accaaatttt ttttattagg ttcacgtgac tattttacct gccggccgcg 9240
   cccgtacaga aacttcaagg acaaccaagc acccaaaaga gtagaaaatc tgccttccta 9300
   tttaggaagg ccagtaaggt ggttgggcca cgcaaaatca aacaaaatgc cgagtgatac 9360
   cgcgtgaagc tgcccctcag cgccccaaaa cccatacgag tgttacaaca tgataatcct 9420
   aatacaacgc cattacgcta ttctgttgaa tatgtcttgt cactacaata aaaaacagct 9480
   ccgatcctag gactttattt cgcctggttt gttcccagcg cacttagcga caagatacgt 9540
   gaggctctca cttaaaagga gttgcgagtt ctcttgcgct gtattcgccc aaaagtgtaa 9600
   attagctcca ttcaagttgc ttgtgatgtt tgcttagagc ctgtttggat acaacagttc 9660
   cgttccgttc ccccatacaa atggcccttt tccttccgtt tcaaaacgga aagaacgctc 9720
   ttttcacagt agaaatggtc gcccgacgga acggccccat ttttgccaaa aaggttctta 9780
   agctttctag ctctcaccat cgagtatgac agtcaaagac attgttggtc aatgggagca 9840
   gaagttcacg tgacctactt agcactcggc gacctgagta cagaaacacg aaagtaacaa 9900
   gtttacaaac tagacaaaag tacaatgcca gtttaaaagt ctacctgtag gatttgtatg 9960
   gaaattttgg cagaagggca cttttgtggg ttcacgcaag ctataactac agttatacct 10020
   tctaaatctc gatacgtcta cagaaattgt tagatctccc acctagaaaa atataggtct 10080
   gtttaaacta gtgcccatgc cgtgaggcca cagaaaaaaa acagggctcc ccatacaaaa 10140
   cacatggcta atgaccaaat atatcaccaa actaggctcg ggtagggtgc tctgtcgggg 10200
   ttgggaatgg agaaaaaaag gggtcagcaa gatcctatgg ggcagtgagc agacggttcg 10260
   tgtgagtgga aaataatgag ttctggcaga tgcaaacaat tctgagtgga tactaaacac 10320
   attatcttac cgacaataag agatcccgag cggatgatta agaattaccc ttagtgaaaa 10380
   ctagctttat agagattact tgaagctccc tgaagccctc cagaatatct gagttggagt 10440
   ttaaagcaga cactaatata cttggtccgg gcccctaatt tctacaactc cctctttgat 10500
   ggggcccggc tagcaatttt cgttttaatt taaattcaca actctctagc ataggaggga 10560
   gaaaatatga agtaaaactg cgggaaatct tatagtatca taccagagat gcacaaatat 10620
   ttaataaaac tatctaaaga cacaacccag gtggtagtaa actccggcac cccccaactg 10680
   atagaggata gttatcaagg gctactggga atagcagaat tgaacgaccc cggccccaac 10740
   aggtccgcga tatctgacaa ggactgttgc caatattaca ttctgagatt agcacatgtc 10800
   agaaacaagc gataacgaaa acatacaatg catgccaact tataccaaac tcttgcaggt 10860
   aatcgctgcc agatgctctt agtctgtaat ggtgggatcc cggcagacct cactaacagc 10920
   gtcaaggaga ggaatcgcaa ctttctcttg cccagcaccc tcaccaaacc ggtccttgag 10980
   aaccacagcc ctcacctggc ggaacgaatt gaaaaagtac gtcgcagcac tatttgagaa 11040
   ttagctttca cctaaagaaa ctttgaagct tgaaagatag cttgcttcag gtgctttaca 11100
   gaggacattt tctggtcccg ggcagagaac ctaaagtagt tttcctcttt agtataatac 11160
   gctttggtca tgatagtaaa 11180
<210> 58
   <211> 1067
   <212> DNA
   <213> Tuber magnatum
<400> 58
   atgtcttccc tctctgtcct tcagatggct gttgaagccg gacctcttat tcaccaacgt        60
   taccacggag tcaacctcag tcaatacctt tccatgtttg ccaataaaac tttttccttc       120
   aatggatcgg atggagctca gctcagtgtt ctccatatgc ttccggccga ctggaagcct       180
   gcctctggac cccttagcgt ttcgtttgat tcggcccccc ataccgtcat cgggactcac       240
   catactatcc gcagccgccc tgtttataag cttagcgacg gatccatcct cctcgtcttc       300
   gaccctgtcg ttaccgacgt cgattttcag ctcactgatc gctctttcga ggacccccag       360
   catgagatta ccggcctaac tcgttttcta cgtcttgcta cagatgaaca agacttcgcc       420
   tctcacggtg ctagtcttag ccttattcgc gctaagggtg tcaagcgccc cgccaatgcg       480
   taagtacaac tttcctgacc tacaggcttc atgcgctcat acagccacag rtttatcctg       540
   ttcaggaccg agaatatgag gactatcaag actcggtatc ctcagatggg caacaatgac       600
   atttgtatgc agctctcatc ccttttatta ataagctctt cattcttagt atcatttcac       660
   taatagtttc aatagcaaaa attctyggtc gcatgtggca gaattcctcg aacgaggcga       720
   aagaggttta taaggtatac cacctaacct aactgttagt tgaaatagct aacacattaa       780
   taggcaaggg cccgtcaact cgcgatttcg cacaagttgg tgaatccgaa ctacaaatac       840
   accccgcgcc gttctcatga aattaggagg agggcctatg cgagaaaggg tctttcgaac       900
   tatgtcgatc tttactctcc tggagctgaa agagatgcca gagagagggc tacaacccac       960
   cttgggaccc ccactgcaca gtttgcctcc gagccgagca ccgtcaccct cggtgaaatt      1020
   atcgaggaag cggagaaggt cttgggttcc tttggtctga ttaattg                   1067
<210> 59
   <211> 4790
   <212> DNA
   <213> Tuber magnatum
<400> 59
   caagttgtat gcaagcagaa atcatccctt cgcttgatgt ccaattccac acctttcaaa 60
   cgaaaaaacc gttaccttat aagtatgata tcacgggtgg gagcttgtgg taaggaggag 120
   agtctgccag cactaccata agaaagtcgc cggaaaaggt gtttagattt tcgttacttc 180
   gaagagtccg gtaaaggacc tgaaggcttt tacttctcca gcattactca cgggcagcaa 240
   aagacagcga aggggttggc caagggggct ctaggatcag cttcgtacgc aactactgta 300
   tgtttgtgag agatattcca gagtattcta gatccaccct cacaaacacc ctcgcaatat 360
   tcatagacgt gtagtatccc cgcctctgat cataccactc acttttccac gaaacaagac 420
   aatcggtact ctttcttccg tatattttcc tcgatgcatc ggtttctatc ctgggagccc 480
   gagcgggagc atcgggccct tttcttcaaa cacccctatc tgacaatatc ttcgcacaga 540
   atcaggagac atagtgcgcc ccttttaagt cccactcatc ctgccaagca tgaagttctc 600
   ggggttgagc tctcaaatac tctcacagcg actttcccaa ttgtccccta gtcctgagcg 660
   agaaatcctg gtggtttaga actggttaga gattgcaatt tctccctgga acccctcccc 720
   ggggggtttt cacactcaca atccagagct cttcctttcg ctataccact acaccatccc 780
   actagcctcg gtatggtcta accaagaacc tctccgaaac catcacgagc aaccgccagc 840
   aatgctaacc acaattcctt aatctccagg ccctttcgct cgcgggctac tgcatagacc 900
   tgctcctttg tccaggagtt ccatctttca aatagcgtcc ttggcagccg caccacccat 960
   cgaatacggt agttaacatt atctatccgc ccgcatgcac cagcgggtag ccgagcgtgg 1020
   ggacatccat cggatcggtt ggtagtgctg ccgaaatatt catcttattt gattggccga 1080
   ggagaaagcc acttctgaat ccgtgtttcg cgtgacgggt tctggggtat tggatatccc 1140
   agaatttaga gaagagcttc cgaatggctg ggcttgagga atcgttttcc agtccgccat 1200
   accgccttat ggaggttatc tatataggat tttccaatta gcatgctacc ttaatccaaa 1260
   taccgaatcg aaaggcaata ttgttcaaga gagcctagat accttacctc tctgccaaag 1320
   aagcaaagta cgggagaagt aggattgagg agacggataa cagaacccac atttctcggc 1380
   tcatcctgtt tcactaattc ttttgttcga aactcctctc gaatatcaaa cagatcttca 1440
   ttaaccacct tgactttttt ctctttgggc atctcgggct gttggagaaa actttttcgc 1500
   cgatcttttc gccaagcatc ttaaaaacct aagggtcgcc ctcttgccct gggaaaaatc 1560
   ttaccacacc atactatgat atggtgtttt cacggcaata ctatgctcag accctggtta 1620
   gggttgctgg agttgtacgt gacggcttct tggtgtcact tcttttgggg agtcttggcg 1680
   tcccccccct tcgtgacggg aggtctatat aagaggtccc aactcccctc ctcctttctt 1740
   ccaccaacaa ccgacaactc ccacaaggtt agattcacag aaacatccaa ccttcctaca 1800
   ggcaattttc ctatcacggc atatttgtca ccctctatct tcaccacctc agcaatgtct 1860
   tccctctctg tccttcagat ggctgttgaa gccggacctc ttattcacca acgttaccac 1920
   ggagtcaacc tcagtcaata cctttccatg tttgccaata aaactttttc cttcaatgga 1980
   tcggatggag ctcagctcag tgttctccat atgcttccgg ccgactggaa gcctgcctct 2040
   ggacccctta gcgtttcgtt tgattcggcc ccccataccg tcatcgggac tcaccatact 2100
   atccgcagcc gccctgttta taagcttagc gacggatcca tcctcctcgt cttcgaccct 2160
   gtcgttaccg acgtcgattt tcagctcact gatcgctctt tcgaggaccc ccagcatgag 2220
   attaccggcc taactcgttt tctacgtctt gctacagatg aacaagactt cgcctctcac 2280
   ggtgctagtc ttagccttat tcgcgctaag ggtgtcaagc gccccgccaa tgcgtaagta 2340
   caactttcct gacctacagg cttcatgcgc tcatacagcc acagrtttat cctgttcagg 2400
   accgagaata tgaggactat caagactcgg tatcctcaga tgggcaacaa tgacatttgt 2460
   atgcagctct catccctttt attaataagc tcttcattct tagtatcatt tcactaatag 2520
   tttcaatagc aaaaattcty ggtcgcatgt ggcagaattc ctcgaacgag gcgaaagagg 2580
   tttataaggt ataccaccta acctaactgt tagttgaaat agctaacaca ttaataggca 2640
   agggcccgtc aactcgcgat ttcgcacaag ttggtgaatc cgaactacaa atacaccccg 2700
   cgccgttctc atgaaattag gaggagggcc tatgcgagaa agggtctttc gaactatgtc 2760
   gatctttact ctcctggagc tgaaagagat gccagagaga gggctacaac ccaccttggg 2820
   acccccactg cacagtttgc ctccgagccg agcaccgtca ccctcggtga aattatcgag 2880
   gaagcggaga aggtcttggg ttcctttggt ctgattaatt gagtgatctt agtggagggg 2940
   gcttatgggt tacttggatg atatactttt atgggattga tgatcttgtt tgctcgtgat 3000
   gggtcgttag ctcagttagg taaatttgta tgatccaaat aaacaaaaat tatcttaaaa 3060
   atgagcccga gaaatccaaa caccaagtcg tgggcaaggt aggagcgagt agtgtatggc 3120
   aaggggcagt gggaacttaa ccattcgagt atggatacag caacccagaa agtttataca 3180
   gtacccgcgc ggccgaattt aagcccgaca ccacccctta taatcgtcac cagccgacca 3240
   ataagaggtg gaactcagat ggaaacttag taaacacata cggtatacca gatagaaaaa 3300
   tgtccaatta catgcatcct aaactatcgt atacctgtat catacacaga aaatttgaaa 3360
   tcaaacactt cctataagaa ttaccacccc caaattaggt gtcaagcccc aattcagtta 3420
   tataagtata tcatttaccc attaaagtta tcgtttcggc catgaagaat aacaagctca 3480
   gattcagcag ttgaggcatc acaacctatt cttttcaatg gtttatcgag ttacatcccc 3540
   cagcttggca aggccatttc tgcataatta ttcgttggct gatcactacc ataagaagtg 3600
   gtgtccggct ttaattcggc cgcgagtatg acctagtttg agtggaaatc ccctctggag 3660
   gtcaaggtgt cgcgtccccc gcattcccac acattactaa accgggtgtt gtgggtaagt 3720
   ctgtcgaact cactttaatt ggctccaggg cctgctcgac cacttaggga acagtattgc 3780
   tccggccact gtgccaagag ttcgacagac ttttttcctt caccaaaaat tagcgtctat 3840
   ggcctgttca gttgcataac tcgcgttgca cacacgttaa ttcaaccaac cagaccagag 3900
   ttcgacagac ttttgcgcac accctaagta caccggccgc cggccggtag gttattctta 3960
   gtaatggcat cagaaaaggc ggccacgaat cgatgcccag tatgggaggc accagccacg 4020
   ccccggccag ctaacacact atcatactca tcgggatcaa aggtctgtcc ccgaggtata 4080
   gtagccagtg gaagcacggc aaccactccc ctccgccagc cttgacgata ggagaattgt 4140
   gttgataggt atcgcttata aatcaaatat ccttagaaaa caagattact agtagcgctg 4200
   agagaagcaa gctgcaatct ttacaaggtg ttgtggctag gttgtttgtg cataggtgaa 4260
   agtcggggac agactatttt tttgccgaat acaatagaaa ccggcgcagt tcggaatggg 4320
   ctagagaagc actggaaaaa tcgtgggtat agtgcacggc cagtagtggc gagaccaggc 4380
   ttaccccata atcagacctc aaatttaggc caaccaaacc atgagaataa acaccggccg 4440
   caccctaatc actagcggta gtgcgacttt tgtgagttta tggtatggcg gaggaacaga 4500
   ggggtgtatc atacgagtaa accaggtggc aaccatgaat gcagccgcta atacgactaa 4560
   aaatgcgcga ggtgcgccag gtgttggagg tcgagtgtgt gtattgagag attgaagaga 4620
   aagtaaagac cgaggaatat aggggggaat aagaagctat atattggaaa cgaagagcgt 4680
   tgatttttct caccgtccta tagttacatg cgccagagac agccattctt atatccgtag 4740
   aaaaccgctt gtgaaccaag tggtgaaaag agaagctccg agctgagcat 4790
<210> 60
   <211> 8221
   <212> DNA
   <213> Tuber magnatum
<400> 60
   caagttgtat gcaagcagaa atcatccctt cgcttgatgt ccaattccac acctttcaaa        60
   cgaaaaaacc gttaccttat aagtatgata tcacgggtgg gagcttgtgg taaggaggag       120
   agtctgccag cactaccata agaaagtcgc cggaaaaggt gtttagattt tcgttacttc       180
   gaagagtccg gtaaaggacc tgaaggcttt tacttctcca gcattactca cgggcagcaa       240
   aagacagcga aggggttggc caagggggct ctaggatcag cttcgtacgc aactactgta       300
   tgtttgtgag agatattcca gagtattcta gatccaccct cacaaacacc ctcgcaatat 360
   tcatagacgt gtagtatccc cgcctctgat cataccactc acttttccac gaaacaagac 420
   aatcggtact ctttcttccg tatattttcc tcgatgcatc ggtttctatc ctgggagccc 480
   gagcgggagc atcgggccct tttcttcaaa cacccctatc tgacaatatc ttcgcacaga 540
   atcaggagac atagtgcgcc ccttttaagt cccactcatc ctgccaagca tgaagttctc 600
   ggggttgagc tctcaaatac tctcacagcg actttcccaa ttgtccccta gtcctgagcg 660
   agaaatcctg gtggtttaga actggttaga gattgcaatt tctccctgga acccctcccc 720
   ggggggtttt cacactcaca atccagagct cttcctttcg ctataccact acaccatccc 780
   actagcctcg gtatggtcta accaagaacc tctccgaaac catcacgagc aaccgccagc 840
   aatgctaacc acaattcctt aatctccagg ccctttcgct cgcgggctac tgcatagacc 900
   tgctcctttg tccaggagtt ccatctttca aatagcgtcc ttggcagccg caccacccat 960
   cgaatacggt agttaacatt atctatccgc ccgcatgcac cagcgggtag ccgagcgtgg 1020
   ggacatccat cggatcggtt ggtagtgctg ccgaaatatt catcttattt gattggccga 1080
   ggagaaagcc acttctgaat ccgtgtttcg cgtgacgggt tctggggtat tggatatccc 1140
   agaatttaga gaagagcttc cgaatggctg ggcttgagga atcgttttcc agtccgccat 1200
   accgccttat ggaggttatc tatataggat tttccaatta gcatgctacc ttaatccaaa 1260
   taccgaatcg aaaggcaata ttgttcaaga gagcctagat accttacctc tctgccaaag 1320
   aagcaaagta cgggagaagt aggattgagg agacggataa cagaacccac atttctcggc 1380
   tcatcctgtt tcactaattc ttttgttcga aactcctctc gaatatcaaa cagatcttca 1440
   ttaaccacct tgactttttt ctctttgggc atctcgggct gttggagaaa actttttcgc 1500
   cgatcttttc gccaagcatc ttaaaaacct aagggtcgcc ctcttgccct gggaaaaatc 1560
   ttaccacacc atactatgat atggtgtttt cacggcaata ctatgctcag accctggtta 1620
   gggttgctgg agttgtacgt gacggcttct tggtgtcact tcttttgggg agtcttggcg 1680
   tcccccccct tcgtgacggg aggtctatat aagaggtccc aactcccctc ctcctttctt 1740
   ccaccaacaa ccgacaactc ccacaaggtt agattcacag aaacatccaa ccttcctaca 1800
   ggcaattttc ctatcacggc atatttgtca ccctctatct tcaccacctc agcaatgtct 1860
   tccctctctg tccttcagat ggctgttgaa gccggacctc ttattcacca acgttaccac 1920
   ggagtcaacc tcagtcaata cctttccatg tttgccaata aaactttttc cttcaatgga 1980
   tcggatggag ctcagctcag tgttctccat atgcttccgg ccgactggaa gcctgcctct 2040
   ggacccctta gcgtttcgtt tgattcggcc ccccataccg tcatcgggac tcaccatact 2100
   atccgcagcc gccctgttta taagcttagc gacggatcca tcctcctcgt cttcgaccct 2160
   gtcgttaccg acgtcgattt tcagctcact gatcgctctt tcgaggaccc ccagcatgag 2220
   attaccggcc taactcgttt tctacgtctt gctacagatg aacaagactt cgcctctcac 2280
   ggtgctagtc ttagccttat tcgcgctaag ggtgtcaagc gccccgccaa tgcgtaagta 2340
   caactttcct gacctacagg cttcatgcgc tcatacagcc acagrtttat cctgttcagg 2400
   accgagaata tgaggactat caagactcgg tatcctcaga tgggcaacaa tgacatttgt 2460
   atgcagctct catccctttt attaataagc tcttcattct tagtatcatt tcactaatag 2520
   tttcaatagc aaaaattcty ggtcgcatgt ggcagaattc ctcgaacgag gcgaaagagg 2580
   tttataaggt ataccaccta acctaactgt tagttgaaat agctaacaca ttaataggca 2640
   agggcccgtc aactcgcgat ttcgcacaag ttggtgaatc cgaactacaa atacaccccg 2700
   cgccgttctc atgaaattag gaggagggcc tatgcgagaa agggtctttc gaactatgtc 2760
   gatctttact ctcctggagc tgaaagagat gccagagaga gggctacaac ccaccttggg 2820
   acccccactg cacagtttgc ctccgagccg agcaccgtca ccctcggtga aattatcgag 2880
   gaagcggaga aggtcttggg ttcctttggt ctgattaatt gagtgatctt agtggagggg 2940
   gcttatgggt tacttggatg atatactttt atgggattga tgatcttgtt tgctcgtgat 3000
   gggtcgttag ctcagttagg taaatttgta tgatccaaat aaacaaaaat tatcttaaaa 3060
   atgagcccga gaaatccaaa caccaagtcg tgggcaaggt aggagcgagt agtgtatggc 3120
   aaggggcagt gggaacttaa ccattcgagt atggatacag caacccagaa agtttataca 3180
   gtacccgcgc ggccgaattt aagcccgaca ccacccctta taatcgtcac cagccgacca 3240
   ataagaggtg gaactcagat ggaaacttag taaacacata cggtatacca gatagaaaaa 3300
   tgtccaatta catgcatcct aaactatcgt atacctgtat catacacaga aaatttgaaa 3360
   tcaaacactt cctataagaa ttaccacccc caaattaggt gtcaagcccc aattcagtta 3420
   tataagtata tcatttaccc attaaagtta tcgtttcggc catgaagaat aacaagctca 3480
   gattcagcag ttgaggcatc acaacctatt cttttcaatg gtttatcgag ttacatcccc 3540
   cagcttggca aggccatttc tgcataatta ttcgttggct gatcactacc ataagaagtg 3600
   gtgtccggct ttaattcggc cgcgagtatg acctagtttg agtggaaatc ccctctggag 3660
   gtcaaggtgt cgcgtccccc gcattcccac acattactaa accgggtgtt gtgggtaagt 3720
   ctgtcgaact cactttaatt ggctccaggg cctgctcgac cacttaggga acagtattgc 3780
   tccggccact gtgccaagag ttcgacagac ttttttcctt caccaaaaat tagcgtctat 3840
   ggcctgttca gttgcataac tcgcgttgca cacacgttaa ttcaaccaac cagaccagag 3900
   ttcgacagac ttttgcgcac accctaagta caccggccgc cggccggtag gttattctta 3960
   gtaatggcat cagaaaaggc ggccacgaat cgatgcccag tatgggaggc accagccacg 4020
   ccccggccag ctaacacact atcatactca tcgggatcaa aggtctgtcc ccgaggtata 4080
   gtagccagtg gaagcacggc aaccactccc ctccgccagc cttgacgata ggagaattgt 4140
   gttgataggt atcgcttata aatcaaatat ccttagaaaa caagattact agtagcgctg 4200
   agagaagcaa gctgcaatct ttacaaggtg ttgtggctag gttgtttgtg cataggtgaa 4260
   agtcggggac agactatttt tttgccgaat acaatagaaa ccggcgcagt tcggaatggg 4320
   ctagagaagc actggaaaaa tcgtgggtat agtgcacggc cagtagtggc gagaccaggc 4380
   ttaccccata atcagacctc aaatttaggc caaccaaacc atgagaataa acaccggccg 4440
   caccctaatc actagcggta gtgcgacttt tgtgagttta tggtatggcg gaggaacaga 4500
   ggggtgtatc atacgagtaa accaggtggc aaccatgaat gcagccgcta atacgactaa 4560
   aaatgcgcga ggtgcgccag gtgttggagg tcgagtgtgt gtattgagag attgaagaga 4620
   aagtaaagac cgaggaatat aggggggaat aagaagctat atattggaaa cgaagagcgt 4680
   tgatttttct caccgtccta tagttacatg cgccagagac agccattctt atatccgtag 4740
   aaaaccgctt gtgaaccaag tggtgaaaag agaagctccg agctgagcat actttcgtcc 4800
   tattgaagaa atactggagt acttcagaca caagatatag ccaaccattg agtgataatc 4860
   tgtgacatta tagatggcaa tcccatatgt tgtattaagt tgcactatta taccaagacc 4920
   ccaacactac actaaatttt tgcctttctg gacccttaat tacctcctcc tgcactcata 4980
   aatggaaaga aagaaattgc cctttatttt tctccctttt gcccacatcc cgacagctct 5040
   tccgcccagt ctggacaagc acctgggagc ttacccgggg actaaaatca cctatttgct 5100
   tgatttacac ttattgcctc tcactctatg gagtgtatca tctgcatttt caggtgcact 5160
   tattccattt ggttgataga acagctatag tatccctttc tcagtttcct gactaggtac 5220
   aagataacag aagtgctaat tcaataaaga tcccagttgt agatgacaag tatagcaagt 5280
   gtcttctcat gatttgtttt ccagaaggct gtgattcaga gcttgcctta cctattccct 5340
   taagatcaag atttcttctt catactattt gagacttatg ccatacattt gcctaaaaaa 5400
   aataggtgga aacaaaggga tcttgaatgc ctcccattga gatgaagcta agtttactta 5460
   agctcctaag aatagacaaa gtcctgttgg taggcaaaat ttaataccta aatatacaat 5520
   actagttgtt tgaagttgta ctctctttgg catcaaattg acagtaacag taatgtacct 5580
   aaaagagcaa gtacagaggg caaacaagta ccttctacaa attaaccaca atttttcaca 5640
   ttccatgcta gatacttact cctctgcact acactctagg aacttttact caacaatatt 5700
   tggtgcagta ttcataactg tttggatatt ctggtagctt tgcagggaga ctataagagg 5760
   actatatcgg ataatactag gattcacaat caattctatc ctgtaactat tgagaacttg 5820
   aatgatttgt tttaacaaaa tctatagctt ggacaataga aatattattt tgttatctta 5880
   gtatcagaac tccaggtgaa acagtagact gctagtacag atttccttta tagaactata 5940
   tagaataatg agttaataga aaatcgataa aaagttgaag tggactatga gaatccagaa 6000
   aatctgacta accagaagcg attgccagca tgggggcata ctgtagctac aaactaagtt 6060
   atagatataa tcatatatac atagttttaa agtatcatac taggaatttt ctagtatttc 6120
   ttttcacatt tctctattgt tttagctatt taaaatatac cataggtata tgctagcaga 6180
   tgtaaaactg ccaagagtta gagtctgttt ggataagact attctattct ttttctttat 6240
   ataaaataac tattatgtga tggaaaaaca atagtattca tatgtgaaaa tgaaactgaa 6300
   tagaacagaa cttatgaaag ggtcatattc aaacaaactc taaaattatt taacttcttt 6360
   tccagtataa gtcatattaa tacaaaaatt ttaatacaag taccaatcct gtgaaaagtt 6420
   actagaaaat aaactattat atactattat acctatcaat agtagtgaac aagtcctgct 6480
   aacttcgata taatttattg tattatagtt gactactagg taatctctac tctctttcta 6540
   caagtttttt gagtttttct actctactct agtaaaagcc tcaaacaggg tcaaattctt 6600
   ctcaattatt aaatattcat ctatattaat acctttatta ctattacttt ctattatatt 6660
   cagaaaaata tagatcagat tatcacaata tattttataa tagtatgata caagtactta 6720
   atagtatgtt atcacataat actaataata atattatcat acttatagag atatagtatt 6780
   taggttttat aagttctaca agtaagattt gctgccttta gcctctatag aatgtataaa 6840
   acttagtggc tatatttcta tacactcttg gcattttttg gcgcattgtg taagtcttgg 6900
   catgtttggt catgtgacta tttctttgaa aagctcttac tttataaaag ggataaataa 6960
   tgctcacagt ctatactacc aaaacttctc tcaaacaccc aacaccagaa cactcgattc 7020
   gacaacatcc tttgcacact tgttagtgga tctgctggta tcagtctcag gcccaagatt 7080
   ggcctctaca catataagat actttttttt tttctttgtc aaaatgcctt taagtatctt 7140
   ctctctacca ggaaatccaa atttaataca gatactaact caaaaaatta gatgaggata 7200
   ttagactccg ccgtgaggca ctattccggg ctggattgat gcttactaga gcaatggatg 7260
   aaactgcctg gaaccctggc aatctaatgg ttggaatggc cattcataaa aatccaagca 7320
   ttactgtggt taaagcagtt aaatcatctg aagttatact taatgaagca gctgtgagtg 7380
   ataatatatt acgcaagatg gatccagtaa atgacctcca gccactggca aatattcaca 7440
   atacactttt acagcttgga gcattaatta ctataaaaca agaactctat gatgcacttc 7500
   aaaatatcaa attacgcctt caggaacttc aagcaaatat gaccaacaac attggccagg 7560
   ccatggcaga agttcatgac tttatctgct ttgtcacatt tgttaacttg attcatccac 7620
   aaaactgcat attaacctct gaatctatca aaaatgctga gattcttgct tccaaaagta 7680
   tattttcaga agctcgtcag gcctttgttg aactccgtca agcttatgac ctgcaaactc 7740
   ctgttccagg ataccgtctg agcaaagcta gagagttacc tgcatttact agctgcatta 7800
   tggaacagtg gcacagaggt tattcttctc atacagctaa aactggaata gtaagtagaa 7860
   aatacctctc ttaacttagt acctatatcc aatatactct actgtatatt atactaacat 7920
   cagagcatag gaaattattc cattccatat catatttgga tggtggagtc agttcaatta 7980
   ccttatttca tctaaacagt atcattaact aacaataaat aatcttggtt tatagtcaat 8040
   taccaacgca agtttattag tagtgctcaa gagaaaacta caccaattgt ggaaaagctt 8100
   atcagccaag cctatatctc gaagtccaag atgccagaag atgaggccca gcaaccatgg 8160
   atgcaggaag ataaggatgc cttcaaggct gcaaccaatc tagccatgtc tatgactata 8220
   c 8221
<210> 61
   <211> 205
   <212> DNA
   <213> Tuber magnatum
<400> 61
   tttggagctg aacccaacaa gtatatgtgg caaaacatcg catctctcta tactgccgct 60
   aggaatcgtg ccgagcccgg tcttgtgctc gaagagttta tcgagaatga attggtgaaa 120
   cacggtcacc caaggacgcc ggagaagctc ttgcgcgaag ctgggttcgt tcccaaggct 180
   ccaattgrca agggcgccgt cgata 205
<210> 62
   <211> 1041
   <212> DNA
   <213> Tuber aestivum
<400> 62
   atgtcaactc tcaccgctct ggagatggct atcgaagccg aggcatttat tcaccagcgt 60
   tacggtggga tctcgttcca cgagtatctc cccttgctca ccgatgggac tttttgcttt 120
   gagggatcgg acggagctca gctccaggtt tcccatatgc ttcctaccaa gtggaaacct 180
   acccccgagc cgctcagggt ttctttcaat ccgtcccccc ataccatcgt cgggactcat 240
   ctcactcttc atgaccgccc tgtctacaag cttagcgatg ggtccattct tgtcgtttcc 300
   gaccccacca ctagccccac cagtttccag ctcacggata tcactttcga tgatcctcag 360
   catgaagtca ctaatgaaaa ccgttttttg cgctttttca tcaaggctgg tcaagacctc 420
   tcatctcatg agatcagtgt tcgcaaaatt caggagaagg gtatcaagcg ccctgccaat 480
   gcgtaagtac aactctacca acctacaagc ttcatacgct catgcagtca cagattcatt 540
   ttgttccgga ccgagaggat gggttccatc aaatcccaat atccggggat tggaaacaat 600
   gacatctgta tgtagttctc attccctctt attaaaactt cattcctagt atcaccttca 660
   ctaatagttt cgatagccaa aattcttggt cgtatgtggc gggattcccc ggacgaggcg 720
   aaggaagtgt ataaggtata gcatctcact caactactag ttgacctggc taacaaatta 780
   ataggcaaag gcccgtcaac tcgcgatctc ccacaagttg atctacccga actacaaata 840
   caccccgcgc cgtccttatg aaattttaag gcgattgaat gggaatgtag atcctgagat 900
   cgtaaacgaa cccggaaata cggttgcaaa tgaccttggg gcgctcaccc aaaggtttat 960
   cgcccatccg accgccgcca ctctcaatga aattatcgcg gaggcagggc agatcatgag 1020
   cgcccttggc tatactgagt a 1041
<210> 63
   <211> 5520
   <212> DNA
   <213> Tuber aestivum
<400> 63
   cgagacaaaa gaagagatgc cgcaccttgc cgggagagcc aacctcaaca ccctagcaaa      60
   cggtgcgatt attcaagttc ttgcggtact cgagcaccca gacactcgcc cttgcccaca     120
   accgggagat tcgagcgtag tattgtatca ctgtaatgac cttcctaaat aggaaggtga     180
   tttactacac ttcagatggc ttggctgtcg ttaaaggtcc tgcattattc gtacaaccgg     240
   cactctccaa ctaactctcg gttacacgca gggatgtcac gcagaatcct agtaggcatc     300
   acgcagacta ctaattatta aggatttttt tatccgctag cctttccgcg accccgatcg     360
   ggggaaatca gtttaagcat tcttgtagac gtgtactgga aacttcacgt cgatgccccc     420
   gcccctgggc gagcaaaaat ataatgcgag aaatgaatat aggagcctca cgtttttccc     480
   ggggagccaa acagcgatga taacactata ccctatctca ctggctctag gtgtctaccc     540
   taaagttaga ggaacggtta attttggtta acctgcgaaa ttatacctgc ggtagagaca     600
   tgtttttcct cctttaactg ctagaatgta gcttcgcccg ggacaagtag tctaggtcca     660
   gactggggac tgaggcagat ggataaacgg atttagtggc tataatacga tagaattgcg     720
   tctttgaatg aaatgccata gtatatgaaa actatatgaa aactcgtagt actccttttg     780
   gcaagcttca ctcatcattg tgcataaaga ataaagtccg aaactagttt tacccccccc     840
   ggcaaggcaa cttcggtatc cgaataaaat acaggtatta agattcgctc ccatcccatg     900
   acttgtaaaa tcctcagctc cgagcgtctc cggatgtttt cacaatgtat cactcgatat     960
   tttattttgc gtgattgttg cacagagatg gaacgggaga caaaaggatt atatgggagg    1020
   acgagtggaa ggaataactt gcccgaactt ctaggtggga taaacacacc gctcaagtgc    1080
   gtatgatata acgatcgtag atgtcaaact gctacttttg aacatctctg atacaatcag    1140
   tgactgtcag cgaaaacccg aacaggattt actccaacat ggttcttttt ttttcctccc    1200
   ctttcccttc cctcctcccc agaaggaaaa cttggactcg tgtctttacg ttagatcaat    1260
   tatataagct aagcgttcag ttctcatgtt gtatccgagt gggtatcgtc ccttcgcttg    1320
   gtgtcgaatt tcacagattt taaacgaagg aaccgttagc aagaaagtat cgtggaggag    1380
   ggaggttgca ggaaaaatga gagtgcacca gtactgtgct atgagccact agcaaaaaaa 1440
   aggagattat atttttatta tgatacttcg aagagtctgg taaaggacct aaagacttct 1500
   ccttctttgg catcactccc aaatagaaaa aacactacga aggaattcac caagtgggcc 1560
   ccgggactgg cctcgtattc aattacggta tttttgtagg agatatttcg ctataatcta 1620
   gaatcccctt cacaaatata cagagacgtg tagtatccct cctctgatga tacaactcgc 1680
   ccttctgcga aacaaaacaa tcaatactct ttcccccgta tagttccctt gacaccttgg 1740
   tttctatccc gggagctcaa gcgggagcgc cgtgcccttt tcctctaacg cccttctctt 1800
   gcgatatctt catacagact catgagaccc agtgtgctcc atttaaatcc cactcatcat 1860
   accaagagta aagttccggg ggttatgcct tcaaatacgt ccccagcgac tttccaaatt 1920
   gccccctagt cctgagcggg aattctaacg gcttagagct ggttagagat tccaattcct 1980
   tcgctgaaaa tcctattcag gggcgttcac actcacaatg caaagctcgt ccttccgtta 2040
   taccaccaat atctttccca ctggtcccag tatggtctag ccgagaacct ctctgaaacc 2100
   atcacgagaa actaccagca atgctagcca caattcctta atctccagac cctctcgccc 2160
   atgggctgct gcatagtact tctcctttgt ttcggagctc catctttcaa atagcgtcct 2220
   tggcagctgc accccccccc cgaataccgc catactcata gccaaattcg actgcatgcg 2280
   ccagcgggta gccgaacgca gggacatcca tcggatcggc cgggagtgcc gaaggatcta 2340
   tctccccctg atcggcagag gagaaagcta cttcgaattc ttgttttcga gtgccggctt 2400
   cttgggtatt gtaagcaacg gcttctggat ggttgggttt aaggtatact tttccagcac 2460
   gcaataccac attatggagg ttatctatat aagaatatct cattagaatt ctatctagat 2520
   ctgtatacag aatcgcaaag caacattgtc agaggggtct tggatagcac acctctctac 2580
   tcaagaagaa atgcacagga aaaaaaagta tcgaggggac aaatagcata acccacattt 2640
   cccagttcat cccccctttg cccaaaactc ctcccggata ccaaatagat cttcattaac 2700
   cgccttgact ttttctgcgg gcctctcgga ctgtaaagag aaaaaaattt cgccgaacat 2760
   tgcagaagcc taagggcccc accctggccc ggggagaatc ctaccacacc atactatgat 2820
   actcccgcaa taatactatg ctcagacctt ggttggggtc gctggggtcg tgcgtgaccg 2880
   gttttttttt ttttcgtacg tgaccggttt ttggtgtcgc cgctttttag ggtctcagcg 2940
   tcccaccttc gtgacgggtg gtctatataa gaagtccaaa ctgcccccct cctttccctc 3000
   tccttcttcc accaacaacc acaggagcga atccccctcc actcgatagc aagttccaac 3060
   cttcctatca acaaatcccc ctttggcagc atattctcca tcacctcgcc agcaatgtca 3120
   actctcaccg ctctggagat ggctatcgaa gccgaggcat ttattcacca gcgttacggt 3180
   gggatctcgt tccacgagta tctccccttg ctcaccgatg ggactttttg ctttgaggga 3240
   tcggacggag ctcagctcca ggtttcccat atgcttccta ccaagtggaa acctaccccc 3300
   gagccgctca gggtttcttt caatccgtcc ccccatacca tcgtcgggac tcatctcact 3360
   cttcatgacc gccctgtcta caagcttagc gatgggtcca ttcttgtcgt ttccgacccc 3420
   accactagcc ccaccagttt ccagctcacg gatatcactt tcgatgatcc tcagcatgaa 3480
   gtcactaatg aaaaccgttt tttgcgcttt ttcatcaagg ctggtcaaga cctctcatct 3540
   catgagatca gtgttcgcaa aattcaggag aagggtatca agcgccctgc caatgcgtaa 3600
   gtacaactct accaacctac aagcttcata cgctcatgca gtcacagatt cattttgttc 3660
   cggaccgaga ggatgggttc catcaaatcc caatatccgg ggattggaaa caatgacatc 3720
   tgtatgtagt tctcattccc tcttattaaa acttcattcc tagtatcacc ttcactaata 3780
   gtttcgatag ccaaaattct tggtcgtatg tggcgggatt ccccggacga ggcgaaggaa 3840
   gtgtataagg tatagcatct cactcaacta ctagttgacc tggctaacaa attaataggc 3900
   aaaggcccgt caactcgcga tctcccacaa gttgatctac ccgaactaca aatacacccc 3960
   gcgccgtcct tatgaaattt taaggcgatt gaatgggaat gtagatcctg agatcgtaaa 4020
   cgaacccgga aatacggttg caaatgacct tggggcgctc acccaaaggt ttatcgccca 4080
   tccgaccgcc gccactctca atgaaattat cgcggaggca gggcagatca tgagcgccct 4140
   tggctatact gagtaagcca cctcggcaga ggggcttgtg tgttccttgg gtgatattct 4200
   tttaatggga gggagggttg tgtctgttct tcatggatca ctatcttagt tagataaatt 4260
   tgtatgatcc aaataaacta aaattgctct aaaaacgtgc gtgagaaacc caaacaccaa 4320
   accatcaatt accatcaagt tcagtagggg tgagtgatgt actcgtgcgt ccgagccaag 4380
   aatgggcgcc tctttcgcaa tagctctccc aaccctgtaa ataatagtat taatgctgtt 4440
   ttttgagtag tgcatatgtg gccactacaa catataataa atatcatgaa cactcataca 4500
   aacatttcca ctattatact aaatctgata cattatgata cgcaataatt gctcctctta 4560
   tagtgcttgt aagactttat tccttcattg cactattcta aatgttaacc ttgaagttaa 4620
   attttaatca caggtatata ctgaccttca ggagctttat ctattcatcc aattcaatgc 4680
   tctaaagtct tcttaagtat tgaaaatatg gtctcacata tgatatcatt ctcaccaggt 4740
   gccagtattt tgtgctatca tcggagctca caaacagtac agccactata tcttttatgg 4800
   tctctgggag cttttccgaa aggggcgccc attcctggct cggacgcgtg agtaccctat 4860
   gggccacagt gccgaaccta caaaaaaccc ctaatatgcc tagacaatta tactgtagga 4920
   tagctcttgc tttcaactaa tgctaaccaa ggggccgctc ggcagtactc gggggtgcat 4980
   gaaagcgacg ggcaccccac gacgtatgtg gcaccagatt atggtgcatg ttcaagcact 5040
   agtcttcagc tctctggtcc tgaccacagg cagtttctaa atggaaaatt acaaaatagt 5100
   aaaatgaaga ttaaatgttg gtcaaggaag actatagcaa tagtgaaaat gaacagtgaa 5160
   ataaaccaca ggacagcgca tggtaaaaag agcaaaaaaa ataccaagac cacaaataca     5220
   aaacaattac acaactcaga agtgggggaa aaactcttca cttcagctac cactctctca     5280
   actgttcaaa atgttgtagg atatggtaca atcttaataa ctctgtatga atatacaatg     5340
   tagtagaaag agtgtattgt gggtaagcta acttattacc tcctatgata atcaatgagt     5400
   atagcaacta caagaaacat atttgaacat cctgcttatt caacttaaag ataagtagac     5460
   atcctatatt ggagtaaggg ttaagtatac catagtcctt tgatcataag cgaatcatct     5520
<210> 64
   <211> 20279
   <212> DNA
   <213> Tuber aestivum
<220>
   <221> misc_feature
   <222> (8849)..(8868)
   <223> n is a, c, g, or t
<220>
   <221> misc_feature
   <222> (17182)..(17201)
   <223> n is a, c, g, or t
<400> 64

## Description

### INVENTION DOMAIN

The invention concerns the setting up of a method for the identification of truffles species and their mating type. The invention is based on the use of mating-specific nucleotide sequences.

An aim of the invention is to control the sexual reproduction of the truffles.

An additional aim of the invention is to distinguish between the truffles species.

The invention concerns the production and the control of truffle inoculated seedlings, with direct impact on the activity and interest of truffles nurseries and agro-food industry.

### STATE OF THE ART AND ENCOUNTED PROBLEM

Truffles are Ascomycete mushrooms belonging to the *Tuber* genus which live in ectomycorrhizal symbiosis with various trees. These mushrooms form fructifications generally named « truffles » which are very appreciated for their organoleptic qualities. Among these truffles species, the Périgord black truffle (*Tuber melanosporum* Vittad.), the Piémont white truffle (*Tuber magnatum* Pico) and the Burgundy Truffle (*Tuber aestivum* Vittad.) are the most appreciated species by the gastronomes and have a large cultural and economic impact. Indeed, their price can vary between 200-400 euro/kg for the Burgundy truffle, 400-1,000 euro/kg for the Périgord truffle and 2,000-6,000 euro/kg for the Piémont white truffle. This very high price is, among other things, the consequence of a high decrease of their production over the last decades. In view to preserve these species and to increase their production, a protocol permitting to inoculate host plantlets, with each of the previously mentioned truffle species has been made to be exploited by truffles nurseries.

This protocol consists to inoculate first young oak, hazel, pin or other host plantlets by mean of spores from several truffles. Each truffle used as spore donor is morphologically controlled to avoid contamination with undesired species. Alternatively, host plants can be inoculated with in vitro grown mycelia.

Then, the development of mycorrhizas on the inoculated plants is controlled 1) to exclude plants that either do not develop mycorrhizas or harbour mycorrhizas from contaminating fungal species according to morphological criteria and 2) to assess whether the percentage of mycorrhization is sufficient. If the level of mycorrhization is sufficient and few contamination are detected the mycorrhized plantlets can be sold. Mycorrhized plantlets means that plantlet roots have been colonized by truffle mycelium and that truffle mycelium and plantlet live in a symbiotic relationship the one with the other.

When these verifications have been met, these mycorrhized plantlets are planted in an orchard. After 5 to 8 years of culturing, it might be obtained truffles of the *Tuber* species that has been inoculated.

The discrimination of *Tuber* vs non *Tuber* mycorrhizas by morphological criteria is often an easy task. However, the discrimination among mycorrhizas of the different *Tuber* species is sometime impossible, such as the typing of mycorrhizas formed by the Périgord Black truffles from those formed by the Chinese black truffles. Mistakes are possible mainly with the Chinese black truffle *Tuber indicum.* Indeed this species is morphologically very similar to the Périgord Black truffle and sometimes their morphological discrimination is impossible. *T. indicum* is a truffle that presents organoleptic qualities lesser pronounced than the ones presented by *T. melanosporum.*

Moreover, despite advances in the inoculation methodology and cultural improvements, some trees and plantations are still sterile without producing truffles. So the quantity of truffles that can be produced by a truffle plant is unpredictable.

### SUMMARY OF THE INVENTION

The inventors have discovered that truffles, and in particular *T*. *melanosporum* and *T. magnatum* are obligate outcrossing fungi, also known as heterothallic fungi. These mushrooms are self-incompatible and therefore need the presence of a partner of opposite mating type to reproduce themselves.

The inventors have discovered that to ensure sexual reproduction of truffle species, the mating between two sexually compatible mycelia is needed. These two mycelia differ for the mating type genes in their genome.

Mating type genes or *MAT* genes are the master loci controlling sexual reproduction and development in fungi.

Sexual reproduction in Ascomycetes filamentous fungi is controlled by two different *MAT* genes that established sexual compatibility: one *MAT* gene codes for a protein with an alpha box domain (MAT1-1-1), whereas the other encodes a high mobility group (HMG) protein (MAT1-2-1). In heterothallic Ascomycetes, there are strains that have only one of these genes in their genome, the other mating type gene is present in different strains at the same site on the chromosome, namely MAT locus. Because this locus contains different genes and other highly divergent sequence, the two versions of a *MAT* locus (MAT1-1 and MAT1-2) are not called allelic and are called idiomorphs (Metzemberg & Glass, 1990, Bioessays 12: 53-59).

To gain insight into the truffle reproduction system, the inventors have tried to identify the nucleotide sequences of the *MAT* locus in each of the most famous truffles species taking advantage from the fact that the genome of the Périgord black truffle has been recently deciphered. Thus, the inventors have first tried to identify, in *T. melanosporum,* the nucleotide sequences corresponding to the two mating type genes. Indeed, due to the high nucleotide sequence divergences between mating type genes within Ascomycetes, the inventors have not immediately found the nucleotide sequences corresponding to the two mating type genes.

The same study has been made to *T. magnatum, T. indicum, T. borchii* and *T. aestivum.*

The inventors have first screened the sequenced *T. melanosporum* genome for the presence of candidate genes encoding for the protein containing the HMG and the alpha-box domain using as queries mating type genes from other Ascomycetes. Following this approach, the inventors have discovered in the *T. melanosporum* genome three nucleotides sequences (three genes: GSTUMT00001090001, GSTUMT00000587001 and GSTUMT00008644001) that have homologies with the HMG containing mating type gene of other Ascomycetes. On the contrary, no *T*. *melanosporum* genes corresponding to the alpha-box mating type genes of other Ascomycetes has been detected, suggesting that *T. melanosporum* is indeed a heterothallic species. For each of the three HMG genes identified in *T.melanosporum* genome, the inventors have designed a specific primer pair to verify their presence in the other *T. melanosporum* strains. Embracing the hypothesis of heterothallism, the putative HMG- mating type gene cannot be present in all *T. melanosporum* strain, since some strains should be of the opposite mating type. Indeed, this prediction was confirmed, as one of the three HMG containing genes (GSTUMT00001090001) was not found in all strains tested. Thus, this gene has been identified as the first mating type gene of *T. melanosporum,* namely *TmelMAT1-2-1.* The HMG box of this first mating type of *T. melanosporum* has as nucleotide sequence the nucleotide sequence SEQ ID N°1.

Having mapped the position of *TmelMAT1-2-1* on the *T. melanosporum* sequenced genome, the inventors have then designed new primers on its flanking regions to amplify the entire idiomorphic sequence both from strains with and without the *TmelMAT1-2-1* gene. In the light of heterothallism in fact, the 5' and 3' regions flanking the two putative idiomorphic structures are expected to be conserved between strains of opposite mating type. Following this approach, the second *MAT* locus (MAT1-11) has been cloned and then sequenced from a strain that lacked the *TmelMAT1-2-1* gene.

Sequence analysis confirmed the identification of the *MAT* idiomorph (MAT1-1) containing a second mating type gene, namely *TmelMAT1-1-1,* said second mating type gene encodes for a putative alpha-box containing transcription factor showing appreciable sequence similarity with the alpha box mating type genes from other Ascomycetes. This alpha box specific to the second mating type gene of *T.melanosporum* has as nucleotide sequence the nucleotide sequence SEQ ID N°2.

Overall, following the above mentioned approaches both MAT idiomorphic regions of *T.melanosporum* have been identified. The first mating type idiomorph MAT1-2 (*TmelMAT1-2*) corresponds to the nucleotide sequence SEQ ID N°3. The second mating type idiomorph MAT1-1 (*TmelMAT1-1*) corresponds to the nucleotide sequence SEQ ID N°4.

The inventors have determined a first genomic region comprising the first mating type gene MAT1-2-1 (or "the first mating type idiomorph MAT1-2") and a second genomic region comprising the second mating type gene MAT1-1-1 (or "the second mating type idiomorph MAT1-1"). Each of these genomic regions comprises also flanking regions that are highly conserved.

The first genomic region has as nucleotide sequence the nucleotide sequence SEQ ID N°5 and the second genomic region has as nucleotide sequence the nucleotide sequence SEQ ID N°6.

By the way of the above identified nucleotide sequences SEQ ID N°1, SEQ ID N°2, SEQ ID N°3, SEQ ID N°4, SEQ ID N°5 and SEQ ID N°6 , the inventors were able to implement an identification method of *T.melanosporum.* But others nucleotide sequences specifics to the two mating type gene of *T.melanosporum* can be used.

The invention permits to differentiate truffles species between them. In particularly, the invention permits to differentiate Chinese truffle (*Tuber indicum* Cook & Massee) with regard to the Périgord Black truffle (T. *melanosporum*).

The invention permits therefore to avoid contamination of plantlets with *T. indicum.* The invention permits to ban the presence of *T. indicum* in the plantlets that will be planted in Europe.

The invention permits also to differentiate other truffles species between them, such as *T. melanosporum, T. magnatum, T. aestivum.*

By the way of the above identified nucleotide sequences, the inventors were also able to screen mycorrhizal plants and determine whether they sustain *T. melanosporum* mycorrhizas of one or both mating types on their roots. More precisely, the method is based on the amplification with mating-type *T. melanosporum* specific primers of DNA isolated from randomly selected pools of *T. melanosporum* mycorrhizas. If the plants harbouring both mating types, the proportion of each mating type can be measured by performing a quantitative PCR analysis or by analyzing randomly selected single ectomycorrhizas and counting the proportion of mycorrhizas harbouring the first or the second mating type.

Likewise, by the way of the above identified nucleotide sequences, the inventors were able to screen *in vitro* cultivated *T.melanosporum* mycelia.

It is also possible to guaranty to the truffle cultivators:
- the mating type(s) of strains present on mycorrhizal plantlets. The methods, in fact, permits to check whether a host plant harbours strains of only one or both mating types of a given *Tuber* species.
- host plant selection according to the mating type of the fungus;
- the setting up of truffle plantations in which the presence of strains of both mating types of a given *Tuber* species is spatially and numerically balanced in the ground. This can be achieved by using plants harbouring strains of both mating type in their roots or by outplanting close each other host plants that harbour in their roots strains of opposite mating type to promote, in open field conditions, the contact and mating between sexually compatible mycelia ;
- to control the presence and the distribution of strains of different mating type in previously realized truffle plantations as well as in natural truffle sites to induce *ex novo* or rescue the truffle production. These analyses permit, in fact, to test whether in a given truffle field the presence of strains of both mating type is spatially and numerically balanced. If not, in order to favour mating between sexually compatible strains, mycorrhized plants harbouring strains with the mating type resulted to be absent or less abundant can be selected and outplanted;
- to control the mating type of mycelia and/or other inocula used for host plant inoculation.

Each mating type gene of a given truffle species can be found with the same method as the one disclosed for obtaining the nucleotide sequence of the *TmelMAT1-2-1* and *TmelMAT1-1-1* as above indicated.

For a given *Tuber* species, it is wanted strains that comprise a first nucleotide sequence that hybridizes with the HMG containing mating type gene of other Ascomycetes. Then, from the flanking regions of this first nucleotide sequence, it is wanted primers in view to amplify a nucleotide sequence corresponding to the second mating type gene of this same given *Tuber* species for the other strains that not contain the first mating type gene.

In an embodiment, each mating type gene of a given truffle species can be identified with the help of parts of nucleotide sequences taken from the first genomic region of *T.melanosporum* and from the second genomic region of *T.melanosporum.* Each mating type gene of a given truffle species can also be identified with the help of parts of nucleotide sequences taken from the first mating type gene of *T.melanosporum* and of the second mating type gene of *T.melanosporum.* These nucleotide sequences can be used as pairs of primers to amplify respectively a first mating type gene and a second mating type gene of a given truffle species.

It is also possible to use the nucleotide sequences of the HMG specific box of *T.melanosporum* and of the alpha specific box of *T.melanosporum* as probes to identify the nucleotide sequences of a first mating type gene and a second mating type gene of a given truffle species.

The invention is used to certify the truffle species, to control fructifications of these species, the identity (and mating type) of their *in vitro* cultured and/or soil living mycelia and mycorrhizas.

The invention permits also to verify the mating type in plantlets inoculated with *T. melanosporum, T. magnatum* and *T. aestivum* which will be sold in the future, and to ensure the presence of mycelia of the two mating types in view to optimize truffles production.

The invention permits to certify the presence of compatible mycelia at the root system level.

The invention also permits to increase fruiting and hence truffle production by controlling *a priori* the presence of mating types on mycorrhized plants used to set a truffle orchard. Also, as reported above, the invention would allow the rescue of man-made and natural plantations by enriching these sites for the presence of strains harbouring the less abundant (or absent) mating type through the introduction of mycorrhized plants specifically selected for the mating type of their fungal partner.

Thus, it is possible to certify to a truffle cultivator the mating type(s) of the truffle strains on the roots of the host plants.

The invention has as object a method for determining the *Tuber* species of a truffle sample, said method comprising identifying whether said truffle sample comprises at least one nucleotide sequence chosen among a first nucleotide sequence associated with a first mating type idiomorph (MAT1-2) and a second nucleotide sequence associated with a second mating type idiomorph (MAT1-1); both nucleotide sequence being specific to a given *Tuber* species.

By truffle sample it is intended that it comprises truffle fructification sample and/or truffle mycelium sample and/or mychorrized plant sample and/or a truffle producing soil sample.

The first nucleotide sequence can be at least one part of the nucleotide sequence SEQ ID N°5. In an example, the first nucleotide sequence can be the nucleotide sequence SEQ ID N°5, or a part of the nucleotide sequence SEQ ID N°5. For example, it can be the nucleotide sequence SEQ ID N°3 or the nucleotide sequence SEQ ID N°1.

The second nucleotide sequence can be at least one part of the nucleotide sequence SEQ ID N°6. In an example, the second nucleotide sequence can be the nucleotide sequence SEQ ID N°6 or a part of the nucleotide sequence SEQ ID N°6. For example, it can be the nucleotide sequence SEQ ID N°4 or the nucleotide sequence SEQ ID N°2.

The nucleotide sequence SEQ ID N°5 is a first genomic region of *T*. *melanosporum* that comprises the first *T. melanosporum* mating type gene *Tmel*MAT1-2-1 or SEQ ID N°3 and the nucleotide sequence SEQ ID N°6 is a second genomic region of *T.melanosporum* that comprises the second *T. melanosporum* mating type gene *Tmel*MAT1-1-1 or SEQ ID N°4.

The invention provides also that the method further comprises determining the fertility of said truffle sample, using the identification in said truffle sample of both the first nucleotide sequence associated with the first mating type idiomorph (MAT1-2) and the second nucleotide sequence associated with a second mating type idiomorph (MAT1-1), the fertility of said truffle sample requiring the presence of both the first and the second nucleotide sequences.

The first nucleotide sequence may comprises at least one part of the nucleotide sequence SEQ ID N°5. In an embodiment, the first sequence may also comprises the nucleotide sequence SEQ ID N°5, or the nucleotide sequence SEQ ID N°3 or the nucleotide sequence SEQ ID N°1. The second nucleotide sequence may comprises at least one part of the second nucleotide sequence SEQ ID N°6. In an embodiment, the second nucleotide sequence may comprises the nucleotide sequence SEQ IDN °6 or the nucleotide sequence SEQ ID N°4 or the nucleotide sequence SEQ ID N°2.

The method may comprises the use of a first pair of oligonucleotide primers to determine the presence of the first mating type idiomorph (MAT1-2) in said truffle sample, each primer of the first pair of oligonucleotide primers comprising from 5 to 50, and preferentially from 10 to 30 successive nucleotides of the first nucleotide sequence. The first nucleotide sequence can be the nucleotide sequence SEQ ID N°5. In an embodiment, the first nucleotide sequence may comprises the nucleotide sequence SEQ ID N°3 or the nucleotide sequence SEQ ID N°1.

A forward primer of this first pair of oligonucleotide primers may comprises the nucleotide sequence SEQ ID N°7. The nucleotide sequence SEQ ID N°7 is taken from the nucleotide sequence SEQ ID N°1. Other specific nucleotide sequences can be used, as the nucleotide sequence SEQ ID N°11 or the nucleotide sequence SEQ ID N°13 that are parts of the nucleotide sequence SEQ ID N°5.

A reverse primer of this first pair of oligonucleotide primers may comprises the nucleotide sequence SEQ ID N°8. The nucleotide sequence SEQ ID N°8 is taken from the flanking region of the nucleotide sequence SEQ ID N°1. Other specific nucleotide sequences can be used, as the nucleotide sequence SEQ ID N°12 or the nucleotide sequence SEQ ID N°14 that are parts of the nucleotide sequence SEQ ID N°5.

The nucleotide sequence SEQ ID N°7 and the nucleotide sequence SEQ ID N°8 serve to amplify a part of the nucleotide sequence SEQ ID N°1.

The nucleotide sequence SEQ ID N°11 and the nucleotide sequence SEQ ID N°12 serve to amplify the nucleotide sequence SEQ ID N°3.

The nucleotide sequence SEQ ID N°13 and the nucleotide sequence SEQ ID N°14 serve to amplify the nucleotide sequence SEQ ID N°5.

The method may comprises the use of a second pair of oligonucleotide primers to determine the presence of the second mating type idiomorph (MAT1-1) in said truffle sample, each primer of the second pair of oligonucleotide primers comprising from 5 to 50, and preferentially from 10 to 30 successive nucleotides of the second nucleotide sequence. As indicated above, the second nucleotide sequence can be the following nucleotide sequences SEQ ID N°2, SEQ ID N°4 or SEQ ID N°6.

A forward primer of this second pair of oligonucleotide primers may comprises the nucleotide sequence SEQ ID N°9. The nucleotide sequence SEQ ID N°9 is taken from the nucleotide sequence SEQ ID N°2. Other specific nucleotide sequences can be used, as the nucleotide sequence SEQ ID N°11 or the nucleotide sequence SEQ ID N°13.

A reverse primer of the second pair of oligonucleotide primers may comprises the nucleotide sequence SEQ ID N°10. The nucleotide sequence SEQ ID N°10 is taken from the nucleotide sequence SEQ ID N°2. Other specific nucleotide sequences can be used, as the nucleotide sequence SEQ ID N°12 or the nucleotide sequence SEQ ID N°14.

The nucleotide sequence SEQ ID N°9 and the nucleotide sequence SEQ ID N°10 serve to amplify a part of the nucleotide sequence SEQ ID N°2.

The nucleotide sequence SEQ ID N°11 and the nucleotide sequence SEQ ID N°12 serve to amplify the nucleotide sequence SEQ ID N°4.

The nucleotide sequence SEQ ID N°13 and the nucleotide sequence SEQ ID N°14 serve to amplify the nucleotide sequence SEQ ID N°6.

The detection of the presence of at least one part of the first nucleotide sequence associated with a first mating type idiomorph of a *Tuber* species and of at least one part of a second nucleotide sequence associated with the second mating type idiomorph of the same *Tuber* species could be done with the help of usual molecular biology tools wheel known from one of ordinary skill in the art.

For example, the detection of this presence can be done in labelling a genomic marker or a specific primer and in hybridizing the genomic marker or the specific primer to an amplified DNA sequence. The marker or the primer can be at least one part of one of the two nucleotide sequences associated with a mating type idiomorph of *T.melanosporum* or an other given *Tuber* species.

The detection of this presence could be done also in labelling cDNA which are able to be detected by at least one part of a first nucleotide sequence associated with a first mating type idiomorph of a *Tuber* species and by at least one part of a second nucleotide sequence associated with a second mating type idiomorph of the same *Tuber* species.

The invention has also as object a nucleotide sequence associated with a first mating type idiomorph (MAT1-2) of a *Tuber* species comprising the nucleotide sequence SEQ ID N°5. The nucleotide sequence SEQ ID N°5 is the first genomic region of *T. melanosporum* that comprises the first mating type gene of *T.melanosporum* and the HMG specific box of *T. melanosporum.*

The invention has also as object a nucleotide sequence associated with a second mating type idiomorph (MAT1-1) of a *Tuber* species comprising the nucleotide sequence SEQ ID N°6. The nucleotide sequence SEQ ID N°6 is the second genomic region of *T. melanosporum* that comprises the second mating type gene of *T.melanosporum* and the alpha specific box of *T. melanosporum.*

The invention has also as object a first pair of oligonucleotide primers associated with a first mating type idiomorph (MAT1-2-1) of a *Tuber* species, wherein each primer comprises from 5 to 50, for preference from 10 to 30 successive nucleotides of the nucleotide sequence SEQ ID N°5.

In an embodiment, this first pair of oligonucleotides primers comprises a forward primer with as nucleotide sequence the nucleotide sequence SEQ ID N°7 and a reverse primer with as nucleotide sequence the nucleotide sequence SEQ ID N°8.

Other forward primers can be used with as nucleotide sequences the nucleotide sequence SEQ ID N° 11 or the nucleotide sequence SEQ ID N°13.

Other reverse primers can be used with as nucleotide sequences the nucleotide sequence SEQ ID N°12 or the nucleotide sequence SEQ ID N°14.

The invention has also as object a second pair of oligonucleotide primers associated with a second mating type idiomorph (MAT1-1-1) of a *Tuber* species, wherein each primer comprises from 5 to 50, for preference from 10 to 30 successive nucleotides of the nucleotide sequence SEQ ID N°6.

In an embodiment, this second pair of oligonucleotides primers comprises a forward primer with as nucleotide sequence the nucleotide sequence SEQ ID N°9, and a reverse primer with as nucleotide sequence the nucleotide sequence SEQ ID N°10.

Other forward primers can be used with as nucleotide sequences the nucleotide sequence SEQ ID N°11 or the nucleotide sequence SEQ ID N°13.

Other reverse primers can be used with as nucleotide sequences the nucleotide sequence SEQ ID N°12 or the nucleotide sequence SEQ ID N°14.

The invention can provides also the development of fast and easy to use a kit to determinate the fertility of a truffle sample, like truffle fructification sample, truffle mycelium sample, mycorrhized plant sample or truffle-producing soil sample; a kit being able to be used even by unspecialized persons. Indeed, it is possible to create a "two in one" kit allowing the identification of the two mating type in a single reaction. The idea is to merge the findings realized by the inventors with the expertise of nanotechnologists to realize such a kit.

The invention has also as object a kit for determining the fertility of a truffle sample, like truffle fructification sample, truffle mycelium sample, mycorrhized plant sample or truffle-producing soil sample, the kit comprising:
- the pair of oligonucleotide primers associated with a first mating type idiomorph (MAT1-2-1) of a *Tuber* species, as indicated above, and
- the pair of oligonucleotide primers associated with a second mating type idiomorph (MAT1-1-1) of a *Tuber* species, as indicated above.

Means for amplifying a nucleotide sequence are also included in this kit. This kit can be use also to identify the truffle species between them.

The nucleotides sequences SEQ ID N°1, SEQ ID N°2, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13 and SEQ ID N°14 are nucleotides sequences that are specifics to the species *T*. *melanosporum.* But others nucleotides sequences specifics to others *Tuber* specie could be used.

The same study has been made to T. indicum.

Since *T. indicum* is an highly polymorphic species (Paolocci et al., 1997 FEMS Microbiology Letters 153: 255-260), some authors regard it as a species-complex (Wang et al., 2006 Mycological Research 110: 1034-1045). The inventors have therefore initially screened a collection of *T. indicum* ascocarps by performing the RFLP analysis of the ITS region described by Paolocci et al. (1997) FEMS Microbiology Letters 153: 255-260 to sort them into the respective molecular RFLP/ITS classes.
The inventors proved that the ascocarps analyzed can be grouped into three ITS classes according to the restriction patterns produced by the endonuclease *RSA*l on ITS1/ITS4 (White *et al.* 1990 Academic Press, New York, pp 315-322) PCR amplicons, hereinafter refereed to as Class A, B1 and B2 .
The inventors then used a set of *T. indicum* ascocarps belonging to each of the three ITS classes for the identification of mating type genes.
The inventors used the *T. melanosporum MAT* primers at low stringent PCR conditions to amplify a portion of *T. indicum* mating type genes. The *T. melanosporum* primer pair SEQ ID N°7/SEQ ID N°8 specific for *TmelMAT1-2-1* gene produced a PCR fragment of approximately 500 bp on some *T. indicum* ascocarps. The *T. melanosporum* primer pair SEQ ID N°9/SEQ ID N°10 specific for *MAT1-1-1* gene produced a PCR fragment of approximately 400 bp, on a different set of *T. indicum* ascocarps. These results were in keeping with the hypothesis of heterothallism in *T. indicum.* As such, the amplification pattern of the *MAT* genes from *T. indicum* ascocarps proved that the gleba of each *T. indicum* samples produced either a 500 bp- or a 400 bp- long PCR fragments and that these PCR fragments shared a high sequence similarity with HMG-box and α-box sequences of *T*. *melanosporum,* respectively.
The inventors have then designed new primers specific to *T. indicum* HMG-box and α-box sequences. As the 5' and 3' regions flanking the two idiomorphs of *T. melanosporum* were expected to be conserved in *T. indicum,* which is phylogenetically related to *T.melanosporum,* the newly designed primers for the *T. indicum HMG-box* and *α-box* genes were then combined with reverse primers designed on the 5' and 3' regions flanking the two idiomorphs of *T. melanosporum* to extend sequence information relative to the *T. indicum MAT* locus. The resulting PCR products from MAT1-2 and MAT1-1 samples were then sequenced. Sequence analysis proved that both *T. indicum* idiomorphs, along with a portion of their flanking regions, were isolated.
As previously observed in *T. melanosporum* (Rubini et al., 2011 New Phytologist 189: 710-722), the spores entrapped within the ascocarp can contribute a slight amount of DNA during the process of nucleic acid isolation from ascocarps. As the spores, differently from the gleba which is only formed by uniparental maternal hyphae, contain DNA of both maternal and paternal origin, the use of MAT specific primers allowed, the inventors to amplify and isolate both MAT indiomorphs from a single *T. indicum* ascocarp. In the light of the high genetic polymorphism of *T. indicum* this approach has enabled the inventors to identify both idiomorphs from samples belonging to the three distinct ITS classes. Thus, both *MAT1-2* and *MAT1-1* idiomorphs were amplified and entirely sequenced from the ascocarps TI_CF10, TI_U983 and TI_U986, belonging to the ITS classes A, B1 and B2, respectively.
The first mating type idiomorph *MAT1-2* corresponds to the nucleotide sequences SEQ ID N° 34, 35 and 36 for TI_U983 (Tind_B1_MAT1-2), TI_U986 (Tind_B2_MAT1-2) and TI_CF10 (Tind_A_MAT1-2) samples, respectively. The second mating type idiomorph *MAT1-1* corresponds to the nucleotide sequences SEQ ID N° 37, 38 and 39 for TI_U983 (Tind_B1_MAT1-1), TI_U986 (Tind_B2_MAT1-1) and TI_CF10 (Tind_A_MAT1-1) samples, respectively.
Within each idiomorph a single *HMG-box* (*MAT1-2-1*) or *α-box* (*MAT1-1-1*) containing gene was identified. The first mating type gene *MAT1-2-1* corresponds to the nucleotide sequences SEQ ID N° 40, 41 and 42 for TI_U983 (*Tind_B1_MAT1-2-1*), TI_U986 (*Tind_B2_MAT1-2-1*) and TI_CF10 (*Tind_A_MAT1-2-1*), respectively. The second mating type gene *MAT1-1-1* corresponds to the nucleotide sequences SEQ ID N° 43, 44 and 45 for TI_U983 (*Tind_B1_MAT1-1-1*), TI_U986 (*Tind_B2_MAT1-1-1*) and TI_CF10 (*Tind_A_MAT1-1-1*), respectively.
The inventors have determined a first genomic region comprising the first mating type idiomorph MAT1-2 which contains the first mating type gene *MAT1-2-1* and a second genomic region comprising the second mating type idiomorph *MAT1-1* which contains the second mating type gene *MAT1-1-1.* Each of these genomic regions comprises also conserved flanking sequences.
The first genomic region correspond to the nucleotide sequences SEQ ID N° 46, 47 and 48 for TI_U983, TI_U986 and TI_CF10, respectively. The second genomic region correspond to the nucleotide sequence SEQ ID N° 49, 50 and 51 for TI_U983, TI_U986 and TI_CF10, respectively.

### BRIEF DESCRIPTION OF THE FIGURES

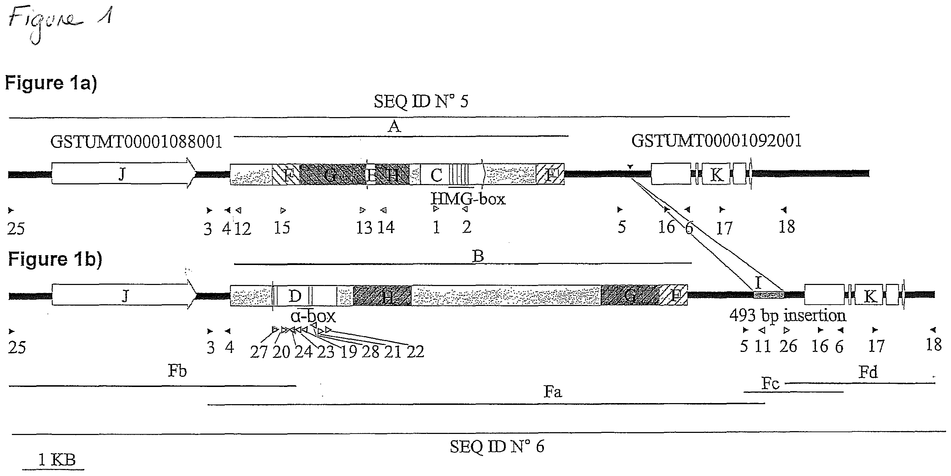

Figures 1 a and 1 b represent schematics representations of *Tuber melanosporum* mating type locus MAT1-2-1 and *Tuber melanosporum* mating type locus MAT1-1-1, respectively.
   A (SEQ ID N°3) and B (SEQ ID N°4) represent respectively the nucleotide sequence for the first mating type idiomorph of *T.melanosporum, Tmel*MAT 1-2, and for the second mating type idiomorph of *T.melanosporum, Tmel*MAT 1-1. The black lines indicate the common flanking regions. C (SEQ ID N°1) and D (SEQ ID N°2) arrows represent respectively the nucleotide sequence for the HMG box (mating type gene *Tmel*MAT 1-2-1) and for the alpha box (mating type gene *Tmel*MAT 1-1-1). An additional putative ORF was detected in the MAT1-2 idiomorph (E arrowed box; gene model: GSTUMT00001089001). The F, G and H boxes represent regions sharing sequence similarities between idiomorphs. The 45° grid pattern indicates the opposite orientation of similar sequences within and between idiomorphs. The flanking region downstream the MAT1-1 idiomorph contains a 493 bp long insertion used to design a backward primer to specifically amplify the MAT1-1 idiomorph (I Box). The arrowed boxes J and K indicate the gene models: GSTUMT00001088001 and GSTUMT00001092001, conserved in the regions flanking the idiomorphs.
   The small black, white and grey arrows indicate the position of common primers or MAT1-1 and MAT1-2 idiomorph-specific primers, respectively. Primers numbers are as in table 1. The black lines at the bottom of the figure 1 indicate the PCR amplicons (Fa, Fb, Fc and Fd) assembled to give the entire MAT1-1-1 contig (15.144 bp).

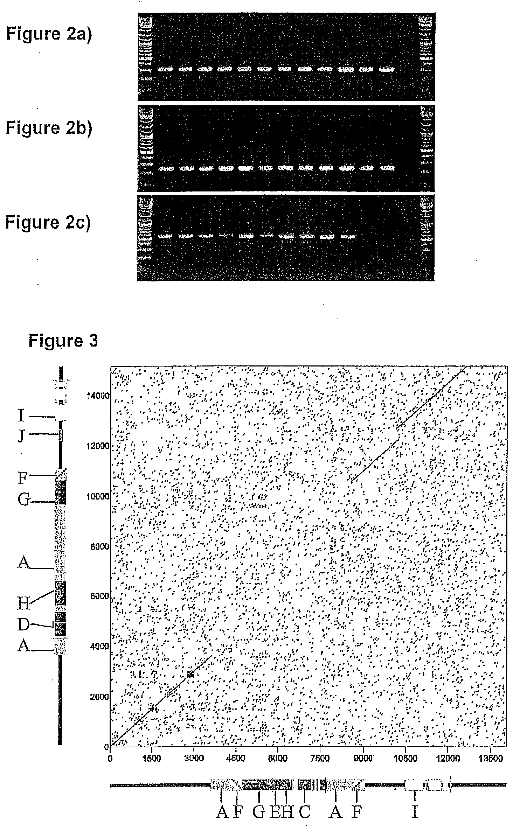

Figures 2a, 2b and 2c represent PCR amplification of three HMG-domain encoding genes in *T. melanosporum.*
   PCR amplifications were performed with the primer pair's p7/p8, p9/p10 and p1/p2 designed on the gene models GSTUMT00008644001 (a), GSTUMT00000587001 (b) and GSTUMT00001090001 (c), respectively.
   Each PCR was performed with genomic DNA from the following ascocarps: lanes 2-13: me443, me448, me458, me459, me66, me67, me68, me300, me303, me225, me206, me151; lane 14: negative control (no DNA template); lanes 1 and 15: DNA ladder mix (Fermentas).
Figure 3 represents a dot-plot comparison of MAT1-1 and MAT1-2 DNA sequences highlighting the extent of the idiomorphic region in each mating type and the repeated sequences. The idiomorphic regions are represented as in figures 1 a and 1 b.
Figures 4a and 4b represent amino acid alignment of conserved regions of *T. melanosporum* MAT proteins with those from others Ascomycetes.

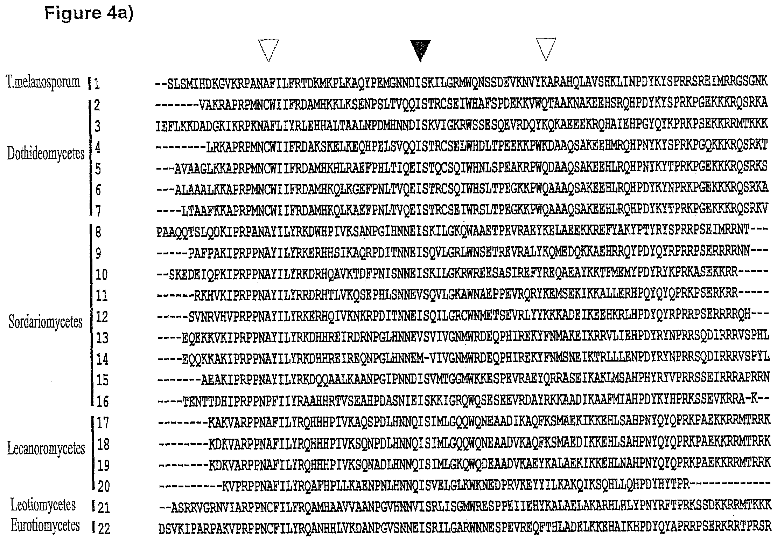

Figure 4a) represents an amino acid alignment of conserved HMG domain of the following species: 1) *Tuber melanosporum*; 2) *Leptosphaeria maculans* (AAO37761); 3) *Mycosphaerella graminicola* (AAL30836); 4) *Pyrenophora teres* (AAY35017); 5) *Cochliobolus sativus* (AAF87724); 6) *Dendryphiella sp.* (AAR83920); 7) *Alternaria alternata* (BAA75908); 8) *Magnaporthe grisea* (BAC65094); 9) *Gibberella fujikuroi* (AAC71056); 10) *Ceratocystis eucalypti* (AAF00498); 11) *Isaria tenuipes* (BAC67543); 12) *Colletotrichum musae* (CAD59615); 13) *Sordaria brevicollis* (CAB63222); 14) *Neurospora crassa* (AAA33598); 15) *Podospora anserina* (CAA45520); 16) *Cryphonectria parasitica* (AAK83343); 17) *Xanthoria flammea* (CAI59780); 18) *Xanthoria elegans* (CAI59778); 19) *Xanthoria polycarpa* (CAI59768); 20) *Cladonia galindezii* (AAT48651); 21) *Pyrenopeziza brassicae* (CAA06843); 22) *Penicillium chrysogenum* (CAP17333).

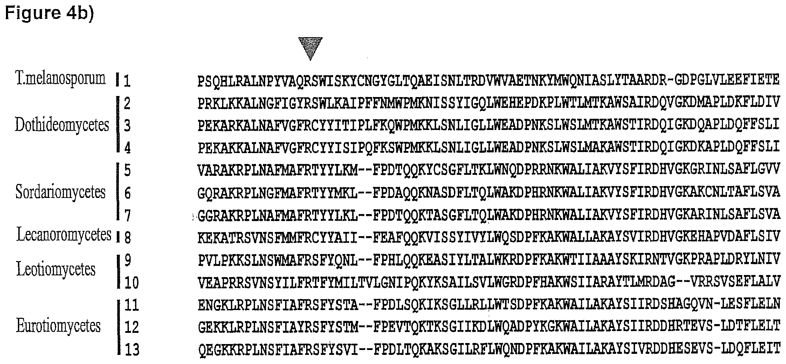

Figure 4b) represents an amino acid alignment of conserved α-box region of the following species: 1) *Tuber melanosporum*; 2) *Phaeosphaeria nodorum* (XP_001791062); 3) *Stemphylium callistephi* (AAR04468); 4) *Alternaria brassicicola* (AAK85542); 5) *Gibberella avenacea* (CAD59608); 6) *Fusarium poae* (CAD59610); 7) *Colletotrichum musae* (CAD59611); 8) *Xanthoria polycarpa* (CAI59771); 9) *Pyrenopeziza brassicae* (CAA06844); 10) *Botryotinia fuckeliana* (XP_001546438); 11) *Ajellomyces capsulatus* (ABO26868); 12) *Penicillium marneffei* (ABC68484); 13) *Neosartorya fischeri* (XP_001263836).
   Ascomycete families are indicated with vertical bars. The position of *T*. *melanosporum* specific introns and those conserved in most of the Ascomycetes are indicated by black and white triangles respectively. In the α-box region alignment, the grey triangles indicate the position of an intron conserved in some Ascomycetes but absent in *T. melanosporum*

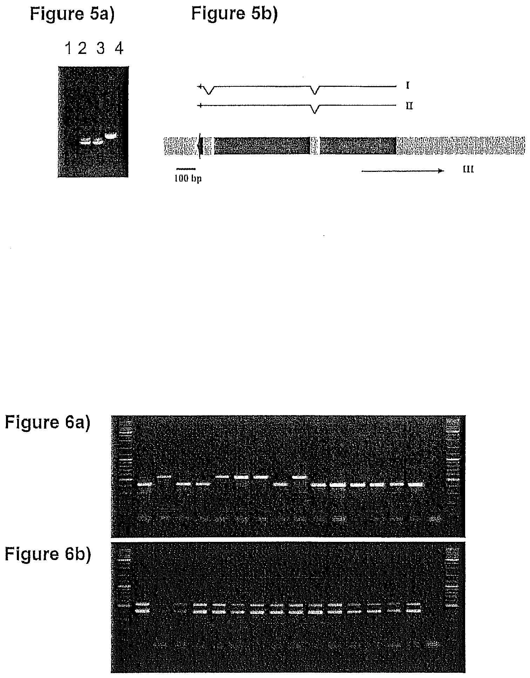

Figures 5a and 5b represent MAT1-1 gene structure as inferred from RT-PCR and RACE analyses. Figure 5a) represents PCR amplification with primers p27 and p28; Lane 1 is a 1 Kb DNA ladder (Fermentas). Lanes 2 and 3 are respectively cDNA from MAT1-1 ascocarps me206 and me151. Lane 4 is a genomic DNA from me206. Figure 5b) represents a schematic representation of MAT1-1 gene structure (dark boxes) and the two different cDNA detected (I and II). The putative antisense transcript (III) is also reported.
Figures 6a and 6b represent respectively a multiplex PCR amplification of DNA from ascocarps and a purified asci with mating type specific primers SEQ ID N°7 (p1); SEQ ID N°8 (p2); SEQ ID N°9 (p19); SEQ ID N°10 (p20).
   Lanes 1 and 18: DNA ladder mix (Fermentas); lanes 2-16: ascocarps me442, me443, me444, me445, me448, me458, me459, me69, me66, me71, me222, me182, me226, me334, me335; lane 17: negative control (no DNA template).

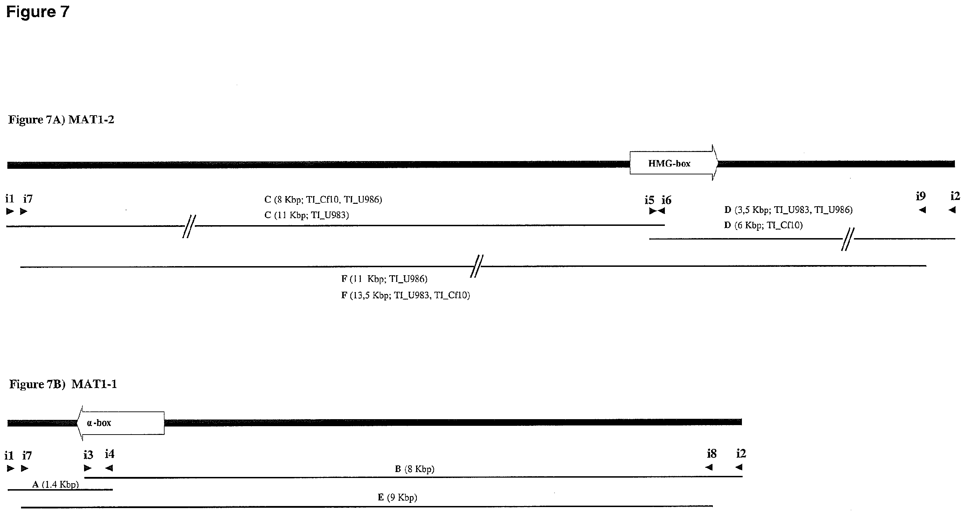

Figure 7 represents the PCR -based strategy followed to isolate the *T*. *indicum MAT1-2* (A) and *MAT1-1* (B) idiomorphs.

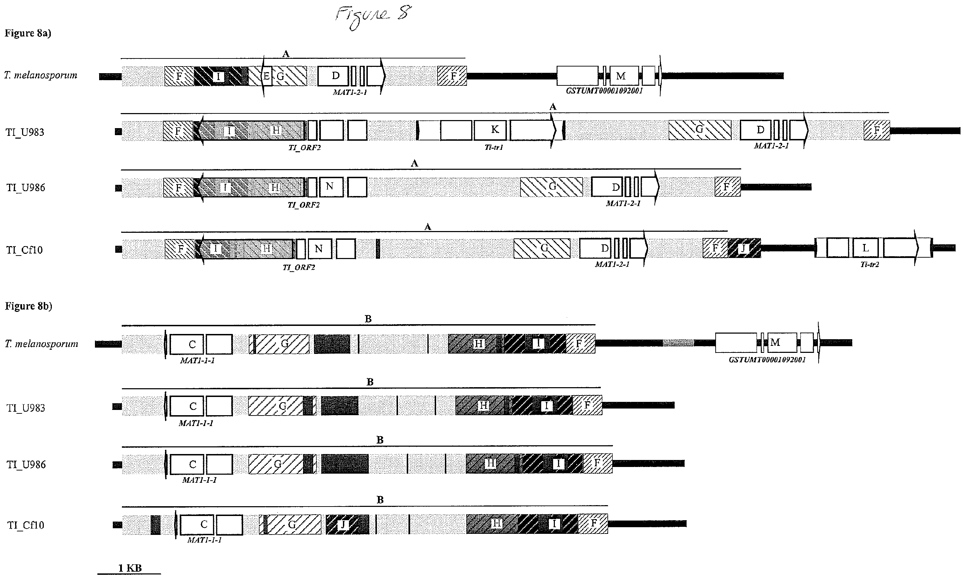

Figure 8 represents the *T. indicum* and *T. melanosporum* mating type regions. A represents the nucleotide sequences (SEQ ID N° 3, 34, 35 and 36) for the first mating type idiomorph (*MAT1-2*) of *T. melanosporum,* and *T. indicum* TI_U983, TI_U986 and TI_CF10 samples, respectively. B represents the nucleotide sequences (SEQ ID N° 4, 37, 38 and 39) for the second mating type idiomorph (*MAT1-1*) of T. *melanosporum* and *T. indicum* TI_U983, TI_U986 and TI_CF10 samples, respectively. The black lines indicate the common flanking regions. C (SEQ ID N°1, 40, 41 and 42) and D (SEQ ID N° 2, 43, 44 and 45) boxes represent the nucleotide sequence of the HMG-box (mating type gene *MAT1-2-1*) and of the α-box (mating type genes *MAT1-1-1*) of *T. melanosporum* and *T. indicum,* respectively. E arrowed box indicates the gene model GSTUMT00001089001. The F, G, H, I and J hatched boxes represent regions sharing sequence similarities between idiomorphs. The 45° grid pattern indicates the opposite orientation of similar sequences within and between idiomorphs. The arrowed boxes K and L indicate the putative transposons Ti_TR1 and TI_TR2, respectively. The arrowed boxes M and N indicates the gene model GSTUMT00001092001 and the TI_ORF2 gene, respectively.

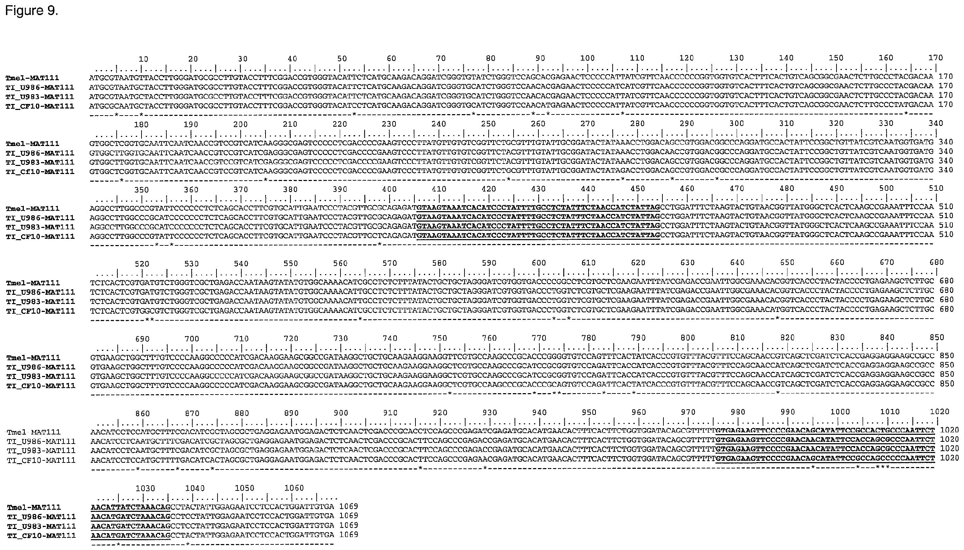

Figure 9 represents the nucleotide alignment of *T. indicum* and *T. melanosporum MAT1-1-1* genes. Polymorphic nucleotide positions are indicated with asterisks; Introns are shown in bold underlined type.

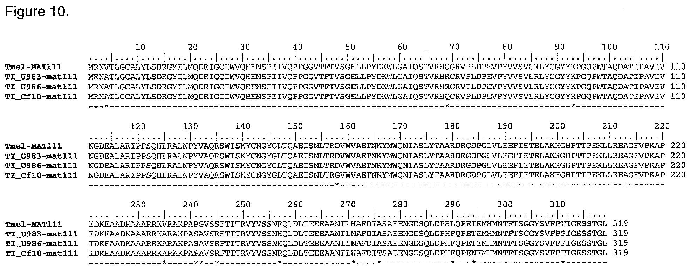

Figure 10 represents the amino acid alignment of *T. indicum* and *T. melanosporum MAT1-1-1* deduced proteins. Polymorphic amino acid positions are indicated with asterisks.

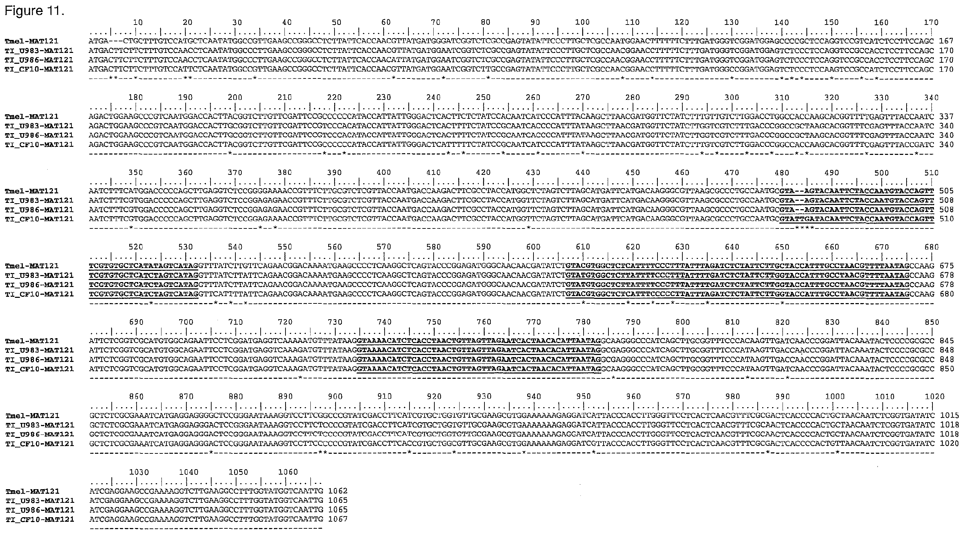

Figure 11 represents the nucleotide alignment of *T. indicum* and *T. melanosporum MAT1-2-1* genes. Polymorphic nucleotide positions are indicated with asterisks; Introns are shown in bold underlined type

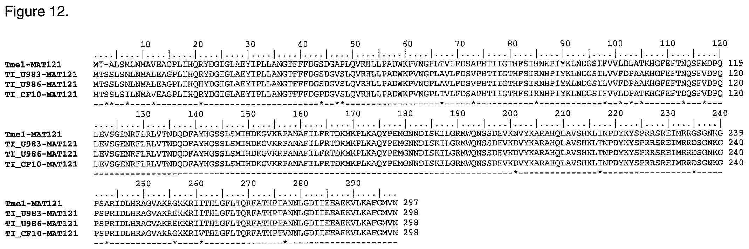

Figure 12 represents the amino acid alignment of *T. indicum* and *T. melanosporum MAT1-2-1* deduced proteins. Polymorphic amino acid positions are indicated with asterisks.

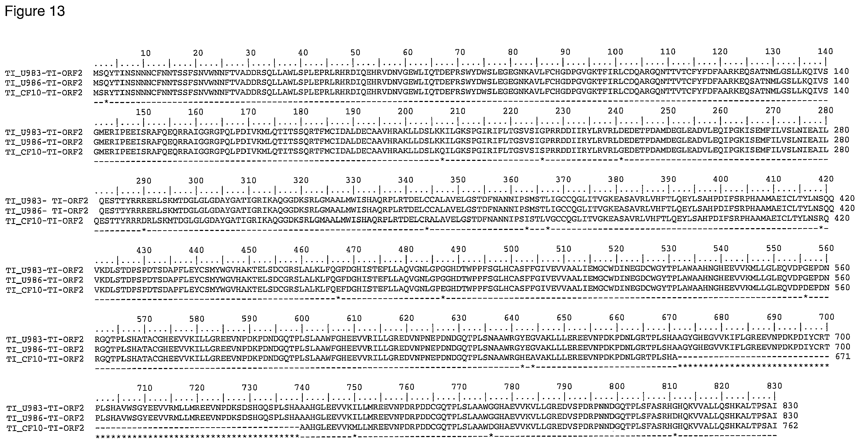

Figure 13 represents the amino acid alignment of *T. indicum* TI_ORF2 hypothetical protein. Polymorphic amino acid positions are indicated with asterisks.

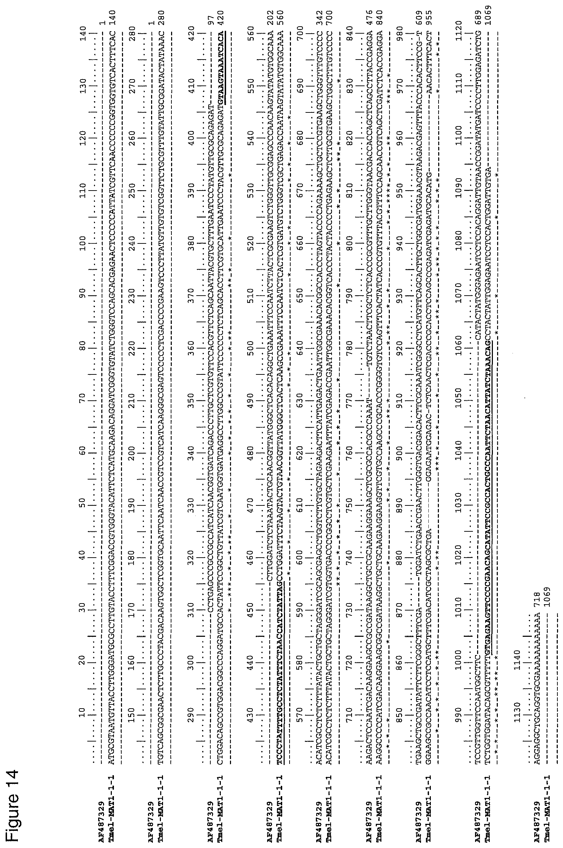

Figure 14 represents the nucleotide alignment of *T. melanosporum MAT1-2-1* gene and *T. borchii* sequence *AF487329.* Polymorphic nucleotide positions are indicated with asterisks. Introns are shown in bold underlined type. Position and length of introns in *T.borchii* were desumed on the basis of the alignment of *AF487329* cDNA with the genomic sequence of *Tmel*MAT1-2-1

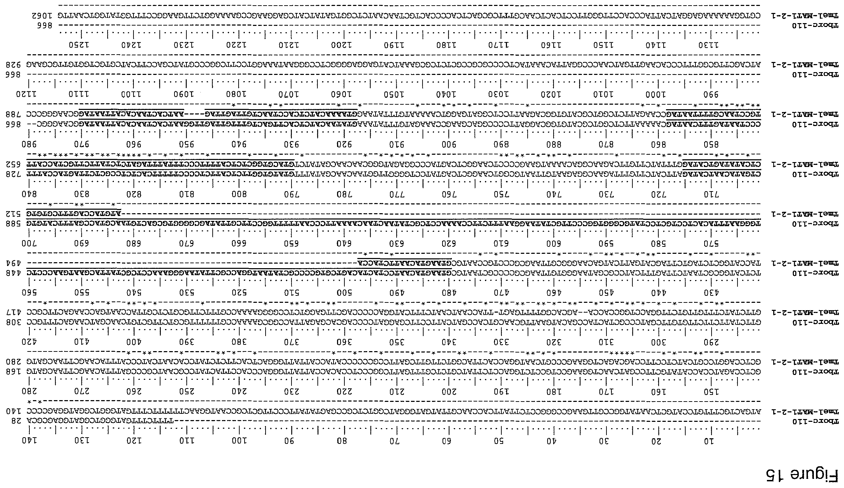

Figure 15 represents the nucleotide alignment of *T. melanosporum MAT1-1-1 gene and T. borchii* sequence obtained with primer pair b3/b4. Polymorphic nucleotide positions are indicated with asterisks. Introns are shown in bold underlined type. Position and length of introns in *T.borchii* were desumed on the basis of the alignment of *T.borchii*MAT1-1-1 with the genomic and cDNA sequences *of Tmel*MAT1-1-1

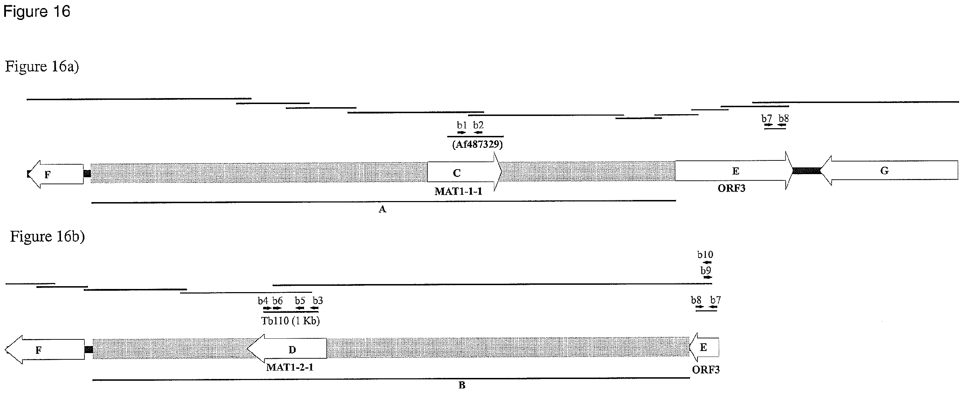

Figure 16 represents the amplification strategy used to sequence the genomic regions containing the *MAT1-1* (a) and *MAT1-2* (b) idiomorphs of *T. borchii.*
   A represents the *MAT1-1* mating type idiomorph whose nucleotide sequence is SEQ ID N° 53.. B represents the *MAT1-2* mating type idiomorph whose nucleotide sequence is SEQ ID N° 55. The thick black lines indicate the common flanking regions. C (SEQ ID N° 52) and D (SEQ ID N° 54) arrowed boxes represent the α-box containing mating type gene (*Tborchii*MAT1-1-1) and the HMG-box containing mating type gene (*Tborchii*MAT1-2-1)*,* respectively. E arrowed boxes indicate genomic regions with homology to *T*. *melanosporum* gene model GSTUMT00001092001 (ORF3). The F and G arrowed boxes represent regions with similarity with an ankyrin repeat-containing protein (XP_0021443441) and a origin recognition complex subunit ORC1 (CBX92828), respectively.
   The thin lines above the schemes of each idiomorph show the fragments, resulting from a combination of Genome walking, Inverse PCR and Long distance PCR procedures, used to assemble these two genomic regions. The small black arrows represent the primers numbered as in Table 1.

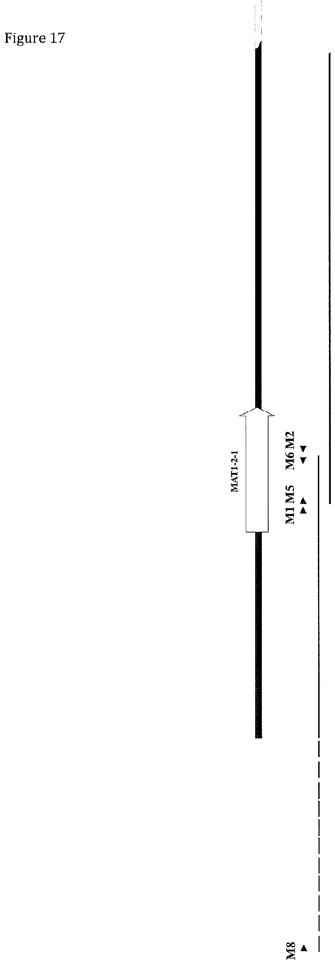

Figure 17 represents the primers M1, M2, M5 and M6 positioned with Mat1-2-1.

### EXPERIMENTATIONS

### A. Tuber melanosporum

### 1. Materials and Methods

### 1.1. PCR amplification and sequencing

The primers' sequences and annealing sites on or near the *MAT* locus are reported in Table 1 and Fig. 1. PCR amplifications were performed in a 50-µl mixture containing 16 mM (NH₄)₂SO₄, 67 mM Tris-HCl (pH 8.8), 0.01% Tween-20, 2.5 mM MgCl₂, 0.2 mM of each dNTP, 10 nM of each primer, 1 U of EuroTaq polymerase (Euroclone, Milano, Italy) and 20 ng of DNA. DNA comes from ascocarps and pools of spores that were isolated according to Paolocci et. al (2006) (complete the references). PCR reactions were performed in a 9700 PCR system (Applied Biosystems, Foster City, CA, USA) with the following thermal profile: 2 min at 94°C followed by 35 cycles of denaturation at 94°C for 30 s, annealing at 55-60°C for 30 s, extension at 72°C for 30 s to 3 min according to the length of the expected amplicon, and a final extension at 72°C for 7 min. Long-range PCR amplification of the *MAT1-1* region (about 10 Kb) was performed using LA Taq DNA polymerase (TaKaRa Bio Inc., Otsu, Japan) and a 2-step thermal profile: 30 s at 94°C, 30-40 cycles of denaturation at 94°C for 30 s, annealing/extension at 68°C for 15 min, and a final extension for 7 min at 72°C. The analysis of simple sequence repeat (SSR) loci was performed according to Riccioni et al. (2008) New Phytologist 180: 466-478.

The PCR amplicons were purified using the EuroGold Cycle-Pure PCR purification kit (Euroclone, Milano, Italy) or cloned into the pGEM T-easy vector (Promega, Madison WI, USA) according to the manufacturer's instructions. Sequences were obtained using the BigDye sequencing kit (Applied Biosystems) according to the manufacturer's protocol. Sequence assembly was performed using Bioedit software v. 7.0.9 (Hall, 1999 Nucleic Acids Symposium. 41: 95-98). Primers were designed with the help of PerlPrimer software v. 1.1.16 (Marshall, 2004 Bioinformatics 20:2471-2472). Sequence comparison and dotplot analysis were carried out using NCBI BLAST and Dotmatcher (EMBOSS Package v. 6.0.1). Gene structure prediction of *MAT1-1-1* was performed with FGENESH software (http://linux1.softberry.com) using *N. crassa* as a reference genome.

### 1.2. RNA isolation, RT-PCR and RACE analyses

Total RNA was isolated from ascocarps stored at -80 °C according to the protocol described by Chang et al. (1999) Plant Molecular Biology Reports 11: 113-116. Rapid amplification of cDNA ends (RACE) was carried out using the SMART-RACE cDNA Amplification Kit (Clontech, Palo Alto, CA, USA) according to the manufacturer's protocol. The 5' and 3' RACE gene-specific primers were p21 and p23, and the nested primers were p22 and p24, respectively (Table 1, Fig. 1). To confirm the structure of *MAT1-1-1* gene, about 5 µg of total RNA isolated from two ascocarps that had gleba with the idiomorph *MAT1-1* were reverse-transcribed using the SuperScript® III RT enzyme (Invitrogen, Carlsbad, CA) and 200 ng of random primers (Invitrogen, Carlsbad, CA) according to the instructions of the enzyme's supplier. First-strand cDNAs were then amplified according to the PCR protocol reported above, using the primers p27 and p28 (Table 1 Figure 1).

**Table 1 list of primers used**

| Primer name | Primer sequence 5'-3' | Primer name | Primer sequence 5'-3' |
|---|---|---|---|
| p1 | CAGGTCCGTCATCTCCTTCCAGCAG (SEQ ID N°7) | p15 | TATCGTACACCACTTTCCTTCTATCTG |
| p2 | CCACATGCGACCGAGAATCTTGGCTA (SEQ ID N°8) | p16 | GATCTTCAGCCACTGGCGAATATCC |
| p3 | CAATCTCTTCCATCGCCCGTCCAG | p17 | GTTCCTCCGATCTTCCAAGAATTCCAG |
| p4 | TGGTATATGTGGATGTATTGATAACTATAAT (SEQ ID N°11) | p18 | TAATTACATTCCCAGAGCTAGGTGCCC (SEQ ID N°14) |
| p5 | AGAGATAGAGAAATAGCATGGCTCGG (SEQ ID N°12) | p19 | CAATCTCACTCGTGATGTCTGGGTC (SEQ ID N°9) |
| p6 | AAGTAACCTTTGTGCCATTGCTCCA | p20 | TCTCGGGCTGGAGGTGCGGGTCGAGT (SEQ ID N°10) |
| p7 | CAGCATGGTCTGGTTGGACGTTTTGAT | p21 | GCCGGAATAGTGGCATCCTGGGCCGTC |
| p8 | CTATGCACCCAAGTGTGATTGAGGAC | p22 | GACGGACGGTTGATTGAATTGCACCGAG |
| p9 | CCATTCGTTGAGATGCGCCGTTGCTA | p23 | GATCGTGGTGACCCCGGCCTCGTGCT |
| p10 | AGGCGGTATCAGAGCTTTTCCATGCAG | p24 | CTTGCGTGAAGCTGGCTTTGTCCCCAAG |
| p11 | GCCAACCTCTAGTTGGGATATTTGTTCAGGAC | p25 | GTGTGCGTGCTTGAAGTGAATTGGAGTAA (SEQ ID N°13) |
| p12 | GTGAATAGACAGACTAGCTTGCCCG | p26 | ATCCTAACTTACAGCATCTCGCCT |
| p13 | AATCTCTTAATGTCCAGACCCTTACC | p27 | TATCGGCTCACAATCCAGTGGAGGATTC |
| p14 | TGAATCAAGGTAGTTTGCTGATTGAGG | p28 | ACGGCCCAGGATGCCACTATTC |

### 2. Results

### 2.1. Searching for mating type genes in T. melanosporum genome.

The *T. melanosporum* genome sequence database "TuberDB" (http://mycor.nancy.inra.fr/IMGC/TuberGenome/) was investigated for the presence of orthologs of Ascomycetes *MAT* genes in the sequenced strain Mel28 using BLASTN and TBLASTX similarity searches. Orthologs, are homologous genes in different species that are similar to each other because they originated from a common ancestor. Using Ascomycetes HMG-containing sequences as queries, 3 candidate gene models were identified in the Mel28 genome: GSTUMT00001090001 (scaffold 247), GSTUMT00000587001 (scaffold 207) and GSTUMT00008644001 (scaffold 49). BLASTP searches against GenBank revealed that the GSTUMT00001090001 predicted protein was the best match for the *MAT1-2-*1 gene from *Diaporthe* sp. (BAE93753, BAE93759), *Cryphonectria parasitica* (AAK83343), *Fusarium sacchari* (BAE94382), and other Ascomycetes. The GSTUMT00000587001 predicted protein showed the best BLASTP match with HMG-box containing proteins, especially with a Ste11 transcription factor of *S*. *pombe* (CAA77507), whereas the GSTUMT00008644001 predicted protein showed similarity with the HMG-box transcriptional regulator of *A. fumigatus* (XP_751745.1). Conversely, BLAST searches using α-box- and homeodomain-containing *MAT* genes from different Ascomycetes as queries did not allow the identification of any significant matches with putative sex genes in the Mel28 genome.

### 2.2. Selection of gene model harbouring the MAT1-2-1 gene and identification of strains harbouring the opposite mating type (figures 1, 2a, 2b and 2c).

To assess for the presence and distribution of the three HMG-box-containing genes in different *T. melanosporum* strains, the primers pairs: p1/p2, p7/p8, and p9/p10, specific to the gene models GSTUMT00001090001, GSTUMT00000587001 and GSTUMT00008644001, respectively, where used to perform a PCR screening on 12 *T*. *melanosporum* ascocarps of different geographical origin.

The GSTUMT00000587001 and GSTUMT00008644001 gene-specific amplicons were obtained from all the ascocarps tested, figures 2a and 2b. Conversely, only ten of the twelve samples screened using the primer pair p1/p2 produced the expected amplicon of 550 bp, figure 2c.

On the basis of these results the GSTUMT00001090001 was selected as the sex- candidate MAT1-2-1 gene and the two samples (me206 and me151) that did not produce the GSTUMT00001090001-specific amplicon regarded to as strains putatively harbouring the opposite mating type idiomorph (*MAT1-1*).

The couple of primers p1/p2 can be used to amplify a nucleotide sequence specific to the first mating type gene of *T. melanosporum* (MAT1-2-1). The primer p1 has, as nucleotide sequence, the nucleotide sequence SEQ ID N°7 and the primer p2 has, as nucleotide sequence, the nucleotide sequence SEQ ID N°8.

### 2.3. Isolation of MAT1-1 idiomorph (figure 1, table 1).

Embracing the hypothesis of heterothallism, the 5' and 3' regions flanking the two putative idiomorphic structures are expected to be conserved between the sequenced strain (Mel28) and strains that lack the *MAT1-2-1* genes such as those that form the gleba of truffles me206 and me151.

Thus, on Mel28 strain, a set of primers were designed upstream and downstream the *MAT1-2-1* gene to search for genomic regions conserved between strains of different mating type (Table 1, figures 2a,2b,2c). To this purpose, on the 5' MAT1-2-1 flanking region, the primer pairs p3/p12, p13/p14, p15/p14 and p3/p14 (Table 1, Fig. 1) were tested for their capacity to yield amplicons of expected length of about 550, 400, 1700 and 2800 bp, respectively, on all samples screened. These primers pairs produced amplicons only when strains containing the *MAT1-2* idiomorph were amplified. Conversely, the primer pair p3/p4 yielded an amplicon of about 370 bp from both *MAT1-2* and *MAT1-1* strains. On the 3' *MAT1-2-1* flanking region of both the *MAT1-2* and *MAT1-1* strains, the primer pairs p16/p6 and p17/p18 yielded amplicons of about 400 and 1000 bp, respectively. The primer pair p5/p6 produced an amplification product of about 1200 bp in *MAT1-2* strains and a fragment of about 1700 bp in MAT1-1 strains. Overall, these preliminary screenings show that the binding capacity of p3, p4 and of p5, p6 primers was conserved in the upstream and downstream *MAT1-2-1* gene regions, respectively, in strains both with and without the *MAT1-2* idiomorph.

The PCR fragments obtained from sample me206 with primer pairs p3/p4 and p5/p6 were cloned and sequenced. The alignments showed that that these two fragments were highly similar (about 96% of sequence identity) to the corresponding regions in the Mel28 genome, although the fragment generated by the primer pair p5/p6 (figure 1, fragment Fc) showed an insertion of 493 bp in the 3' region downstream the MAT1-1 idiomorph (figure 1b).

On this insertion, the primer p11, specific for *MAT1-1* strains, was designed and used in combination with the primer p3 to amplify the genomic region containing the *MAT1-1* idiomorph from strain me206.

The amplification with the primer p11 and the primer p3 yielded a fragment of about 9 Kbp (figure 1, fragment Fa) whose sequencing confirmed the identification of the *MAT1-1* idiomorph. Indeed, BLASTX search against GenBank revealed the presence of about 200 bp region with similarity to the α-box domain that typifies the *MAT1-1-1* gene of filamentous Ascomycetes. In particular, BLASTX matches were observed with α-box domains of MAT1-1 proteins of *Alternaria brassicae* (AAK85543.1), *Talaromyces stipitatus* (XP_002486084), *Aspergillus nidulans* (XP_660359), *Pyrenophora sp.* (CAP08782), and *Penicillium marneffei* (ABC68484).

The genomic fragment containing the *MAT1-1* idiomorph was further extended by PCR at the 5' and 3' ends using the primers p24 and p26 coupled with the MAT1-2 primers p25 and p18, respectively. The sequenced fragments Fa, Fb, Fc, and Fd were assembled in a 15,144 bp contig (figure 1).

The couple of p19/p20 was then designated and can be used to amplify a nucleotide sequence specific to the second mating type gene of *T*. *melanosporum* (MAT1-1-1). The primer p19 has, as nucleotide sequence, the nucleotide sequence SEQ ID N°9. The primer p20 has, as nucleotide sequence, the nucleotide sequence SEQ ID N°10.

Mating type specific amplification can be also obtained using any others couples of primers that are specific to the first and/or to the second mating type idiomorph of *T.melanosporum.*

The entire *MAT1-2* idiomorph sequence or SEQ ID N°3 nucleotide sequence is available in *T. melanosporum* genome database "TuberDB" (http://mycor.nancy.inra.fr/IMGC/TuberGenome/).

The entire MAT1-1 idiomorph sequence or SEQ ID N°4 was deposited in GenBank under the accession number HM370280.

### 2.4. Structure of MAT idiomorphs.

Sequence alignment and dotplot analyses of the two genomic regions showed that the *MAT1-2* and *MAT1-1* idiomorphs were about 5550 bp and 7430 bp long, respectively (A in figure 1a, B in figure 1b and figure 3). The MAT1-2 idiomorph has as nucleotide sequence the nucleotide sequence SEQ ID N°3 and the MAT1-1 idiomorph has as nucleotide sequence the nucleotide sequence SEQ ID N°4. The sequences flanking these idiomorphs were highly conserved: pairwise sequence alignment showed that the 5' flanking regions (3603 bp) share 99,1% sequence identity and the 3' flanking regions (3586 bp), excluding the 493-bp long insertion of MAT1-1, share 91,3% of identity.

Each idiomorph contains only a single *MAT* gene (HMG or α-box containing gene).

However, blast and dotplot analyses (figures 4 and 3) revealed that the idiomorphic regions were not completely different. In fact, the presence of shared sequences arranged in inverse orientations was observed (figure 1). Pairwise alignments showed 960 bp- (figure 1: G box) and 950 bp- (figure 1: H box) long sequences sharing 76.5% and 71.1% sequence identity, respectively. Additionally, BLASTX analysis revealed a region within the 960-bp sequence (G box) with similarity to conserved ankyrin repeat domains. An EST sequence (SY0AAA61YO16FM1) from Mel28 mycelium relative to GSTUMT00001089001 proved that the transcript of this gene extends its 3' end into the ankyrin-containing region, but that this last region is not included in the predicted CDS because it is only 57 amino acids long. Furthermore, no ORF could be identified in the corresponding region of the MAT1-1 idiomorph.

Finally, a short repeated sequence of about 460 bp was present in inverse orientation in the *MAT1-2* idiomorph (figure. 1: F boxes). A similar sequence (84% sequence identity) was also present at the extreme 3' end of the MAT1-1 idiomorph.

### 2.5. Structure of MAT genes.

The analysis of EST produced by the *Tuber* Genome Consortium showed that the *T. melanosporum MAT1-2-1* gene (*TmelMAT1-2-1*) consisted of 4 exons since it showed 2 additional introns to the one conserved in most of the Ascomycetes (figures 1a and 4a). The deduced amino acid sequence was 297 residues long and contained a conserved HMG-box motif typical of the *MAT1-2-1* genes of Ascomycetes (figure 4a).

Gene prediction analysis (performed with FGENESH in the *MAT1-1* idiomorph) suggested the presence of 3 exons within the *T. melanosporum MAT 1-1-1* gene (*TmelMAT1-1-1*), figure 1b. RACE and RT-PCR analyses were performed on RNA isolated from fruiting bodies harbouring the *MAT1-1* idiomorph to confirm the predicted gene structure. Interestingly, these analyses showed that the second intron could be differentially spliced to give putative proteins of either 319 or 399 aminoacids, (figures 5a and 5b). When RACE analysis was performed using the polyA-anchored primer in combination with the primers for 5' RACE (p21, p22) on the 5' extended cDNA pools as control, it revealed the presence of an antisense transcript partially overlapping the MAT1-1-1 CDS (figures 5a and 5b).

### 2.6. MAT genes are expressed both during the vegetative and reproductive phases.

RT-PCR analyses along with the availability of ESTs (http://mycor.nancy.inra.fr/) from fruit body (FB) free living mycelia (FLM) and ectomycorrhizae (ECM) (Martin et al. 2010, Nature 464:1033-1038) allowed the inventors to evaluate transcription of MAT genes at different phases of the *T. melanosporum* life cycle.

The analysis of EST datasets confirmed that in FLM of the Mel28 strain used for genome sequencing, only the *MAT1-2-1* gene was transcribed. On the contrary, both *MAT1-1-1* and *MAT1-2-1* transcripts were detected in the EST libraries obtained from FB and ECMs.

### 2.7. MAT idiomorphs are equally distributed among truffles within single populations.

The presence and distribution of mating type genes in the ascomata collected from natural populations were assessed using a multiplex-PCR approach. To this end, the α-box-specific primer pair p19 (SEQ ID N°9) / p20 (SEQ ID N°10) was designed to produce an amplicon of 421 bp and combined with the HMG box-specific primer pair p1 (SEQ ID N°7) / p2 (SEQ ID N°8) that produced an amplicon of 550 bp. As shown in Table 2, truffles harbouring either the *MAT1-2* or the *MAT1-1* idiomorphs were identified within each local population.

### 2.8. All T. melanosporum truffles results from outcrossing.

To test the hypothesis that all *T. melanosporum* truffles were derived from outcrossing and that *T. melanosporum* is a "truly" heterothallic spp., the multiplex PCR approach described above was carried out on asci and gleba isolated from single, selected ascocarps.

When screened with SSR and single nucleotide polymorphism (SNP) markers, the asci of most ascocarps did not display any additional alleles with respect to their corresponding gleba (Riccioni et al., 2008, New Phytologist 180: 466-478). Whether these truffles resulted from the crossing of two parents or just from selfing of homothallic strains remained elusive. Multiplex PCR-based mating type screening showed that the gleba of these ascomata had either the MAT1-2- or MAT1-1-specific allele (figure 6a). In stark contrast, the pool of spores always presented, with an approximately equal intensity, of both MAT1-2- and MAT1-1-specific amplicons, irrespective of the truffle samples processed, the mating type of the corresponding gleba, and the number of PCR cycles performed (figure 6b).

Altogether, these results suggest that the additional mating idiomorph detected in the asci with respect to the surrounding gleba must be contributed by a partner with a different sexual polarity, and that this condition is common to all truffles screened.

**Table 2 : List of truffles whose gleba was genotyped using mating-specific primers**

| **Sample** | **Population** | **Mating type** |
|---|---|---|
| 442 | 2 | α-box |
| 443 | 2 | hmg |
| 444 | 2 | α-box |
| 445 | 2 | α-box |
| 446 | 2 | α-box |
| 448 | 2 | hmg |
| 458 | 2 | hmg |
| 471 | 2 | α-box |
| 478 | 2 | α-box |
| 459 | 2 | hmg |
| 476 | 2 | α-box |
| 482 | 2 | hmg |
| 280 | 2 | α-box |
| 284/287 | 2 | hmg |
| 281 | 2 | α-box |
| 206 | 2 | α-box |
| 151 | 2 | α-box |
| 66 | 6 | hmg |
| 69 | 6 | α-box |
| 67 | 6 | hmg |
| 68 | 6 | hmg |
| 71 | 6 | α-box |
| 70 | 6 | hmg |
| 296 | 7 | hmg |
| 300/303 | 7 | hmg |
| 298 | 7 | α-box |
| 180 | 10 | α-box |
| 224 | 10 | hmg |
| 222/182/226/334/335/227 | 10 | α-box |
| 225 | 10 | hmg |
| 336 | 10 | α-box |
| 337 | 10 | hmg |
| 183 | 10 | α-box |
| 294 | 12 | hmg |
| 367 | 12 | α-box |
| 81 | 12 | hmg |
| 83 | 12 | hmg |

### B. Tuber indicum

### 1. Materials and Methods

### 1.1. Fungal samples and PCR analysis of ITS

*T. indicum* ascocarps used in this study are reported in Table 3. The genomic DNA was isolated from ascocarps according to Paolocci et al. (2006) Applied and environmental microbiology 72: 2390-2393. The PCR amplification and RFLP analysis of ITS region were performed using ITS1 and ITS4 primers (White *et al.* 1990 Academic Press, New York, pp 315-322) and *RSA*I endonuclease according to Paolocci et al. (1997) FEMS Microbiology Letters 153: 255-260.

**Table 3. List of T. indicum ascocarps**

| *T. indicum* (code) | ITS/RFLP class | MAT* |
|---|---|---|
| TI_C69 | A | 1-1 |
| TI_CF11 | A | 1-2 |
| TI_C80 | A | 1-1 |
| TI_D5 | A | 1-1 |
| TI_D9 | A | 1-2 |
| TI_CF10 | A | 1-2 |
| TI_TC37 | A | 1-1 |
| TI_RIBC | B1 | 1-2 |
| TI_C20 | B1 | 1-1 |
| TI_D54 | B1 | 1-1 |
| TI_U983 | B1 | 1-2 |
| TI_F7 | B1 | 1-2 |
| TI_LI2 | B1 | 1-1 |
| TI_LI8 | B2 | 1-2 |
| TI_CF7 | B2 | 1-1 |
| TI_C8 | B2 | 1-2 |
| TI_C18 | B2 | 1-2 |
| TI_C38 | B2 | 1-2 |
| TI_TC61 | B2 | 1-1 |
| TI_U986 | B2 | 1-2 |

| | | |
|---|---|---|
| *PCR performed with *T. melanosporum* primers P1/P2 (*MAT1-2*) and P19/P20 (*MAT1-*1) | | |

### 1.2 PCR amplification and sequencing of mating types

The primers used in this study are reported in Table 4 and Fig. 7. PCR amplification of HMG-box and α-box containing fragments of *T. indicum* was performed as described above for *T. melanosporum* except that the annealing temperature was lowered to 50°C.

Long distance PCR amplification of MAT1-1 and MAT1-2 fragments and of the entire idiomorph containing regions was performed using LA-Taq DNA polymerase (TaKaRa Bio Inc., Otsu, Japan) using a 2 step thermal profile: 30 s at 94 °C followed by 25-30 cycle of denaturation at 94 °C for 30 s and annealing/extension at 68 °C for 10-15 min according to the length of the amplicons, and final extension of 7 min at 72 °C.

In order to amplify both idiomorphs from a single ascocarp, a long distance PCR was performed as described above by using either the *MAT1-2* or the *MAT1-1* idiomorph specific primer pairs reported above. The number of PCR cycles was increased up to 50.

Sequencing, sequence assembly and analysis and primers design were performed as described above for *T. melanosporum.*

**Table 4. List of primers**

| Primer name | Primer sequence 5'- 3' |
|---|---|
| i1 | ACGTGGCAYTCTCTATTGGAGGATTCTG |
| i2 | ATYTTGCGCAGTTTATKGTCGCTCACAG |
| i3 | CCAGAAGTGAAAGTGTTCATGTGCATCTCG |
| i4 | CCCTACTACCCCTGAGAAGCTCTTGCG |
| i5 | ATGGTTCTATCTTGGTCGTCTTTGACCCG |
| i6 | TGGTAGGCGAAGTCTTGGTCATTGGTAAC |
| i7 | TCCTGCCTTAGGCCAATCCGCTTT |
| l8 | TGGACATGGGATCGGGTGGGAATAC |
| l9 | GGGATTGGGTGGGAGTATTGATCCTCG |

### 2. Results

PCR amplification of the ITS region produced an amplicons of approximately 600 bp in all *T. indicum* samples. The RFLP analysis of the ITS amplicons allowed the identification of three genetic classes (Table 3) that were named *T. indicum* A, *T. indicum* B1 and *T. indicum* B2 according to Paolocci et al. (1997) FEMS Microbiology Letters 153: 255-260.

### 2.1 Identification of T. indicum MAT genes

Amplification of a portion of the mating type genes from *T. indicum* samples was first performed by using, under low stringent PCR conditions (see M&M), *T. melanosporum MAT* primers pairs P1/P2 (SEQ ID N°7 / SEQ ID N°8) and P19/P20 (SEQ ID N° 9 / SEQ ID N° 10) specific for *MAT1-2* and *MAT1-1* genes, respectively (Table 4). The primer pair P1/P2 produced an amplicon (approximately 500 bp) on 11 out of the 20 *T. indicum* ascocarps analyzed. These 11 ascocarps were then considered *MAT1-2.* The remaining nine ascocarps were classified as *MAT1-1* because a PCR amplicon (about 400 bp) was obtained when amplified with the primer pair P19/P20 (Table 3).

The PCR fragments from three *MAT1-2* ascocarps (TI_U983, TI_U986 and TI_CF10) and from three *MAT1-1* ascocarps (TI_TC61, TI_LI2, and TI_TC37), one representative of each of the three ITS/RFLP classes, were cloned and sequenced. Sequence analysis showed that these PCR fragments shared high similarity with HMG-box and α-box sequences of *T*. *melanosporum,* respectively.

### 2.2. Isolation of T. indicum MAT idiomorphs.

In order to isolate the *T. indicum* idiomorphs, two *MAT1-2-1* (named i5 and i6) and two *MAT1-1-1* (named i3 and i4) primers were then designed and combined with the primers i1 and i2 designed on the 5' and 3' flanking regions of *T. melanosporum* (Table 4, Figure 7). Using these primers, two fragments, spanning the entire idiomorphic region, were amplified from each of the six *T. indicum* samples. More specifically, the primer pairs i1/i4 and i2/i3 produced fragments of about 1.3 Kbp and 8 Kbp, respectively, in all three *T. indicum MAT1-1* samples (TI_LI2, TI_TC61 and TI_TC37). The primer pair i1/i6 produced a fragment of about 8 Kbp from *MAT1-2* samples TI_U986 and TI_CF10 and a fragment of 11 Kbp from TI_U983. The primer pair i2/i5 produced a fragment of 3.5 Kbp from TI_U983 and TI_U986 samples and a fragment of 6.5 Kbp from TI_CF10 sample (Figure 7).
This PCR-based analysis showed a similar orientation of *MAT1-2-1* and *MAT1-1-1* genes between *T. indicum* and *T. melanosporum* and length polymorphism in the *MAT1-2-1* idiomorphs of *T. indicum* samples.

In order to selectively amplify the complete *T. indicum* regions containing the *MAT1-2* and *MAT1-1* idiomorphs, the 5' and 3' ends of *T. indicum* PCR amplicons A, B, C and D (Figure 7) were sequenced and aligned. Then the *MAT1-2-* and *MAT1-1-* specific primers i8 and i9 were designed on the 3' flanking region and the primer i7 common to both idiomorphs on the 5' flanking region (Table 4, Figure 7).The flanking regions were identified by comparison with *T. melanosporum MAT* locus.

The amplification pattern of *MAT* genes in *T. indicum* is consistent with a heterothallic organization of the *MAT* locus because the gleba of different *T. indicum* ascocarps produced the *MAT1-1* or *MAT1-2* specific amplicon. However, as previously observed in *T. melanosporum* (Rubini et al., 2011 New Phytologist 189: 710-722), the spores entrapped within the ascocarp contribute a slight amount of paternal DNA during the process of nucleic acid isolation. By using appropriate PCR conditions and MAT specific primers, DNA contributed from the spores in addition to that contributed by the gleba was PCR amplified enabling the isolation of both mating type sequences from a single ascocarp.
In turn, this approach ensures that both idiomorphs belong indeed to the same biological species. Whether in fact all the genetic variants exhibited by ascocarps showing the *T. indicum* morphotype belong to the same species is still a matter of debate among mycologists (Wang et al., 2006 Mycological Research 110: 1034-1045; Chen et al., 2011 PLoS ONE 6: e14625). It remains to be elucidate in fact whether or not *T. indicum* strains of different ITS classes are cross-fertile.
Thus, the *T. indicum* ascocarps TI_U983, TI_U986 and TI_CF10 were selected and by using the *MAT1-1* idiomorph-specific primer pair i7/i8 a 9 Kbp long amplicon was obtained from each of the three ascocarps.
Similarly, by using the *MAT1-2* idiomorph-specific primer pair i7/i9, an amplicon of about 13.5 Kbp was obtained from TI_U983 and TI_CF10 samples, whereas a 11 Kbp amplicon was obtained from TI_U986 (Figure 7, Figure 8).
These six amplicons were completely sequenced and deposited in GenBank under the accession N°.

### 3. Structure of T. indicum MAT idiomorphs and comparison with their hortologs from T. melanosporum

Sequence alignment, BLAST and gene prediction analyses were performed to evaluate length and structure of *MAT1-1* and *MAT1-2* idiomorphs and to identify the putative coding regions. The organization of *T. indicum MAT* idiomorphs, compared with those of *T. melanosporum* is reported in Figure 8.

Blast analysis revealed that, like in *T. melanosporum,* the *T. indicum* idiomorphic regions were not completely different each other for the presence of shared sequences arranged in inverse (Figure 8; "G", "H", "I" and "F" hatched boxes) or similar (Figure 8 "J" hatched box) orientation. Most of these regions were also shared with *T. melanosporum.* The exceptions were region "J" which is present only in the idiomorphs of *T. indicum* TI_CF10, and region "H" that was absent in *T. melanosporum MAT1-2.*

The idiomorph sequences of *T. indicum* differed each other and from that of *T. melanosporum* for the presence of some indels ranging from few bp to approximately 160 bp. These indels are indicated with grey boxes in Figure 8.

### 3.1. T. indicum MAT1-1

*T. indicum MAT1-1* idiomorph is 7.583 bp long in TI_U983, 7.741 bp in TI_U986 and 7.666 bp in TI_CF10. The structure and sequence is similar to that of *T. melanosporum MAT1-1* which is 7.430 bp long.

Gene prediction analysis of *MAT1-1* showed the presence of a single gene (Figure 8b) exhibiting high similarity (97% of sequence identity) with *T*. *melanosporum MAT1-1-1* gene (Figure 9). The deduced *MAT1-1-1* encoded protein is 319 amino acids long in each of the three *T. indicum* strains. *T. indicum* TI_U983 and TI_U986 shared identical protein sequences whereas *T. indicum* TI_CF10 contained nine polymorphic sites with respect to the other two strains. *T. indicum MAT1-1-1* proteins of TI_U986 and TI_U983 differed from *T. melanosporum MAT1-1-1* for 9 amino acids, whereas TI_CF10 for 7.

### 3.2. T. indicum MAT1-2

*T. indicum MAT1-2* idiomorph is 9.783 bp, 10.096 bp and 12.131 bp long in TI_U986, TI_CF10 and TI_U983, respectively. Compared to *T*. *melanosporum* which is only 5.550 bp long, it contains a large insertion (about 4.300 bp in TI_U986 and TI_CF10 and about 6.650 bp in TI_U983) between the "I" and "G" regions (Figure 8a).

Differently from *MAT1-1,* where a single ORF was identified, two ORFs were predicted within TI_U986 and TI_CF10 and three ORFs in TI_U983 *MAT1-2* idiomorph (Figure 8a).

In each of the three *MAT1-2* amplicons, gene prediction analysis confirmed the presence of an ORF exhibiting high similarity (about 94% of sequence identity) with *MAT1-2-1* gene of *T. melanosporum* (Figure 8a, Figure 11). *T. indicum MAT1-2-1* gene encoded a hypothetical protein of 298 amino acids with TI_U983 differing from TI_U986 for only one amino acid. Conversely, TI_CF10 differed for thirteen and fourteen amino acids from TI_U983 and TI_U986, respectively... *T. indicum MAT1-2-1* deduced proteins of TI_U986, TI_U983 and TI_CF10 differed from *T. melanosporum MAT1-2-1* for 19, 17 and 13 amino acids, respectively, and for an insertion of a single amino acid in the N-terminal region (Figure 12).
The second ORF identified in each *T. indicum MAT1-2* was named *TI_orf2.* IT consists of 4 exons and was in reverse orientation respect to *MAT1-2-1* (Figure 8a). The *TI_orf2* codified for a hypothetical protein of 830 amino acids in TI_U986 and TI_U983 and 762 amino acids in TI_CF10 (Figure 13). BlastP analysis against GenBank database showed high similarity (E-value < 8e -45) with some uncharacterized Ankyrin containing proteins of ascomycetes (i.e. XP_002487679, XP_002382023, XP_002144344). Searches against the Conserved Domains Database (CCD) at NCBI revealed the presence of a conserved ankyrin domain (ANK) at the C-termini of the predicted amino acidic sequence. Amino acid alignment showed that the length difference between the ORF2 proteins of TI_U986/TI_U983 and TI_CF10 resides in the C-terminal region and consists of a variation on the number of ankyrin repeat units.
The 4^{th} exon of *TI_orf2* gene in the MAT1-2 idiomorph spans the regions "H" and "I" (Figure 8a). A partially similar region (about 85% nucleotide identity) was also present in inverse orientation in *MAT1-1* idiomorph of both *T. indicum* and *T. melanosporum.* However, the *orf2* is not functional in *MAT1-1* because the first three exons were completely absent and the region corresponding to the 4^{th} exon contains several stop codons.

A third additional ORF was identified in TI_U983 *MAT1-2.* In this sample the *MAT1-2* idiomorph was longer than those of the other two samples for the presence of an insertion of 2.350 bp. Gene prediction analysis and BlastX searches against GenBank database allowed the identification within this insertion of a putative 1.819 bp gene consisting of three exons and resembling a transposable element, which was named *Ti-tr1* (Figure 8a).
A 1.868 bp long insertion resembling a second transposon element, named *Ti-tr2,* was also identified in the 3' flanking region of the *TI_CF10 MAT1-2* idiomorph (Figure 8a).

### C. Tuber borchii

### 1. Identification of T. borchii MAT genes

The inventors used the sequences of *T. melanosporum* mating type genes *MAT1-2-1* and *MAT1-1-1* as queries to search for ortholog *T. borchii* sequences deposited in public databases (GenBank and Expressed Sequence Tags (EST) NCBI databases).

By using the *T. melanosporum MAT1-1-1* sequence as a blastn query, the *T. borchii* sequence AF487329 with 85% sequence identity was identified in the NCBI EST database. Conversely, neither Blastn nor tblastn searches identified any *T. borchii* sequence with a significant similarity to the *T. melanosporum MAT1-2-1* nucleotidic and amino acidic sequences, respectively.

The AF487329 sequence of *T. borchii* was aligned with the corresponding *MAT1-1-1* gene of *T. melanosporum* (Figure 14) and *T. borchii MAT1-1-1* specific primers b1 and b2 (Table 5, Figure 16) were designed and used to analyze a set of 5 *T. borchii* ascocarps and 4 mycelial isolates given in Table 2. The *T. borchii MAT1-1-1* specific primers produced the expected amplicon of about 530 bp from two ascocarps and from two of the four *in vitro* isolated mycelia (Table 6). At the light of a possible heterothallism in this species, the strains that produced the expected 530 bp-long amplicon were regarded to as strains containing the *MAT-1-1-1* gene, those that did not as strains harboring the alternative *MAT* gene *MAT1-2-1* (Table 6).

To identify the second mating type gene (*MAT1-2-1)* in *T. borchii,* the inventors screened the *T. borchii* ascocarps and mycelia reported in Table 6 with the PCR primers P1 and P2 specific to the *MAT1-2-1* gene of *T. melanosporum* (Rubini et al., 2011 New Phytologist 189: 710-722). Since these primers did not produce any detectable amplicon, the inventors designed the primer pair b3/b4 on the most conserved HMG-box region of *MAT1-2-1* genes of different *Tuber* spp. and tested it under low stringent PCR condition. This primer pair produced an amplicon of approximately 1 Kbp only on those *T.borchii* strains that failed to produce the *MAT1-1-1* specific amplicon (Table 2). The PCR amplicon from ascocarp TB110 was selected, cloned and sequenced. Blastx search against the *T. melanosporum* Gene Models database (htt://mycor.nancy.inra.fr/IMGC/TuberGenome/blast.php) confirmed a high similarity (68% amino acidic identity) of the TB110 *MAT1-2-1* fragment with the *MAT1-2-1* predicted protein of *T. melanosporum.* The alignment of the two above mentioned sequences is given in Figure 15.

The inventors then designed the primer pair b5/b6 specific to *T. borchii MAT1-2-1* (Table 5; Figure 16). This primer pair produced a band of about 670 bp on the *MAT1-2* samples but not on the *MAT1-1* samples (Table 6) confirming that *T. borchii,* like other *Tuber* spp. is an heterothallic fungus. Moreover, since the mycelia have been isolated from single ectomycorrhizas, these results proved that the mycelium forming *T. borchii* mycorrhizas is haploid. The same conclusion was previously drawn for *T. magnatum* and *T. melanosporum* mycorrhizas (Paolocci et al., (2006) Applied and Environmental Microbiology 72: 2390-2393; Rubini et al., (2011) New Phytologist 189: 723-735.

### 2. Identification of T. borchii MAT idiomorphs

The inventors used the two haploid mycelial strains TB16 and TB15, harboring *MAT1-1-1* and *MAT1-2-1* genes, respectively, to clone and sequence the corresponding *MAT* idiomorphs.

To clone the idiomorph containing the *MAT1-1-1* gene, the sequence (AF487329) retrieved from GenBank was extended towards the 5' and 3' ends following a mixture of genome walking and inverse PCR-based strategies using DNA from TB16 as a target. Genome walking was performed using the Universal GenomeWalker™ Kit (Takara Bio Europe/Clontech Saint-Germain-en-Laye France) according to the supplier's instruction; inverse PCR basically as described in Ochman et al., (1988) Genetics 120: 621-623. Long distance PCR was performed using LA-Taq DNA polymerase (Takara Bio Europe Saint-Germain-en-Laye France). Following these approaches the inventors assembled a 11,4 kb long contig containing the *MAT1-1* idiomorph. Within the *MAT1-1* idiomorph a single α-box (*MAT1-1-1*) containing gene was identified (Figure 16a).

The mating type idiomorph *MAT1-1* of *T. borchii* (*Tborchii*MAT1-1) corresponds to the nucleotide sequences SEQ ID N°52. The *MAT1-1-1* gene of *T. borchii* (*T.borchii*MAT1-1-1) corresponds to the nucleotide sequences SEQ ID N°53.

To sequence the genomic region corresponding to the *MAT1-2* idiomorph the inventors extended the 1 Kb long fragment resulting from the PCR amplification of TB110 ascocarp with the primer pair b3/b4 toward its 5' end following the genome walking approach described above and using DNA from TB15 as target. Conversely, the 3' end of the same idiomorph was cloned using sequence information derived from the TB16 sequence. The inventors have in fact identified within the TB16 contig a sequence corresponding to the *T. melanosporum* gene model GSTUMT00001092001 denoted as ORF3, which is conserved on the 3' flanking region of both *MAT1-1* and *MAT1-2* idiomorphs of *T. melanosporum* and *T. indicum* (Figure 16a). The inventors thought this sequence could also be conserved on the *T*. *borchii MAT1-2* downstream region. Following this rationale, the inventors first designed the primer pair b7/b8 on the ORF3 gene of TB16 (Table 5, Figure 16a). These primers produced an amplicon of approximately 320 bp not only on TB16, used as control, but also on TB15 as predicted. Sequence analysis confirmed that b7/b8-generated amplicons from TB15 and TB16 have similar sequences (about 90% of sequence identity) and share homology with a portion of the *T. melanosporum* gene model GSTUMT00001092001. The inventors then designed the primers b9 and b10, specific to TB15 fragment, and used them in combination with the HMG-box forward primer b6 (Table 5, Figure 16b). Whereas the b6/b9 primer pair did not produce any PCR amplicon on TB15, a 6870 kb long amplicon was obtained using the b6/b10 primer pair on the same target. The inventors interpreted these results to mean that ORF3 sequence is indeed present in the TB15 genomic region downstream the *MAT1-2-1* gene but it shows an inverted orientation with respect to the ORF3 sequence detected downstream the *T. borchii MAT1-1* idiomorph. All the TB15 sequences obtained following the above reported strategies were assembled on a 14,6 Kb long contig that embraces the *MAT1-2* idiomorph and the *MAT1-2-1* gene (Figure 16b)

The mating type idiomorph *MAT1-2* of *T. borchii* (*Tborchii*MAT1-2) corresponds to the nucleotide sequences SEQ ID N°54. The *MAT1-2-1* gene of *T. borchii* (*T.borchii*MAT1-2-1) corresponds to the nucleotide sequences SEQ ID N°55.

The inventors have determined a first genomic region comprising the mating type idiomorph *MAT1-1* which contains the mating type gene *MAT1-1-1* and a second genomic region comprising the mating type idiomorph *MAT1-2* which contains the mating type gene *MAT1-2-1.* Each of these genomic regions comprises also conserved flanking sequences.
The first genomic region correspond to the nucleotide sequences SEQ ID N° 56, and the second genomic region correspond to the nucleotide sequence SEQ ID N°57.

**Table 5. List of primers used in this study**

| Primer name | Primer sequence 5' - 3' |
|---|---|
| b1 | TCAACGGTGATCAGACCCTTGCTCGTG |
| b2 | GAGCGAAGTTAGACAATTTGGGCGT |
| B3 | TTTCTTTGATGGGTCGGATGGAG |
| B4 | GCCCTTGCCTATTAATGTGTTAGTG |
| B5 | ATTGGGACCCACCTTACTATCCGCAATC |
| B6 | CCGGGGAATCCTGCCACATGCGACCGAGA |
| B7 | TTTACTATCATGACCAAAGCGTAT |
| B8 | TTGCAGGTAATGGCTGCCAGAT |
| B9 | GGGATCCCGGCAGACCTCACTAACAG |
| B10 | GGCTGTGGTTCTCAAGGACCGGTTT |

**Table 6. list of mycelial strains and ascocarps used in this study**

| code | Species | source | Mating type |
|---|---|---|---|
| Tb6 | *T.borchii* | mycelium | MAT1-1 |
| Tb7 | *T.borchii* | mycelium | MAT1-2 |
| Tb15 | *T.borchii* | mycelium | MAT1-2 |
| Tb16 | *T.borchii* | mycelium | MAT1-1 |
| tb98 | *T. borchii* | ascocarp | MAT1-2 |
| tb101 | *T. borchii* | ascocarp | MAT1-2 |
| tb102 | *T. borchii* | ascocarp | MAT1-1 |
| tb110 | *T. borchii* | ascocarp | MAT1-2 |
| tb100 | *T. borchii* | ascocarp | MAT1-1 |

### D. Tuber magnatum

### Identification of the mating type genes and of the MAT1-2 idiomorph in T.magnatum

The inventors used the sequences of both *T. melanosporum* and *T. borchii MAT1-2-1* and *MAT1-1-1* genes to design on their conserved HMG-box and α-box regions the primer pairs M1/M2 and M3/M4, respectively (Table 1). The inventors then used these two primer pairs to amplify the DNA isolated from different *T. magnatum* ascocarps. Those ascocarps that produced an amplicon of about 300 bp with the primers M1/M2 did not yield any amplicon with the primer pair M3/M4. Similarly, ascocarps producing a band of about 500 bp with the primers pair M3/M4 did not yield any amplicon with the primers M1/M2. These results are in keeping with the arrangement of *MAT* genes shown by heterothallic ascomycetes and among them by the *Tuber* spp. analysed thus far, in that strains harbouring either the *MAT1-2-1* or the *MAT1-1-1* genes have been observed. Sequencing of the two *T. magnatum* products obtained as reported above confirmed the homology with the HMG-box and α-box sequences of the other Tuber spp for the 300 and 500 bp long products, respectively.
The inventors have then designed the primers M5 and M6 (Figure 17) based on the sequences of *T. magnatum* HMG-box (*MAT1-2-1*).The inventors also designed the primers M7 and M8 on the *T. melanosporum* genes GSTUMT00001092001 and GSTUMT00001088001 present at the 3' and 5' extremities, respectively, of both *MAT* idiomorphs of *T. melanosporum.* By reasoning that genomic regions flanking the MAT idiomorphs are expected to be conserved among *Tuber* spp. the inventors used the primer combinations M5/M7 and M6/M8 (Table 7) to clone the entire *MAT1-2* idiomorph from *T. magnatum.* Indeed, using the primer combination M5/M7 the inventors were able to PCR amplify a fragment of about 5600 bp containing the 3' end of *MAT1-2-1* gene and the relative flanking sequence, including a portion of a gene homologous to GSTUMT00001092001 of *T. melanosporum* (Figure 17). Conversely, no PCR amplicons were obtained when the primer combination M6/M8 was used. Therefore, to clone the 5' end of the *MAT1-2* idiomorph, the inventors performed a genome walking procedure by using the Universal GenomeWalker™ Kit (Clontech) according to the supplier's instruction and the primer M6 as starting point for walking. This approach has allowed the inventors to clone a PCR fragment of about 2500 bp, whose sequencing proved the successful cloning of the 5' end of *T. magnatum MAT1-2-1* gene along with its upstream sequences.
Overall, merging the results of the 5' and 3' walking reactions a genomic sequence of about 7700 bp has been identified. This genomic region contains the *T. magnatum MAT1-2-1* gene and the *MAT1-2* idiomorph. The *T. magnatum MAT1-2-1* gene (*Tmagn*MAT1-2-1) corresponds to the nucleotide sequence SEQ ID N°58.
The mating type idiomorph *MAT1-2* (*Tmagn*MAT1-2) corresponds to the nucleotide sequence SEQ ID N°59.

The inventors have determined the genomic region comprising the first mating type gene *MAT1-2-1* and the first mating type idiomorph *MAT1-2* of *T. magnatum.* This genomic region comprises also flanking regions that are highly conserved between *Tuber* species. The entire genomic region has as nucleotide sequence the nucleotide sequence SEQ ID N°60.

The inventors have also determined the nucleotide sequence of the conserved
α-box region within the *MAT1-1* gene. This region has as nucleotide sequence the nucleotide sequence SEQ ID N°61.

**Table 7 primers used**

| Primer name | Sequence 5'-3' |
|---|---|
| M1 | GTTYGATTCSGCCCYCCAYACCATTAT |
| M2 | CCGGGGAATCCTGCCACATGCGACCGAGA |
| M3 | CYTTCCTTCTTGCRGCAGCCTTATC |
| M4 | ARTACTGYAACGGTTATGGGCTCAC |
| M5 | ATCGGGACTCACCATACTATCCGCAGC |
| M6 | AAAGGGATGAGAGCTGCATACAAATGTCA |
| M7 | TTGRCGGAGTTCRACAAAKGCCTGACGAGC |
| M8 | CAATCTCTTCCATCGCCCGTCCAG |

### E. Tuber aestivum

### Identification of the MAT1-2 idiomorph of Tuber aestivum

The inventors have screened the first assembly of *T. aestivum* genome for the presence of candidate genes encoding for proteins containing the HMG or the alpha-box domain using the mating type genes of *T melanosporum* as queries. Following this approach, the inventors have discovered in the *T. aestivum* genome one nucleotide sequence on the scaffold 88 that has homology with the HMG containing mating type gene of *T. melanosporum* (GSTUMT00001090001). On the contrary, no *T. aestivum* genes corresponding to the alpha-box mating type genes have been detected, suggesting that this is indeed a heterothallic species. The inventors have also identified in the same *T. aestivum* scaffold the two gene models GSTUMT00001088001 and GSTUMT00001092001 present at the 5' and 3' extremities of the *T. melanosporum MAT1-2* idiomorph, respectively. This allows the inventors to confirm that this region corresponds to the mating type *MAT1-2* of *T. aestivum.* The *MAT1-2-1* gene of *T. aestivum* (*Taest*MAT1-2-1), containing the HMG-box region, has as nucleotide sequence SEQ ID N°62.

Overall, following the above mentioned approaches *MAT1-2* idiomorphic region of *T. aestivum* has been identified. The mating type idiomorph *MAT1-2* (*Taest*MAT1-2) corresponds to the nucleotide sequence SEQ ID N°63.

The inventors have determined the genomic region comprising the first mating type gene *MAT1-2-1* and the first mating type idiomorph *MAT1-2.* This genomic region comprises also flanking regions that are highly conserved between *Tuber* species. The genomic region has as nucleotide sequence the nucleotide sequence SEQ ID N°64.

## Claims

1. A method for determining the *Tuber* species of a truffle sample, said method comprising identifying whether said truffle sample comprises at least one nucleotide sequence chosen among a first nucleotide sequence associated with a first mating type idiomorph (MAT1-2) and a second nucleotide sequence associated with a second mating type idiomorph (MAT1-1); both nucleotide sequences being specific to a given *Tuber* species.

2. The method of claim 1, further comprising determining the fertility of said truffle sample, using the identification in said truffle sample of both the first nucleotide sequence associated with the first mating type idiomorph (MAT1-2) and the second nucleotide sequence associated with a second mating type idiomorph (MAT1-1), the fertility of said truffle sample requiring the presence of both the first and the second nucleotide sequences.

3. The method of one of the claims 1 to 2, wherein the first nucleotide sequence comprises the nucleotide sequence SEQ ID N°5 or SEQ ID N°46 or SEQ ID N° 47 or SEQ ID N° 48 or SEQ ID N° 56 or SEQ ID N° 59 and the second nucleotide sequence comprises the respectively nucleotide sequence SEQ ID N°6 or SEQ ID N° 49 or SEQ ID N° 50 or SEQ ID N° 51 or SEQ ID N° 57 or SEQ ID N° 61.

4. The method of one of the claims 1 to 3, using a first pair of oligonucleotide primers to determine the presence of the first mating type idiomorph (MAT1-2) in said truffle sample, each primer of the first pair of oligonucleotide primers comprising from 5 to 50 successive nucleotides of the first nucleotide sequence.

5. The method of claim 4, wherein each primer of the first pair of oligonucleotide primers comprises from 10 to 30 successive nucleotides of the first nucleotide sequence.

6. The method of claim 4 or 5, wherein a forward primer of the first pair of oligonucleotide primers comprises the nucleotide sequence SEQ ID N°7.

7. The method of one of the claims 4 to 6, wherein a reverse primer of the first pair of oligonucleotide primers comprises the nucleotide sequence SEQ ID N°8.

8. The method of one of the claims 1 to 7, using a second pair of oligonucleotide primers to determine the presence of the second mating type idiomorph (MAT1-1) in said truffle sample, each primer of the second pair of oligonucleotide primers comprising from 5 to 50 successive nucleotides of the second nucleotide sequence.

9. The method of claim 8, wherein each primer of the second pair of oligonucleotide primers comprises from 10 to 30 successive nucleotides of the second nucleotide sequence.

10. The method of claim 8 or 9, wherein a forward primer of the second pair of oligonucleotide primers comprises the nucleotide sequence SEQ ID N°9.

11. The method of one of the claims 8 to 10, wherein a reverse primer of the second pair of oligonucleotide primers comprises the nucleotide sequence SEQ ID N°10.

12. Nucleotide sequence associated with a first mating type idiomorph (MAT1-2) of a *Tuber* species comprising the nucleotide sequence SEQ ID N°5.

13. Nucleotide sequence associated with a second mating type idiomorph (MAT1-1) of a *Tuber* species comprising the nucleotide sequence SEQ ID N°6.

14. Kit for determining the fertility of a truffle sample, like truffle fructification sample, truffle mycelium sample, mycorrhized plant sample or truffle-producing soil sample, and/or for identifying truffle species between them, the kit comprising:
- a pair of oligonucleotide primers to determine a first mating type idiomorph (MAT1-2) of a *Tuber* species, wherein each primer comprises from 5 to 50 successive nucleotides of the nucleotide sequence SEQ ID N°5, and
- a pair of oligonucleotide primers to determine a second mating type idiomorph (MAT1-1) of a *Tuber* species, wherein each primer comprises from 5 to 50 successive nucleotides of the nucleotide sequence SEQ ID N°6.

15. Kit for determining the fertility of a truffle sample, like truffle fructification sample, truffle mycelium sample, mycorrhized plant sample or truffle-producing soil sample, and/or for identifying truffle species between them, the kit comprising:
- a pair of oligonucleotide primers to determine a first mating type idiomorph (MAT1-2) of *Tuber indicum,* wherein each primer comprises from 5 to 50 successive nucleotides of the nucleotide sequence SEQ ID N°46 or SEQ ID N°47 or SEQ ID N°48, and
- a pair of oligonucleotide primers to determine a second mating type idiomorph (MAT1-1) of a *Tuber indicum,* wherein each primer comprises from 5 to 50 successive nucleotides of the nucleotide sequence SEQ ID N°49 or SEQ ID N°50 or SEQ ID N°51.

16. Kit for determining the fertility of a truffle sample, like truffle fructification sample, truffle mycelium sample, mycorrhized plant sample or truffle-producing soil sample, and/or for identifying truffle species between them, the kit comprising:
- a pair of oligonucleotide primers to determine a first mating type idiomorph (MAT1-2) of *Tuber borchii,* wherein each primer comprises from 5 to 50 successive nucleotides of the nucleotide sequence SEQ ID N°56 and
- a pair of oligonucleotide primers to determine a second mating type idiomorph (MAT1-1) of a *Tuber borchii,* wherein each primer comprises from 5 to 50 successive nucleotides of the nucleotide sequence SEQ ID N°57.

17. Kit for determining the fertility of a truffle sample, like truffle fructification sample, truffle mycelium sample, mycorrhized plant sample or truffle-producing soil sample, and/or for identifying truffle species between them, the kit comprising:
- a pair of oligonucleotide primers to determine a first mating type idiomorph (MAT1-2) of *Tuber magnatum,* wherein each primer comprises from 5 to 50 successive nucleotides of the nucleotide sequence SEQ ID N°60 and
- a pair of oligonucleotide primers to determine a second mating type idiomorph (MAT1-1) of a *Tuber magnatum,* wherein each primer comprises from 5 to 50 successive nucleotides of the nucleotide sequence SEQ ID N°61.

## Patentansprüche

1. Verfahren zur Bestimmung der *Tuber*-Spezies einer Trüffelprobe, wobei man bei dem Verfahren identifiziert, ob die Trüffelprobe mindestens eine Nukleotidsequenz umfasst, die aus einer ersten, mit einer ersten Paarungstyp-Idiomorphe (MAT1-2) assoziierten Nukleotidsequenz und einer zweiten, mit einer zweiten Paarungstyp-Idiomorphe (MAT1-1) assoziierten Nukleotidsequenz ausgewählt ist, wobei beide Nukleotidsequenzen für eine bestimmte *Tuber-*Spezies spezifisch sind.

2. Verfahren nach Anspruch 1, bei dem man weiterhin die Fruchtbarkeit der Trüffelprobe unter Verwendung der Identifizierung von sowohl der ersten, mit der ersten Paarungstyp-Idiomorphe (MAT1-2) assoziierten Nukleotidsequenz als auch der zweiten, mit einer zweiten Paarungstyp-Idiomorphe (MAT1-1) assoziierten Nukleotidsequenz in der Trüffelprobe bestimmt, wobei die Fruchtbarkeit der Trüffelprobe das Vorhandensein von sowohl der ersten als auch der zweiten Nukleotidsequenz erfordert.

3. Verfahren nach einem der Ansprüche 1 bis 2, wobei die erste Nukleotidsequenz die Nukleotidsequenz SEQ ID NO: 5 oder SEQ ID NO: 46 oder SEQ ID NO: 47 oder SEQ ID NO: 48 oder SEQ ID NO: 56 oder SEQ ID NO: 59 umfasst beziehungsweise die zweite Nukleotidsequenz die Nukleotidsequenz SEQ ID NO: 6 oder SEQ ID NO: 49 oder SEQ ID NO: 50 oder SEQ ID NO: 51 oder SEQ ID NO: 57 oder SEQ ID NO: 61 umfasst.

4. Verfahren nach einem der Ansprüche 1 bis 3, das ein erstes Paar von Oligonukleotid-Primern zur Bestimmung des Vorhandenseins der ersten Paarungstyp-Idiomorphe (MAT1-2) in der Trüffelprobe verwendet, wobei jeder Primer des ersten Paars von Oligonukleotid-Primern 5 bis 50 aufeinanderfolgende Nukleotide der ersten Nukleotidsequenz umfasst.

5. Verfahren nach Anspruch 4, wobei jeder Primer des ersten Paars von Oligonukleotid-Primern 10 bis 30 aufeinanderfolgende Nukleotide der ersten Nukleotidsequenz umfasst.

6. Verfahren nach Anspruch 4 oder 5, wobei ein Vorwärts-Primer des ersten Paars von Oligonukleotid-Primern die Nukleotidsequenz SEQ ID NO: 7 umfasst.

7. Verfahren nach einem der Ansprüche 4 bis 6, wobei ein reverser Primer des ersten Paars von Oligonukleotid-Primern die Nukleotidsequenz SEQ ID NO: 8 umfasst.

8. Verfahren nach einem der Ansprüche 1 bis 7, das ein zweites Paar von Oligonukleotid-Primern zur Bestimmung des Vorhandenseins der zweiten Paarungstyp-Idiomorphe (MAT1-1) in der Trüffelprobe verwendet, wobei jeder Primer des zweiten Paars von Oligonukleotid-Primern 5 bis 50 aufeinanderfolgende Nukleotide der zweiten Nukleotidsequenz umfasst.

9. Verfahren nach Anspruch 8, wobei jeder Primer des zweiten Paars von Oligonukleotid-Primern 10 bis 30 aufeinanderfolgende Nukleotide der zweiten Nukleotidsequenz umfasst.

10. Verfahren nach Anspruch 8 oder 9, wobei ein Vorwärts-Primer des zweiten Paars von Oligonukleotid-Primern die Nukleotidsequenz SEQ ID NO: 9 umfasst.

11. Verfahren nach einem der Ansprüche 8 bis 10, wobei ein reverser Primer des zweiten Paars von Oligonukleotid-Primern die Nukleotidsequenz SEQ ID NO: 10 umfasst.

12. Nukleotidsequenz, die mit einer ersten Paarungstyp-Idiomorphe (MAT1-2) einer *Tuber*-Spezies assoziiert ist, umfassend die Nukleotidsequenz SEQ ID NO: 5.

13. Nukleotidsequenz, die mit einer zweiten Paarungstyp-Idiomorphe (MAT1-1) einer *Tuber*-Spezies assoziiert ist, umfassend die Nukleotidsequenz SEQ ID NO: 6.

14. Kit zum Bestimmen der Fruchtbarkeit einer Trüffelprobe, wie einer Trüffel-Fruktifizierungsprobe, Trüffel-Myzelprobe, mykorrhizierten Pflanzenprobe oder Trüffel-produzierenden Bodenprobe, und/oder zum Identifizieren der Trüffel-Spezies unter diesen, wobei das Kit Folgendes umfasst:
- ein Paar von Oligonukleotid-Primern zum Bestimmen einer ersten Paarungstyp-Idiomorphe (MAT1-2) einer *Tuber*-Spezies, wobei jeder Primer 5 bis 50 aufeinanderfolgende Nukleotide der Nukleotidsequenz SEQ ID NO: 5 umfasst, und
- ein Paar von Oligonukleotid-Primern zum Bestimmen einer zweiten Paarungstyp-Idiomorphe (MAT1-1) einer *Tuber*-Spezies, wobei jeder Primer 5 bis 50 aufeinanderfolgende Nukleotide der Nukleotidsequenz SEQ ID NO: 6 umfasst.

15. Kit zum Bestimmen der Fruchtbarkeit einer Trüffelprobe, wie einer Trüffel-Fruktifizierungsprobe, Trüffel-Myzelprobe, mykorrhizierten Pflanzenprobe oder Trüffel-produzierenden Bodenprobe, und/oder zum Identifizieren der Trüffel-Spezies unter diesen, wobei das Kit Folgendes umfasst:
- ein Paar von Oligonukleotid-Primern zum Bestimmen einer ersten Paarungstyp-Idiomorphe (MAT1-2) von *Tuber indicum,* wobei jeder Primer 5 bis 50 aufeinanderfolgende Nukleotide der Nukleotidsequenz SEQ ID NO: 46 oder SEQ ID NO: 47 oder SEQ ID NO: 48 umfasst, und
- ein Paar von Oligonukleotid-Primern zum Bestimmen einer zweiten Paarungstyp-Idiomorphe (MAT1-1) eines *Tuber indicum,* wobei jeder Primer 5 bis 50 aufeinanderfolgende Nukleotide der Nukleotidsequenz SEQ ID NO: 49 oder SEQ ID NO: 50 oder SEQ ID NO: 51 umfasst.

16. Kit zum Bestimmen der Fruchtbarkeit einer Trüffelprobe, wie einer Trüffel-Fruktifizierungsprobe, Trüffel-Myzelprobe, mykorrhizierten Pflanzenprobe oder Trüffel-produzierenden Bodenprobe, und/oder zum Identifizieren der Trüffel-Spezies unter diesen, wobei das Kit Folgendes umfasst:
- ein Paar von Oligonukleotid-Primern zum Bestimmen einer ersten Paarungstyp-Idiomorphe (MAT1-2) von *Tuber borchii,* wobei jeder Primer 5 bis 50 aufeinanderfolgende Nukleotide der Nukleotidsequenz SEQ ID NO: 56 umfasst, und
- ein Paar von Oligonukleotid-Primern zum Bestimmen einer zweiten Paarungstyp-Idiomorphe (MAT1-1) eines *Tuber borchii,* wobei jeder Primer 5 bis 50 aufeinanderfolgende Nukleotide der Nukleotidsequenz SEQ ID NO: 57 umfasst.

17. Kit zum Bestimmen der Fruchtbarkeit einer Trüffelprobe, wie einer Trüffel-Fruktifizierungsprobe, Trüffel-Myzelprobe, mykorrhizierten Pflanzenprobe oder Trüffel-produzierenden Bodenprobe, und/oder zum Identifizieren der Trüffel-Spezies unter diesen, wobei das Kit Folgendes umfasst:
- ein Paar von Oligonukleotid-Primern zum Bestimmen einer ersten Paarungstyp-Idiomorphe (MAT1-2) von *Tuber magnatum,* wobei jeder Primer 5 bis 50 aufeinanderfolgende Nukleotide der Nukleotidsequenz SEQ ID NO: 60 umfasst, und
- ein Paar von Oligonukleotid-Primern zum Bestimmen einer zweiten Paarungstyp-Idiomorphe (MAT1-1) eines *Tuber magnatum,* wobei jeder Primer 5 bis 50 aufeinanderfolgende Nukleotide der Nukleotidsequenz SEQ ID NO: 61 umfasst.

## Revendications

1. Procédé de détermination de l'espèce *Tuber* d'un échantillon de truffe, ledit procédé comprenant l'identification du fait que ledit échantillon de truffe comprend ou non au moins une séquence de nucléotides choisie parmi une première séquence de nucléotides associée à un premier idiomorphe de type sexuel (MAT1-2) et une deuxième séquence de nucléotides associée à un deuxième idiomorphe de type sexuel (MAT1-1) ; les deux séquences de nucléotides étant spécifiques d'une espèce *Tuber* donnée.

2. Procédé selon la revendication 1, comprenant en outre la détermination de la fertilité dudit échantillon de truffe, en utilisant l'identification dans ledit échantillon de truffe à la fois de la première séquence de nucléotides associée au premier idiomorphe de type sexuel (MAT1-2) et de la deuxième séquence de nucléotides associée au deuxième idiomorphe de type sexuel (MAT1-1), la fertilité dudit échantillon de truffe nécessitant la présence à la fois de la première et de la deuxième séquence nucléotidique.

3. Procédé selon l'une des revendications 1 à 2, dans lequel la première séquence de nucléotides comprend la séquence de nucléotides SEQ ID N° 5 ou SEQ ID N° 46 ou SEQ ID N° 47 ou SEQ ID N° 48 ou SEQ ID N° 56 ou SEQ ID N° 59 et la deuxième séquence de nucléotides comprend respectivement la séquence de nucléotides SEQ ID N° 6 ou SEQ ID N° 49 ou SEQ ID N° 50 ou SEQ ID N° 51 ou SEQ ID N° 57 ou SEQ ID N° 61.

4. Procédé selon l'une des revendications 1 à 3, utilisant une première paire d'amorces oligonucléotidiques pour déterminer la présence du premier idiomorphe de type sexuel (MAT1-2) dans ledit échantillon de truffe, chaque amorce de la première paire d'amorces oligonucléotidiques comprenant de 5 à 50 nucléotides successifs de la première séquence de nucléotides.

5. Procédé selon la revendication 4, dans lequel chaque amorce de la première paire d'amorces oligonucléotidiques comprend de 10 à 30 nucléotides successifs de la première séquence de nucléotides.

6. Procédé selon la revendication 4 ou 5, dans lequel une amorce sens de la première paire d'amorces oligonucléotidiques comprend la séquence de nucléotides SEQ ID N° 7.

7. Procédé selon l'une des revendications 4 à 6, dans lequel une amorce antisens de la première paire d'amorces oligonucléotidiques comprend la séquence de nucléotides SEQ ID N° 8.

8. Procédé selon l'une des revendications 1 à 7, utilisant une deuxième paire d'amorces oligonucléotidiques pour déterminer la présence du deuxième idiomorphe de type sexuel (MAT1-1) dans ledit échantillon de truffe, chaque amorce de la deuxième paire d'amorces oligonucléotidiques comprenant de 5 à 50 nucléotides successifs de la deuxième séquence de nucléotides.

9. Procédé selon la revendication 8, dans lequel chaque amorce de la deuxième paire d'amorces oligonucléotidiques comprend de 10 à 30 nucléotides successifs de la deuxième séquence de nucléotides.

10. Procédé selon la revendication 8 ou 9, dans lequel une amorce sens de la deuxième paire d'amorces oligonucléotidiques comprend la séquence de nucléotides SEQ ID N° 9.

11. Procédé selon l'une des revendications 8 à 10, dans lequel une amorce antisens de la deuxième paire d'amorces oligonucléotidiques comprend la séquence de nucléotides SEQ ID N° 10.

12. Séquence nucléotidique associée à un premier idiomorphe de type sexuel (MAT1-2) d'une espèce *Tuber* comprenant la séquence de nucléotides SEQ ID N° 5.

13. Séquence nucléotidique associée à un deuxième idiomorphe de type sexuel (MAT1-1) d'une espèce *Tuber* comprenant la séquence de nucléotides SEQ ID N° 6.

14. Kit pour la détermination de la fertilité d'un échantillon de truffe, tel qu'un échantillon de fructification de truffe, un échantillon de mycélium de truffe, un échantillon de plante mycorrhizée ou un échantillon de sol de production de truffe, et/ou pour l'identification d'une espèce de truffe parmi ceux-ci, le kit comprenant :
- une paire d'amorces oligonucléotidiques pour déterminer un premier idiomorphe de type sexuel (MAT1-2) d'une espèce *Tuber,* dans laquelle chaque amorce comprend de 5 à 50 nucléotides successifs de la séquence de nucléotides SEQ ID N° 5, et
- une paire d'amorces oligonucléotidiques pour déterminer un deuxième idiomorphe de type sexuel (MAT1-1) d'une espèce *Tuber,* dans laquelle chaque amorce comprend de 5 à 50 nucléotides successifs de la séquence de nucléotides SEQ ID N° 6.

15. Kit pour la détermination de la fertilité d'un échantillon de truffe, tel qu'un échantillon de fructification de truffe, un échantillon de mycélium de truffe, un échantillon de plante mycorrhizée ou un échantillon de sol de production de truffe, et/ou pour l'identification d'une espèce de truffe parmi ceux-ci, le kit comprenant :
- une paire d'amorces oligonucléotidiques pour déterminer un premier idiomorphe de type sexuel (MAT1-2) de *Tuber indicum,* dans laquelle chaque amorce comprend de 5 à 50 nucléotides successifs de la séquence de nucléotides SEQ ID N° 46 ou SEQ ID N° 47 ou SEQ ID N° 48, et
- une paire d'amorces oligonucléotidiques pour déterminer un deuxième idiomorphe de type sexuel (MAT1-1) de *Tuber indicum,* dans laquelle chaque amorce comprend de 5 à 50 nucléotides successifs de la séquence de nucléotides SEQ ID N° 49 ou SEQ ID N° 50 ou SEQ ID N° 51.

16. Kit pour la détermination de la fertilité d'un échantillon de truffe, tel qu'un échantillon de fructification de truffe, un échantillon de mycélium de truffe, un échantillon de plante mycorrhizée ou un échantillon de sol de production de truffe, et/ou pour l'identification d'une espèce de truffe parmi ceux-ci, le kit comprenant :
- une paire d'amorces oligonucléotidiques pour déterminer un premier idiomorphe de type sexuel (MAT1-2) de *Tuber borchii,* dans laquelle chaque amorce comprend de 5 à 50 nucléotides successifs de la séquence de nucléotides SEQ ID N° 56 et
• une paire d'amorces oligonucléotidiques pour déterminer un deuxième idiomorphe de type sexuel (MAT1-1) de *Tuber borchii,* dans laquelle chaque amorce comprend de 5 à 50 nucléotides successifs de la séquence de nucléotides SEQ ID N° 57.

17. Kit pour la détermination de la fertilité d'un échantillon de truffe, tel qu'un échantillon de fructification de truffe, un échantillon de mycélium de truffe, un échantillon de plante mycorrhizée ou un échantillon de sol de production de truffe, et/ou pour l'identification d'une espèce de truffe parmi ceux-ci, le kit comprenant :
- une paire d'amorces oligonucléotidiques pour déterminer un premier idiomorphe de type sexuel (MAT1-2) de *Tuber magnatum,* dans laquelle chaque amorce comprend de 5 à 50 nucléotides successifs de la séquence de nucléotides SEQ ID N° 60 et
- une paire d'amorces oligonucléotidiques pour déterminer un deuxième idiomorphe de type sexuel (MAT1-1) de *Tuber magnatum,* dans laquelle chaque amorce comprend de 5 à 50 nucléotides successifs de la séquence de nucléotides SEQ ID N° 61.
